(19) Europäisches Patentamt
European Patent Office
Office européen des brevets

(11) EP 4 585 595 A1

(12) **EUROPEAN PATENT APPLICATION**
published in accordance with Art. 153(4) EPC

(43) Date of publication:
**16.07.2025 Bulletin 2025/29**

(21) Application number: **23862396.1**

(22) Date of filing: **05.09.2023**

(51) International Patent Classification (IPC):
***C07D 413/14*** *(2006.01)* ***C07D 413/10*** *(2006.01)*
***C07D 405/12*** *(2006.01)* ***C07D 405/14*** *(2006.01)*
***C07D 487/04*** *(2006.01)* ***C07D 401/14*** *(2006.01)*
***C07D 403/10*** *(2006.01)* ***C07D 473/34*** *(2006.01)*
***C07D 513/04*** *(2006.01)* ***A61K 31/538*** *(2006.01)*
***A61K 31/506*** *(2006.01)* ***A61K 31/5355*** *(2006.01)*
***A61K 31/519*** *(2006.01)* ***A61K 31/52*** *(2006.01)*
***A61K 31/542*** *(2006.01)* ***A61K 31/527*** *(2006.01)*
***A61P 3/00*** *(2006.01)* ***A61P 9/00*** *(2006.01)*
***A61P 29/00*** *(2006.01)* ***A61P 31/00*** *(2006.01)*
***A61P 37/00*** *(2006.01)*

(52) Cooperative Patent Classification (CPC):
**A61K 31/506; A61K 31/519; A61K 31/52; A61K 31/527; A61K 31/5355; A61K 31/538; A61K 31/542; A61P 3/00; A61P 9/00; A61P 29/00; A61P 31/00; A61P 35/00; A61P 37/00; A61P 37/02; C07D 401/14;** (Cont.)

(86) International application number:
**PCT/CN2023/117094**

(87) International publication number:
**WO 2024/051702 (14.03.2024 Gazette 2024/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.09.2022 CN 202211080914**
**09.03.2023 CN 202310223786**

(71) Applicant: **TYK Medicines, Inc.**
**Huzhou, Zhejiang 313100 (CN)**

(72) Inventors:
- **LIANG, Apeng**
  **Huzhou, Zhejiang 313100 (CN)**
- **WANG, Kai**
  **Huzhou, Zhejiang 313100 (CN)**
- **CHEN, Shaoqing**
  **Huzhou, Zhejiang 313100 (CN)**
- **LI, Jun**
  **Huzhou, Zhejiang 313100 (CN)**
- **WU, Yusheng**
  **Huzhou, Zhejiang 313100 (CN)**
- **LI, Meihua**
  **Huzhou, Zhejiang 313100 (CN)**
- **YIN, Zhou**
  **Huzhou, Zhejiang 313100 (CN)**
- **LONG, Yi**
  **Huzhou, Zhejiang 313100 (CN)**
- **NIU, Chengshan**
  **Huzhou, Zhejiang 313100 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) COMPOUND USED AS INHIBITOR OF CDK4 KINASE AND USE THEREOF

(57) The present invention relates to a compound used as an inhibitor of a CDK4 kinase and use thereof. Specifically, the compound of the present invention has a structure represented by formula (I), wherein the definitions of each group and each substituent group are as described in the specification. The compound of the



Processed by Luminess, 75001 PARIS (FR)

(Cont. next page)

present invention can be used as an inhibitor of a cyclin-dependent kinase (CDK), is used for treating or preventing proliferative diseases (such as cancer), and is in particular used for modulating and treating related diseases caused by the abnormal activity of the cyclin-dependent kinase (CDK).

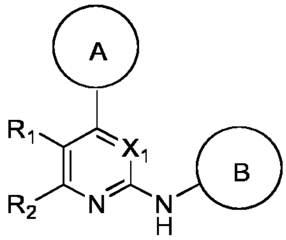

Formula I

(52) Cooperative Patent Classification (CPC): (Cont.)
**C07D 403/10; C07D 405/12; C07D 405/14; C07D 413/04; C07D 413/10; C07D 413/14; C07D 471/04; C07D 473/00; C07D 473/34; C07D 487/04; C07D 487/10; C07D 513/04; C07D 519/00**

## Description

### THCHNICAL FIFLD

The present invention relates to the field of pharmaceutical technology, and specifically to compounds used as CDK4 kinase inhibitors, and their application in modulating CDK4 kinase activity or in the treatment of CDK4-related diseases, in particular cancer.

### BACKGROUND ART

Cyclin-dependent kinases (CDKs) belong to the serine/threonine kinase family, which exert physiological functions by binding to the corresponding cyclins to form active dimeric complexes that cause cell growth and proliferation. More than 20 types of CDKs have been identified, which are categorized into two main groups according to their functions: CDKs that regulate the cell cycle and CDKs that regulate cellular transcription, among which CDKs 1-6 and 14-18 are involved in cell cycle regulation, and CDKs 7-13 and 19-20 are involved in the transcriptional regulation of cells. It has now been shown that CDK inhibitors can be used in the treatment of cancer.

CDK4 and CDK6 are involved in regulating the cell cycle from G1 to S phase upon binding to Cyclin D. Abnormalities in the CyclinD-CDK4/6-Rb pathway have been reported to be associated with progression of resistance to endocrine therapies. Currently, several CDK4/6 inhibitors, such as palbociclib, ribociclib, and abemaciclib, have been approved and marketed in combination with endocrine therapies for the treatment of hormone receptor (HR)-positive, human epidermal growth factor 2 (HER2)-negative advanced or metastatic breast cancer. However, hematologic toxicities such as neutropenia and/or gastrointestinal toxicities often occur during treatment with CDK4/6 inhibitors, leading to discontinuation or intermittent dosing, which seriously affects drug efficacy and adherence. Currently, there are research data suggesting that the activity of cyclin D3-CDK6 may be associated with these side effects. Given the current situation of toxic side effects associated with dual-target CDK4/6 inhibitors, the development of a selective CDK4 inhibitor may have better safety and efficacy.

### SUMMARY OF THE INVENTION

The present invention provides a new compound with CDK4 kinase inhibitory activity and better pharmacodynamic and pharmacokinetic properties.

In the first aspect of the present invention, it provides a compound for use as a CDK4 kinase inhibitor, wherein the compound is a compound of formula I, or a pharmaceutically acceptable salt, stereoisomer, tautomer, hydrate, solvate, isotope compound, or prodrug thereof,

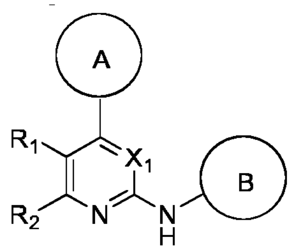

formula I

wherein,

$X_1$ is selected from the group consisting of N, and $CR_3$;
$R_1$ is selected from the group consisting of H, $CF_3$, F, Cl, Br, methyl, ethyl, isopropyl, and cyclopropyl;
$R_2$ is selected from the group consisting of H, F, Cl, Br, $CF_3$, $CF_2H$, $NH_2$, and methyl;
or, $R_1$ and $R_2$ together with the C to which they are attached form a 5-membered heteroaryl containing two N atoms;
$R_3$ is selected from the group consisting of H, $CF_3$, F, Cl, Br, methyl, and cyano;
Ring A is selected from the group consisting of

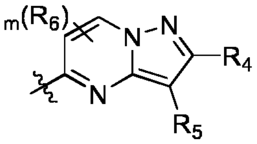,

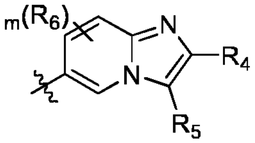, 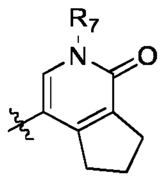, 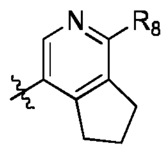, 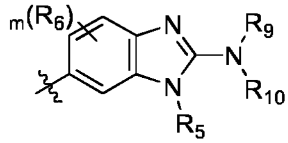,

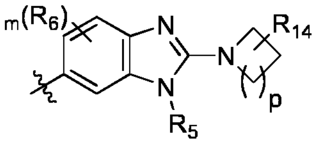, 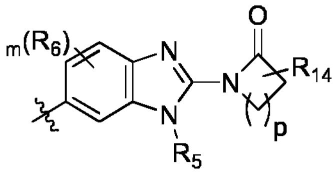, 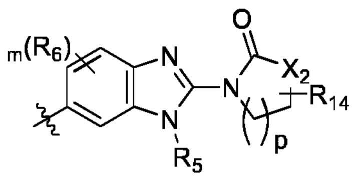,

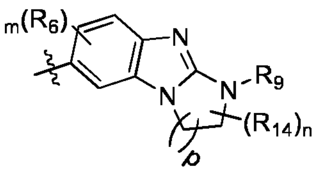, 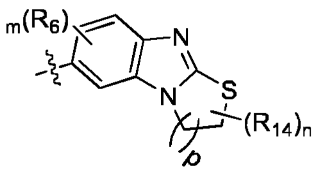, 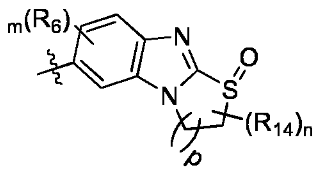,

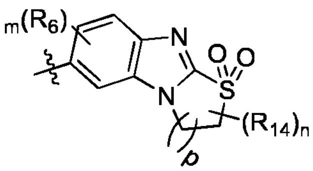, 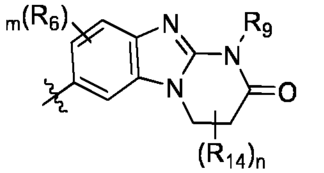, 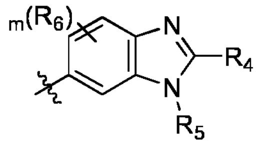,

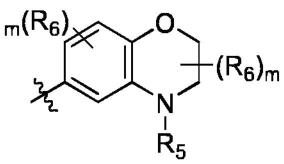, 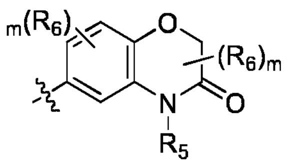, 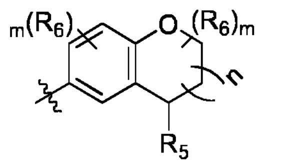,

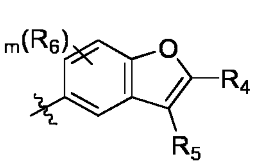, 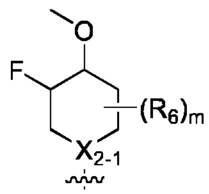, 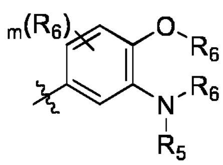, and 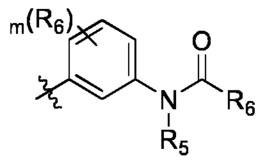,

wherein, $X_2$ is O or NR;

$X_{2-1}$ is selected from the group consisting of N, and $CR_{11}$;
Ring B is selected from the group consisting of

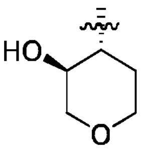, 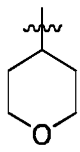, 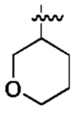,

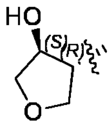, 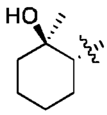, 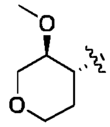, 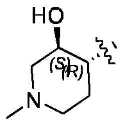, 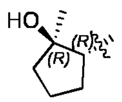, 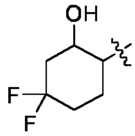, 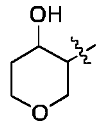,

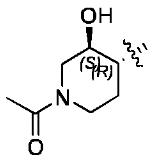, 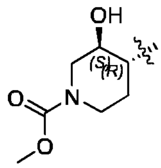, 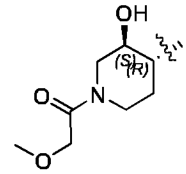, 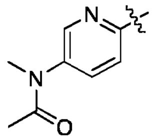, 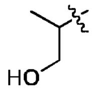, 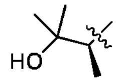,

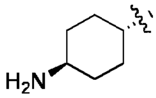, 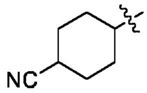, 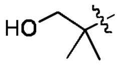, 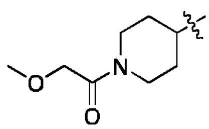, 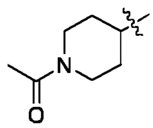, 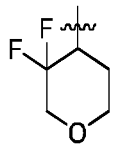,

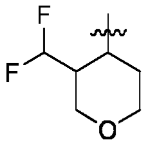, 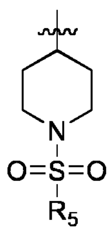, 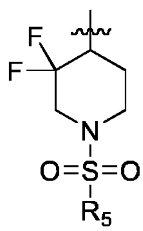, 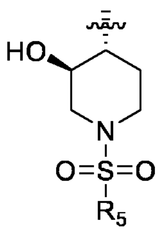, 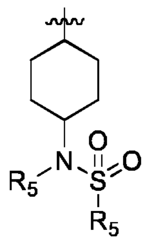, 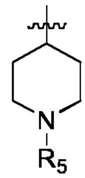,

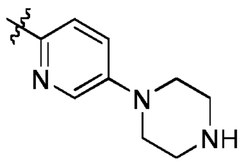, 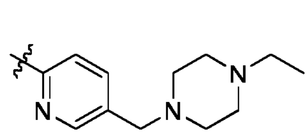, 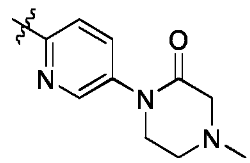,

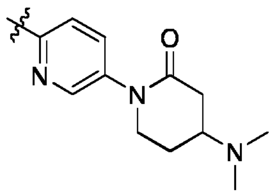, 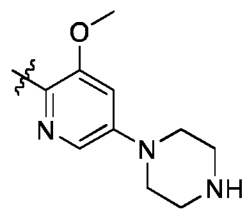, 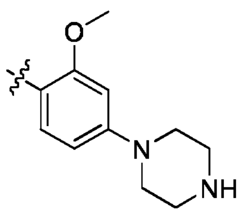, 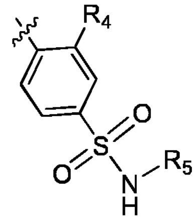,

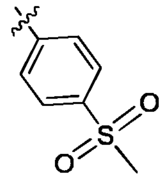, 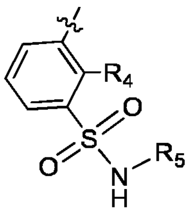, 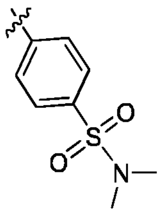, 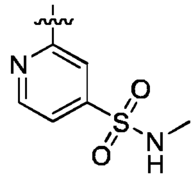, 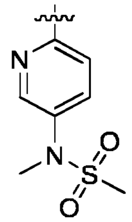,

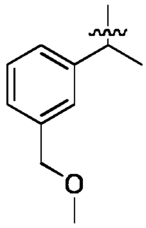, 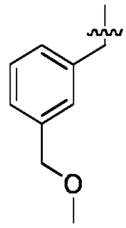, 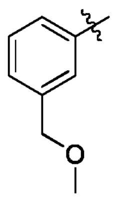, 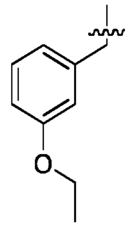, 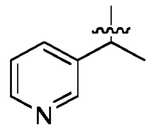, 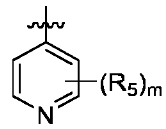, 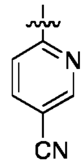,

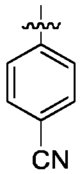, 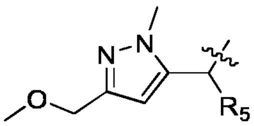, 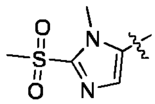, 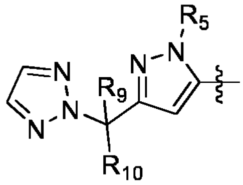, 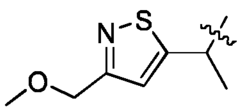,

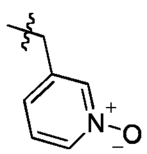, 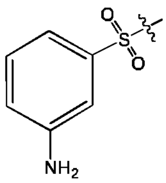, 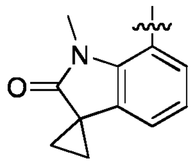, 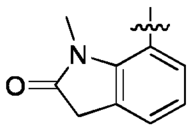, 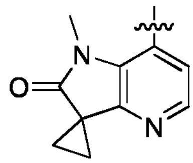,

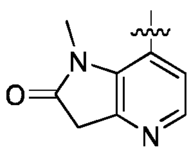, 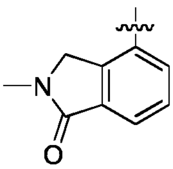, 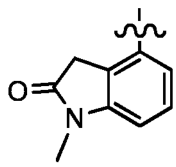, 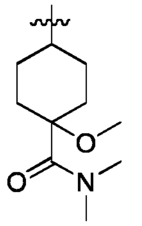, 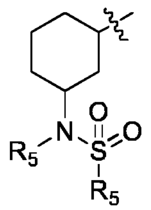,

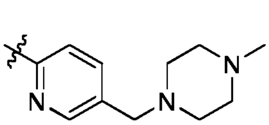, 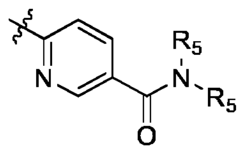, 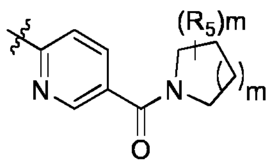, 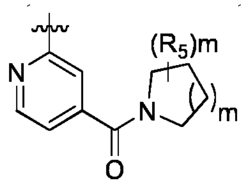,

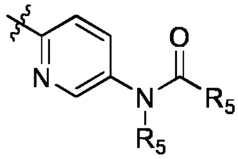, 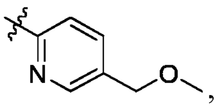, 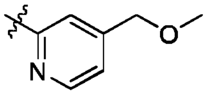, 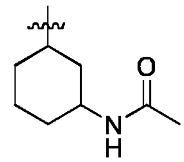, 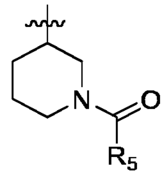,

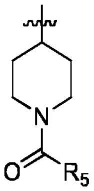, 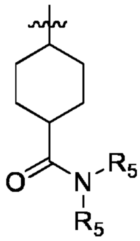, 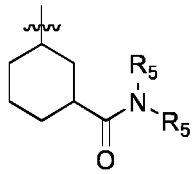, 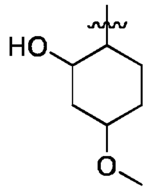, 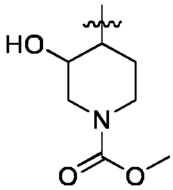, 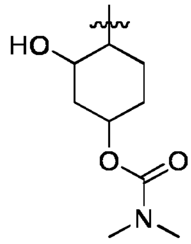,

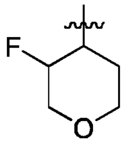, 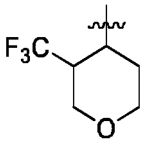, 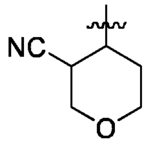, 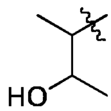, and 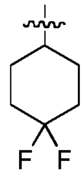;

wherein, each $R_4$ is independently selected from the group consisting of H, cyano, halogen, substituted or unsubstituted C1-C6 alkyl, substituted or unsubstituted C3-C6 cycloalkyl, $-C(O)NR_9R_{10}$, and substituted or unsubstituted $-NR_mR_n$,

each $R_5$ and $R_6$ is each independently selected from the group consisting of H, halogen, hydroxyl, amino,

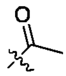, 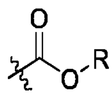, 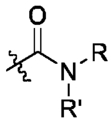,

substituted or unsubstituted C1-C6 alkyl, substituted or unsubstituted C3-C6 cycloalkyl, substituted or unsubstituted phenyl, substituted or unsubstituted $C_{1-6}$ alkoxy, and substituted or unsubstituted $-NR_mR_n$;

$R_7$ and $R_{11}$ are each independently selected from the group consisting of H, and substituted or unsubstituted $C_{1-6}$ alkyl;

$R_8$ is selected from the group consisting of H, $C_{1-6}$ alkoxy, halogenated $C_{1-6}$ alkoxy, $-NR_mR_n$, halogenated $-NR_mR_n$, cyano, substituted or unsubstituted $C_{1-6}$ alkyl, substituted or unsubstituted $C_{3-6}$ cycloalkyl, and $-C(O)NR_9R_{10}$;

each $R_9$ and $R_{10}$ is each independently selected from the group consisting of H, halogen, hydroxyl, amino,

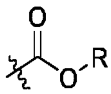, 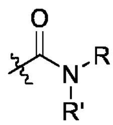,

substituted or unsubstituted C1-C6 alkyl, substituted or unsubstituted C3-C6 cycloalkyl, substituted or unsubstituted phenyl, $-C(O)R_{12}$, and $-C(O)OR_{13}$; or $R_9$ and $R_{10}$ together with the attached N form a substituted or unsubstituted 5-7 membered heterocyclic ring; or either $R_9$ or $R_{10}$ together with $R_5$ form a 5-7 membered ring;

each $R_{14}$ is independently selected from the group consisting of H, halogen, hydroxyl, amino,

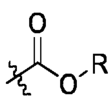, 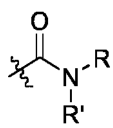,

substituted or unsubstituted C1-C6 alkyl, substituted or unsubstituted C3-C6 cycloalkyl, substituted or unsubstituted phenyl, and oxo; or two $R_{14}$ groups attached to the same C together with the C to which they are attached form C3-C6 cycloalkyl;

or $R_4$ and $R_5$ together with the C to which they are attached form a 5-7membered heterocyclic ring containing 1, 2 or 3 heteroatoms selected from N, O, S;

$R_{12}$ and $R_{13}$ are each independently selected from the group consisting of H, substituted or unsubstituted $C_{1-6}$ alkyl, and substituted or unsubstituted $C_{3-6}$ cycloalkyl;
each R and R' is each independently selected from the group consisting of H, C1-C6 alkyl, halogenated C1-C6 alkyl, C3-C6 cycloalkyl, halogenated C3-C6 cycloalkyl, and phenyl;
the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;
each $R_m$ is selected from the group consisting of H, $C_{1-6}$ alkyl, $C_{3-6}$ cycloalkyl, $C_{6-10}$ aryl, and 6-10 membered heteroaryl containing 1, 2, or 3 heteroatoms selected from N, O, and S;
each $R_n$ is $C_{1-6}$ alkyl;
or $R_m$ and $R_n$ together with the N atom to which they are attached form a 3-10 membered N-containing monocyclic or bicyclic heterocyclic group;
each m, n, p is independently selected from the group consisting of 0, 1, 2, 3, 4, and 5;
wherein, when ring A is

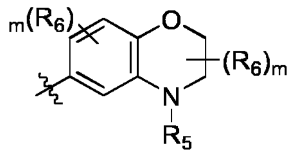,

ring B is selected from the group consisting of

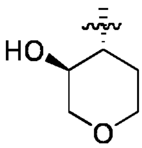, 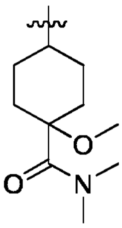, 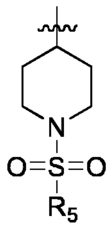, 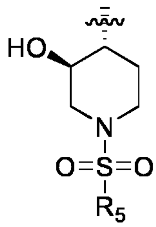, 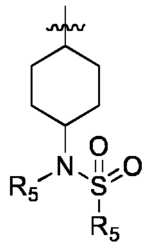,

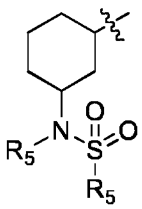, 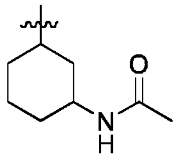, 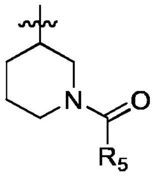, 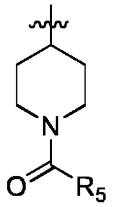, 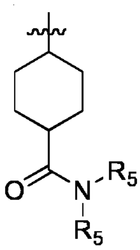, 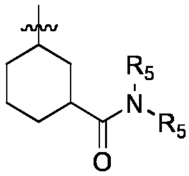,

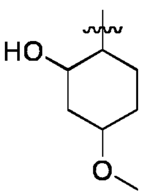, 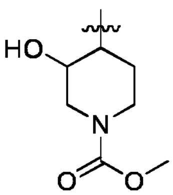, 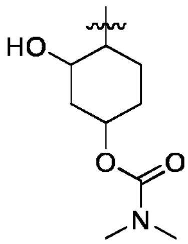, 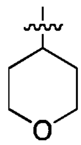, 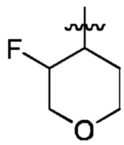, 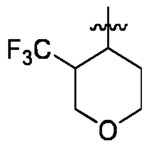,

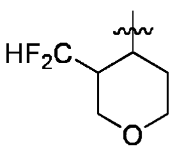, 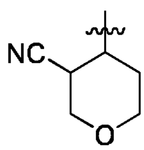, and 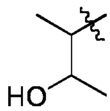.

In another preferred embodiment, $X_1$ is selected from the group consisting of N, and $CR_3$;

$R_1$ is selected from the group consisting of H, $CF_3$, F, Cl, Br, methyl, ethyl, isopropyl, and cyclopropyl;
$R_2$ is selected from the group consisting of H, $CF_3$, F, Cl, Br, and methyl;
$R_3$ is selected from the group consisting of H, $CF_3$, F, Cl, Br, methyl, and cyano;
Ring A is selected from the group consisting of

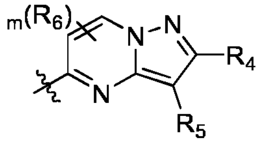,

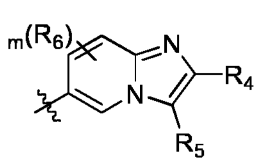, 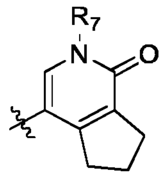, 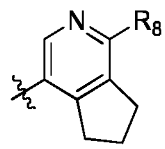, 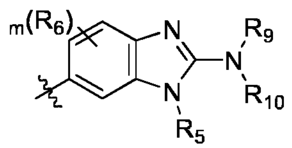,

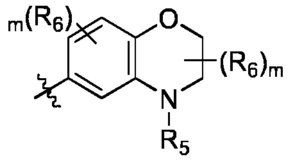, 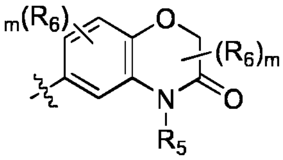, 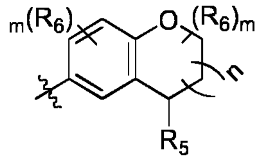,

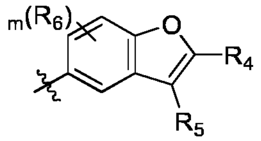, 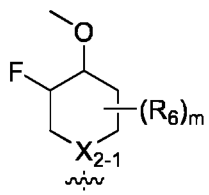, 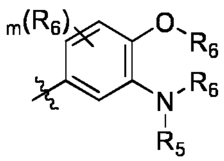, and 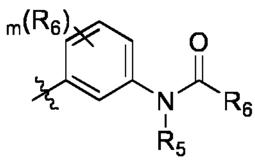,

wherein, $X_{2-1}$ is selected from the group consisting of N and $CR_{11}$;

$R_4$ is selected from the group consisting of H, substituted or unsubstituted $C_{1-6}$ alkyl, substituted or unsubstituted $C_{3-6}$ cycloalkyl, $-C(O)NR_9R_{10}$, substituted or unsubstituted $-NR_mR_n$;
each $R_5$ is independently selected from the group consisting of H, substituted or unsubstituted $C_{1-6}$ alkyl, substituted or unsubstituted $C_{1-6}$ alkoxy, substituted or unsubstituted $C_{3-6}$ cycloalkyl, substituted or unsubstituted $-NR_mR_n$;
each $R_6$ is respectively independently selected from the group consisting of halogen, substituted or unsubstituted $C_{1-6}$ alkyl, substituted or unsubstituted $C_{3-6}$ cycloalkyl;
$R_7$ and $R_{11}$ are each independently selected from the group consisting of H and substituted or unsubstituted $C_{1-6}$ alkyl;
$R_8$ is selected from the group consisting of H, $C_{1-6}$ alkoxy, halogenated $C_{1-6}$ alkoxy, $-NR_mR_n$, halogenated $-NR_mR_n$, cyano, substituted or unsubstituted $C_{1-6}$ alkyl, substituted or unsubstituted $C_{3-6}$ cycloalkyl, and $-C(O)NR_9R_{10}$;
each $R_9$ and $R_{10}$ is independently selected from the group consisting of H, substituted or unsubstituted $C_{1-6}$ alkyl, substituted or unsubstituted $C_{3-6}$ cycloalkyl, $-C(O)R_{12}$, and $-C(O)OR_{13}$; or $R_9$ and $R_{10}$ together with the N to which they are attached form a substituted or unsubstituted 5-7 membered heterocyclic ring; or either $R_9$ or $R_{10}$ together with $R_5$ form a 5-7 membered ring;
or $R_4$ and $R_5$ together with the C to which they are attached form a 5-7membered heterocyclic ring containing 1, 2 or 3 heteroatoms selected from N, O, S;
$R_{12}$ and $R_{13}$ are each independently selected from the group consisting of H, substituted or unsubstituted $C_{1-6}$ alkyl, and substituted or unsubstituted $C_{3-6}$ cycloalkyl;
the "substituted" each independently means to be substituted with 1, 2, 3 or 4 substituents selected from the group

consisting of deuterium, halogen, hydroxyl, amino, $-NR_mR_n$, and $C_{1-6}$ alkoxy;
each $R_m$ is selected from the group consisting of H, $C_{1-6}$ alkyl, $C_{3-6}$ cycloalkyl, $C_{6-10}$ aryl, and 6-10 membered heteroaryl containing 1, 2 or 3 heteroatoms selected from N, O, S;
each $R_n$ is $C_{1-6}$ alkyl;
each m, n is independently selected from the group consisting of 0, 1, 2, 3, 4, and 5;
Ring B is selected from the group consisting of

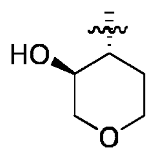, 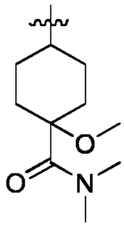,

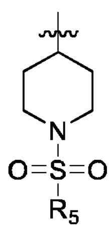, 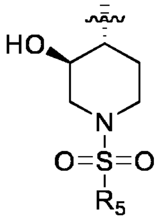, 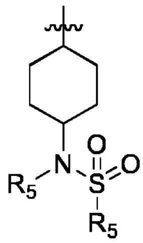, 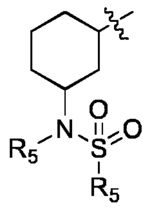, 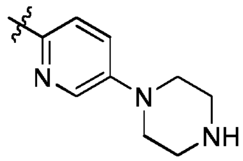,

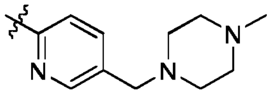, 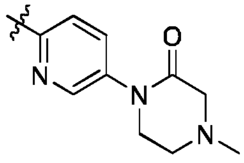, 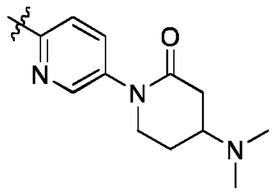, 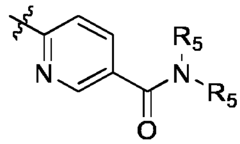,

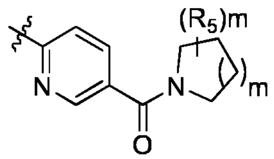, 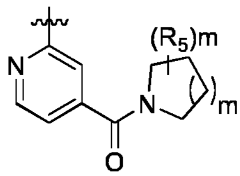, 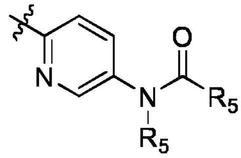, 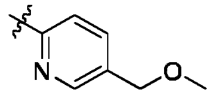,

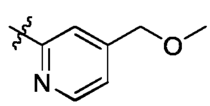, 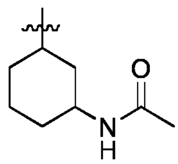, 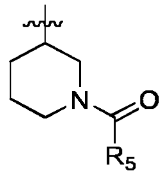, 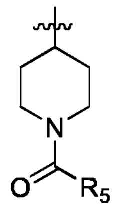, 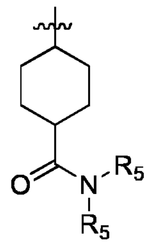,

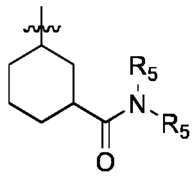, 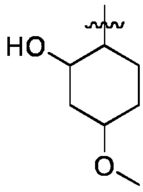, 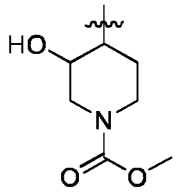, 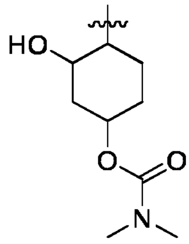, 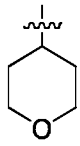, 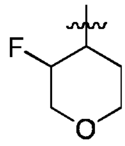,

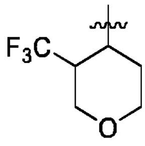, 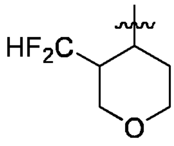, 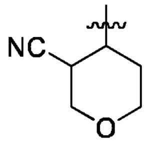, and 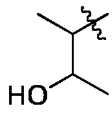;

wherein, when ring A is

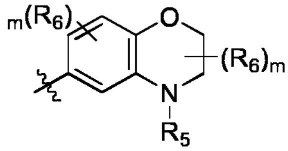,

ring B is selected from the group consisting of

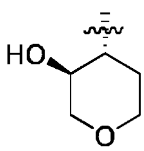, 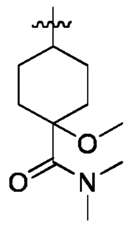, 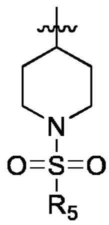, 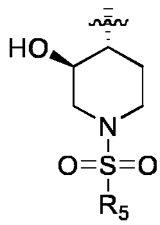, 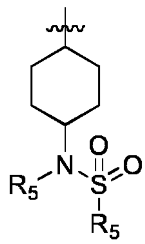,

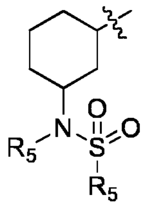, 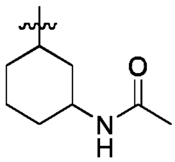, 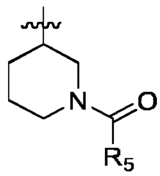, 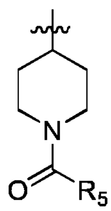, 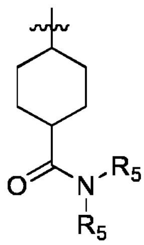, 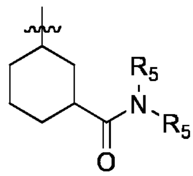,

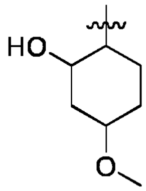, 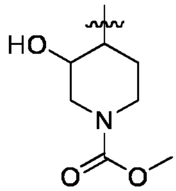, 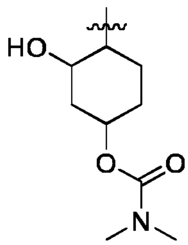, 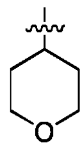, 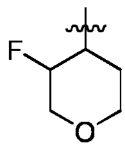, 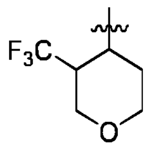,

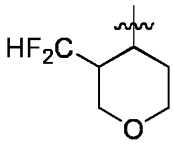, 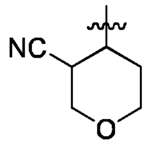, and 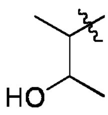.

In the second aspect of the present invention, it provides a compound for use as a CDK4 kinase inhibitor, wherein the compound is a compound of formula II, or a pharmaceutically acceptable salt, stereoisomer, tautomer, hydrate, solvate, isotope compound, or prodrug thereof,

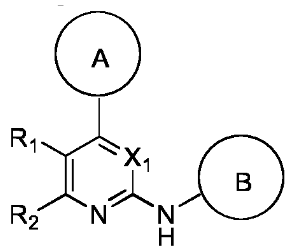
Formula II

wherein,

$X_1$ is selected from the group consisting of N and $CR_3$;
$R_1$ is selected from the group consisting of H, $CF_3$, F, Cl, Br, methyl, ethyl, isopropyl, and cyclopropyl;
$R_2$ is selected from the group consisting of H, F, $CF_3$, $CF_2H$, $NH_2$, and methyl;
or $R_1$ and $R_2$ together with the C to which they are attached form a 5-membered heteroaryl containing 2 N atoms;
$R_3$ is selected from the group consisting of H, $CF_3$, F, Cl, Br, methyl, and cyano;
Ring A is selected from the group consisting of

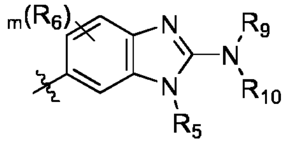,

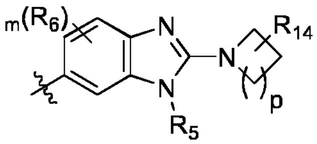, 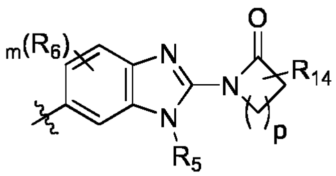, 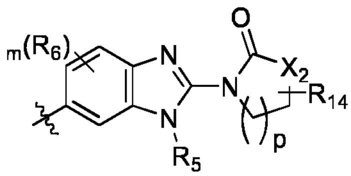,

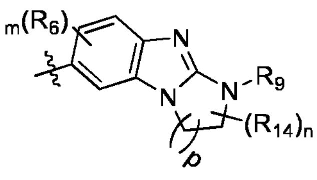, 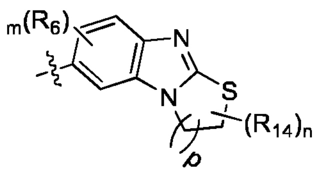, 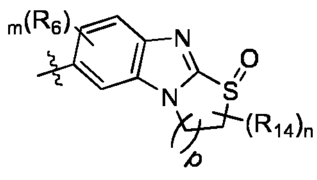,

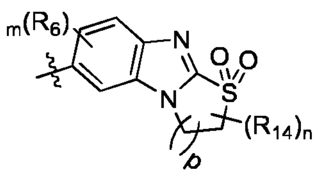, 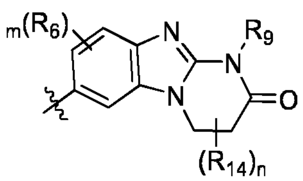, and 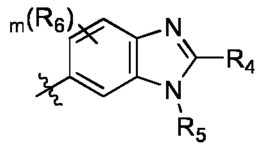,

wherein, $X_2$ is O or NR;
Ring B is selected from the group consisting of

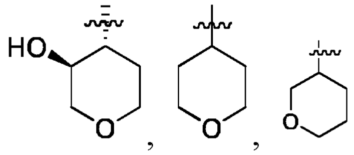 , , ,

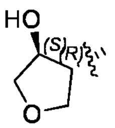 , 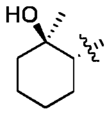 , 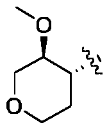 , 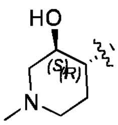 , 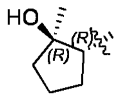 , 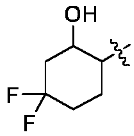 , 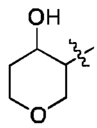 ,

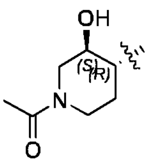 , 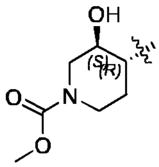 , 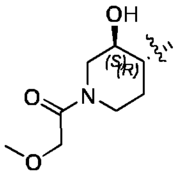 , 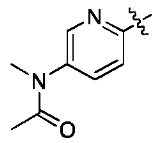 , 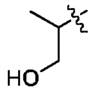 , 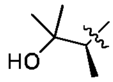 ,

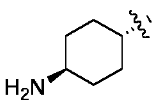 , 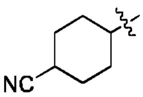 , 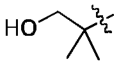 , 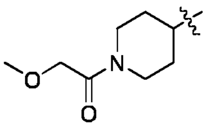 , 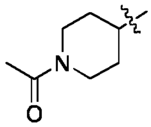 , 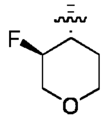 ,

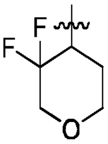 , 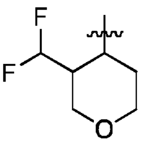 , 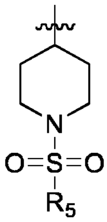 , 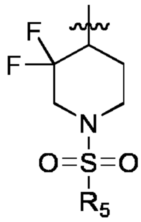 , 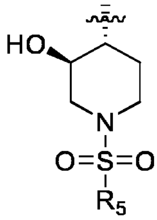 , 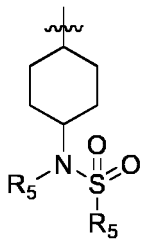 , 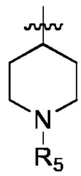 ,

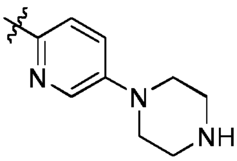 , 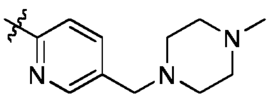 , 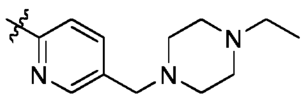 ,

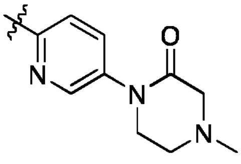 , 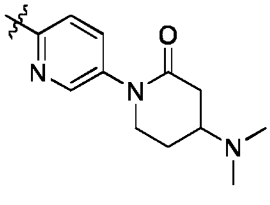 , 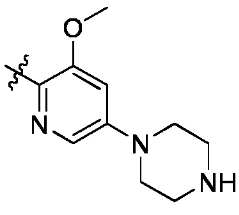 , 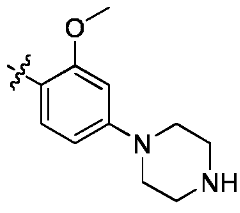 ,

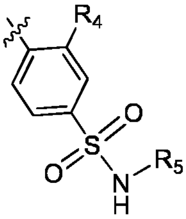 , 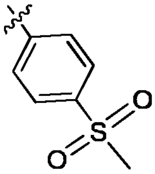 , 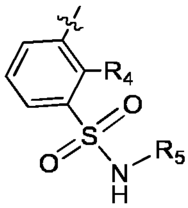 , 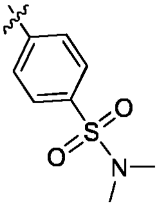 , 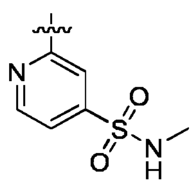 ,

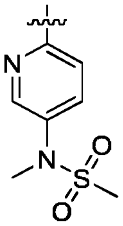, 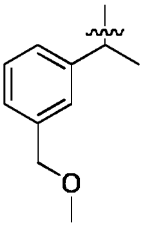, 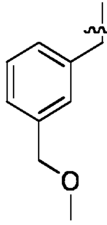, 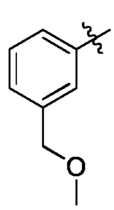, 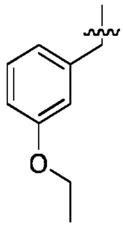, 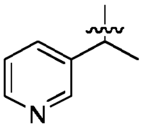, 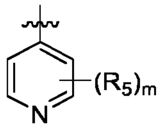,

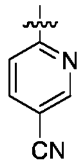, 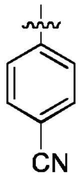, 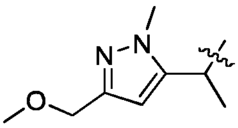, 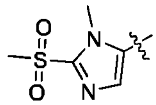, 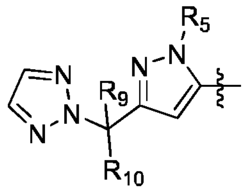,

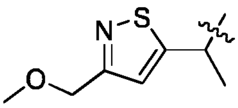, 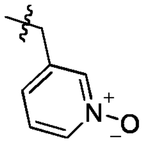, 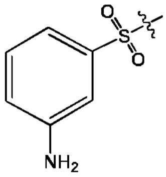, 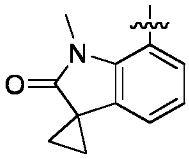, 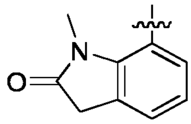,

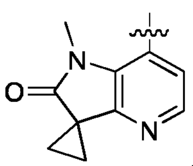, 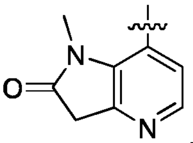, 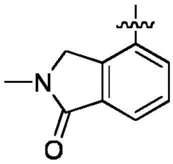, 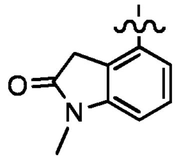, and 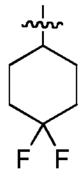;

wherein, each $R_4$ is independently selected from the group consisting of H, cyano, halogen, substituted or unsubstituted C1-C6 alkyl, and substituted or unsubstituted C3-C6 cycloalkyl;

each $R_5$, $R_6$, $R_9$, $R_{10}$, and $R_{14}$ is each independently selected from the group consisting of H, halogen, hydroxyl, amino,

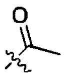, 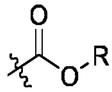, 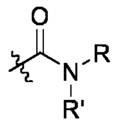,

oxo, substituted or unsubstituted C1-C6 alkyl, substituted or unsubstituted C3-C6 cycloalkyl, substituted or unsubstituted phenyl, substituted or unsubstituted $C_{1-6}$ alkoxy, and substituted or unsubstituted $-NR_mR_n$; or two $R_{14}$ attached to the same C together with the C to which they are attached form C3-C6 cycloalkyl;

or $R_9$ and $R_{10}$ together with N to which they are attached form

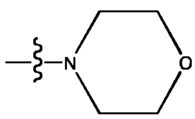 or 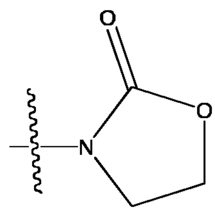;

or when ring A is

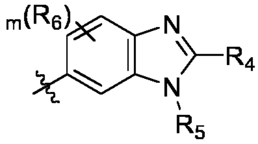,

$R_4$ and $R_5$ together with the ring to which they are attached form a 5-7-membered heterocycle containing 1, 2 or 3 heteroatoms selected from N, O, or S;

each R and R' is independently selected from the group consisting of H, C1-C6 alkyl, halogenated C1-C6 alkyl, C3-C6 cycloalkyl, halogenated C3-C6 cycloalkyl, and phenyl;

each $R_m$ is selected from the group consisting of H, $C_{1-6}$ alkyl, $C_{3-6}$ cycloalkyl, $C_{6-10}$ aryl, 6-10-membered heteroaryl containing 1, 2 or 3 heteroatoms selected from N, O, or S;

each $R_n$ is C1-6 alkyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each m, n, p is independently selected from the group consisting of 0, 1, 2, 3, and 4.

In another preferred embodiment,

$R_1$ is selected from the group consisting of H, $CF_3$, F, Cl, Br, methyl, ethyl, isopropyl, and cyclopropyl;

$R_2$ is selected from the group consisting of H, F, $CF_3$, $CF_2H$, $NH_2$, and methyl;

Ring A is selected from the group consisting of

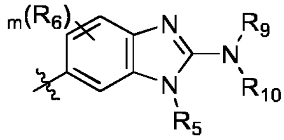,

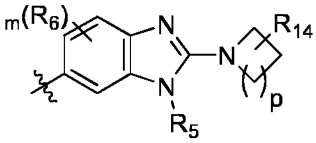, 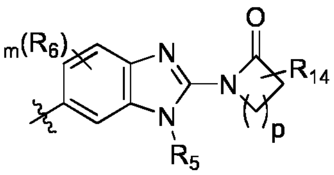, 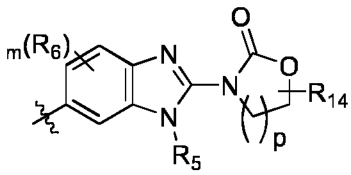,

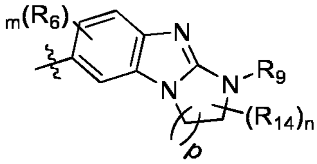, and 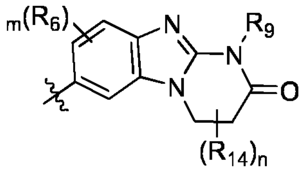,

Ring B is selected from the group consisting of

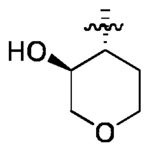, 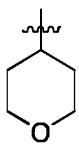, 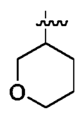,

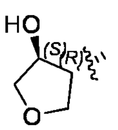, 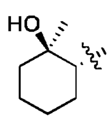, 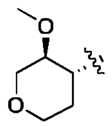, 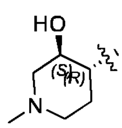, 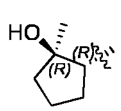, 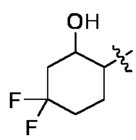, 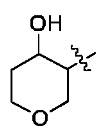,

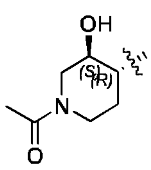, 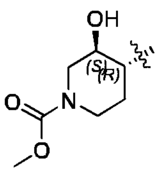, 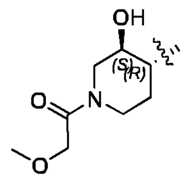, 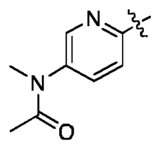, 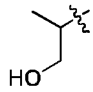, 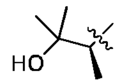,

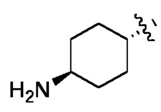, 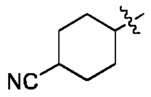, 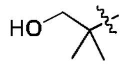, 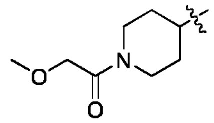, 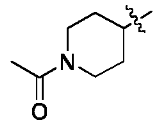, 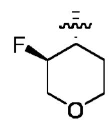,

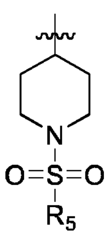, 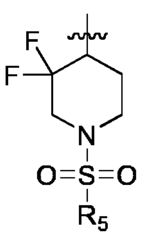, 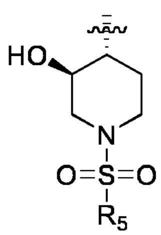, 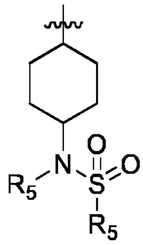, 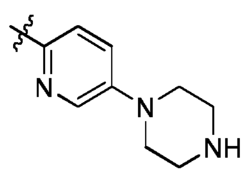,

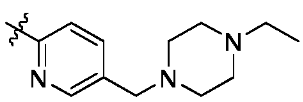, 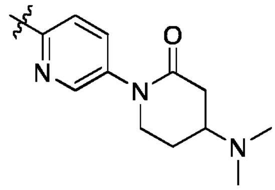, 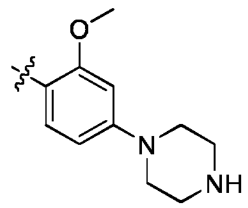, 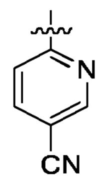,

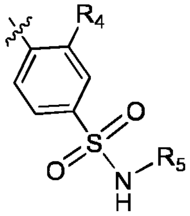, 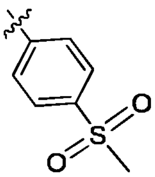, 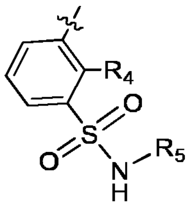, 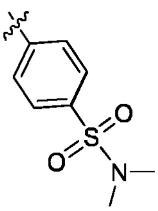, 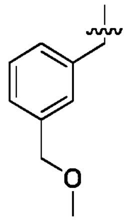, 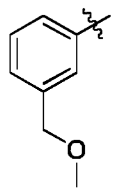,

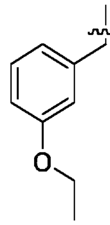, 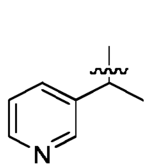, 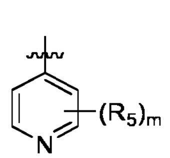, 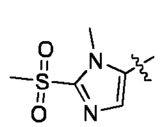, 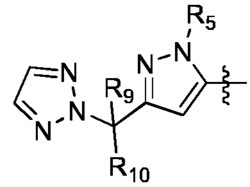,

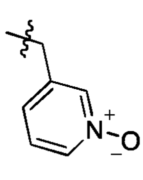, 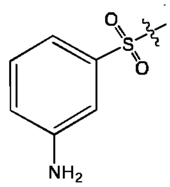, 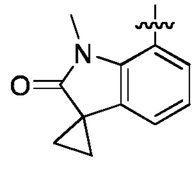, 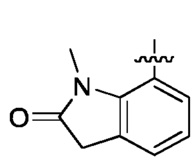, 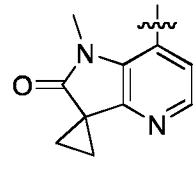,

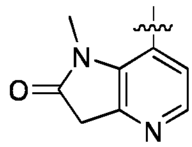, 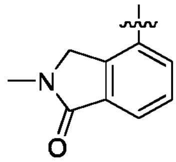, and 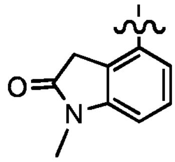;

wherein,

each $R_5$, $R_6$, $R_9$, $R_{10}$ and $R_{14}$ is independently selected from the group consisting of H, halogen, hydroxyl, amino,

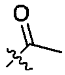, 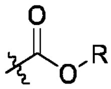, 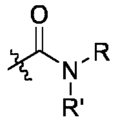,

substituted or unsubstituted C1-C6 alkyl, substituted or unsubstituted C3-C6 cycloalkyl, and substituted or unsubstituted phenyl;
or $R_9$, $R_{10}$ together with N to which they are attached form

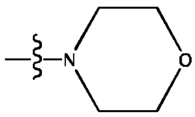;

R and R' are each independently selected from the group consisting of H, C1-C6 alkyl, halogenated C1-C6 alkyl, C3-C6 cycloalkyl, halogenated C3-C6 cycloalkyl, and phenyl;
the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, cyano;
each m, n, p is independently selected from the group consisting of 0, 1, 2, 3, and 4.

In another preferred embodiment,

$R_1$ is selected from the group consisting of Cl and Br;
$R_2$ is H;
Ring A is selected from the group consisting of

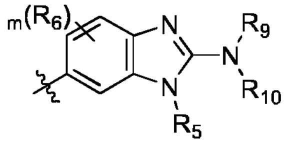,

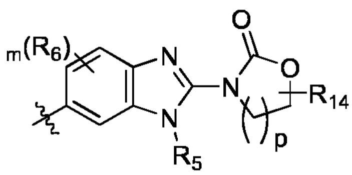, 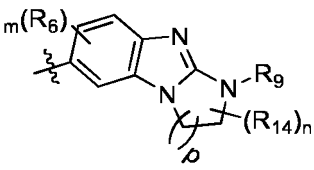, and 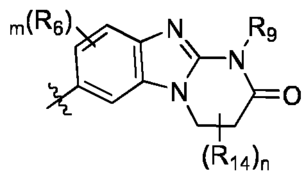;

Ring B is selected from the group consisting of

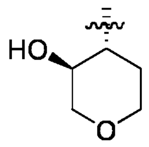, 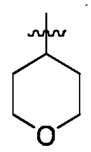,

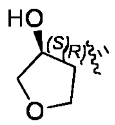, 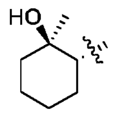, 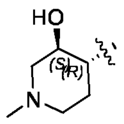, 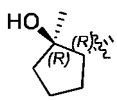, 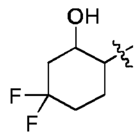, 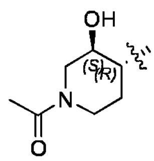, 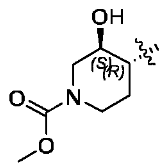,

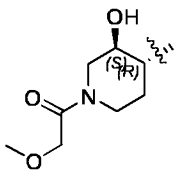, 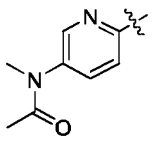, 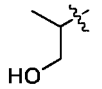, 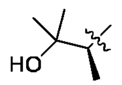, 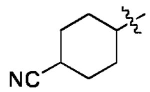, 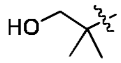,

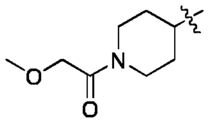, 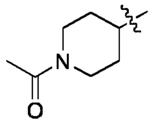, 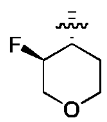, 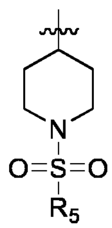, 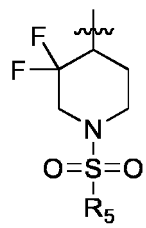, 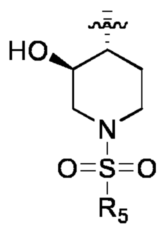,

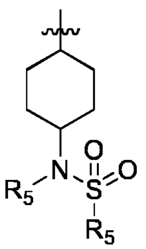, 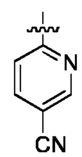, 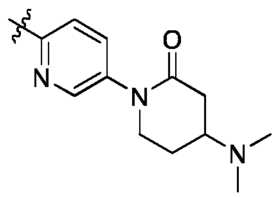, 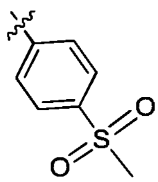, 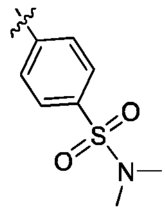,

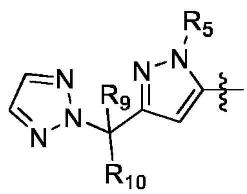, and 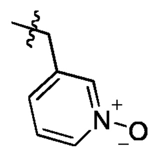;

wherein,

each $R_5$, $R_6$, $R_9$, $R_{10}$, and $R_{14}$ is each independently selected from the group consisting of H, halogen, hydroxyl, amino,

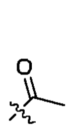, 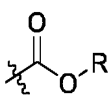, 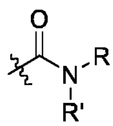,

substituted or unsubstituted C1-C6 alkyl, substituted or unsubstituted C3-C6 cycloalkyl, and substituted or

unsubstituted phenyl;
or $R_9$ and $R_{10}$ together with N to which they are attached form

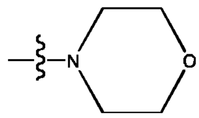;

R and R' is independently selected from the group consisting of H, C1-C6 alkyl, halogenated C1-C6 alkyl, C3-C6 cycloalkyl, halogenated C3-C6 cycloalkyl, and phenyl;
the substituted in $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;
each m, n, p is independently selected from the group consisting of 0, 1, 2, 3, and 4.

In another preferred embodiment,

$R_1$ and $R_2$ together with the C to which they are attached form a 5-membered heteroaryl containing two N atoms;

Ring A is selected from the group consisting of

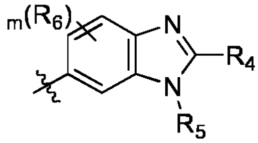,

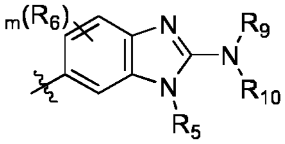, 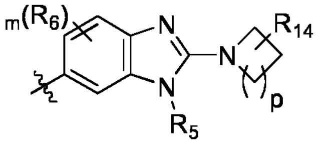, 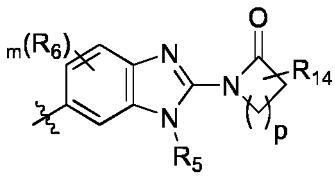,

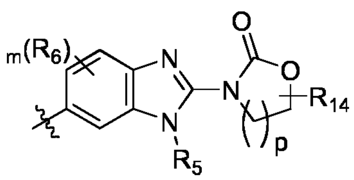, 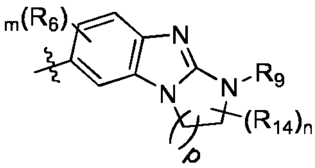, and 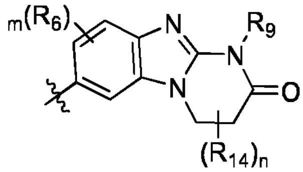,

Ring B is selected from the group consisting of

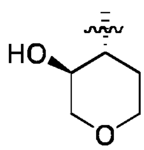, 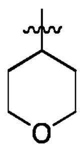, 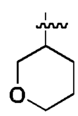,

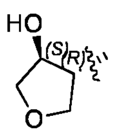, 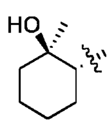, 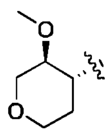, 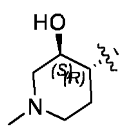, 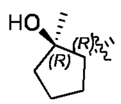, 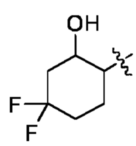, 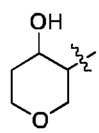,

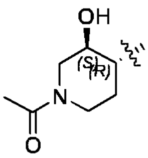, 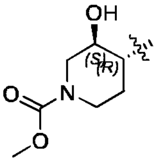, 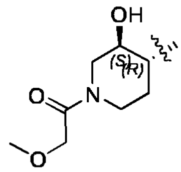, 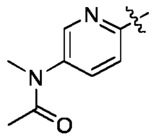, 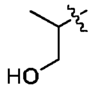, 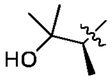,

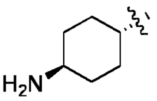, 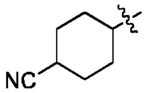, 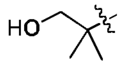, 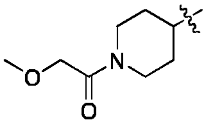, 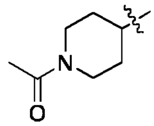, 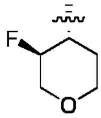,

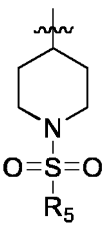, 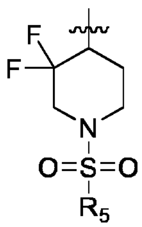, 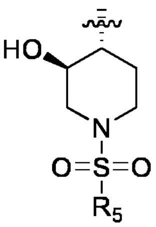, 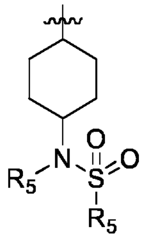, 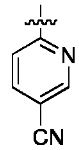, 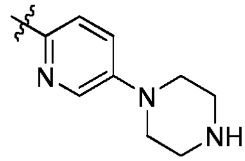,

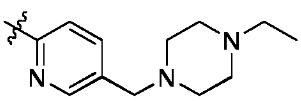, 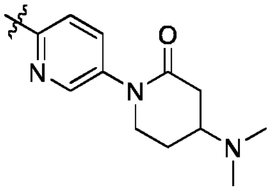, 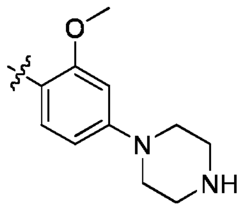, 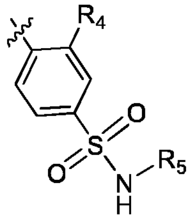,

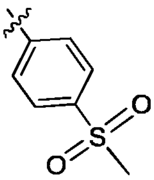, 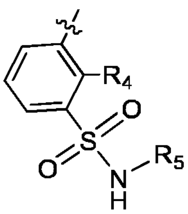, 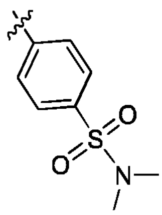, 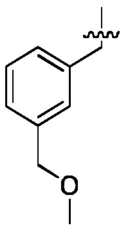, 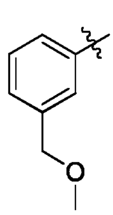, 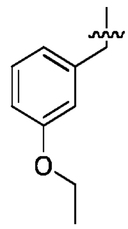,

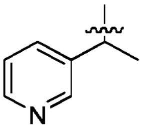, 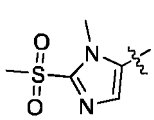, 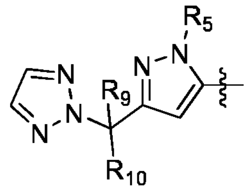, 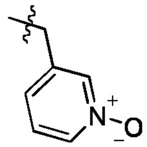, 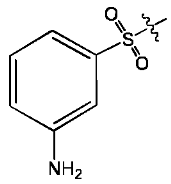,

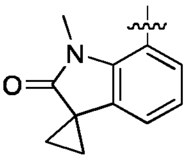, 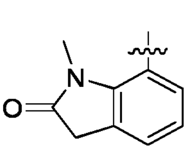, 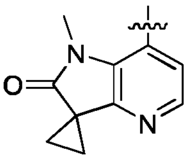, 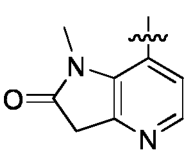, 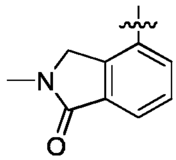,

and

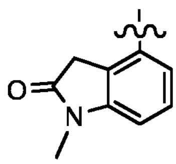
;

wherein, each $R_4$ is independently selected from the group consisting of H, substituted or unsubstituted C1-C6 alkyl, and substituted or unsubstituted C3-C6 cycloalkyl;

each $R_5$, $R_6$, $R_9$, $R_{10}$, and $R_{14}$ is each independently selected from the group consisting of H, halogen, hydroxyl, amino,

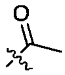
,
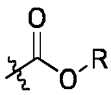
,
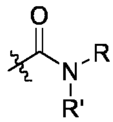
,

substituted or unsubstituted C1-C6 alkyl, substituted or unsubstituted C3-C6 cycloalkyl, and substituted or unsubstituted phenyl;

R and R' is independently selected from the group consisting of H, C1-C6 alkyl, halogenated C1-C6 alkyl, C3-C6 cycloalkyl, halogenated C3-C6 cycloalkyl, and phenyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each m, n, p is independently selected from the group consisting of 0, 1, 2, 3, and 4.

In another preferred embodiment,

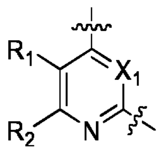

has the following structure:

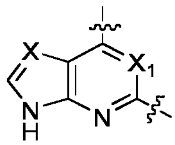
;

X is selected from the group consisting of N and $CR_3$;

$R_3$ is selected from the group consisting of H, $CF_3$, F, Cl, Br, methyl, and cyano.

In the third aspect of the present invention, it provides a compound for use as a CDK4 kinase inhibitor, wherein the compound is a compound of formula I, or a pharmaceutically acceptable salt, stereoisomer, tautomer, hydrate, solvate, isotope compound, or prodrug thereof, wherein,

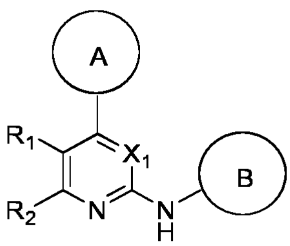
formula I

$X_1$ is selected from the group consisting of N and $CR_3$;
$R_1$ is selected from the group consisting of H, $CF_3$, F, Cl, Br, methyl, ethyl, isopropyl, and cyclopropyl;
$R_2$ is selected from the group consisting of H, F, $CF_3$, $CF_2H$, $NH_2$, and methyl;
or, $R_1$ and $R_2$ together with the C to which they are attached form a 5-membered heteroaryl containing 2 N atoms;
$R_3$ is selected from the group consisting of H, $CF_3$, F, Cl, Br, methyl, and cyano;
Ring A is selected from the group consisting of

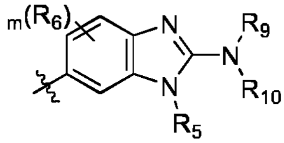 ,

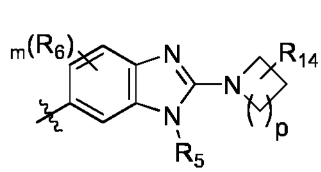 , 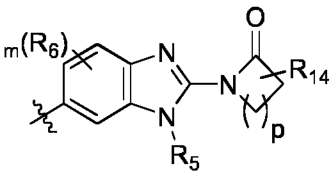 , 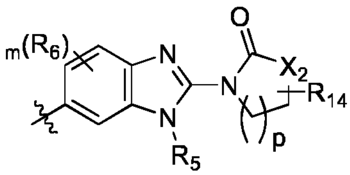 ,

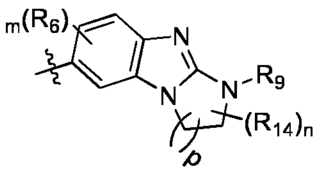 , 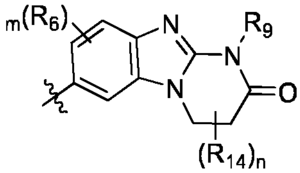 , and 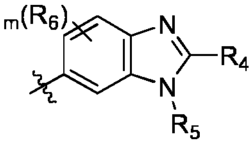 ,

wherein, $X_2$ is O or NR;

Ring B is selected from the group consisting of

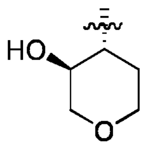 , 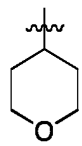 ,

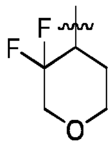 , 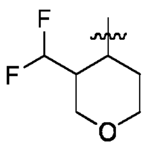 , 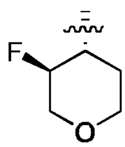 , 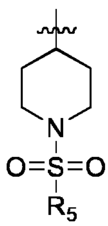 , 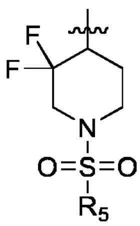 , 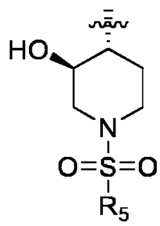 , 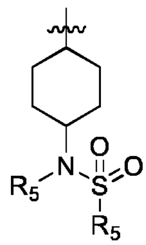 ,

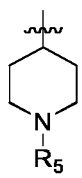 , 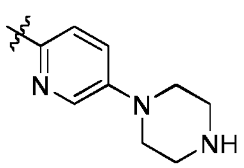 , 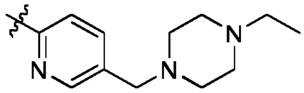 , 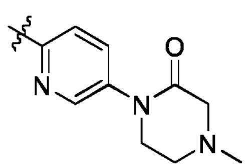 ,

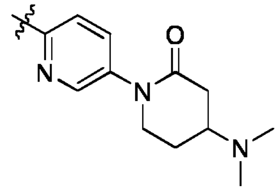, 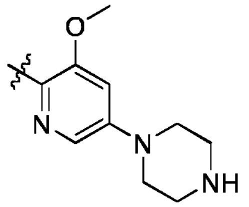, 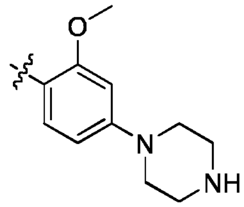, 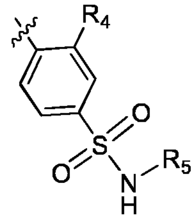,

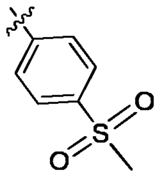, 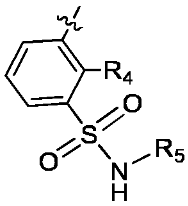, 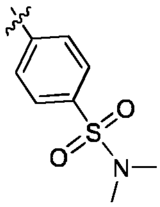, 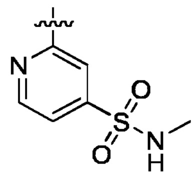, 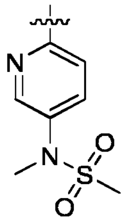,

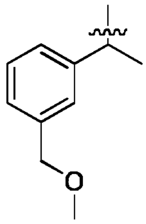, 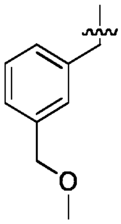, 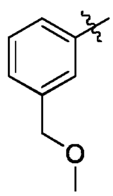, 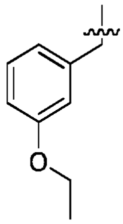, 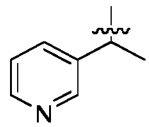, 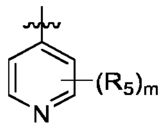, 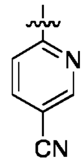,

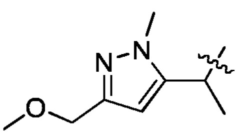, 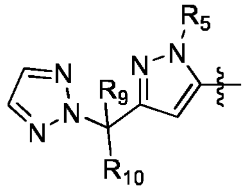, 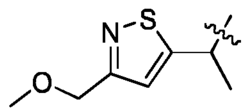, 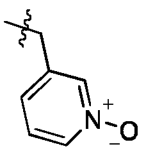,

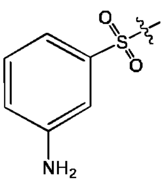, 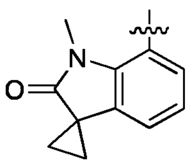, 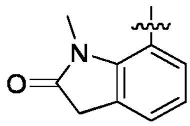, 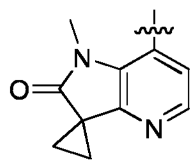, 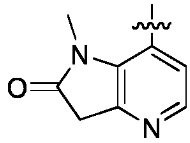,

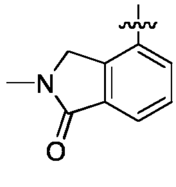, 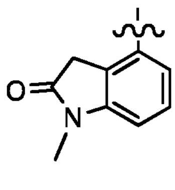, and 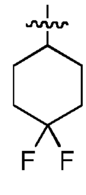;

wherein, each $R_4$ is independently selected from the group consisting of H, cyano, halogen, substituted or unsubstituted C1-C6 alkyl, substituted or unsubstituted C3-C6 cycloalkyl,
each $R_5$, $R_6$ , $R_9$, $R_{10}$ and $R_{14}$ is each independently selected from the group consisting of H, halogen, hydroxyl, amino,

, 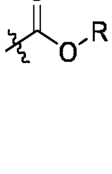, 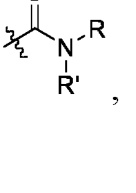,

substituted or unsubstituted C1-C6 alkyl, substituted or unsubstituted C3-C6 cycloalkyl, substituted or unsubstituted phenyl;

or $R_9$ and $R_{10}$ together with N to which they are attached form

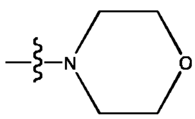 or 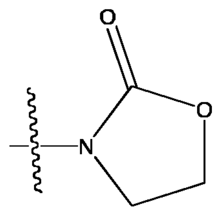 ;

each R and R' is independently selected from the group consisting of H, C1-C6 alkyl, halogenated C1-C6 alkyl, C3-C6 cycloalkyl, halogenated C3-C6 cycloalkyl, and phenyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each m, n, p is independently selected from the group consisting of 0, 1, 2, 3, and 4.

In another preferred embodiment,

$R_1$ is selected from the group consisting of H, $CF_3$, F, Cl, Br, methyl, ethyl, isopropyl, and cyclopropyl;

$R_2$ is selected from the group consisting of H, F, $CF_3$, $CF_2H$, $NH_2$, and methyl:

Ring A is selected from the group consisting of

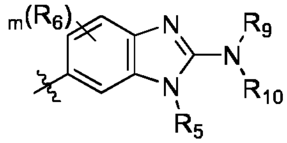 ,

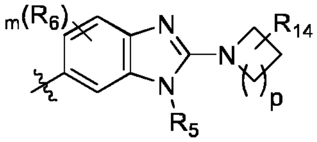 , 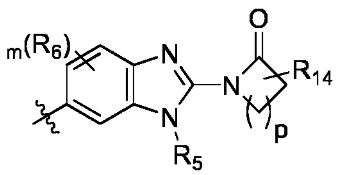 , 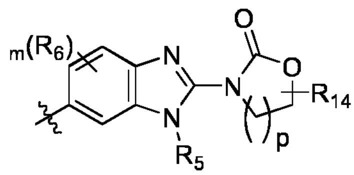 ,

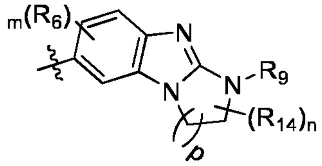 , and 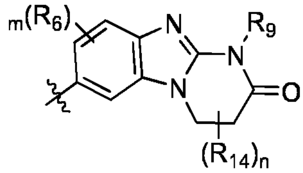 ,

Ring B is selected from the group consisting of

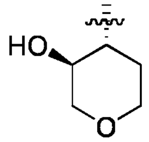 , 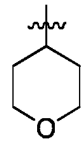 ,

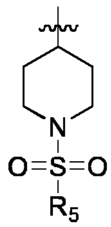, 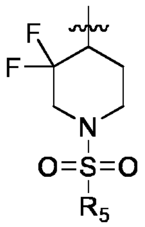, 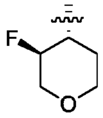, 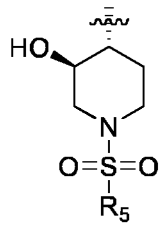, 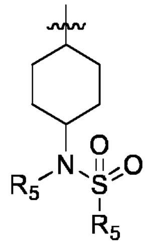, 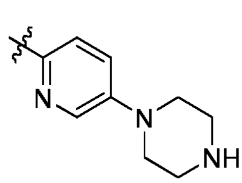,

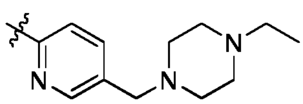, 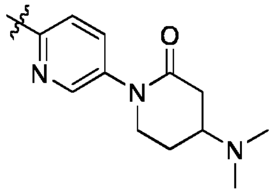, 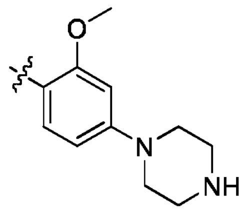, 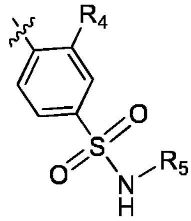,

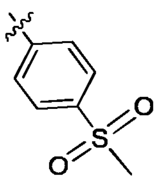, 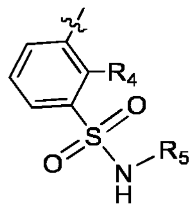, 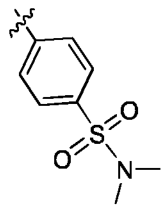, 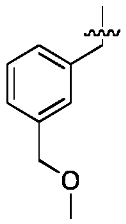, 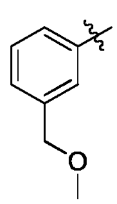, 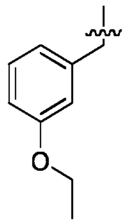,

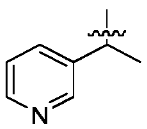, 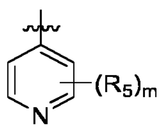, 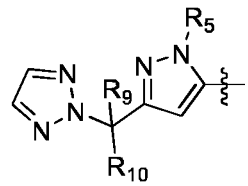, 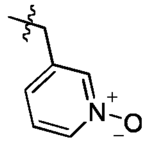, 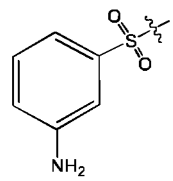,

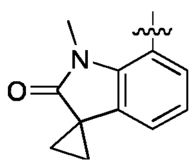, 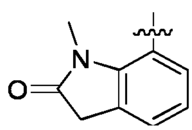, 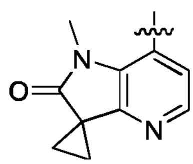, 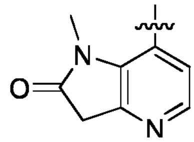, 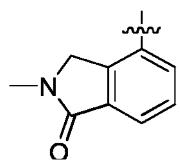,

and

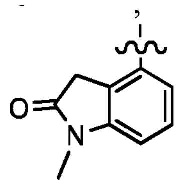;

wherein, each $R_4$ is independently selected from the group consisting of H, cyano, halogen, substituted or unsubstituted C1-C6 alkyl, and substituted or unsubstituted C3-C6 cycloalkyl;

each $R_5$, $R_6$, $R_9$, $R_{10}$ and $R_{14}$ is independently selected from the group consisting of H, halogen, hydroxyl, amino,

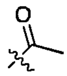, 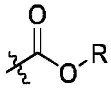, 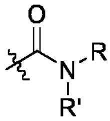,

substituted or unsubstituted C1-C6 alkyl, substituted or unsubstituted C3-C6 cycloalkyl, and substituted or unsubstituted phenyl;

or $R_9$, $R_{10}$ together with N to which they are attached form

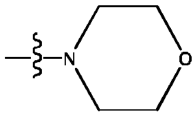;

R and R' are each independently selected from the group consisting of H, C1-C6 alkyl, halogenated C1-C6 alkyl, C3-C6 cycloalkyl, halogenated C3-C6 cycloalkyl, and phenyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, cyano;

each m, n, p is independently selected from the group consisting of 0, 1, 2, 3, and 4.

In another preferred embodiment,

$R_1$ is selected from the group consisting of $CF_3$, F, Cl, Br;

$R_2$ is H;

Ring A is selected from the group consisting of

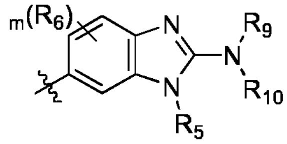,

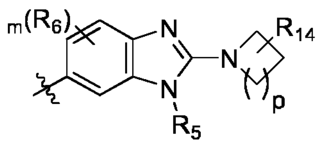, 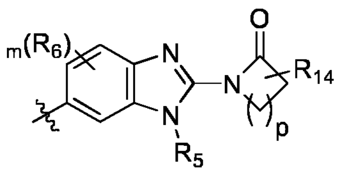, 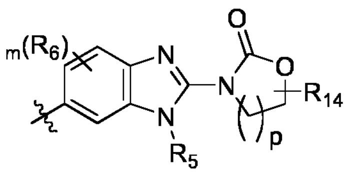,

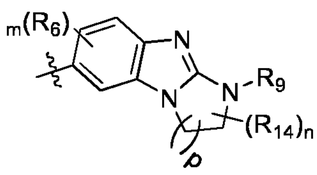, and 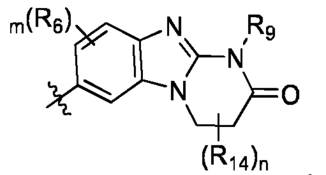,

Ring B is selected from the group consisting of

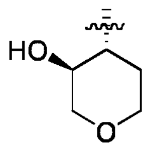, 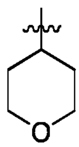,

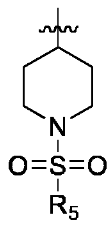, 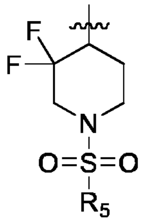, 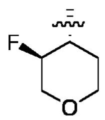, 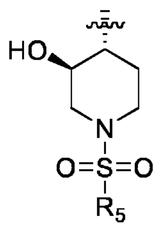, 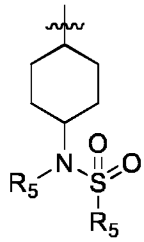, 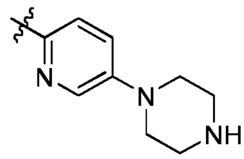,

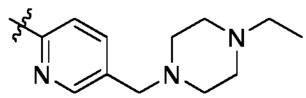, 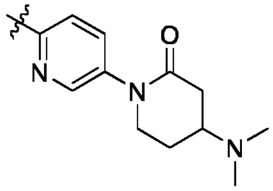, 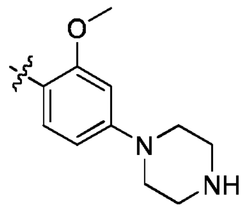, 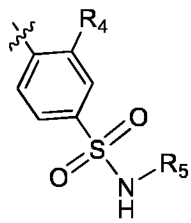,

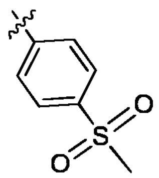, 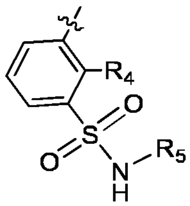, 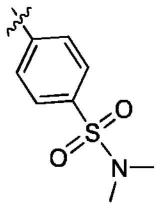, 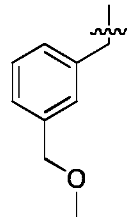, 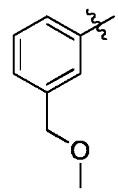, 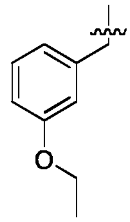,

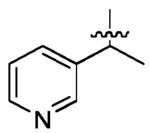, 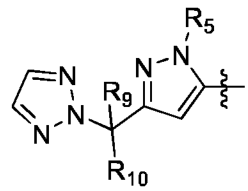, 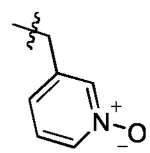, 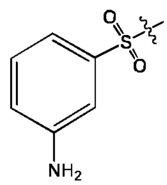, 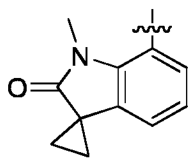,

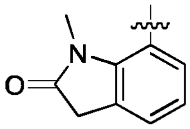, 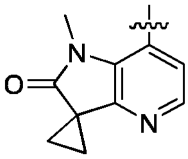, 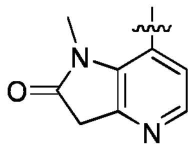, 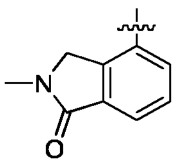, and 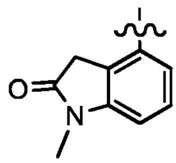;

wherein, each $R_4$ is independently selected from the group consisting of H, substituted or unsubstituted C1-C6 alkyl, and substituted or unsubstituted C3-C6 cycloalkyl;

each $R_5$, $R_6$, $R_9$, $R_{10}$ and $R_{14}$ is independently selected from the group consisting of H, halogen, hydroxyl, amino,

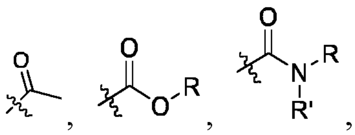

substituted or unsubstituted C1-C6 alkyl, substituted or unsubstituted C3-C6 cycloalkyl, and substituted or unsubstituted phenyl;

or $R_9$, $R_{10}$ together with N to which they are attached form

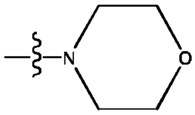
;

R and R' are each independently selected from the group consisting of H, C1-C6 alkyl, halogenated C1-C6 alkyl, C3-C6 cycloalkyl, halogenated C3-C6 cycloalkyl, and phenyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each m, n, p is independently selected from the group consisting of 0, 1, 2, 3, and 4.

In another preferred embodiment,

$R_1$ and $R_2$ together with the C to which they are attached form a 5-membered heteroaryl group containing two N atoms;

Ring A is selected from the group consisting of

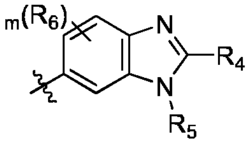
,

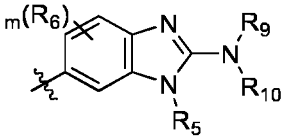
,
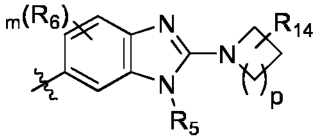
,
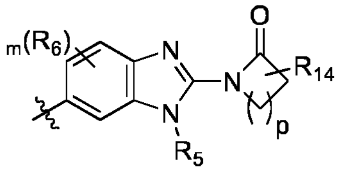
,

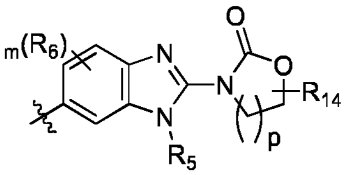
,
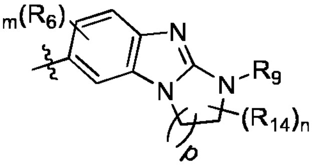
, and
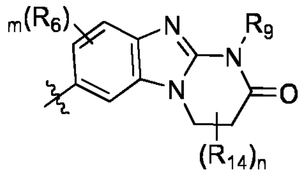
,

Ring B is selected from the group consisting of

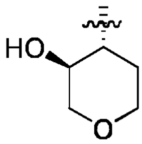
,
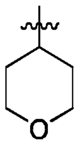
,

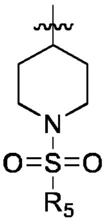
,
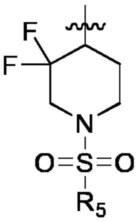
,
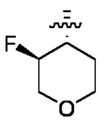
,
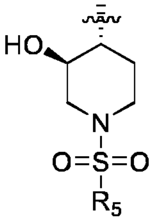
,
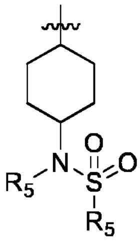
,
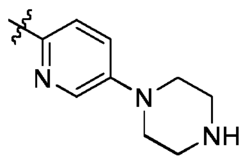
,

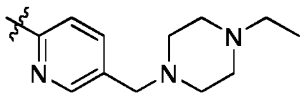, 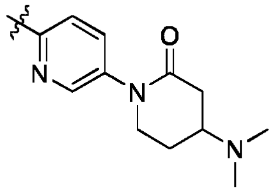, 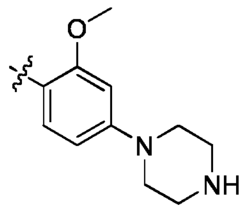, 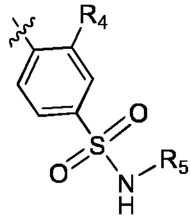,

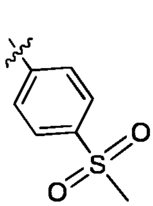, 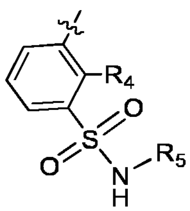, 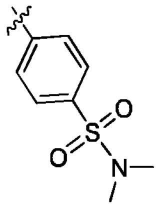, 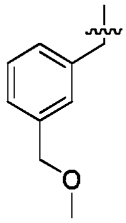, 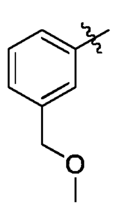, 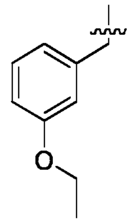,

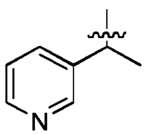, 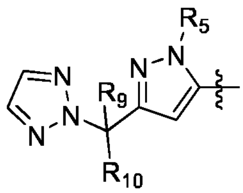, 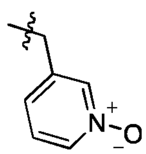, 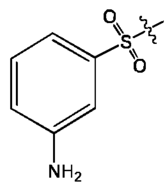, 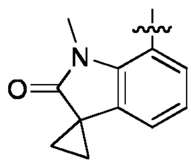,

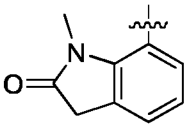, 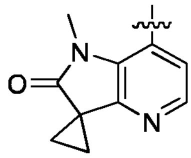, 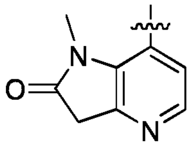, 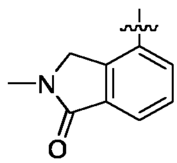, and 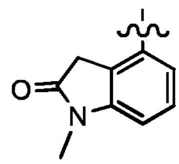;

wherein, each $R_4$ is independently selected from the group consisting of H, substituted or unsubstituted C1-C6 alkyl, and substituted or unsubstituted C3-C6 cycloalkyl;
each $R_5$, $R_6$, $R_9$, $R_{10}$ and $R_{14}$ is independently selected from the group consisting of H, halogen, hydroxyl, amino,

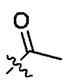, 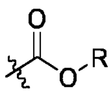, 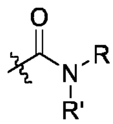,

substituted or unsubstituted C1-C6 alkyl, substituted or unsubstituted C3-C6 cycloalkyl, and substituted or unsubstituted phenyl;
R and R' are each independently selected from the group consisting of H, C1-C6 alkyl, halogenated C1-C6 alkyl, C3-C6 cycloalkyl, halogenated C3-C6 cycloalkyl, and phenyl;
the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;
each m, n, p is independently selected from the group consisting of 0, 1, 2, 3, and 4.

In another preferred embodiment,

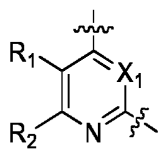

has the following structure:

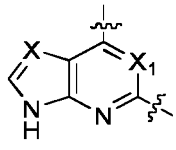
;

X is selected from the group consisting of N and $CR_3$;
$R_3$ is selected from the group consisting of H, $CF_3$, F, Cl, Br, methyl, and cyano.

In another preferred embodiment, the compound is selected from the group consisting of:

| | | |
|---|---|---|
| 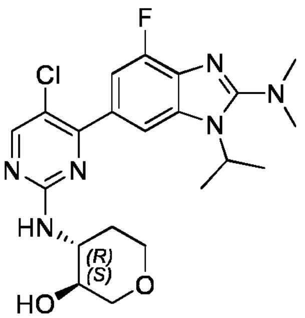 TB-1 | 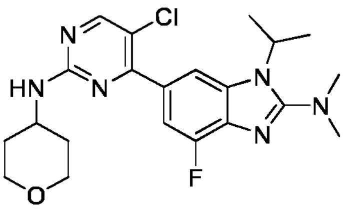 TB-2 | 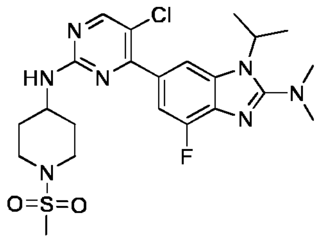 TB-3 |
| 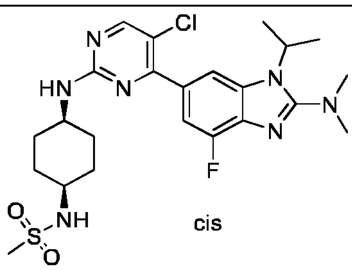 cis TB-4 | 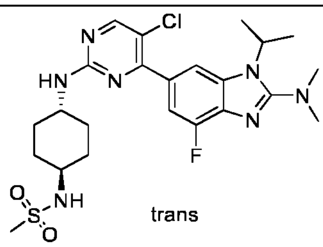 trans TB-5 | 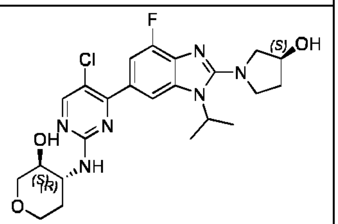 TB-6 |
| 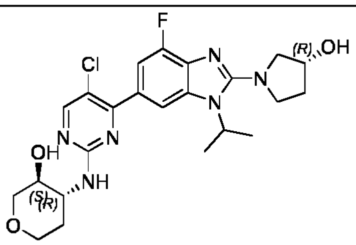 TB-7 | 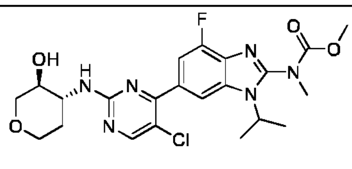 TB-8 | 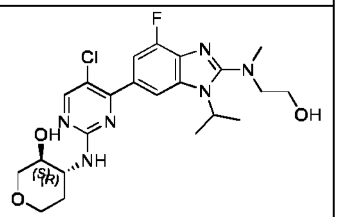 TB-9 |

| | | |
|---|---|---|
| 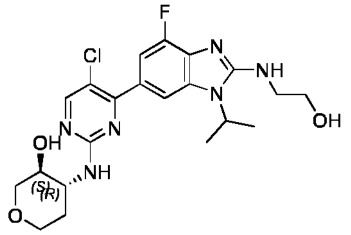 TB-10 | 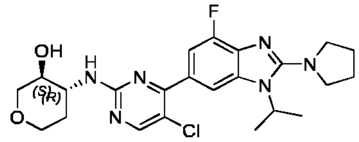 TB-11 | 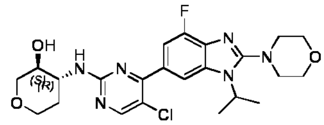 TB-12 |
| 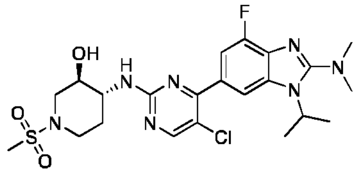 TB-13 | 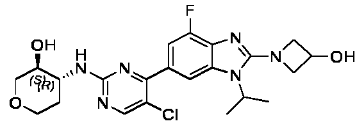 TB-14 | 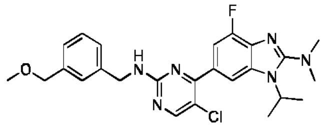 TB-15 |
| 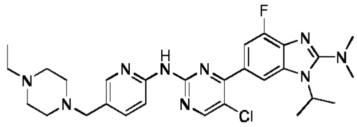 TB-16 | 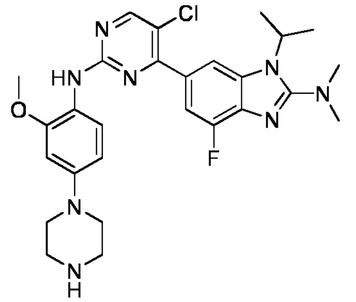 TB-17 | 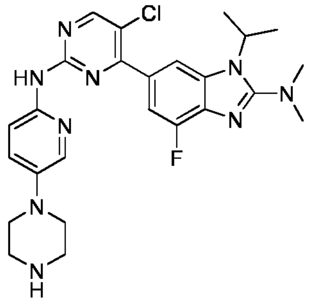 TB-18 |
| 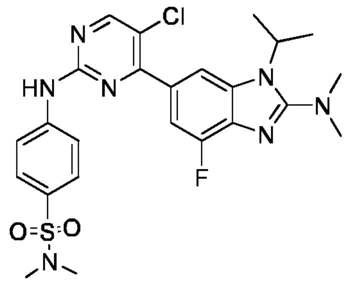 TB-19 | 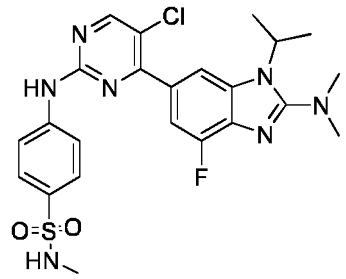 TB-20 | 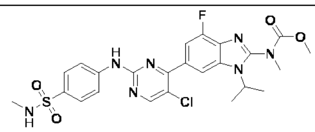 TB-21 |
| 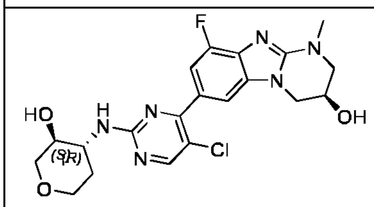 TB-22 | 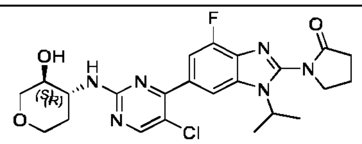 TB-23 | 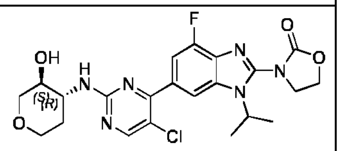 TB-24 |
| 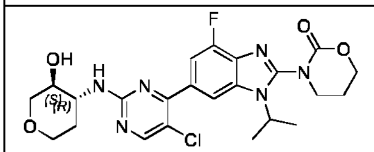 TB-25 | 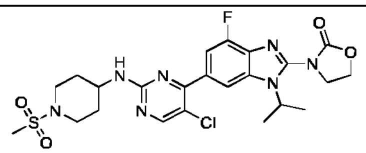 TB-26 | 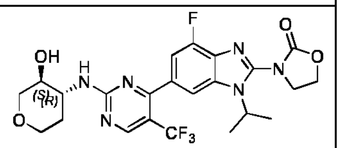 TB-27 |

| | | |
|---|---|---|
| 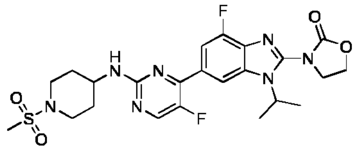<br>TB-28 | 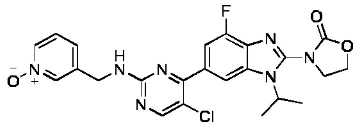<br>TB-29 | 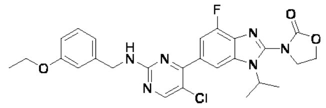<br>TB-30 |
| 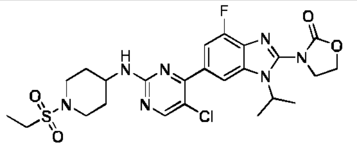<br>TB-31 | 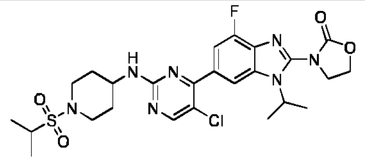<br>TB-32 | 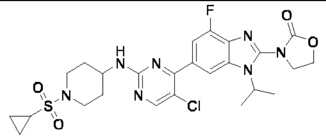<br>TB-33 |
| 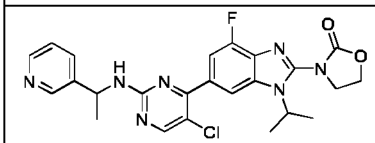<br>TB-34 | 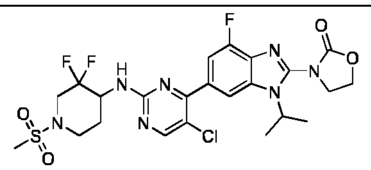<br>TB-35 | 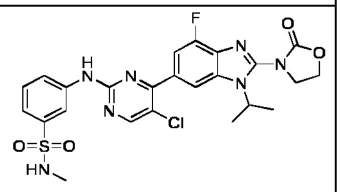<br>TB-36 |
| 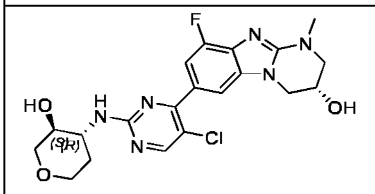<br>TB-37 | 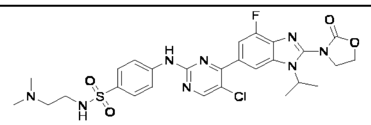<br>TB-38 | 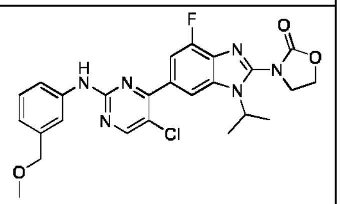<br>TB-39 |
| 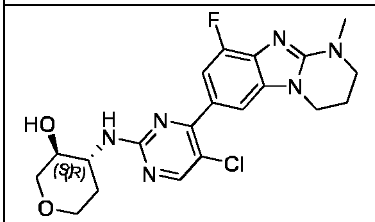<br>TB-40 | 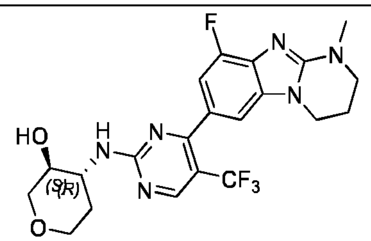<br>TB-41 | 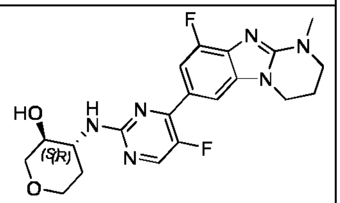<br>TB-42 |
| 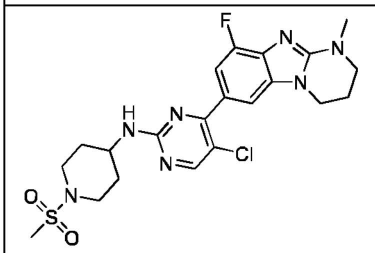<br>TB-43 | 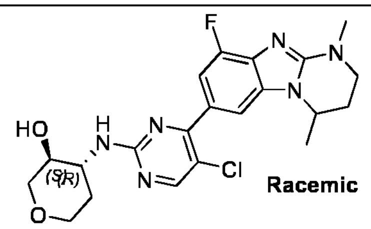<br>Racemic<br>TB-44 | 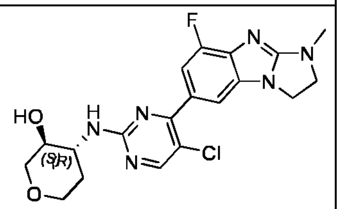<br>TB-45 |

| | | |
|---|---|---|
| 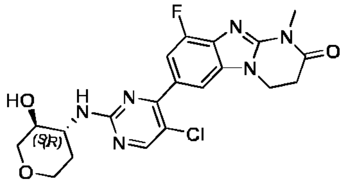 TB-46 | 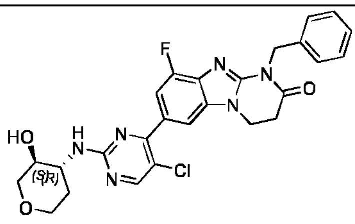 TB-47 | 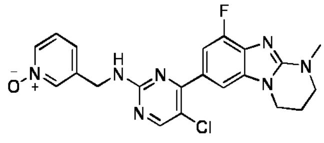 TB-48 |
| 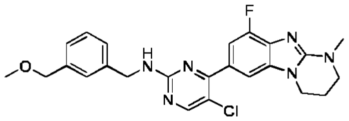 TB-49 | 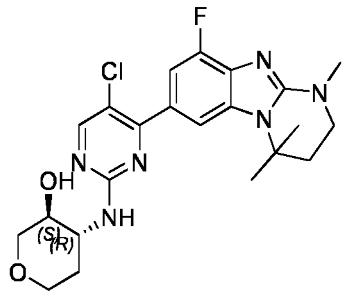 TB-50 | 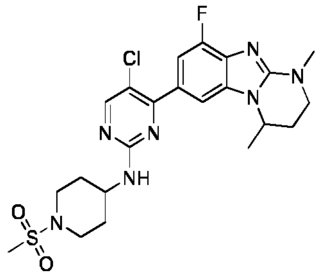 TB-51 |
| 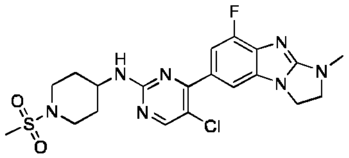 TB-52 | 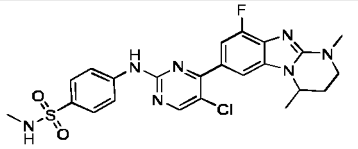 TB-53 | 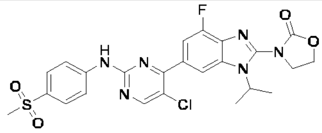 TB-54 |
| 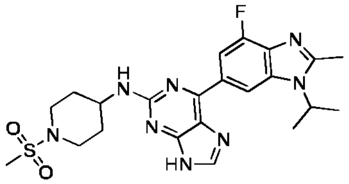 TB-55 | 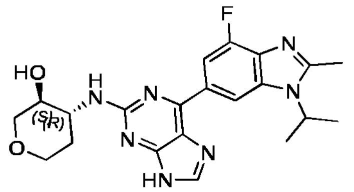 TB-56 | 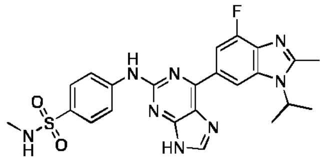 TB-57 |
| 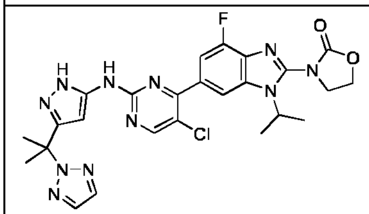 TB-58 | 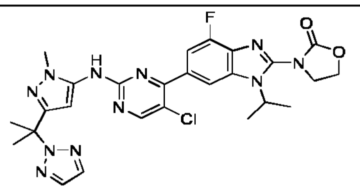 TB-59 | 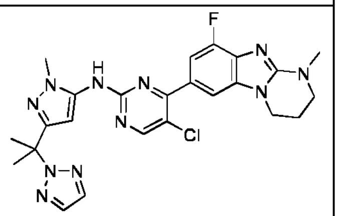 TB-60 |

| | | |
|---|---|---|
| 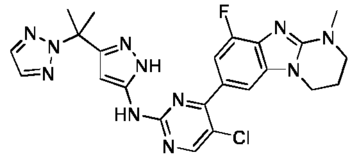 TB-61 | 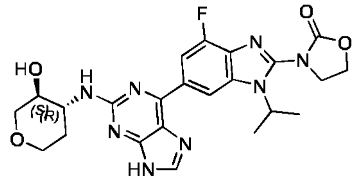 TB-62 | 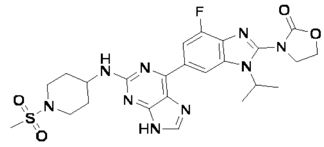 TB-63 |
| 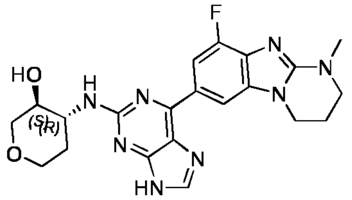 TB-64 | 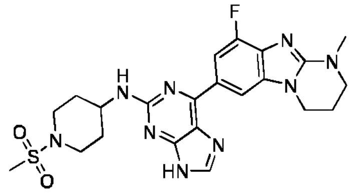 TB-65 | 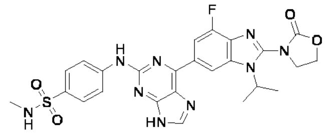 TB-66 |
| 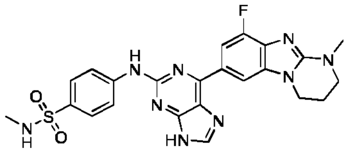 TB-67 | 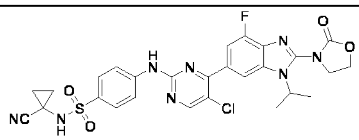 TB-68 | 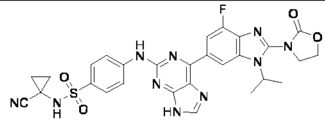 TB-69 |
| 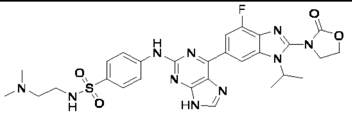 TB-70 | 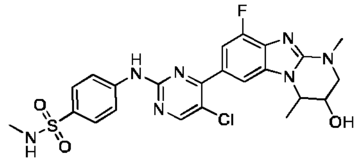 TB-71 | 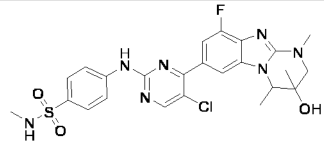 TB-72 |
| 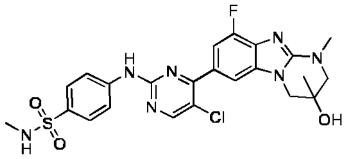 TB-73 | 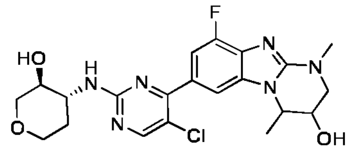 TB-74 | 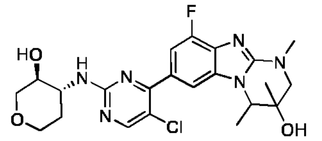 TB-75 |
| 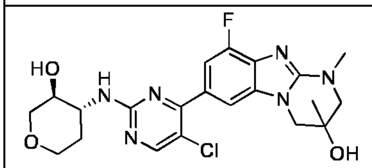 TB-76 | 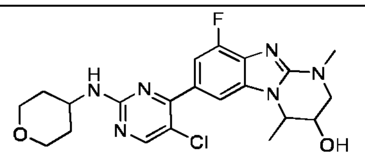 TB-77 | 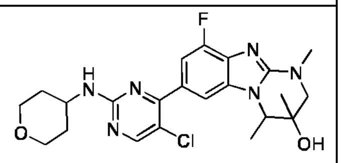 TB-78 |

| | | |
|---|---|---|
| 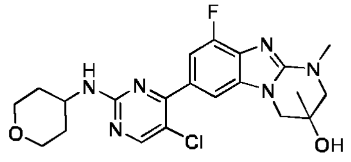 TB-79 | 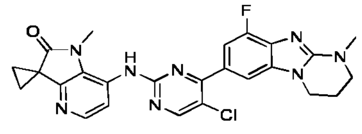 TB-80 | 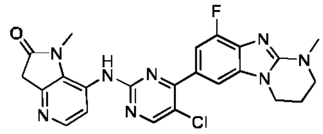 TB-81 |
| 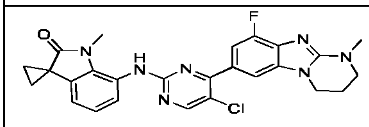 TB-82 | 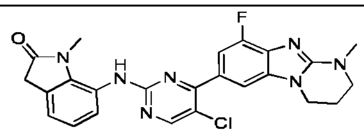 TB-83 | 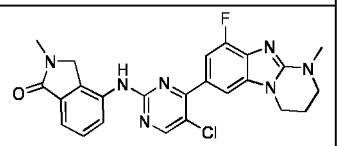 TB-84 |
| 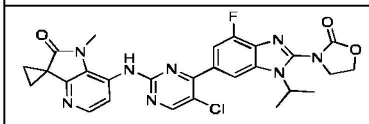 TB-85 | 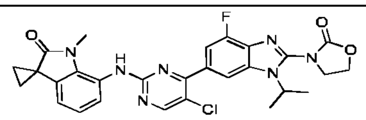 TB-86 | 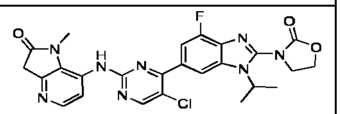 TB-87 |
| 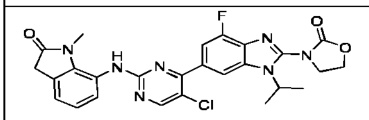 TB-88 | 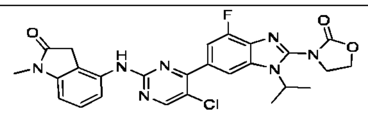 TB-89 | 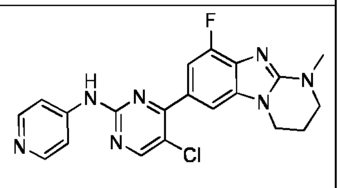 TB-90 |
| 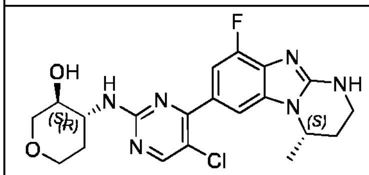 TB-91' | 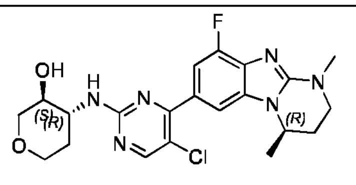 TB-91 | 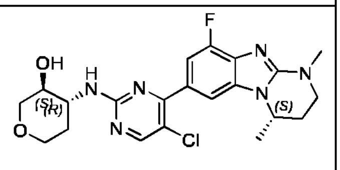 TB-92 |
| 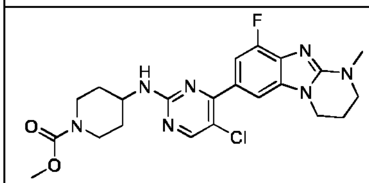 TB-93 | 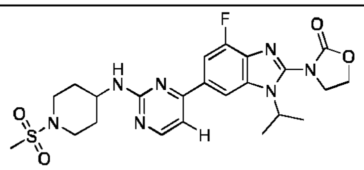 TB-94 | 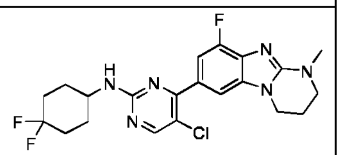 TB-95 |
| 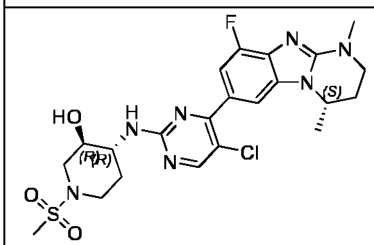 TB-96 | 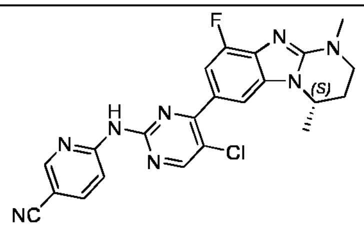 TB-97 | 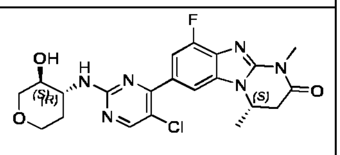 TB-98 |

| | | |
|---|---|---|
| 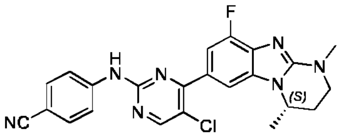 TB-99 | 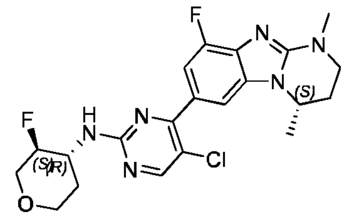 TB-100 | 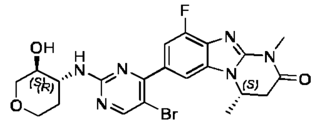 TB-101 |
| 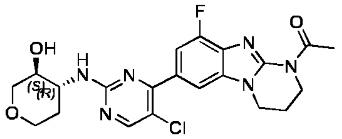 TB-102 | 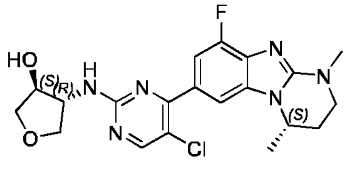 TB-103 | 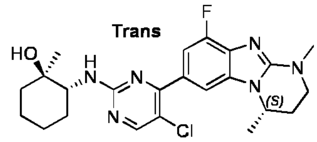 TB-104 |
| 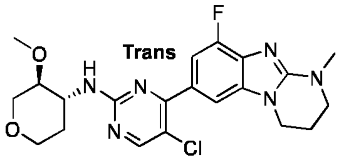 TB-105 | 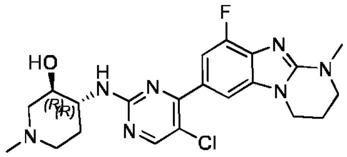 TB-106 | 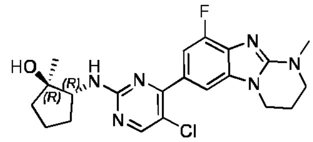 TB-107 |
| 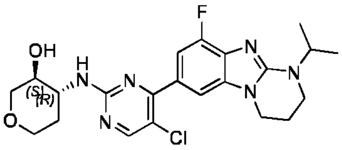 TB-108 | 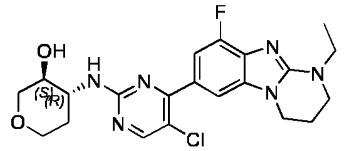 TB-109 | 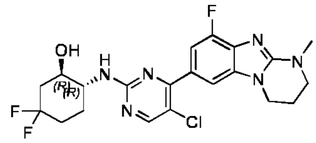 TB-110 |
| 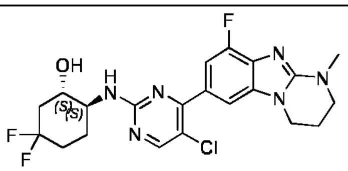 TB-111 | 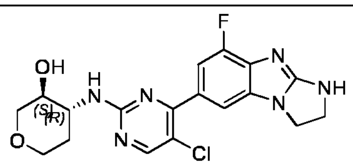 TB-112 | 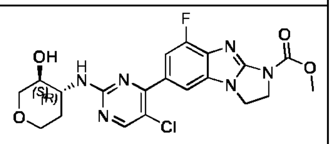 TB-113 |
| 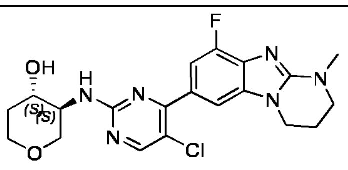 TB-114 | 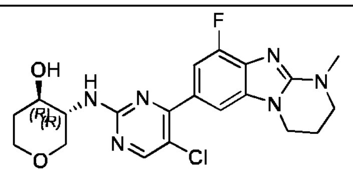 TB-115 | 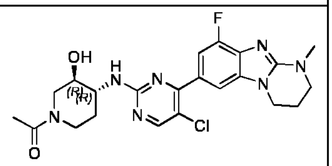 TB-116 |
| 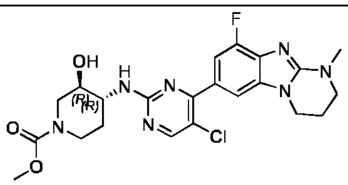 TB-117 | 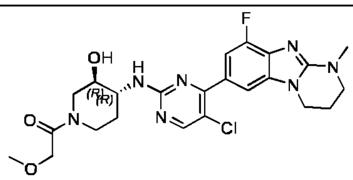 TB-118 | 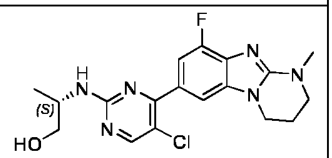 TB-119 |
| 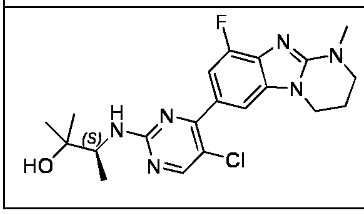 | 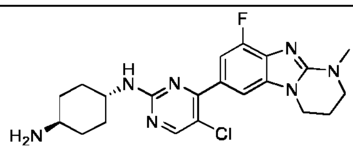 TB-121 | 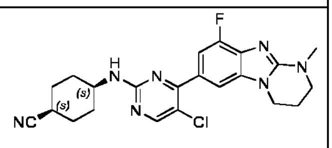 TB-122 |

| | | |
|---|---|---|
| TB-120 | | |
| 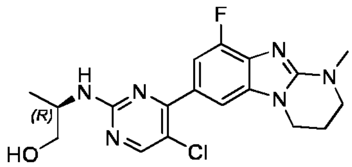 TB-123 | 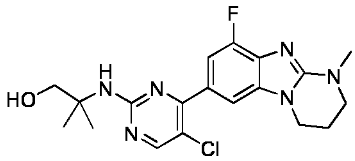 TB-124 | 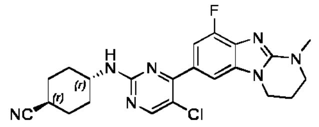 TB-125 |
| 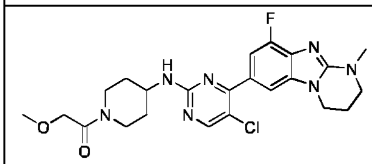 TB-126 | 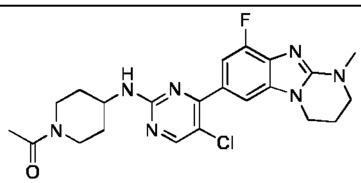 TB-127 | 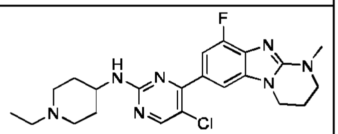 TB-128 |
| 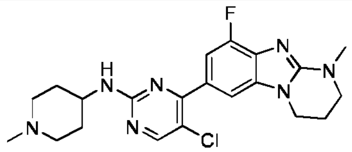 TB-129 | 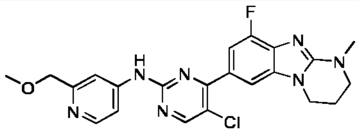 TB-130 | 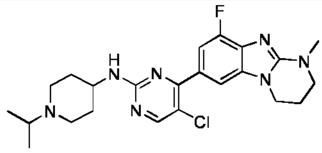 TB-131 |
| 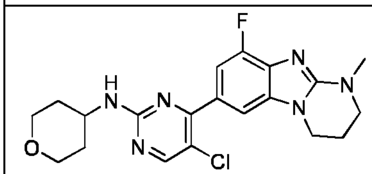 TB-132 | 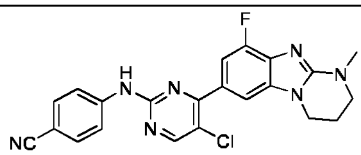 TB-133 | Racemic 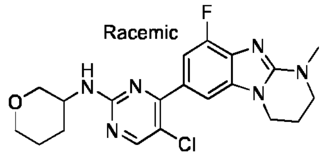 TB-134 |
| 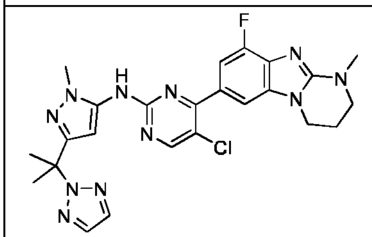 TB-135 | Trans 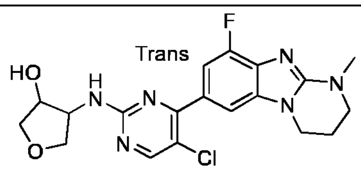 TB-136 | 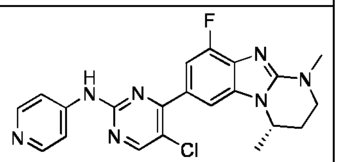 TB-137 |
| 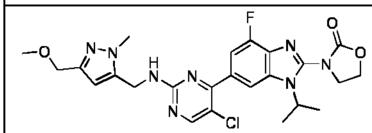 TB-138 | 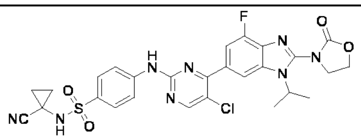 TB-139 | 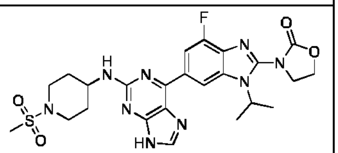 TB-140 |
| 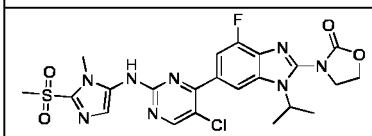 TB-141 | 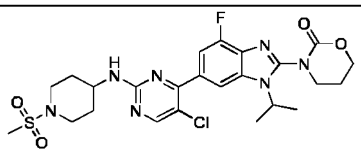 TB-142 | 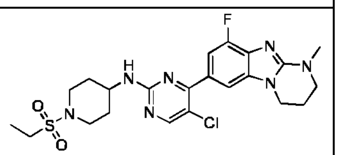 TB-143 |

| | | |
|---|---|---|
| 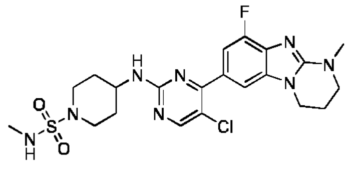 TB-144 | 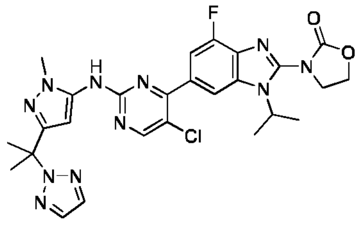 TB-145 | 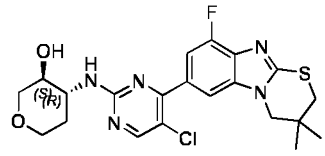 TB-146 |
| 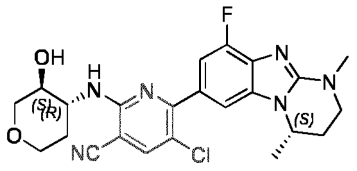 TB-147 | 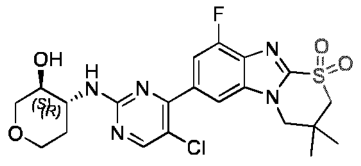 TB-148 | 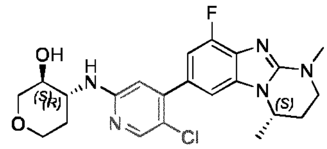 TB-149 |
| 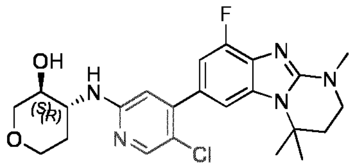 TB-150 | 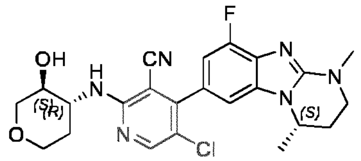 TB-151 | 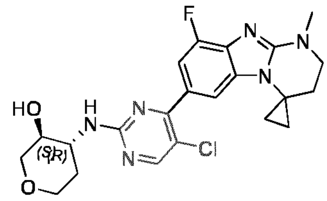 TB-152 |
| 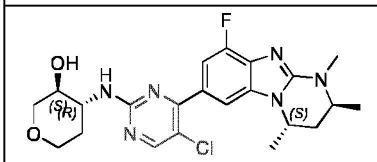 TB-153 | 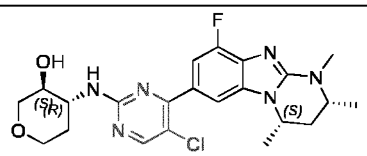 TB-154 | 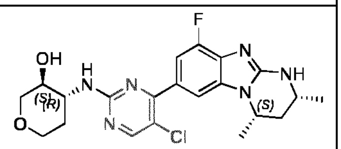 TB-155 |
| 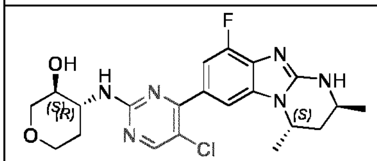 TB-156 | 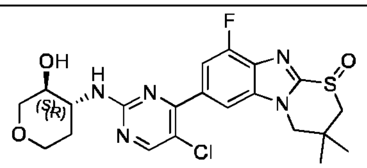 TB-157 | 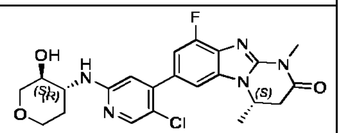 TB-158 |
| 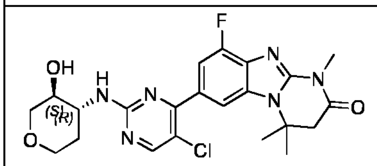 TB-159 | 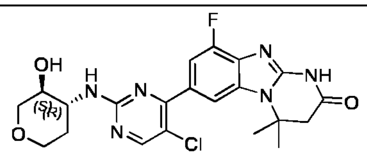 TB-160 | 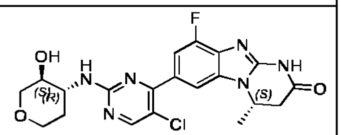 TB-161 |
| 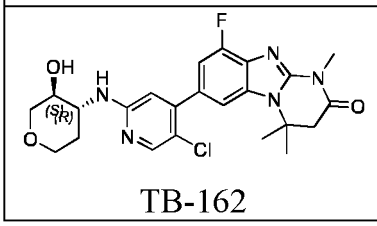 TB-162 | 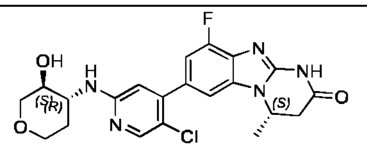 TB-163. | |

In the fourth aspect of the present invention, it provides a compound for use as a CDK4 kinase inhibitor, the compound is a compound of formula I, or a pharmaceutically acceptable salt, stereoisomer, tautomer, hydrate, solvate, isotope compound, or prodrug thereof,

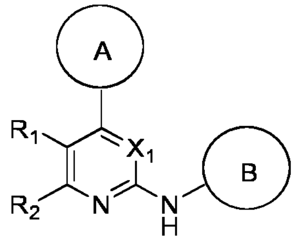

formula I

wherein,

$X_1$ is selected from the group consisting of N and $CR_3$;
$R_1$ is selected from the group consisting of H, $CF_3$, F, Cl, Br, methyl, ethyl, isopropyl, and cyclopropyl;
$R_2$ is selected from the group consisting of H, $CF_3$, F, Cl, Br, and methyl;
$R_3$ is selected from the group consisting of H, $CF_3$, F, Cl, Br, methyl, and cyano;
Ring A is selected from the group consisting of

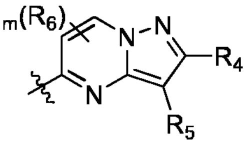

,

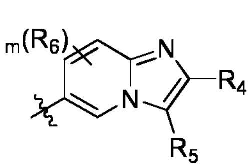

,

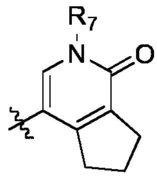

,

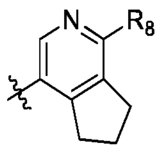

,

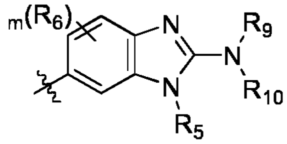

,

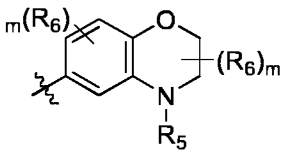

,

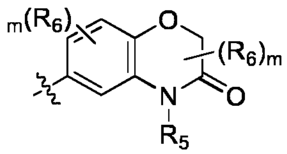

,

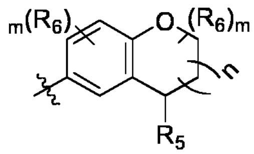

,

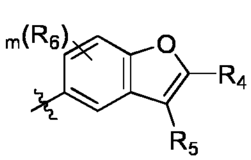

,

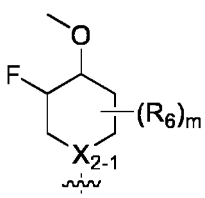

,

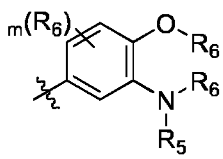

, and

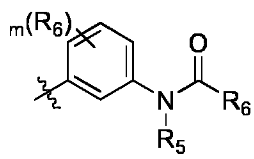

,

wherein,

$X_{2-1}$ is selected from the group consisting of N and $CR_{11}$;
$R_4$ is selected from the group consisting of H, substituted or unsubstituted $C_{1-6}$ alkyl, substituted or unsubstituted $C_{3-6}$ cycloalkyl, $-C(O)NR_9R_{10}$, substituted or unsubstituted $-NR_mR_n$, and substituted or unsubstituted $-NR_aR_b$;
each $R_5$ is independently selected from the group consisting of H, substituted or unsubstituted $C_{1-6}$ alkyl, substituted or unsubstituted $C_{1-6}$ alkoxy, substituted or unsubstituted $C_{3-6}$ cycloalkyl, substituted or unsubstituted $-NR_aR_b$, and substituted or unsubstituted $-NR_mR_n$;
each $R_6$ is each independently selected from the group consisting of halogen, substituted or unsubstituted $C_{1-6}$ alkyl, substituted or unsubstituted $C_{3-6}$ cycloalkyl;
$R_7$ and $R_{11}$ are each independently selected from the group consisting of H, and substituted or unsubstituted $C_{1-6}$ alkyl;
$R_8$ is selected from the group consisting of H, $C_{1-6}$ alkoxy, halogenated $C_{1-6}$ alkoxy, $-NR_mR_n$, halogenated

$-NR_mR_n$, cyano, substituted or unsubstituted $C_{1-6}$ alkyl, substituted or unsubstituted $C_{3-6}$ cycloalkyl, and $-C(O)NR_9R_{10}$;

each $R_9$ and $R_{10}$ is each independently selected from the group consisting of H, substituted or unsubstituted $C_{1-6}$ alkyl, substituted or unsubstituted $C_{3-6}$ cycloalkyl, $-C(O)R_{12}$, and $-C(O)OR_{13}$; or $R_9$ and $R_{10}$ together with N to which they are attached form a substituted or unsubstituted 5-7 membered heterocyclic ring; or either $R_9$ or $R_{10}$ together with $R_5$ form a 5-7 membered ring;

or $R_4$ and $R_5$ together with the C to which they are attached form a 5-7 membered heterocycle containing 1, 2, or 3 heteroatoms selected from N, O, or S;

$R_{12}$, and $R_{13}$ are each independently selected from the group consisting of H, substituted or unsubstituted $C_{1-6}$ alkyl, and substituted or unsubstituted $C_{3-6}$ cycloalkyl;

the "substituted" each independently refers to be substituted by 1, 2, 3 or 4 substituents selected from the group consisting of deuterium, halogen, hydroxyl, amino, $-NR_mR_n$, $C_{1-6}$ alkoxy;

each $R_m$ is selected from the group consisting of H, $C_{1-6}$ alkyl, $C_{3-6}$ cycloalkyl, $C_{6-10}$ aryl, and 6-10-membered heteroaryl containing 1, 2, or 3 heteroatoms selected from N, O, or S;

each $R_n$ is $C_{1-6}$ alkyl;

each $R_a$ is selected from the group consisting of H, $C_{1-6}$ alkyl, $C_{3-6}$ cycloalkyl;

each $R_b$ is $C_{3-6}$ cycloalkyl;

or $R_a$ and $R_b$ together with the N to which they are attached form a 3-10 membered N-containing monocyclic or bicyclic heterocyclic group;

each m, n is independently selected from the group consisting of 0, 1, 2, 3, 4, and 5;

Ring B is selected from the group consisting of

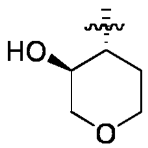 , 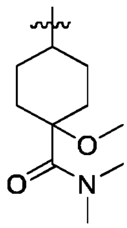 ,

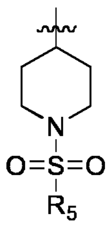 , 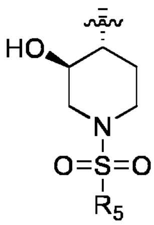 , 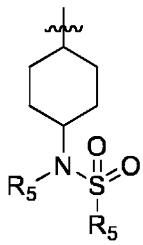 , 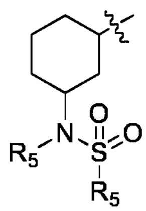 , 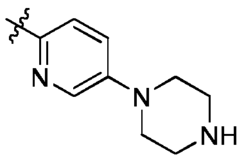 ,

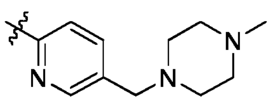 , 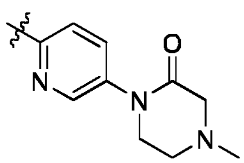 , 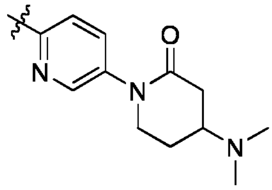 , 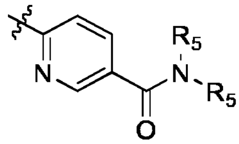 ,

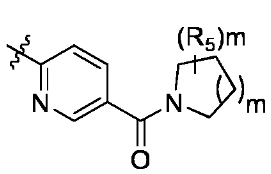 , 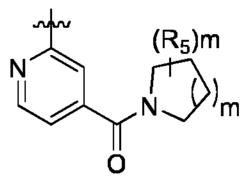 , 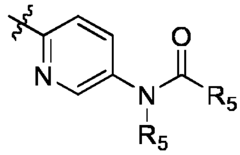 , 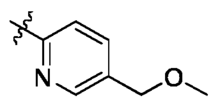 ,

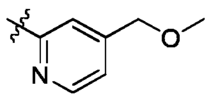, 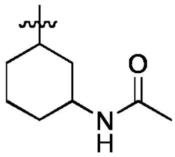, 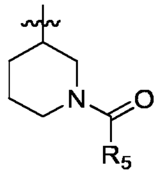, 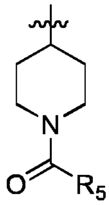, 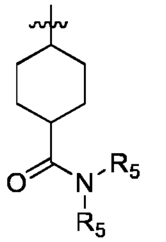,

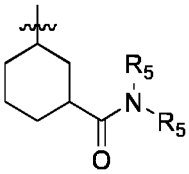, 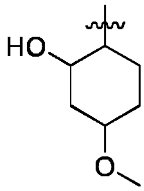, 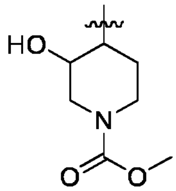, 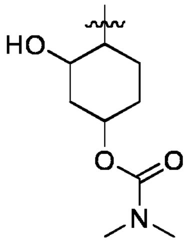, 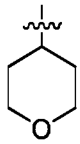, 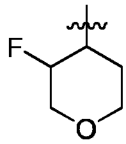,

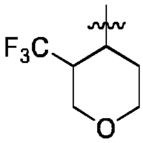, 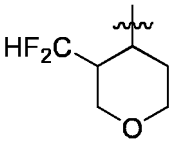, 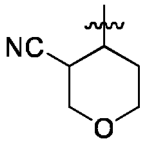, and 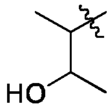;

wherein, when ring A is

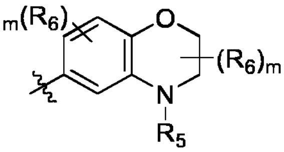,

ring B is selected from the group consisting of

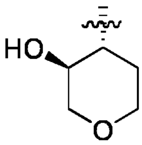, 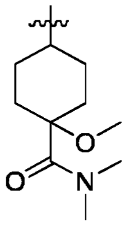, 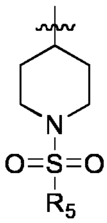, 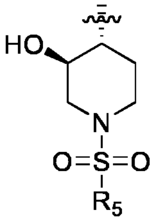, 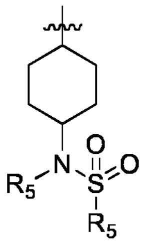,

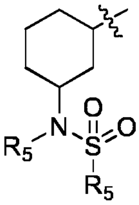, 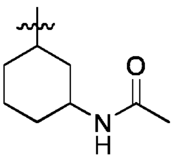, 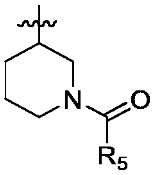, 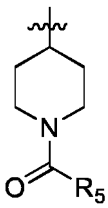, 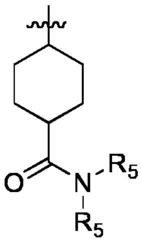, 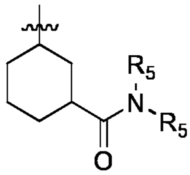,

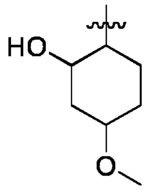, 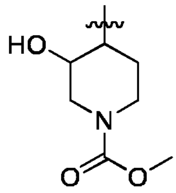, 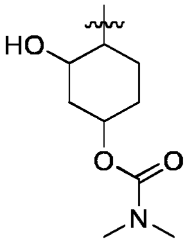, 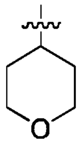, 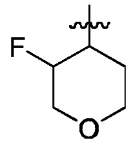, 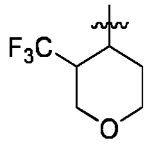,

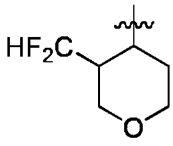, 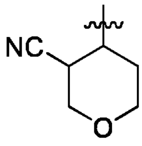, and 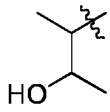.

In another preferred embodiment,

Ring A is selected from the group consisting of

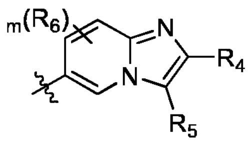,

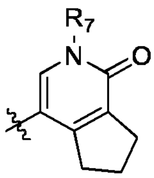, 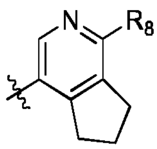, 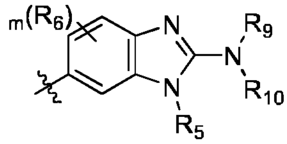, 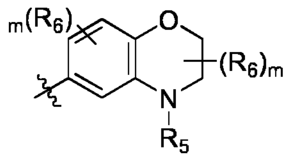,

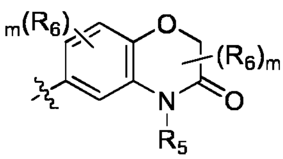, 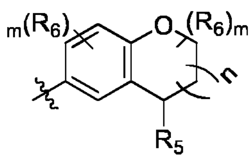, 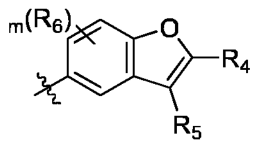, 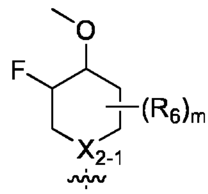,

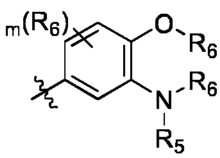, and 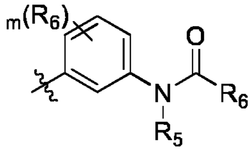;

Ring B is selected from the group consisting of

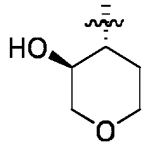, 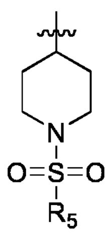,

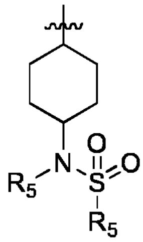 , 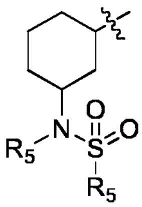 , 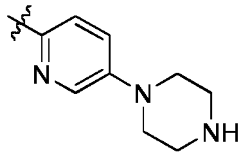 , 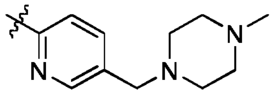 , 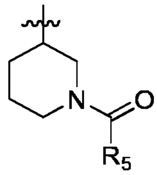 ,

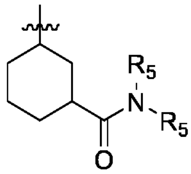 , 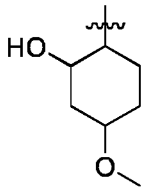 , 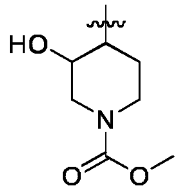 , 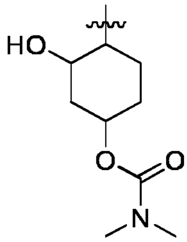 , 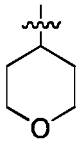 ,

and

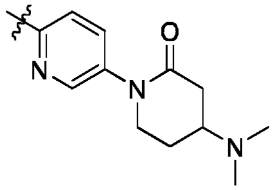 ;

wherein, $X_{2-1}$, $R_4$, $R_5$, $R_6$, $R_7$, $R_8$, $R_9$, $R_{10}$, m, and n are as defined above.

In another preferred embodiment,

Ring A is selected from the group consisting of

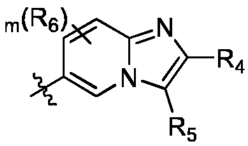 ,

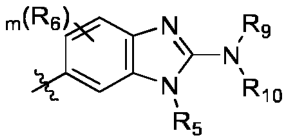 , and 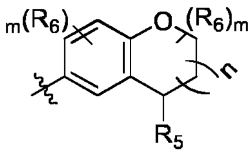 ;

Ring B is selected from the group consisting of

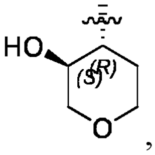 , 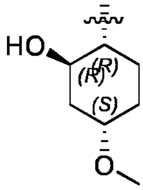 ,

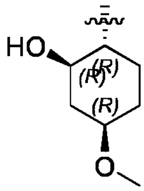, 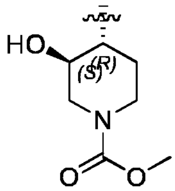, 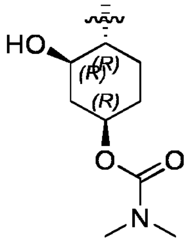, 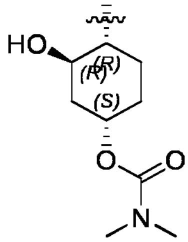, 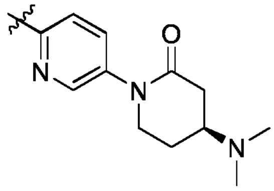,

and

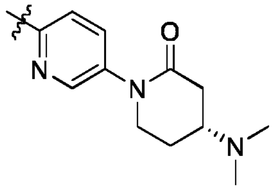;

wherein, $R_4$, $R_5$, $R_6$, $R_9$, $R_{10}$, m, and n are as defined above.

In another preferred embodiment,

Ring A is selected from the group consisting of

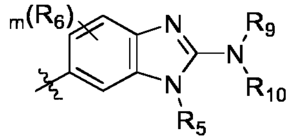

and

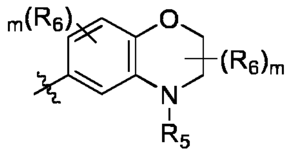;

Ring B is selected from the group consisting of

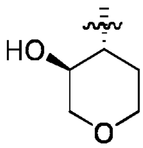 and 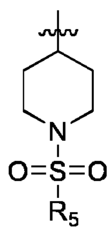;

wherein, $R_5$, $R_6$, $R_9$, $R_{10}$, and m are as defined above.

In another preferred embodiment, the compound is selected from the group consisting of:

| | | |
|---|---|---|
| 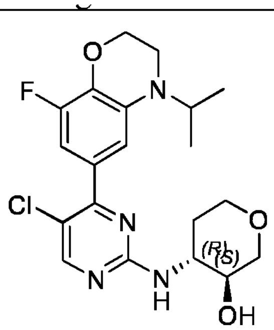 TA-1 | 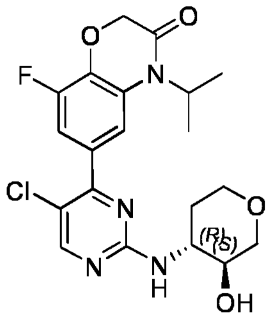 TA-2 | 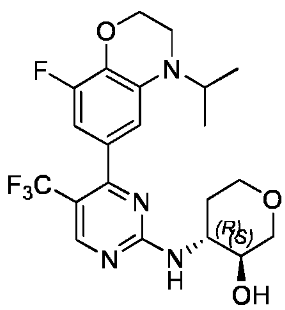 TA-3 |
| 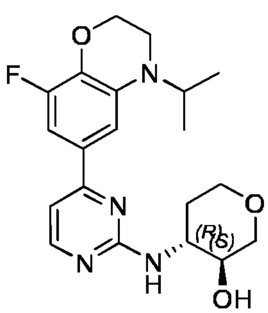 TA-4 | 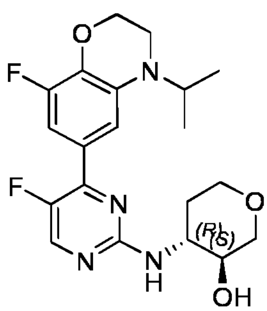 TA-5 | 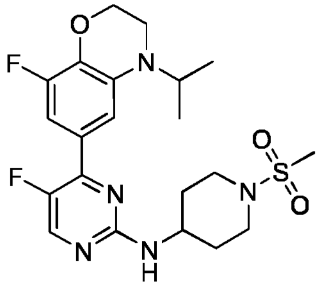 TA-6 |
| 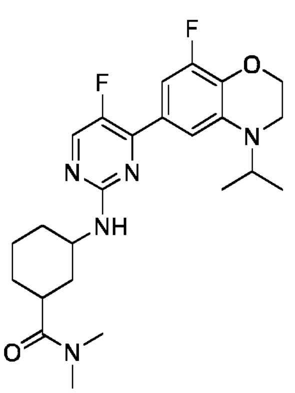 TA-7 | 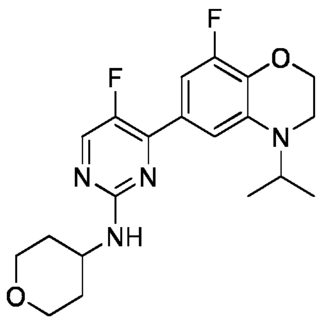 TA-8 | 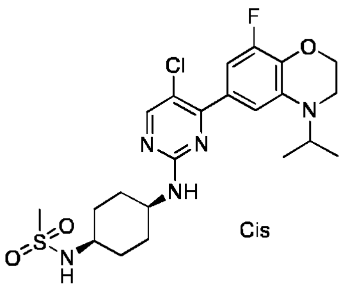 Cis TA-9 |
| 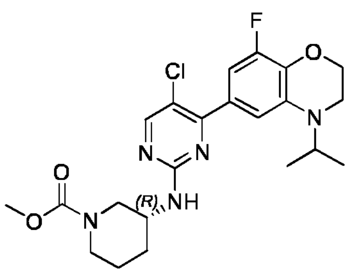 TA-10 | 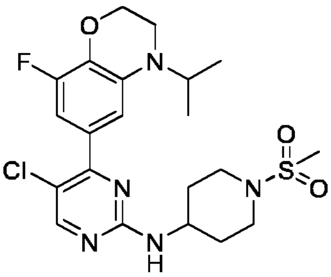 TA-11 | 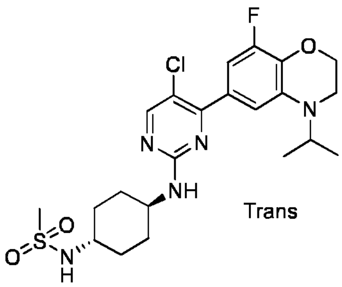 Trans TA-12 |

| | | |
|---|---|---|
| 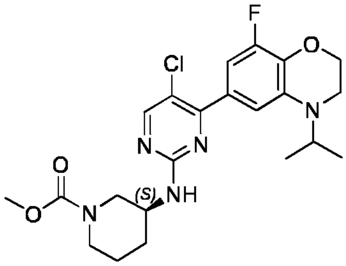 TA-13 | 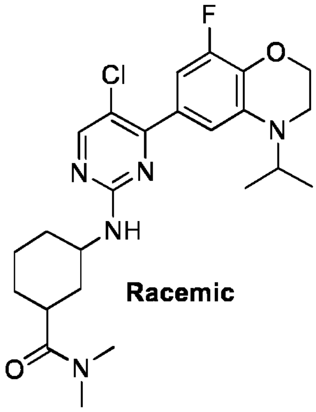 Racemic TA-14 | 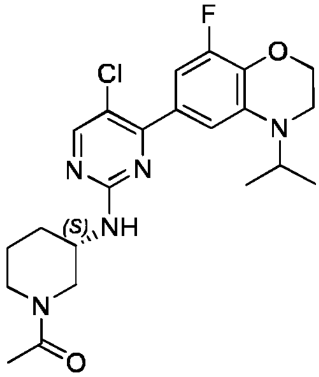 TA-15 |
| 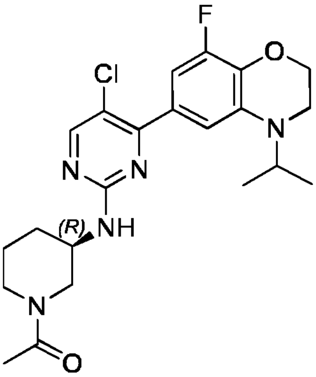 TA-16 | 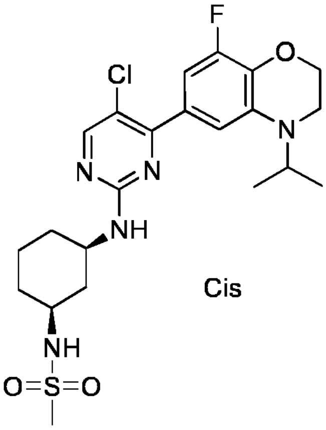 Cis TA-17 | 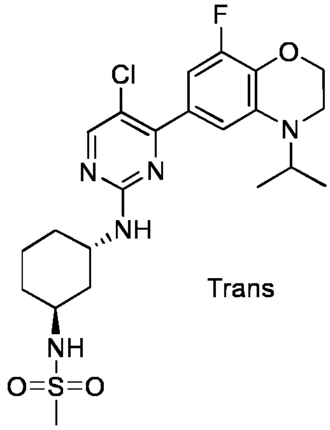 Trans TA-18 |
| 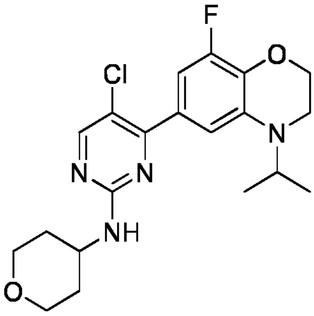 TA-19 | 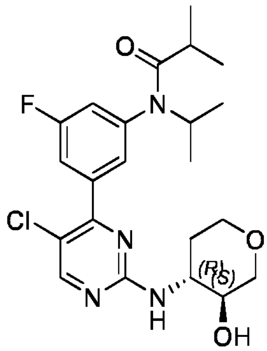 TA-20 | 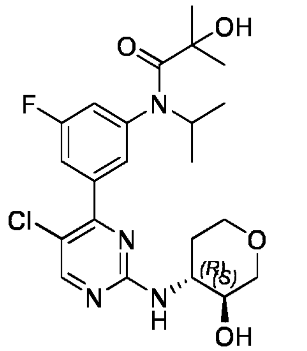 TA-21 |
| 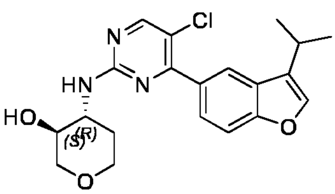 TA-22 | 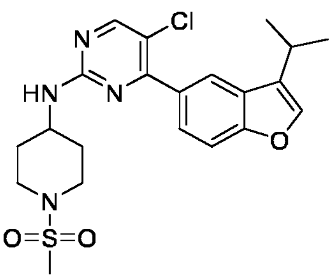 TA-23 | 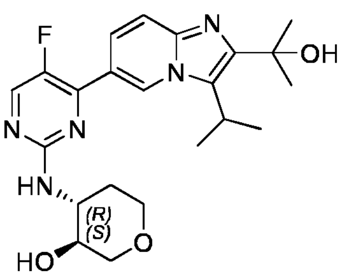 TA-24 |

| | | |
|---|---|---|
| 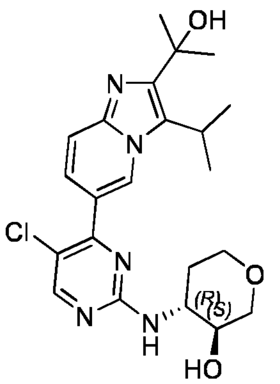 TA-25 | 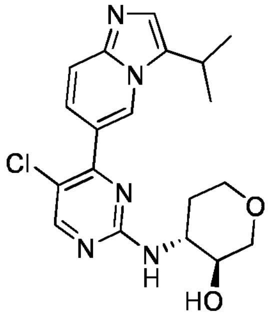 TA-26 | 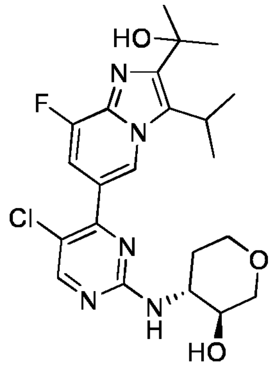 TA-27 |
| 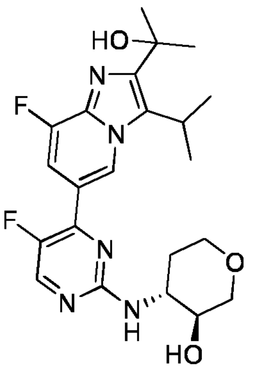 TA-28 | 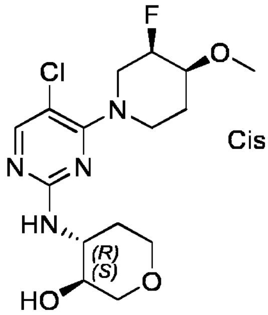 TA-30 | 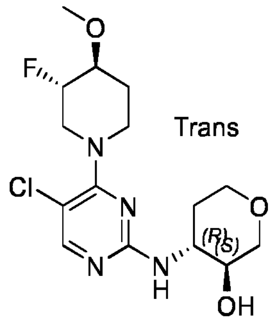 TA-31 |
| 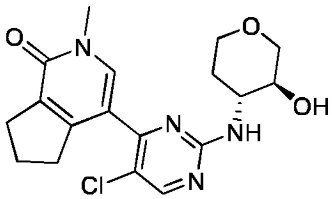 TA-32 | 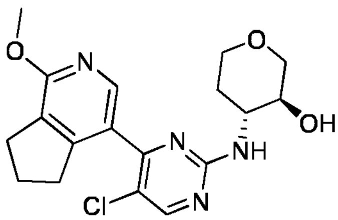 TA-33 | 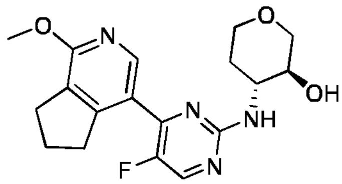 TA-34 |
| 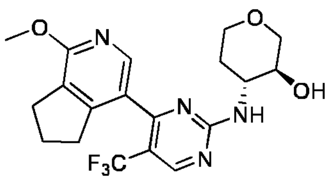 TA-35 | 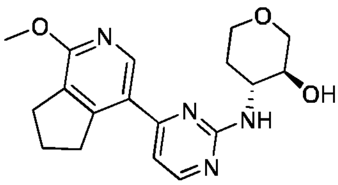 TA-36 | 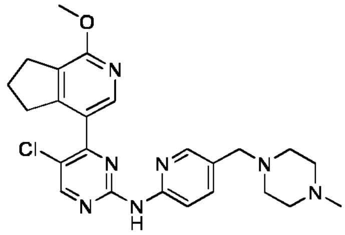 TA-37 |

| | | |
|---|---|---|
| 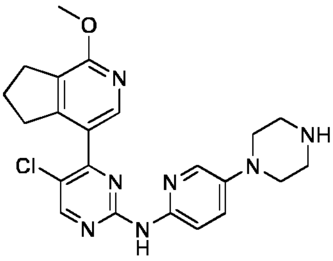<br>TA-38 | 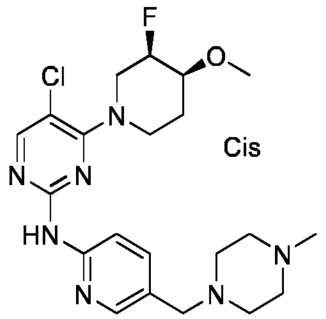<br>TA-39 | 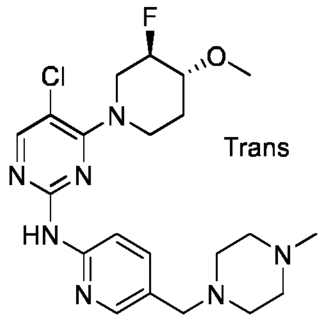<br>TA-40 |
| 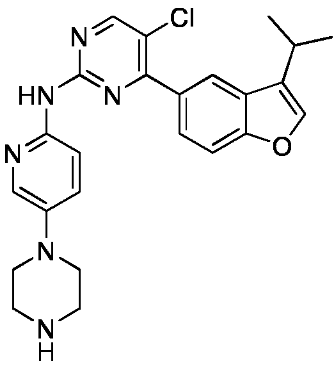<br>TA-41 | 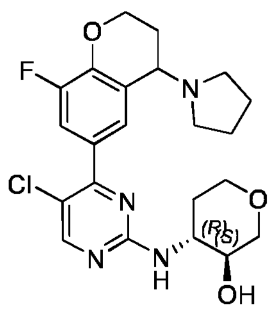<br>TA-42 | 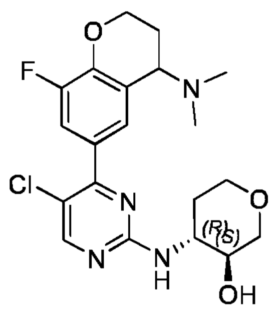<br>TA-43 |
| 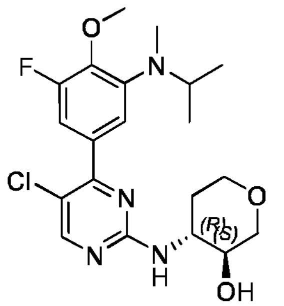<br>TA-44 | 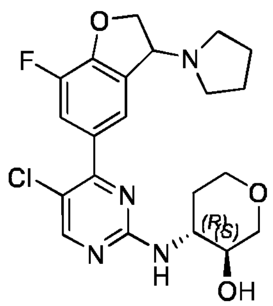<br>TA-45 | 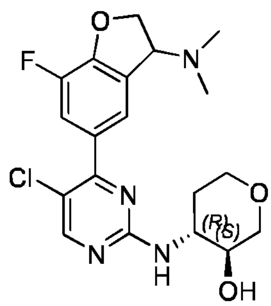<br>TA-46 |
| 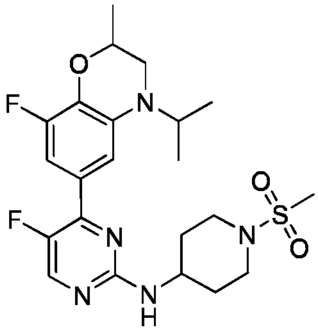<br>TA-47 | 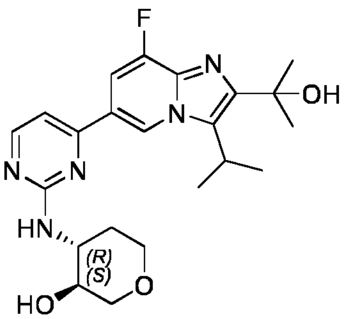<br>TA-48 | 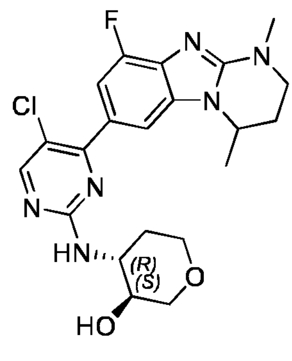<br>TA-50 |

| | | |
|---|---|---|
| 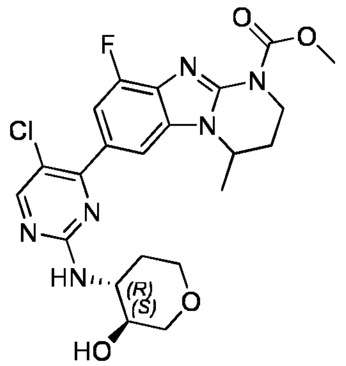 TA-52 | 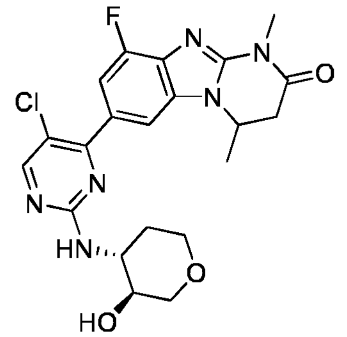 TA-53 | 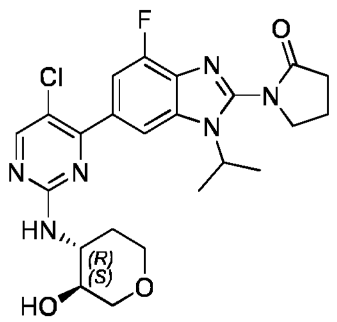 TA-54 |
| 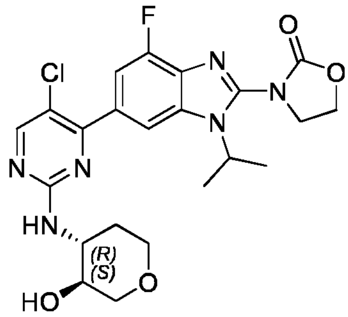 TA-55 | 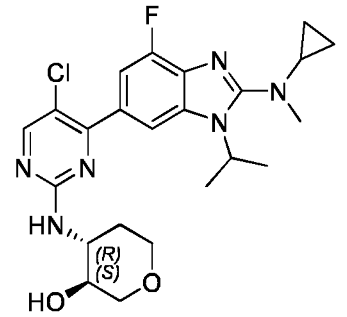 TA-56 | 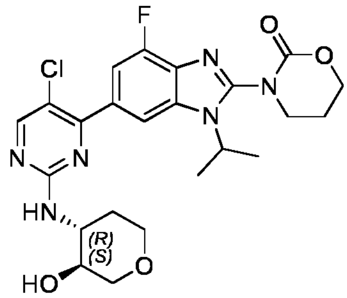 TA-57 |
| 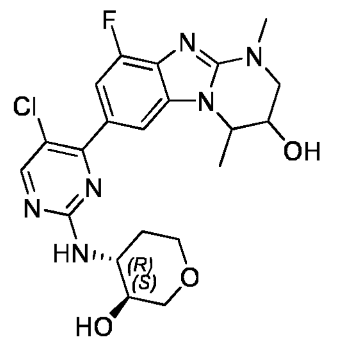 TA-58 | 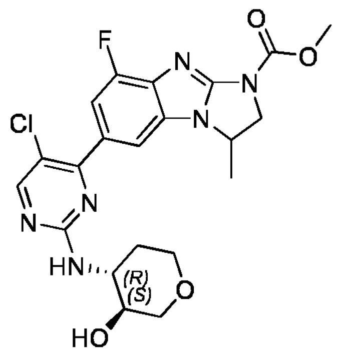 TA-59 | 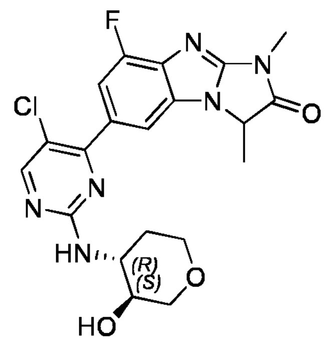 TA-60. |

In another preferred embodiment, the pharmaceutically acceptable salt is an inorganic acid salt or an organic acid salt.

In another preferred embodiment, the inorganic acid salt is selected from the group consisting of hydrochloride, hydrobromide, hydriodate, sulphate, bisulphate, nitrate, phosphate, and acid phosphate.

In another preferred embodiment, the organic acid salt is selected from the group consisting of formate, acetate, trifluoroacetate, propionate, pyruvate, hydroxyacetate, oxalate, malonate, fumarate, maleate, lactate, malate, citrate, tartrate, methane sulfonate, ethanesulfonate, benzenesulfonate, p-toluene sulfonate, salicylate, picrate, glutamate, ascorbate, Camphorates, and camphor sulfonates.

In the fifth aspect of the present invention, it provides a pharmaceutical composition comprising a safe and effective amount of the compound described in the first aspect of the present invention, and a pharmaceutically acceptable carrier.

In the sixth aspect of the present invention, it provides a use of the compound described in the first aspect of the present invention for the preparation of a drug for use as a CDK4 kinase inhibitor.

In the seventh aspect of the present invention, it provides a use of the compound described in the first aspect of the present invention for preparing a drug for use in modulating CDK4 kinase activity or for treating a CDK4-related disease.

In another preferred embodiment, the CDK4-related disease is selected from the group consisting of inflammation, cancer, cardiovascular disease, infection, immune disease, metabolic disease.
In another preferred embodiment, the cancer is selected from the group consisting of lung cancer, breast cancer, prostate cancer, colorectal cancer, liver cancer, pancreatic cancer, ovarian cancer, leukemia, neuroblastoma, gastric cancer, renal cancer, esophageal cancer, uterine cancer.
In another preferred embodiment, the inflammation is selected from the group consisting of dermatitis, keratitis, conjunctivitis, prostatitis, hepatitis, enteritis.
In another preferred embodiment, the infection is selected from the group consisting of bacterial infection and viral infection.
In another preferred embodiment, the bacterial infection is selected from the group consisting of gram-positive bacterial infection, gram-negative bacterial infection, mycoplasma infection, fungal infection.
In another preferred embodiment, the viral infection is selected from the group consisting of coronavirus infection, influenza A, influenza B, avian influenza.
In another preferred embodiment, the immune disease is selected from the group consisting of systemic lupus erythematosus, rheumatoid arthritis, scleroderma, ulcerative colitis.
It should be understood that in the present invention, any of the technical features specifically described above and below (such as in the Examples) can be combined with each other, so as to constitute new or preferred technical solutions. Due to space limitations, it will not redundantly be described one by one herein.

## DETAILED DESCRIPTION OF THE INVENTION

After long-term and in-depth research, the present inventors unexpectedly discovered a class of compounds with better CDK4 kinase inhibitory activity. In addition, the compounds have excellent inhibitory activity and selectivity for CDK4 kinase and have better pharmacodynamic/pharmacokinetic properties. On above basis, the present invention has been accomplished.

## TERMS

Unless otherwise specified, the following terms used in this application (including the specification and claims) have the definitions given below.
When a substituent is described by a conventional chemical formula written from left to right, the substituent also includes a chemically equivalent obtained substituent when the structural formula is written from right to left. For example, $-CH_2O-$ is equivalent to $-OCH_2-$.
"Alkyl (alone or as part of other groups)" refers to a monovalent linear or branched saturated hydrocarbon group containing 1 to 12 carbon atoms composed only of carbon and hydrogen atoms. The alkyl is preferably C1-C6 alkyl (i.e. containing 1, 2, 3, 4, 5 or 6 carbon atoms). Examples of alkyl include but are not limited to methyl, ethyl, propyl, isopropyl, isobutyl, sec-butyl, tert-butyl, amyl, n-hexyl, octyl and dodecyl, etc. In the present application, alkyl is also intended to comprise a substituted alkyl, i.e. one or more positions in the alkyl are substituted, in particular 1-4 substituents, which may be substituted at any position. "Haloalkyl" refers to an alkyl as defined herein in which one or more hydrogen is replaced by the same or different halogens. Examples of haloalkyl include $-CH_2Cl$, $-CH_2CF_3$, $-CH_2CCl_3$, perfluoroalkyl (e.g.$-CF_3$), etc.
"Alkylene" refers to divalent group of alkyl, e.g.$-CH_2-$, $-CH_2CH_2-$ and $-CH_2CH_2CH_2-$.
"Alkoxy (alone or as part of other groups)" refers to alkyl to which an oxy is attached with a formula alkyl O-, wherein the alkyl has the definition described above, and preferably the alkoxy is C1-C6 alkoxy. Alkoxy includes but is not limited to methoxy, ethoxy, propoxy, tert-butoxy, etc. "Haloalkoxy" refers to the formula -OR, wherein, R is a haloalkyl as defined herein. Examples of haloalkoxy include but are not limited to trifluoromethoxy, difluoromethoxy, and 2, 2, 2-trifluoroethoxy, etc.
"Thioalkyl" refers to that the carbon in the alkyl is substituted by S, S (O) or $S(O)_2$.
"Alkenyl (alone or as part of other groups)" refers to aliphatic group containing at least one double bond, typically having between 2 and 20 carbon atoms. In the present invention, "C2-C6 alkenyl" refers to alkenyl containing 2, 3, 4, 5 or 6 carbon atoms. The alkenyl includes but is not limited to for example, vinyl, propenyl, butenyl, 1-methyl-2-buten-1-yl, etc. In the present invention, the alkenyl includes substituted alkenyl.
"Alkenylene" refers to alkenyl having two connection points. For example, "vinylidene" refers to the group -CH = CH-. The alkenylene may also be in an unsubstituted form or in a substituted form having one or more substituents.
"Alkynyl (alone or as part of other groups)" refers to a straight or branched hydrocarbon chain containing more than 2 carbon atoms and characterized by one or more triple bonds, typically having 2 to 20 carbon atoms. In the present invention, "C2-6 alkynyl" refers to alkynyl having 2, 3, 4, 5 or 6 carbon atoms. Alkynyl includes but is not limited to ethynyl, propargyl and 3-hexynyl. One of the triple-bond carbons can optionally be the connection point of alkynyl substituent. In the invention, the alkynyl also includes substituted alkynyl.

"Alkynylene" refers to alkynyl having two connecting points. For example, "ethynylene" refers to the group: -C≡C-. The alkynylene may also be in an unsubstituted form or in a substituted form having one or more substituents.

"Aliphatic group" refers to straight-chain, branched or cyclic hydrocarbon group including saturated and unsaturated group, such as alkyl, alkenyl and alkynyl.

"Aromatic ring system" refers to monocyclic, bicyclic or polycyclic hydrocarbon ring system in which at least one ring is aromatic.

"Aryl (alone or as part of other groups)" refers to a monovalent group of an aromatic ring system. Representative aryl includes all aromatic ring systems, such as phenyl, naphthyl and anthryl; and ring systems in which aromatic carbocyclic rings are fused with one or more non-aromatic carbocyclic rings, such as indanyl, phthalimide, naphthylimide or tetrahydronaphthyl, etc. In the present invention, the aryl is preferably C6-C12 aryl. In the present invention, aryl is also intended to comprise substituted aryl.

"Arylalkyl" or "aralkyl" refers to an alkyl moiety in which the alkyl hydrogen atom is substituted by an aryl. An aralkyl includes a group in which one or more hydrogen atoms are substituted by aryl, wherein aryl and alkyl are defined as above. Examples of "arylalkyl" or "aralkyl" include benzyl, 2-phenylethyl, 3-phenylpropyl, 9-fluorenyl, diphenylmethyl and triphenylmethyl, etc.

"Aryloxy" refers to -O-(aryl), wherein the aryl moiety is as defined herein.

"Heteroalkyl" refers to a substituted alkyl having one or more skeleton chain atoms selected from atoms other than carbon, such as oxygen, nitrogen, sulfur, phosphorus, or a combination thereof. Numeric ranges may be given for example C1-C6 heteroalkyl refers to the number of carbons in the chain which includes 1 to 6 carbon atoms. For example, $-CH_2OCH_2CH_3$ is called "C3" heteroalkyl. The connection with the rest of the molecule can be through heteroatoms or carbons in heteroalkyl chain. "Heteroalkylene" refers to an optionally substituted divalent alkyl having one or more skeleton chain atoms selected from atoms other than carbon, such as oxygen, nitrogen, sulfur, phosphorus, or a combination thereof.

"Carbocyclic system" refers to a monocyclic, dicyclic or polycyclic hydrocarbon ring system in which each ring is fully saturated or contains one or more unsaturated units, but none of the rings are aromatic.

"Carbocyclic group" refers to a monovalent group of a carbocyclic system. These include, for example, cycloalkyl (cyclopentyl, cyclobutyl, cyclopropyl, cyclohexyl, etc.) and cycloalkenyl (e.g. cyclopentenyl, cyclohexenyl, cyclopentadienyl, etc.).

"Cycloalkyl" refers to a monovalent saturated carbocyclic group consisting of monocyclic or dicyclic ring having 3-12, preferably 3-10, more preferably 3-8 ring atoms. The cycloalkyl may optionally be substituted with one or more substituents, wherein each substituent is independently a hydroxyl, alkyl, alkoxy, halogen, haloalkyl, amino, monoalkylamino or dialkylamino. Examples of cycloalkyl include but are not limited to cyclopropyl, cyclobutyl, cyclopentyl, cyclohexyl and cycloheptyl, etc.

"Cycloalkoxy" refers to the formula -OR, wherein R is a cycloalkyl as defined herein. Examples of cycloalkyloxy include cyclopropyloxy, cyclobutyloxy, cyclopentyloxy and cyclohexyloxy, etc. "Cycloalkyl alkyl" means -(cycloalkyl)-alkyl in which cycloalkyl and alkyl are as disclosed herein. "Cycloalkyl alkyl" is bonded to the parent molecular structure by a cycloalkyl.

"Heteroaromatic ring system" refers to a monocyclic (e.g. 5 or 6 membered), dicyclic (6-12 membered), or polycyclic system in which at least one ring is both aromatic and contains at least one heteroatom (e.g. N, O, or S). And none of the other rings are heterocyclyl (as defined below). In some cases, rings that are aromatic and contain heteroatoms contain 1, 2, 3 or 4 ring heteroatoms in the ring. At least one ring is heteroaromatic, and the remaining rings may be saturated, partially unsaturated or completely unsaturated.

"Heteroaryl" refers to a monocyclic (e.g. 5 or 6 membered), dicyclic (e.g. 8-10 membered) or tricyclic group with 5 to 12 ring atoms, and it contains at least one aromatic ring containing 1, 2 or 3 cyclic heteroatoms selected from N, O or S, and the remaining ring atoms are C. It should be clear that the connection point of the heteroaryl should be located on the aromatic ring. Examples of heteroaryl include but are not limited to imidazolyl, aoxazolyl, isoxazolyl, thiazolyl, isothiazolyl, oxadiazolyl, thiadiazolyl, pyrazinyl, thienyl, furanyl, pyranyl, pyridyl, pyrrolyl, pyrazolyl, pyrimidinyl, quinolinyl, isoquinolinyl, benzofuranyl, benzothienyl, benzothiopyranyl, benzimidazolyl, benzoxazolyl, benzoxadiazolyl, benzothiazolyl, benzothiadiazolyl, benzopyranyl, indolyl, isoindolyl, triazolyl, triazinyl, quinoxalinyl, purinyl, quinazolinyl, quinazinyl, naphthyridinyl, pterridinyl, carbazolyl, azepinyl, diazepinyl and acridinyl, etc.. Heteroarylene refers to heteroaryl having two connecting points.

"Heterocyclic system" refers to monocyclic, bicyclic and polycyclic systems where at least one ring is saturated or partially unsaturated (but not aromatic) and the ring contains at least one heteroatom. The heterocyclic system can be attached to the side group of any heteroatom or carbon atom to produce a stable structure and any ring atom can be optionally substituted.

"Heterocyclyl" refers to a monovalent group of a heterocyclic system. It usually refers to stable monocyclic (such as 3-8 membered, i.e. 3 membered, 4 membered, 5 membered, 6 membered, 7 membered or 8 membered) or bicyclic (such as 5-12 membered, i.e. 5 membered, 6 membered, 7 membered, 8 membered, 9 membered, 10 membered, 11

membered or 12 membered) or polycyclic (such as 7-14 membered, i.e. 7 membered, 8 membered, 9 membered, 10 membered, 11 membered, 12 membered, 13 membered or 14 membered), including fused ring, spiro ring and/or bridge ring structure, which is saturated and partially unsaturated and contains carbon atoms and 1, 2, 3 or 4 heteroatoms independently selected from N, O or S. Representative heterocyclyl include the following ring systems, where (1) each ring is non-aromatic and at least one ring contains a heteroatom, for example, tetrahydrofuryl, tetrahydropyranyl, tetrahydrothienyl, pyrrolidinyl, pyrrolidonyl, piperidinyl, pyrrolinyl, decahydroquinolinyl, oxazolidinyl, piperazinyl, dioxanyl, dioxolanyl, diazepinyl, oxazepinyl, thiazepinyl, morpholinyl and quinuclidinyl. (2) At least one ring is non-aromatic and contains heteroatoms and at least one other ring is an aromatic carbocyclic ring, for example, 1, 2, 3, 4-tetrahydroquinolinyl, 1, 2, 3, 4-tetrahydroisoquinolinyl. And (3) at least one ring is non-aromatic and contains a heteroatom and at least one other ring is aromatic and contains a heteroatom, for example, 3, 4-dihydro-1H-pyrano [4, 3-c] pyridine and 1, 2, 3, 4-tetrahydro-2, 6-naphthyridine. Heterocyclylene refers to heterocyclyl having two connecting points. In the present invention, the heterocyclylene is preferably bicyclic, wherein one ring is a heteroaryl and connected to the other parts of the general formula through the heteroaryl. In the present invention, the heterocyclylene is preferably a 5-6-membered monocyclic heterocyclylene or an 8-10-membered bicyclic heterocyclylene.

"Heterocyclyl alkyl" refers to an alkyl substituted with a heterocyclyl, wherein the heterocyclyl and alkyl are defined as above.

"Alkylamino" refers to a group having a structure of alkyl-NR-, where R is H, or an alkyl, cycloalkyl, aryl, heteroaryl, etc. as described above.

"Cycloalkylamino" refers to the formula -NRaRb, wherein Ra is H, an alkyl as defined herein or a cycloalkyl as defined herein, and Rb is a cycloalkyl as defined herein; or Ra and Rb together with their attached N atoms form a 3-10-membered N-containing monocyclic or bicyclic heterocyclyl, such as tetrahydropyrrolyl. As used herein, C3-C8 cycloalkylamino refers to an amino containing 3-8 carbon atoms.

As used herein, an "ester" refers to -C (O)-O-R or R-C (O)-O-, wherein R independently represents hydrogen, alkyl, cycloalkyl, aryl, heteroaryl, heterocyclyl as defined above.

As used herein, the term "amido" refers to a group with a structure of - CONRR', wherein R and R' independently represent hydrogen, alkyl or substituted alkyl, cycloalkyl or substituted cycloalkyl, aryl or substituted aryl, heterocyclyl or substituted heterocyclyl with the definitions as described above. R and R' may be the same or different in the dialkylamino segments.

As used herein, the term "sulfonamido" refers to a group with a structure of $-SO_2NRR'$, wherein R and R' independently represent hydrogen, alkyl or substituted alkyl, cycloalkyl or substituted cycloalkyl, aryl or substituted aryl, heterocyclyl or substituted heterocyclyl with the definitions as described above. R and R' may be the same or different in the dialkylamino segments.

"Ketocarbonyl" refers to R-C (= O)-, wherein R is alkyl, cycloalkyl, etc. with the definitions as described above.

When the substituent is a non-terminal substituent, it is a subunit of the corresponding substituent, such as alkyl corresponding to alkylene, cycloalkyl corresponding to cycloalkylene, heterocyclyl corresponding to heterocyclylene, alkoxy corresponding to alkyleneoxy, etc..

In the present invention, each of the above-mentioned groups of alkyl, alkoxy, cycloalkyl, heteroalkyl, aryl, heteroaryl, cycloheteroalkyl, alkenyl, alkynyl, heterocycle, heterocyclyl, etc. may be substituted or unsubstituted.

As used herein, the term "substituted" refers to the substitution of one or more hydrogen atoms on a specific group by specific substituent. The specific substituents are the substituents described correspondingly in the foregoing, or the substituents appearing in the respective examples. Unless otherwise specified, a substituted group may have a substituent selected from a specific group at any substitutable position of the group, and the substituent may be the same or different at each position. Those skilled in the art should understand that the combinations of substituents contemplated by the present invention are those that are stable or chemically achievable. Typical substituents include but are not limited to one or more of the following groups: such as hydrogen, deuterium, halogen (such as monohalogenated substituent or polyhalogenated substituents, and the latter such as trifluoromethyl or alkyl containing $Cl_3$), cyano, nitro, oxo (= O), trifluoromethyl, trifluoromethoxy, cycloalkyl, alkenyl, alkynyl, heterocycle, aromatic ring, $OR_a$, $SR_a$, $S(=O)R_e$, $S(=O)_2R_e$, $P(=O)_2R_e$, $S(=O)_2OR_e$, $P(=O)_2OR_e$, $NR_bR_c$, $NR_bS(=O)_2R_e$, $NR_bP(=O)_2R_e$, $S(=O)_2NR_bR_c$, $P(=O)_2NR_bR_c$, $C(=O)OR_d$, $C(=O)R_a$, $C(=O)NR_bR_c$, $OC(=O)R_a$, $OC(=O)NR_bR_c$, $NR_bC(=O)OR_e$, $NR_4C(=O)NR_bR_c$, $NR_dS(=O)_2NR_bR_c$, $NR_dP(=O)_2NR_dR_c$, $NR_bC(=O)R_a$ or $NR_bP(=O)_2R_e$, wherein $R_a$ can independently represent hydrogen, deuterium, alkyl, cycloalkyl, alkenyl, alkynyl, heterocycle or aromatic ring, $R_b$, $R_c$ and $R_d$ can independently represent hydrogen, deuterium, alkyl, cycloalkyl, heterocycle or aromatic ring, or $R_b$ and $R_c$ together with the N atom form a heterocycle, $R_e$ can independently represent hydrogen, alkyl, cycloalkyl, alkenyl, alkynyl, heterocycle or aromatic ring. The above typical substituents, such as alkyl, cycloalkyl, alkenyl, cycloalkenyl, alkynyl, heterocyclyl or aromatic ring, may be optionally substituted. The substituent is such as (but is not limited to): halogen, hydroxyl, cyano, carboxy (-COOH), C1-C6 alkyl, C2-C6 alkenyl, C2-C6 alkynyl, C3-C8 cycloalkyl, 3-12 membered heterocyclyl, aryl, heteroaryl, C1-C8 aldehydyl, C2-C10 acyl, C2-C10 ester, amino, C1-C6 alkoxy, C1-C10 sulfonyl, and C1-C6 uramido, etc..

"Cyano" refers to -CN.

"Nitro" refers to $-NO_2$.
"Hydroxyl" refers to -OH.
"Amino" refers to $-NH_2$ or RNH-, wherein R is ketocarbonyl, sulfonyl, sulfonamido, $R_a$-C(=O)-, $R_aR_b$N-C (=O)- and so on, wherein $R_a$ and $R_b$ are alkyl, cycloalkyl, aryl or heteroaryl, etc.
"Halogen (halogenated)" refers to any halogen group, e.g. -F, -Cl, -Br or -I.
"Deuterated compound" refers to the compound obtained by replacing one hydrogen atom (H) or multiple hydrogen atoms (H) with deuterium atoms (D) in a compound.
In the present invention, the term "more" independently refers to 2, 3, 4, or 5.

## ACTIVE INGREDIENT

As used herein, the terms "compound of the invention" or "active ingredient of the invention" are used interchangeably and refer to a compound of Formula I, or a pharmaceutically acceptable salt, hydrate, solvate, isotopic compound (e.g., deuterated compound) or prodrug thereof. The term also includes racemates, optical isomers.
The Formula I compound has the following structure:

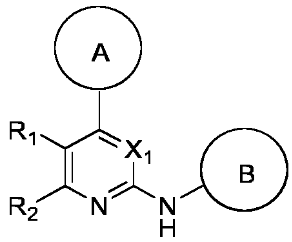
Formula I

wherein each group is defined as described above.
The salts that may be formed by the compound in the present invention are also within the scope of the present invention. Unless otherwise stated, the compound in the present invention is understood to include its salt. The term "salt" as used herein refers to a salt formed in the form of acid or base from inorganic or organic acid and base. Further, when the compound in the present invention contains a base fragment which includes, but is not limited to pyridine or imidazole, when it contains an acid segment which includes, but is not limited to carboxylic acid. The zwitter-ion that may form "inner salt" is included within the range of the term "salt". Pharmaceutically acceptable (i.e., non-toxic, physiologically acceptable)salt is preferred, although other salts are also useful and may be used, for example, in the separation or purification steps of the preparation process. The compound of the present invention may form a salt, for example, compound I is reacted with a certain amount (such as an equivalent amount) of an acid or base, and precipitated in a medium, or freeze-dried in aqueous solution.
The base fragment contained in the compounds of the present invention includes but is not limited to amines or pyridine or imidazole rings, and may form salt with organic or inorganic acid. Typical acids that form salts include acetate (such as acetic acid or trihalogenated acetic acid, such as trifluoroacetic acid), adipate, alginate, ascorbate, aspartate, benzoate, benzene sulfonate, disulfate, borate, butyrate, citrate, camphorate, camphor sulfonate, cyclopentane propionate, diethylene glycolate, lauryl sulfate, ethane sulphonate, fumarate, gluceptate, glycerophosphate, hemisulphate, enanthate, caproate, hydrochloride, hydrobromide, hydriodate, isethionate(e.g., 2-hydroxyl-ethesulfonate), lactate, maleate, mesylate, naphthalenesulfonate (e.g., 2-naphthalenesulfonate), nicotinate, nitrate, oxalate, pectate, persulfate, phenylpropionate (e.g., 3-phenylpropionate), phosphate, picrate, pivalate, propionate, salicylate, succinate, sulfate (e.g., formed with sulfuric acid), sulfonate, tartrate, thiocyanate, toluenesulfonate(e.g., tosilate), dodecanoate, etc..
Some compounds of the invention may contain acidic fragments including, but not limited to carboxylic acid which may form salts with various organic or inorganic bases. Salt formed by typical base includes ammonium salt, alkali metal salt (such as sodium, lithium and potassium salts), alkaline earth metal salt (such as calcium and magnesium salts), and salt formed by organic bases (such as organic amines), such as benzathine, dicyclohexylamine, hydrabamine (salt formed with *N,N*- bis (dehydroabietyl) ethylenediamine), N-methyl-D-glucosamine, *N*-methyl-D-glucoamide, tert-butyllamine, and the salt formed with amino acids such as arginine, lysine, etc.. Basic nitrogen-containing groups can form quaternary ammonium salts with halides, such as small molecular alkyl halides (such as chlorides, bromides and iodides of methyl, ethyl, propyl and butyl), dialkyl sulfate (such as dimethyl, diethyl, dibutyl, and dipentyl sulfates), long chain halides (such as chlorides, bromides and iodides of decyl, dodecyl, tetradecyl, and tetradecyl), aralkyl halides (such as bromides of benzyl and phenyl), etc..
The prodrug and solvate of the compound in the present invention are also included within the scope of the present invention.
The term "prodrug" herein refers to a compound, which produces a compound, salt, or solvate in the present

invention resulting from the chemical transformation of a metabolic or chemical process when used in the treatment of an associated disease. The compounds of the invention include solvates such as hydrates.

Compound, salt or solvate in the present invention, may be present in tautomeric forms such as amide and imino ether. All of these tautomers are part of the present invention.

Stereisomers of all compounds (e.g., those asymmetric carbon atoms that may be present due to various substitutions), including their enantiomeric forms and non-enantiomed forms, all belong to the protection scope of the present invention. The independent stereoisomer in the present invention may not coexist with other isomers (e.g., as a pure or substantially pure optical isomer with special activity), or may be a mixture (e.g., racemate), or a mixture formed with all other stereoisomers or a part thereof. The chiral center of the present invention has two configurations of S or R, which is defined by International Union of Pure and Applied Chemistry (IUPAC) in 1974. The racemization form can be solved by physical methods, such as fractional crystallization, or separation crystallization by derivation into diastereomers, or separation by chiral column chromatography. Individual optical isomer can be obtained from racemate by appropriate methods, including but not limited to conventional methods, such as recrystallization after salting with optically active acids.

Weight content of compound in the present invention obtained by preparation, separation and purification in turn is equal to or greater than 90%, such as equal to or greater than 95%, equal to or greater than 99% ("very pure" compound), which is listed in the description of the text. In addition, the "very pure" compound of the present invention is also part of the present invention.

All configuration isomers of the compound of the present invention are within the scope, whether in mixture, pure or very pure form. The definition of the compound of the present invention comprises cis (Z) and trans (E) olefin isomers, and cis and trans isomers of carbocycle and heterocycle.

In the entire specification, the groups and substituents can be selected to provide stable fragments and compounds.

Specific functional groups and chemical term definitions are described in detail below. For the purposes of the present invention, the chemical elements are consistent with Periodic Table of the Elements, CAS version, Handbook of Chemistry and Physics, 75th Ed. The definition of a particular functional group is also described therein. In addition, the basic principles of Organic Chemistry as well as specific functional groups and reactivity are described in "Organic Chemistry", Thomas Sorrell, University Science Books, Sausalito: 1999, the entire content of which is incorporated herein by reference.

Some compounds of the present invention may exist in specific geometric or stereoisomer forms. The present invention covers all compounds, including their cis and trans isomers, R and S enantiomers, diastereomers, (D) type isomers, (L) type isomers, racemic mixtures and other mixtures. In addition, asymmetric carbon atom can represent substituent, such as alkyl. All isomers and mixtures thereof are included in the present invention.

According to the invention, mixtures of isomers may contain a variety of ratios of isomers. For example, mixtures with only two isomers may have the following combinations: 50:50, 60:40, 70:30, 80:20, 90:10, 95:5, 96:4, 97:3, 98:2, 99:1, or 100:0, all ratios of the isomers are within the scope of the present invention. Similar ratios readily understood by those of ordinary skill in the art and ratios for mixtures of more complex isomers are also within the scope of the present invention.

The invention also includes isotope labeled compounds, which are equivalent to the original compounds disclosed herein. However, in practice, it usually occurs when one or more atoms are replaced by atoms with a different atomic weight or mass number. Examples of compound isotopes that may be listed in the present invention include hydrogen, carbon, nitrogen, oxygen, phosphorus, sulfur, fluorine and chlorine isotopes, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$ respectively. Compound, or enantiomer, diastereomer, isomer, or pharmaceutically acceptable salt or solvate, the above compound containing isotopes or other isotope atoms are all within the scope of the invention. Some isotope-labeled compounds in the present invention, such as the radioactive isotopes of $^{3}H$ and $^{14}C$, are also included and are useful in experiments on the tissue distribution of drugs and substrates. Tritium ($^{3}H$) and Carbon-14 ($^{14}C$), which are relatively easy to prepare and detect, are the preferred choice. In addition, heavier isotope substitutions such as deuterium, i.e. $^{2}H$, have advantages in certain therapies due to their good metabolic stability, such as increased half-life or reduced dosage in vivo, and thus may be preferred in certain situations. Isotope-labeled compounds can be prepared by conventional methods through replacing non-isotopic reagents with readily available isotope-labeled reagents using the disclosed scheme shown in the Example.

If the synthesis of a specific enantiomer of the compound of the invention is to be designed, it can be prepared by asymmetric synthesis, or derivatized with chiral auxiliary reagent, separating the resulting diastereomeric mixture and removing the chiral auxiliary reagent to obtain a pure enantiomer. In addition, if a molecule contains a basic functional group, such as an amino acid, or an acidic functional group, such as a carboxyl group, a diastereomer salt can be formed with a suitable optically active acids or bases, and it can be separated by conventional means, such as crystallization or chromatography, to obtain a pure enantiomer.

As described herein, the compound in the present invention may be substituted with any number of substituents or functional groups to extend its scope. In general, whether the term "substituted" appears before or after the term

"optional", the general formula that includes substituents in the compound of the present invention means the substitution of a specified structural substituent for a hydrogen radical. When multiple locations in a particular structure are replaced by multiple specific substituents, each location of the substituents can be the same or different. The term "substituted" as used herein includes all substitution that allows organic compounds to be substituted. Broadly speaking, the allowable substituents include non-cyclic, cyclic, branched, non-branched, carbocyclic and heterocyclic, aromatic ring and non-aromatic organic compounds. In the present invention, such as heteroatomic nitrogen, its valence state may be supplemented by a hydrogen substituent or by any permitted organic compound described above. Furthermore, the invention does not unintentionally limit the substituted organic compounds in any way. The present invention considers that a combination of substituents and variable groups is good for the treatment of diseases in the form of stable compounds. The term "stable" herein refers to a stable compound which is sufficient for maintaining the integrity of the compound structure within a sufficiently long time, preferably being effective in a sufficiently long time, which is hereby used for the above purposes.

The metabolites of the compounds of the present application and their pharmaceutically acceptable salts, and prodrugs that can be converted into the compounds of the present application and their pharmaceutically acceptable salts thereof in vivo, are also included in the claims.

In another preferred embodiment, in the compound, any one of ring A, ring B, $R_1$, $R_2$, $X_1$ is a corresponding group in the specific compound, respectively.

**Preparation Method**

Methods for preparing compounds of formula I are described in the following schemes and examples. Raw materials and intermediates are purchased from commercial sources, prepared by known steps, or otherwise described. In some cases, the sequence of steps to perform the reaction scheme may be changed to facilitate the reaction or avoid unwanted side reaction products.

The preparation method of the compound of formula I of the present invention is more specifically described below, but these specific methods do not constitute any limitation of the invention. The compound of the invention may also optionally be conveniently prepared by combining the various synthetic methods described in this specification or known in the art, such a combination may be easily performed by a skilled person in the art to which the invention belongs.

Generally, in the preparation process, each reaction is usually carried out under the protection of inert gas and in an appropriate solvent at 0 to 150 °C, and the reaction time is usually 2-24 hours.

Preferably, the preparation method is as follows:

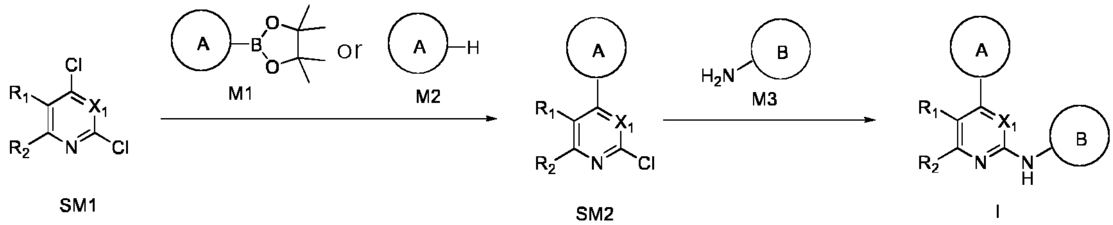

Step 1: In solvents (e.g., 1,2-dichloroethane, dioxane, tetrahydrofuran), SM1 reacts with M1 or M2 to form SM2 at 70-120 °C by palladium-catalyzed coupling or nucleophilic substitution reactions;

Step 2: In an inert solvent (e.g., DMF, dioxane, ethylene glycol dimethyl ether, N-methylpyrrolidone, tetrahydrofuran, etc.), under basic (e.g., diisopropylethylamine, potassium acetate, DBU, LiHDMS, etc.) conditions or in the presence of catalysts and ligands (e.g., $Pd(PPh_3)_4$, $Pd_2(dba)_3$\t-BuXphos, etc.), M3 reacts with SM2 to form T (i.e., the compound of formula I).

In each of the above formulas, ring A, ring B, $R_1$, $R_2$, and $X_1$ are defined as described above.

If not otherwise indicated, the above starting materials are commercially available or synthesized in accordance with the reported literature.

**Pharmaceutical Compositions and Administration Methods**

The pharmaceutical compositions of the present invention are used to prevent and / or treat the following diseases: inflammation, cancer, cardiovascular disease, infection, immune disease, metabolic disease.

The compounds of formula I can be used in combination with other drugs known to treat or improve similar conditions. When administered in combination, the original administration method and dosage for the drug can remain

unchanged, while the compound of formula I may be administered simultaneously or subsequently. Pharmaceutical composition simultaneously containing one or more known drugs and the compound of formula I may be preferred when administered in combination with one or more other drugs. The drug combination also includes administering the compound of formula I and other one or more known drugs at overlapping time. When the compound of formula I is combined with other one or more drugs, the dose of the compound of formula I or known drug may be lower than that of their individual use.

The drug or active ingredients that can be used in pharmaceutical use with the compounds of formula I include but are not limited to PD-1 inhibitor (such as nivolumab, pembrolizumab, JS-001, SHR-120, BGB-A317, IBI-308, GLS-010, GB-226, STW204, HX008, HLX10, BAT 1306, AK105, LZM 009 or the biological analogue thereof, etc.), PD-L1 inhibitor (such as durvalumab, atezolizumab, CS1001, KN035, HLX20, SHR-1316, BGB-A333, JS003, CS1003, KL-A167, F 520 , GR1405, MSB2311 or the biological analogue thereof, etc.), CD20 antibody (such as rituximab, obinutuzumab, ofatumumab, tositumomab, ibritumomab, etc.), CD47 antibody (such as Hu5F9-G4, CC-90002, TTI-621, TTI-622, OSE-172, SRF-231, AlX-148, NI-1701, SHR-1603, IBI188, IMM01), ALK inhibitor (such as Ceritinib, Alectinib, Brigatinib, Lorlatinib, Ocatinib), PI3K inhibitor (such as Idelalisib, Dactolisib, Taselisib, Buparlisib, etc.), BTK inhibitor (such as Ibrutinib, Tirabrutinib, Acalabrutinib, etc.), EGFR inhibitor (such as Afatinib, Gefitinib, Erlotinib , Lapatinib, Dacomitinib, Icotinib, Canertinib, etc.), VEGFR inhibitor (such as Sorafenib, Pazopanib, Regorafenib, Cabozantinib, Sunitinib, Donafenib, etc.), HDAC inhibitor (such as Givinostat, Droxinostat, Entinostat, Dacinostat, Tacedinaline, etc.), MEK inhibitor (such as Selumetinib (AZD6244), Trametinib (GSK1120212), PD0325901, U0126, AS-703026, PD184352 (CI-1040) , etc.), mTOR inhibitor (such as Vistusertib, etc.), SHP2 inhibitor (such as RMC-4630, JAB-3068, TNO 155, etc.), IGF-1R inhibitor (such as Ceritinib, Okatinib, Linsitinib, BMS-754807, GSK1838705A, etc.), ER antagonist or degradant (such as tamoxifen, fulvestrant, etc.), aromatase inhibitor (such as letrozole, etc.), BCL2 or BCL-XL inhibitor (such as ABT-199, ABT-263, etc.), Hedgehog inhibitor (such as vismodegib, cyclopamine, etc.), chemotherapy drug (such as cisplatin, etoposide, topotecan, etc.), PARP inhibitor (such as Olaparib, Veliparib, Rucaparib, etc.), ATR/ATM inhibitor (such as Ceralasertib, Berzosertib, etc.) or combinations thereof.

The dosage forms of the pharmaceutical composition of the present invention include (but are not limited to) : injection, tablet, capsule, aerosol, suppository, pellicle, dripping pill, liniment for external use, controlled release or sustained-release or nano formulation.

The pharmaceutical composition of the present invention comprises a safe and effective amount of a compound of the present invention or a pharmacologically acceptable salt thereof, and a pharmacologically acceptable excipient or carrier. In which, "safe and effective amount" is meant that the amount of the compound is sufficient to significantly improve the condition without causing serious side effects. Generally, the pharmaceutical composition contains 1-2000 mg of the compound of the present invention/dose, more preferably, 10-1000 mg of the compound of the present invention/dose. Preferably, the "dose" is a capsule or tablet.

"Pharmaceutically acceptable carrier" means one or more compatible solid or liquid fillers or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc.

The administration mode of the compound or pharmaceutical composition of the present invention is not particularly limited, and representative administration modes include, but are not limited to, oral, intratumoral, rectal, parenteral (intravenous, intramuscular or subcutaneous) and topical administration.

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxylmethyl cellulose, alginate, gelatin, polyvinylpyrrolidone, sucrose and arabic gum; (c) humectant, such as, glycerol; (d) disintegrating agent, such as agar, calcium carbonate, potato starch or tapioca starch, alginic acid, certain composite silicates, and sodium carbonate; (e) dissolution-retarding agents, such as paraffin; (f) absorption accelerators, such as quaternary ammonium compounds; (g) wetting agents, such as cetyl alcohol and glyceryl monostearate; (h) adsorbents, such as kaolin; and (i) lubricants such as talc, stearin calcium, magnesium stearate, solid polyethylene glycol, lauryl sodium sulfate, or the mixtures thereof. In capsules, tablets and pills, the dosage forms may also contain buffering agents.

Solid dosage forms such as tablets, dragees, capsules, pills and granules can be prepared with coatings and shells such as enteric coatings and other materials known in the art. They may contain opacifying agents and the release of the active compound or compound in such compositions may be released in a portion of the digestive tract in a delayed

manner. Examples of embedding components that can be employed are polymeric materials and waxy materials. If necessary, the active compound may also be in microencapsulated form with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the compositions may contain adjuvants such as wetting agents, emulsifying and suspending agents, sweetening agents, flavoring agents and spices.

In addition to the active compound, the suspension may contain suspending agent, such as ethoxylated isooctadecanol, polyoxyethylene sorbitol and dehydrated sorbitan ester, microcrystalline cellulose, aluminum methoxide and agar, or the mixture thereof etc..

The compositions for parenteral injection may comprise physiologically acceptable sterile aqueous or anhydrous solutions, dispersions, suspensions or emulsions and sterile powders which can be re-dissolved into sterile injectable solutions or dispersions. Suitable aqueous and non-aqueous carriers, diluents, solvents or excipients include water, ethanol, polyols and any suitable mixtures thereof.

Dosage forms for the compounds of the invention for topical administration include ointments, powders, patches, propellants and inhalants. The active ingredient is mixed under sterile conditions with a physiologically acceptable carrier and any preservatives, buffers, or propellants which may be required if necessary.

The treatment method of the present invention can be administered alone or in combination with other treatment means or therapeutic drugs.

When the pharmaceutical composition is used, a safe and effective amount of the compound of the present invention is administered to a mammal (such as a human) in need of treatment, wherein the dosage at the time of administration is the pharmaceutically effective dosage, for people having a body weight of 60kg, the daily dose is usually 1~2000mg, preferably 50~ 1000mg. Of course, specific doses should also consider factors such as the administration route, the health of the patient, etc., which are within the skill of the skilled physician.

The present invention also provides a preparation method of pharmaceutical composition comprising the step of mixing a pharmaceutically acceptable carrier with the compound of formula I or crystalline form, pharmacically acceptable salt, hydrate or solvate thereof of the present invention, thus forming the pharmaceutical composition.

The invention also provides a treatment method comprising the step of administering the compound of formula I, or its crystalline form, pharmaceutically acceptable salt, hydrate or solvate thereof, or the pharmaceutical composition of the invention to a subject in need thereof to inhibit CDK4.

Compared with the prior art, the present invention has the following main advantages:

(1) The compounds of the present invention have excellent inhibitory ability and selectivity for CDK4 kinase;
(2) The compounds of the present invention have lower toxic side effects;
(3) The compounds of the present invention have better pharmacodynamic and pharmacokinetic properties.

The present invention will be further illustrated below with reference to the specific examples. It should be understood that these examples are only to illustrate the invention but not to limit the scope of the invention. Experimental methods in which the specific conditions are not specified in the following examples are usually in accordance with conventional conditions such as the conditions described in Sambrook et al., Molecular Cloning: Laboratory Manual (New York: Cold Spring Harbor Laboratory Press, 1989), or in accordance with the conditions recommended by the manufacturer. Unless indicated otherwise, percentage and parts are calculated by weight.

Unless otherwise defined, all professional and scientific terminology used in the text have the same meanings as known to the skilled in the art. In addition, any methods and materials similar or equal with the recorded content can apply to the methods of the invention. The preferred embodiments and the materials described herein are only for demonstration purposes.

**Example A1**

Synthesis of the compound TA-1 of the present invention:

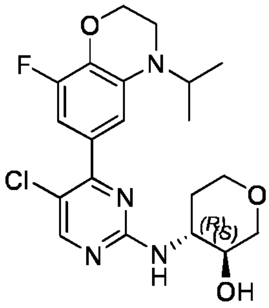

The synthesis route is as follows:

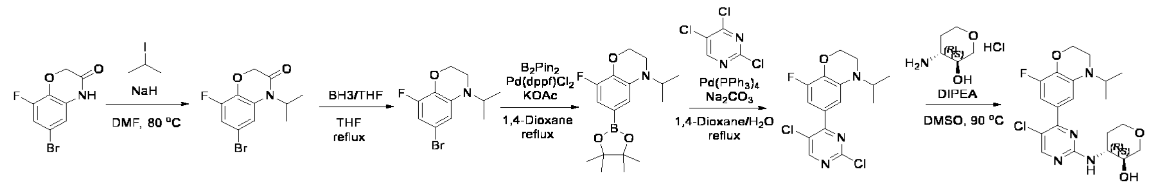


SM 1 SM 2 SM 3 SM 4 SM 5 TA- 1

The experimental procedure is as follows:

Step 1:

SM 1 (200 mg, 1.0 e.q.) was added to a 25 mL three-necked flask under nitrogen, solubilized in 5 mL of ultra-dry DMF, placed in an ice-water bath, NaH (32.5 mg, 2.0 e.q.) was added to the three-necked flask and after stirring for half an hour 2-iodopropane (138.2 mg, 2.0 e.q.) was added, and the reaction was subsequently heated up to 80 °C. TLC showed that the reaction was completed, cooled to room temperature, extracted and partitioned with saturated NaCl solution and EA, and the organic phase was again extracted and partitioned with water, the organic phase was collected, dried over anhydrous sodium sulfate, and concentrated, then separated and purified by silica gel column chromatography to obtain 100 mg of the compound SM 2 in a yield of 42.9%.
$^1$H NMR (400 MHz, Chloroform-d) $\delta$ 7.05 (t, $J$ = 4.0 Hz, 1H), 7.01 (dd, $J$ = 8.0, 4.0 Hz, 1H), 4.62 (p, $J$ = 8.0 Hz, 1H), 4.54 (s, 2H), 1.55 (d, $J$ = 8.0 Hz, 6H).

Step 2:

SM 2 (100 mg, 1.0 e.q.) was added to a 10 mL reaction branch pipe under nitrogen, solubilized in ultra-dry THF, and borane tetrahydrofuran solution (1.56 ml, 5.0 e.q.) was added, and the reaction was refluxed by heating up to 70 °C. After TLC showed that the reaction was completed, the reaction solution was cooled to room temperature and quenched with an appropriate amount of ice water. The reaction system was extracted and partitioned with saturated $NH_4Cl$ solution and EA, the organic phase was dried over anhydrous sodium sulfate, concentrated and purified by silica gel column chromatography to obtain 82 mg of compound SM 3 in 86.3% yield.
$^1$H NMR (400 MHz, Chloroform-d) $\delta$ 6.64 (s, $J$ = 4.0 Hz, 1H), 6.58 (dd, $J$ = 8.0, 4.0 Hz, 1H), 4.27 - 4.21 (m, 2H), 3.99 (m, 1H), 3.25 (t, $J$ = 4.0 Hz, 2H), 1.18 (d, $J$ = 8.0 Hz, 6H).

Step 3:

SM 3 (82 mg, 1.0 e.q.), $B_2Pin_2$ (98.7 mg, 1.3 e.q.), $Pd(dppf)Cl_2$ (10.9 mg, 0.05 e.q.), and KOAc (88.0 mg, 3.0 e.q.) were sequentially added to a 10 mL reaction branch pipe under nitrogen, 1,4-dioxane was added, nitrogen was displaced and warmed up to reflux reaction. At the end of the reaction, the reaction solution was cooled to room temperature. After being extracted and partitioned with ethyl acetate and water, the organic phase was dried over anhydrous sodium sulfate, concentrated and purified by silica gel column chromatography to give 50 mg of compound SM 4 in 52.0% yield, LCMS: $[M + H]^+$ = 322.4.

Step 4:

Compound SM 4 (265 mg, 1.0 e.q.), 2,4,5-trichloropyrimidine (196.7 mg, 1.3 e.q.), $Pd(PPh_3)_4$ (95.0 mg, 0.1 e.q.),

and sodium carbonate (262.4 mg, 3.0 e.q.) were sequentially added to a 25 mL three-necked flask, followed by the addition of 12 mL of 1,4-dioxane/$H_2O$ mixed solvent (v:v = 3:1), nitrogen was displaced for three times and then heated up to reflux. At the end of the reaction, the reaction solution was cooled to room temperature, extracted and partitioned with EA and water, and the organic phase was dried over anhydrous sodium sulfate, concentrated and purified by silica gel column chromatography to obtain 130 mg of the compound SM 5, LCMS: [ M + H ]$^+$ = 342.1, yield 46%.

Step 5:

Compound SM 5 (160 mg, 1.0 e.q.), (3s, 4r)-4-aminooxan-3-ol hydrochloride (93.4 mg, 1.3 e.q.) and DIPEA (211.6 mg, 3.5 e.q.) were sequentially added to 3.0 mL of DMSO solvent, and heated up to 90 °C under nitrogen protection. At the end of the reaction, the reaction solution was cooled down to room temperature, extracted and partitioned with saturated NaCl solution, water and EA sequentially, and the organic phase was dried over anhydrous sodium sulfate, concentrated and then separated and purified by silica gel column chromatography to obtain 75 mg of the target compound TA-1 in 38% yield and 99.4% purity by HPLC, LCMS: [ M + H ]$^+$ = 423.2.

$^1$H NMR (400 MHz, Chloroform-d) $\delta$ 8.26 (s, 1H), 7.00 (d, *J* = 4.0 Hz, 1H), 6.97 (dd, *J* = 12.0, 4.0 Hz, 1H), 5.22 (d, *J* = 8.0 Hz, 1H), 4.99 (s, 1H), 4.43 - 4.28 (m, 2H), 4.05 (dd, *J* = 12.0, 4.0 Hz, 1H), 4.01 - 3.95 (m, 1H), 3.86 - 3.77 (m, 1H), 3.68 - 3.56 (m, 1H), 3.45 (td, *J* = 12.0, 4.0 Hz, 1H), 3.29 (t, *J* = 4.0, 2H), 3.17 (t, *J* = 8.0 Hz, 1H), 2.02 (d, *J* = 12.0 Hz, 1H), 1.76 - 1.64 (m, 2H), 1.21 (dd, *J* = 8.0, 4.0s Hz, 6H).

Referring to the synthesis method of Example A1, the following compounds were synthesized:

| Compound | Structure | LC-MS | NMR |
|---|---|---|---|
| TA-2 | 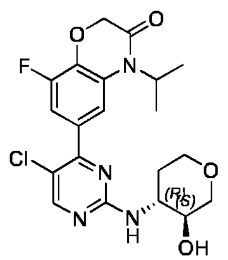 **6-(5-chloro-2-(((3*S*,4*R*)-3-hydroxytetrahydro-2*H*-pyran-4-yl)amino)pyrimidin-4-yl)-8-fluoro-4-isopropyl-2*H*-benzo-[*b*][1,4]oxazin-3(4*H*)-one** | 437.1 | $^1$H NMR (400 MHz, Chloroform-d) $\delta$ 8.32 (s, 1H), 7.45 (s, 1H), 7.38 (dd, *J* = 12.0, 4.0 Hz, 1H), 5.30 (d, *J* = 8.0 Hz, 1H), 4.81 (p, *J* = 8.0 Hz, 1H), 4.63 (s, 2H), 4.57 (s, 1H), 4.05 (dd, *J* = 12.0, 4.0 Hz, 1H), 3.99 (dd, *J* = 12.0, 4.0 Hz, 1H), 3.85 (m, 1H), 3.63 (m, 1H), 3.46 (td, *J* = 12.0, 4.0 Hz, 1H), 3.17 (t, *J* = 8.0, 1H), 2.14 - 1.98 (m, 1H), 1.71 (dd, *J* = 12.0, 4.0 Hz, 1H), 1.57 (dd, *J* = 8.0, 4.0 Hz, 6H). |
| TA-3 | 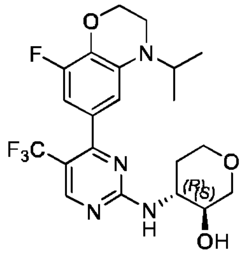 **(3*S*,4*R*)-4-((4-(8-fluoro-4-isopropyl-3,4-dihydro-2*H*-benzo[*b*][1,4]oxazin-6-yl)-5-(trifluoromethyl)pyrimidin-2-yl)amino)tetrahydro-2*H*-pyran-3-ol** | 457.3 | $^1$H NMR (400 MHz, Chloroform-d) $\delta$ 8.53 (s, 1H), 6.71 (s, 1H), 6.68 (dd, *J* = 12.0, 4.0 Hz, 1H), 5.67 (s, 1H), 4.70 (d, 1H), 4.41 - 4.23 (m, 2H), 4.11 - 3.85 (m, 4H), 3.61 (td, *J* = 8.0, 4.0 Hz, 1H), 3.44 (td, *J* = 12.0, 4.0 Hz, 1H), 3.34 - 3.24 (m, 2H), 3.19 - 3.10 (m, 1H), 2.12 - 1.96 (m, 1H), 1.19 (d, *J* = 8.0 Hz, 6H). |

(continued)

| Compound | Structure | LC-MS | NMR |
|---|---|---|---|
| TA-4 | 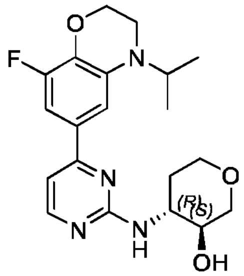 **(3*S*,4*R*)-4-((4-(8-fluoro-4-isopropyl-3,4-dihydro-2H-benzo[b][1,4]oxazin-6-yl)pyrimidin-2-yl)amino)tetra- hydro-2*H*-pyran-3-ol** | 389.4 | $^1$H NMR (400 MHz, Chloroform-d) $\delta$ 8.24 (d, *J* = 4.0 Hz, 1H), 7.04 (d, *J* = 12.0 Hz, 1H), 6.92 (d, *J* = 4.0 Hz, 1H), 5.32 (s, 1H), 4.33 (s, 2H), 4.21 (p, *J* = 8.0 Hz, 1H), 4.08 (dd, *J* = 12.0, 4.0 Hz, 1H), 4.00 (dd, *J* = 12.0, 4.0 Hz, 1H), 3.83 (dt, *J* = 12.0, 4.0 Hz, 1H), 3.63 (td, *J* = 8.0, 4.0 Hz, 1H), 3.55 - 3.40 (m, 1H), 3.29 (t, *J* = 4.0 Hz, 2H), 3.18 (t, *J* = 12.0 Hz, 1H), 2.02 (dd, *J* = 12.0, 4.0 Hz, 1H), 1.75 (tt, *J* = 12.0, 4.0 Hz, 2H), 1.22 (d, *J* = 8.0 Hz, 6H). |
| TA-5 | 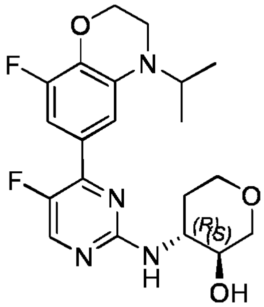 **(3*S*,4*R*)-4-((5-fluoro-4-(8-fluoro-4-isopropyl-3,4-dihydro-2*H*-benzo[*b*][1,4]oxazin-6-yl)pyrimidin-2-yl)amino) tetrahydro -2*H*-pyran-3-ol** | 407.1 | $^1$H NMR (400 MHz, Chloroform-d) $\delta$ 8.15 (d, *J* = 4.0 Hz, 1H), 7.30 (s, 1H), 7.20 (d, *J* = 12.0 Hz, 1H), 5.17 (d, *J* = 4.0 Hz, 1H), 4.42 - 4.30 (m, 2H), 4.17 (m, 1H), 4.11 - 4.03 (m, 1H), 4.03 - 3.93 (m, 1H), 3.80 (m, 1H), 3.62 (td, *J* = 8.0, 4.0 Hz, 1H), 3.46 (td, *J* = 12.0, 4.0 Hz, 1H), 3.29 (t, *J* = 4.0 Hz, 2H), 3.18 (t, *J* = 12.0 Hz, 1H), 2.09 - 2.00 (m, 1H), 1.73 (td, *J* = 12.0, 4.0 Hz, 2H), 1.22 (d, *J* = 8.0 Hz, 6H). |
| TA-6 | 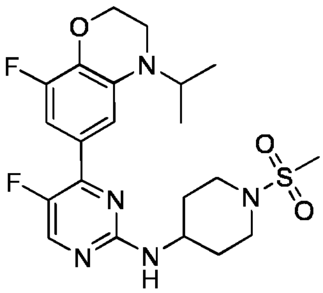 **5-fluoro-4-(8-fluoro-4-isopropyl-3,4-dihydro-2*H*-benzo [*b*][1,4]oxazin-6-yl)-*N*-(1-(methylsulfonyl)piperidin-4-yl) pyrimidin-2-amine** | 468.2 | $^1$H NMR (400 MHz, Chloroform-d) $\delta$ 8.16 (d, *J* = 4.0 Hz, 1H), 7.32 (s, 1H), 7.23 *(d, J* = 12.4 Hz, 1H), 5.02 (d, *J* = 8.0 Hz, 1H), 4.35 (t, *J* = 4.0, 2H), 4.19 - 4.09 (m, 1H), 3.95 (m, 1H), 3.76 (dt, *J* = 12.0, 4.0 Hz, 2H), 3.29 (t, *J* = 4.0 Hz,2H), 2.94 (td, *J* = 12.0, 4.0 Hz, 2H), 2.82 (s, 3H), 2.27 - 2.13 (m, 2H), 1.68 (m, 2H), 1.22 (d, *J* = 4.0 Hz, 6H). |

(continued)

| Compound | Structure | LC-MS | NMR |
|---|---|---|---|
| TA-7 | 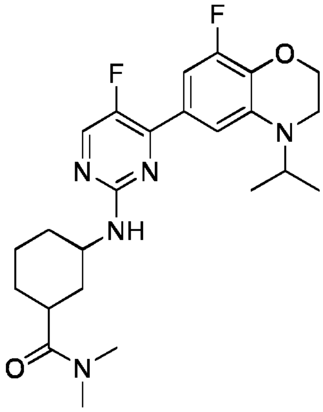 **3-((5-fluoro-4-(8-fluoro-4-isopropyl-3,4-dihydro-2*H*-benzo[b][1,4]oxazin-6-yl)pyrimidin-2-yl)amino)-*N,N*-dimethylcyclohexane-1-carboxamide** | 460.2 | $^1$H NMR (400 MHz, Chloroform-d) $\delta$ 8.14 (d, *J* = 4.0 Hz, 1H), 7.33 (s, 1H), 7.24 (*d, J* = 12.0 Hz, 1H), 5.01 (d, *J* = 8.0 Hz, 1H), 4.34 (t, *J* = 4.0 Hz, 2H), 4.16 (p, *J* = 8.0 Hz, 1H), 3.84 (m, 1H), 3.29 (t, *J* = 4.0 Hz, 2H), 3.07 (s, 3H), 2.93 (s, 3H), 2.71 (m, 1H), 2.18 (t, *J* = 12.0 Hz, 2H), 1.97 - 1.87 (m, 1H), 1.77 (m, 2H), 1.56 - 1.41 (m, 4H), 1.21 (dd, *J* = 8.0, 4.0 Hz, 6H). |
| TA-8 | 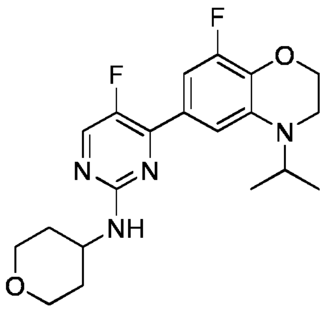 **5-fluoro-4-(8-fluoro-4-isopropyl-3,4-dihydro-2*H*-benzo[*b*][1,4]oxazin-6-yl)-*N*-(tetrahydro-2*H*-pyran-4-yl)pyrimidin-2-amine** | 390.9 | $^1$H NMR (400 MHz, Chloroform-*d*) $\delta$ 8.16 (d, *J* = 4.0 Hz, 1H), 7.36 (s, 1H), 7.24 (d, *J* = 8.0 Hz, 1H), 5.06 (d, *J* = 8.0 Hz, 1H), 4.43 - 4.29 (m, 2H), 4.15 (p, *J* = 6.6 Hz, 1H), 4.04 - 3.99 (m, 2H), 3.55 (td, *J* = 12.0, 4.0 Hz, 2H), 3.35 - 3.21 (m, 2H), 2.08 (dd, *J* = 12.0, 4.0 Hz, 2H), 1.62 - 1.54 (m, 2H), 1.22 (d, *J* = 8.0 Hz, 6H). |
| TA-9 | 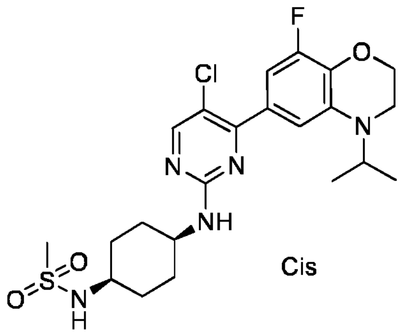 Cis **N-(Cis-4-((5-chloro-4-(8-fluoro-4-isopropyl-3,4-dihydro-2*H*-benzo[*b*][1,4]oxazin-6-yl)pyrimidin-2-yl)amino)cyclohexyl)methanesulfonamid** e | 498.1 | $^1$H NMR (400 MHz, Chloroform-d) $\delta$ 8.25 (s, 1H), 7.05 (s, 1H), 6.98 (d, *J* = 12.0 Hz, 1H), 5.25 (d, *J* = 8.0 Hz, 1H), 4.49 (d, *J* = 8.0 Hz, 1H), 4.34 (t, *J* = 4.0 Hz, 2H), 4.10 (m, 1H) , 3.98 (s, 1H), 3.55 (s, 1H), 3.29 (t, *J* = 4.0 Hz, 2H), 3.00 (s, 3H), 1.84 (m, 4H), 1.72 (m, 4H), 1.21 (d, *J* = 8.0 Hz, 6H). |

(continued)

| Compound | Structure | LC-MS | NMR |
|---|---|---|---|
| TA-10 | 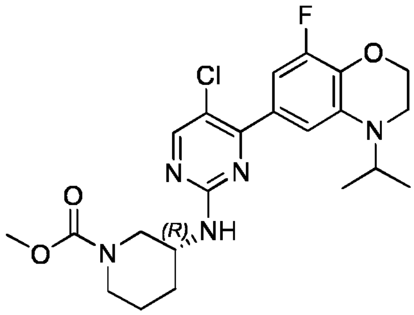 **methyl (*R*)-3-((5-chloro-4-(8-fluoro-4-isopropyl-3,4-dihydro-2*H*-benzo[*b*][1,4]oxazin-6-yl)pyrimidin-2-yl)amino)piperidine-1-carboxylate** | 464.0 | $^1$H NMR (400 MHz, Chloroform-d) δ 8.26 (s, 1H), 7.08 (s, 1H), 7.01 (s, 1H), 5.20 (s, 1H), 4.34 (t, *J* = 4.0 Hz, 2H), 4.13 (m, 1H), 3.98 (s, 2H), 3.69 (s, 3H), 3.66 - 3.47 (m, 1H), 3.29 (t, *J* = 4.0 Hz, 2H), 1.97 (s, 1H), 1.64 (s, 4H), 1.21 (dd, *J* = 8.0, 4.0 Hz, 6H). |
| TA-11 | 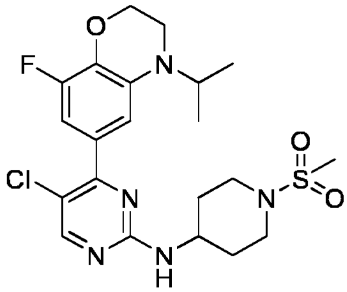 **5-chloro-4-(8-fluoro-4-isopropyl-3,4-dihydro-2*H*-benzo[*b*][1,4]oxazin-6-yl)-*N*-(1-(methylsulfonyl)piperidin-4-yl)pyrimidin-2-amine** | 484.3 | $^1$H NMR (400 MHz, Chloroform-d) δ 8.26 (s, 1H), 7.05 (s, 1H), 6.98 (d, *J* = 12.0 Hz, 1H), 5.11 (d, *J* = 8.0 Hz, 1H), 4.34 (t, *J* = 4.0 Hz, 2H), 4.10 (m, 1H), 4.03 - 3.91 (m, 1H), 3.80 - 3.70 (m, 2H), 3.29 (t, *J* = 4.0 Hz, 2H), 2.93 (td, *J* = 12.0, 4.0 Hz, 2H), 2.80 (s, 3H), 2.18 (dd, *J* = 12.0, 4.0 Hz, 2H), 1.67 (dd,*J* = 12.0, 4.0 Hz, 2H), 1.21 (d, *J* = 8.0 Hz, 6H). |
| TA-12 | 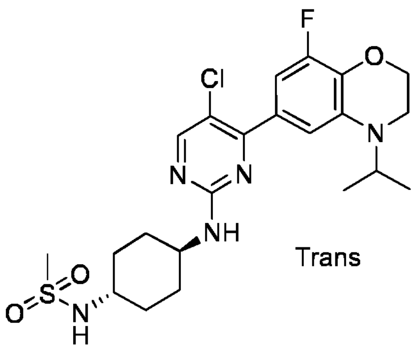 | 498.1 | $^1$H NMR (400 MHz, Chloroform-d) δ 8.24 (s, 1H), 7.05 (s, 1H), 6.98 (d, *J* = 12.0 Hz, 1H), 5.06 (d, *J* = 8.0 Hz, 1H), 4.42 - 4.30 (m, 2H), 4.25 (d, *J* = 8.0 Hz, 1H), 4.18 - 4.04 (m, 1H), 3.85 - 3.70 (m, 1H), 3.35 (dp, *J* = 12.0, 4.0 Hz, 1H), 3.31 - 3.21 (m, 2H), 2.99 (s, 3H), 2.16 (m, 4H), 1.44 - 1.30 (m, 4H), 1.21 (d, *J* = 8.0 Hz, 6H). |
| TA-13 | 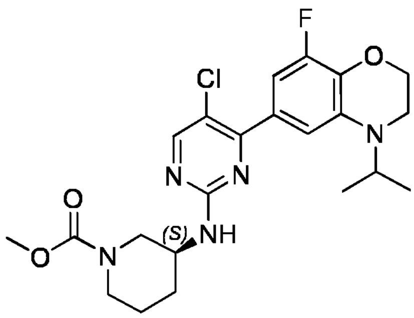 | 464.1 | $^1$H NMR (400 MHz, Chloroform-d) δ 8.26 (s, 1H), 7.08 (s, 1H), 7.00 (s, 1H), 5.19 (s, 1H), 4.34 (t, *J* = 4.0 Hz, 2H), 4.12 (s, 1H), 3.98 (s, 2H), 3.69 (s, 3H), 3.50 (s, 1H), 3.29 (t, *J* = 4.0 Hz, 2H), 3.15 (s, 1H) 1.97 (s, 1H), 1.75 (s, 1H), 1.21 (dd, *J* = 8.0, 4.0 Hz, 6H). |

(continued)

| Compound | Structure | LC-MS | NMR |
|---|---|---|---|
| TA-14 | 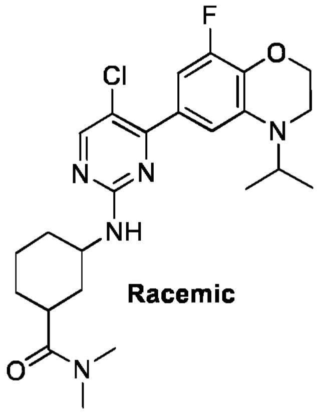 Racemic<br>**3-((5-chloro-4-(8-fluoro-4-isopropyl-3,4-dihydro-2*H*-benzo[*b*][1,4]oxazin-6-yl)pyrimidin-2-yl)amino)-N,N-dimethylcyclohexane-1-carboxamide** | 476.1 | $^1$H NMR (400 MHz, Chloroform-d) $\delta$ 8.23 (s, 1H), 7.06 (s, 1H), 6.97 (d, *J* = 12.0 Hz, 1H), 5.14 (d, *J* = 8.0 Hz, 1H), 4.34 (t, *J* = 4.0 Hz, 2H), 4.10 (m, 1H), 3.87 (m, 1H), 3.29 (t, *J* = 4.0 Hz, 2H), 3.06 (s, 3H), 2.93 (s, 3H), 2.69 (t, *J* = 12.0 Hz, 1H), 2.14 (dd, *J* = 24.0, 12.0 Hz, 2H), 1.96 - 1.87 (m, 1H), 1.74 (s, 1H), 1.48 (m, 4H), 1.20 (d, *J* = 8.0 Hz, 6H). |
| TA-15 | 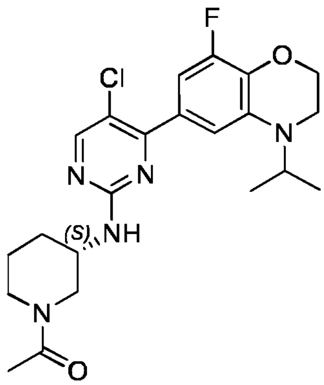<br>**(*S*)-1-(3-((5-chloro-4-(8-fluoro-4-isopropyl-3,4-dihydro-2*H*-benzo[*b*][1,4]oxazin-6-yl)pyrimidin-2-yl)amino)piperidin-1-yl)ethan-1-one** | 448.1 | |
| TA-16 | 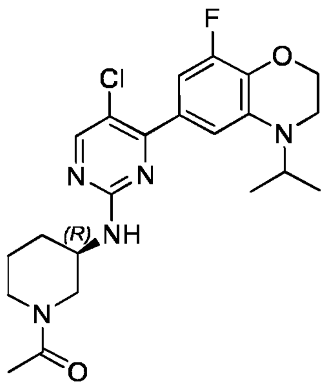<br>**(*R*)-1-(3-((5-chloro-4-(8-fluoro-4-isopropyl-3,4-dihydro-2*H*-benzo[*b*][1,4]oxazin-6-yl)pyrimidin-2-yl)amino)piperidin-1-yl)ethan-1-one** | 448.1 | |

(continued)

| Compound | Structure | LC-MS | NMR |
|---|---|---|---|
| TA-17 | 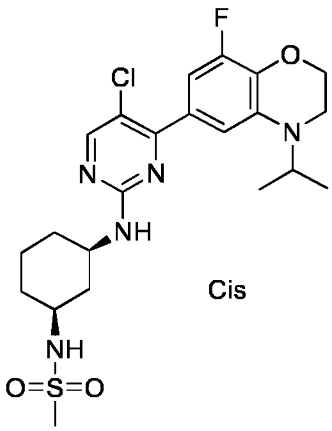 Cis | 498.1 | 1H NMR (400 MHz, Chloroform-d) δ 8.25 (s, 1H), 7.05 (s, 1H), 6.97 (d, *J* = 12.0 Hz, 1H), 5.17 (d, *J*= 8.0 Hz, 1H), 4.34 (t, *J* = 4.0 Hz, 2H), 4.09 (m, 1H), 3.88 (m, 1H), 3.41 (m, 1H), 3.29 (t, *J* = 4.0 Hz, 2H), 2.99 (s, 3H), 2.48 (d, *J* = 12.0 Hz, 1H), 2.08 (d, *J* = 12.0 Hz, 2H), 1.85 (*dt, J* = 12.0, 4.0 Hz, 1H), 1.53 - 1.38 (m, 2H), 1.21 (dd, *J* = 6.6, 1.6 Hz, 6H), 1.15 - 1.08 (m, 2H). |
| TA-18 | 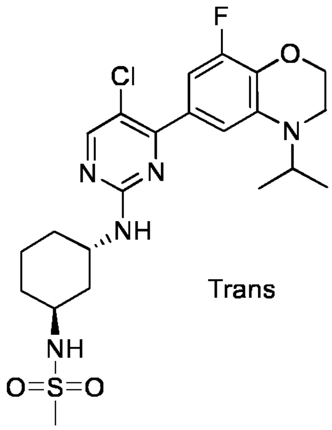 Trans | 498.1 | |
| TA-19 | 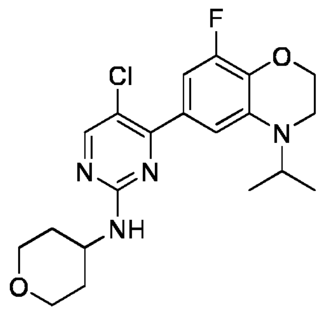 **5-chloro-4-(8-fluoro-4-isopropyl-3,4-dihydro-2*H*-benzo [*b*][1,4]oxazin-6-yl)-*N*-(tetrahydro-2*H*-pyran-4-yl)pyrimidin-2-amine** | 407.2 | 1H NMR (400 MHz, Chloroform-d) δ 8.25 (s, 1H), 7.06 (s, 1H), 7.00 (d, *J* = 12.0 Hz, 1H), 5.13 (d, *J* = 8.0 Hz, 1H), 4.34 (t, *J* = 4.0, 2H), 4.15 - 4.03 (m, 2H), 4.02 - 3.94 (m, 2H), 3.53 (td, *J* = 12.0, 4.0 Hz, 2H), 3.328 (t, *J* = 4.0, 2H), 2.16 - 1.96 (dt, *J* = 12.0, 4.0, 2H), 1.60 - 1.53 (m, 2H), 1.21 (d, J= 8.0 Hz, 6H). |

**Example A2**

Synthesis of the compound TA-20 of the present invention:

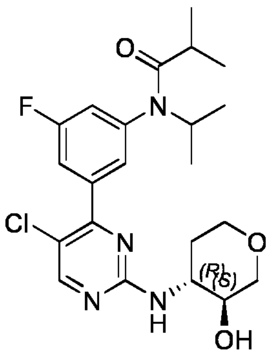

The synthesis route is as follows:

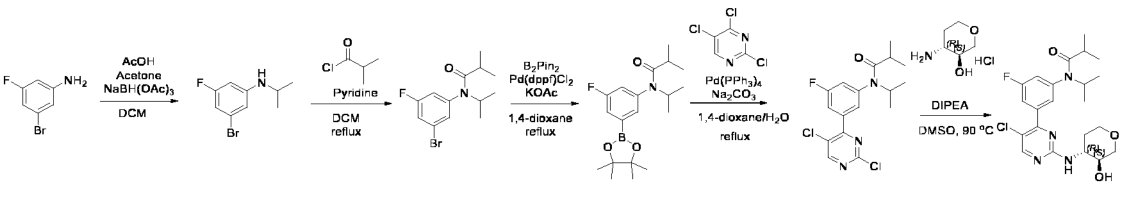


SM 1 SM 2 SM 3 SM 4 SM 5 TA-20

The experimental procedure is as follows:

1. Synthesis of compound SM2:

Compound SM 1 (5.97 g, 1.0 e.q.) was solubilized in 120 mL of DCM under nitrogen, cooled down in an ice-water bath, acetone (10.4 mL, 4.5 e.q.) and glacial acetic acid (9 mL, 5.0 e.q.) were added sequentially, and the reaction was kept warm for 0.5 h. Sodium borohydride acetate (26.6 g, 4.0 e.q.) was added in batches, and the reaction was moved to room temperature at the end of addition. At the end of the reaction, the reaction was quenched by adding an appropriate amount of ammonium chloride solution, extracted and partitioned with EA and water, and the organic phase was dried over anhydrous sodium sulfate, concentrated and then separated and purified by silica gel column chromatography to obtain 2.8 g of the compound SM 2 in a yield of 38.4%.

2. Synthesis of compound SM3:

Under the condition of nitrogen, compound SM 2 (1.48 g, e.q.) and pyridine (1.01 g, 2.0 e.q.) were sequentially added to 15 mL of dichloromethane solvent, and then stirred for ten minutes in an ice-water bath, and isobutyryl chloride (2.05 g, e.q.) was slowly added dropwise, and then the reaction was heated up to 50 °C after the dropwise addition. At the end of the reaction, the reaction solution was extracted with ethyl acetate and water, and the organic phase was dried over anhydrous sodium sulfate, concentrated and purified by silica gel column chromatography to obtain 1.62 g of compound SM 3 in 83.9% yield.

3. Synthesis of compound SM4:

Compound SM 3 (95 mg, e.q.), pinacol bis(boronic acid) ester (159.6 mg, 2.0 e.q.), Pd(dppf)$Cl_2$ (11.5 mg, 0.05 e.q.), and potassium acetate (92.5 mg, 3.0 e.q.) were added sequentially to a 25 mL three-necked flask under nitrogen, followed by the addition of 5 mL of super-dry 1,4-dioxane. Nitrogen was replaced three times and the temperature was raised to reflux the reaction. At the end of the reaction, the reaction solution was cooled to room temperature, extracted and partitioned with ethyl acetate and water, and the organic phase was dried over anhydrous sodium sulfate, and then concentrated to give the crude product containing compound SM 4, which was directly used in the next step, LCMS: $[M + H]^+ = 350.2$.

4. Synthesis of compound SM5:

Crude compound SM 4 (110 mg, 1.0 e.q.), 2,4,5-trichloropyrimidine (81.7 mg, 1.3 e.q.), $Pd(PPh_3)_4$ (39.6 mg, 0.1 e.q.), and sodium carbonate (109.0 mg, e.q.) were sequentially added to a 25 mL triple-necked flask, and then 8 mL of a

solvent mixture of 1,4-dioxane and water ( v:v = 3:1), nitrogen was displaced three times, and the temperature was raised to reflux the reaction. At the end of the reaction, the reaction solution was cooled to room temperature, the reaction solution was extracted with ethyl acetate and water, and the organic phase was dried over anhydrous sodium sulfate, concentrated and then separated and purified by silica gel column chromatography to obtain 43 mg of compound SM 5, LCMS: $[M+H]^+$ = 370.0.

5. Synthesis of compound TA-20:

Compound SM 5 (43 mg, 1.0 e.q.), (3s, 4r)-4-aminooxan-3-ol hydrochloride (23.2 mg, 1.3 e.q.) and DIPEA (52.5 mg, 3.5 e.q.) were sequentially added to 3.0 mL of DMSO solvent, and heated under nitrogen protection up to 90 °C. At the end of the reaction, the reaction solution was cooled down to room temperature, and extracted and partitioned with saturated NaCl solution, water, and EA sequentially, and the organic phase was dried over anhydrous sodium sulfate, concentrated and then separated and purified by silica gel column chromatography to obtain 25 mg of the target compound TA-20 in 47.9% yield and 99.4% purity by HPLC, LCMS: $[M+H]^+$ = 451.10.

$^1$H NMR (400 MHz, Chloroform-d) $\delta$ 8.35 (s, 1H), 7.55 (d, J= 8.0 Hz, 1H), 7.37 (s, 1H), 6.97 (dt, *J* = 8.0, 2.2 Hz, 1H), 5.30 (d, *J* = 6.3 Hz, 1H), 5.03 (p, *J* = 6.8 Hz, 1H), 4.34 (s, 1H), 4.05 (dd, *J* = 11.4, 4.9 Hz, 1H), 4.02 - 3.93 (m, 1H), 3.87 (tt, *J* = 11.2, 5.8 Hz, 1H), 3.63 (tt, *J* = 9.0, 4.2 Hz, 1H), 3.46 (td, *J* = 11.9, 2.3 Hz, 1H), 3.18 (t, *J* = 10.6 Hz, 1H), 2.38 - 2.24 (m, 1H), 2.13 - 2.00 (m, 1H), 1.72 (td, *J* = 12.1, 4.8 Hz, 1H), 1.08 (dd, *J* = 7.0, 4.3 Hz, 6H), 1.02 (dd, *J* = 6.6, 2.8 Hz, 6H).

Referring to the synthesis method of Example A2, the following compound was synthesized:

| Compound | Structure | LC-MS | NMR |
|---|---|---|---|
| TA-21 | 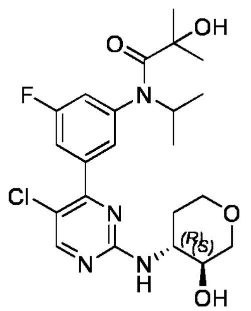 ***N*-(3-(5-chloro-2-(((3*S*,4*R*)-3-hydroxytetrahydro-2*H*-pyran-4-yl)amino)pyrimidin-4-yl)-5-fluorophenyl)-2-hydroxy-*N*-isopropyl-2-methylpropanamide** | 467.2 | |

**Example A3**

compound TA-22 synthesized in the present invention:

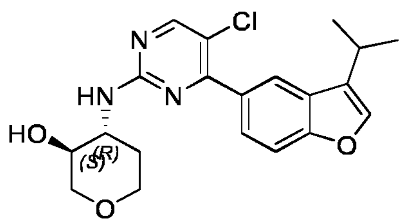

The synthesis route is as follows:

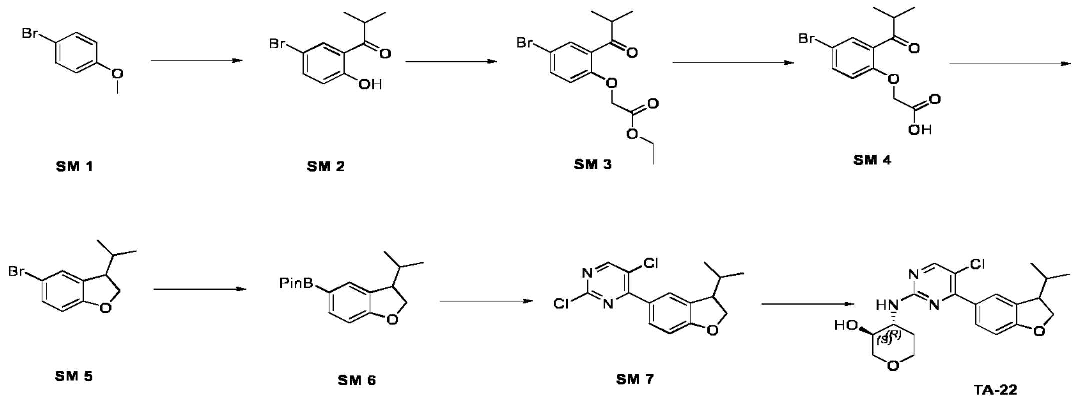

SM 1 SM 2 SM 3 SM 4

SM 5 SM 6 SM 7 TA-22

The experimental procedure is as follows:

1. Synthesis of compound SM2:

Under nitrogen, to a dry 50 mL three-necked flask, p-bromoanisole SM 1 (2.0 g, 1.0 e.q.) was added, solubilized by adding 20 mL of 1,2-dichloroethane, placed in an ice-water bath, and after the temperature was stabilized, isobutyryl chloride (1.72 g, 1.5 e.q.) and anhydrous aluminum trichloride (2.84 g, 2.0 e.q.) were added in sequence. After the reaction solution was returned to room temperature, the reaction solution was heated up to reflux temperature. After being controlled by liquid chromatography, the raw materials were completely reacted, the reaction solution was cooled to room temperature, quenched by slowly pouring into ice water and extracted and partitioned with dichloroethane, the organic phase was dried over anhydrous sodium sulfate, concentrated under reduced pressure, and then separated and purified by column chromatography to yield 2.0 g of a light yellow oily liquid, SM 2, in 79% yield.
$^1$H NMR (400 MHz, Chloroform-d) $\delta$ 12.43 (s, 1H), 7.88 (d, $J$ = 2.4 Hz, 1H), 7.54 (dd, $J$ = 8.0, 2.4 Hz, 1H), 6.91 (d, $J$ = 8.0 Hz, 1H), 3.54 (m, $J$ = 8.0 Hz, 1H), 1.25 (d, $J$ = 8.0 Hz, 6H).

2. Synthesis of compound SM3:

Under the condition of nitrogen, compound SM 2 (2.5 g, 1.0 e.q.) was dissolved in 35 mL of acetone solvent, ethyl bromoacetate (3.4 mL, 3.0 e.q.) and potassium carbonate (4.26 g, 3.0 e.q.) were added sequentially and the reaction was carried out at room temperature. After TLC showed that the raw material was disappeared, the reaction was ended. The reaction solution was suction filtered under reduced pressure, the filter cake was washed with appropriate amount of ethyl acetate, extracted and partitioned with water, the organic phase was dried over anhydrous sodium sulfate, concentrated under reduced pressure and then separated and purified by silica gel column chromatography to obtain 2.16 g of the compound SM 3, $[M + H]^+$ = 330.9, $[M + Na]^+$ = 350.9, the yield was 80%.

3. Synthesis of compound SM 4:

Compound SM 3 (2.16 g, 1.0 e.q.) was placed in a 50 ml three-necked flask under nitrogen, and an aqueous solution of sodium carbonate (2.08 g, 3.0 e.q.) was added, and the reaction was carried out at 90 °C for 3 h. After TLC showed that the raw material was disappeared, the reaction was ended. The reaction solution was cooled to room temperature, quenched with 1 M HCl under the condition of ice water bath and adjusted PH to 3~4. Appropriate amount of ethyl acetate was added to extract and partition, the organic phase was dried over anhydrous sodium sulfate and then concentrated to obtain 1.85 g of light yellow solid SM 4, $[M + H]^+$ = 303.1, $[M + Na]^+$ = 325.1, yield 94%.

4. Synthesis of compound SM 5:

Compound SM 4 (1.8 g, 1.0 e.q.) was dissolved in 18 ml of acetic anhydride under nitrogen, sodium acetate (2.7 g, 5.5 e.q.) was added, nitrogen was displaced three times, and the reaction was carried out overnight at 140 °C. After TLC showed that the raw material was disappeared, the reaction was ended and the reaction solution was cooled to room temperature. The reaction solution was extracted and partitioned with appropriate amounts of ethyl acetate and water, the organic phase was dried over anhydrous sodium sulfate and concentrated, and purified by column chromatography to obtain 1.01 g of the compound SM 5 as a colorless oil in 71.1% yield.

5. Synthesis of compound SM 6:

Compound SM 5 (200 mg, 1.0 e.q.) was dissolved in 2 ml of dioxane under nitrogen, and bis(boronic acid) pinacol ester (430 mg, 2.0 e.q.), Pd(dppf)$Cl_2$ (31 mg, 0.05 e.q.), and KOAc (250 mg, 3.0 e.q.) were added sequentially with nitrogen displacing three times and the reaction was carried out overnight at 80 °C. After TLC showed that the raw material was disappeared, the reaction was ended and the reaction solution was cooled to room temperature. The reaction solution was extracted and partitioned with appropriate amount of ethyl acetate and water, and the organic phase was dried over anhydrous sodium sulfate and concentrated, and directly put into the next step.

6. Synthesis of compound SM 7:

Compound SM 6 (crude 240 mg, 1.0e.q.) was dissolved in dioxane/water (10V:2V) under nitrogen, 2,4,5-trichloropyrimidine (230 mg, 1.5e.q.), $Pd(pph_3)_4$ (97 mg, 0.1e.q.), and sodium carbonate (267 mg, 3.0e.q.) were added sequentially with nitrogen displacing three times. The reaction was carried out at 90°C for 4 h. After TLC control showed the disappearance of the raw material, the reaction was terminated and the reaction solution was cooled to room temperature. The reaction solution was extracted and partitioned with appropriate amounts of ethyl acetate and water, the organic phase was dried over anhydrous sodium sulfate and concentrated, and purified by column chromatography to obtain 180 mg of the colorless oily compound SM 7 in 70% yield.

7. Synthesis of compound TA-22:

Compound SM 7 (180 mg, 1.0e.q.) was dissolved in 2 ml of DMSO under nitrogen, (3S,4R)-4-amino oxacyclohexan-3-ol hydrochloride (135 mg, 1.5e.q.) and DIPEA (0.4 ml, 3.5e.q.) were added, and the reaction was carried out overnight at 80 °C. After TLC showed that the raw material was disappeared, the reaction was ended and the reaction solution was cooled down to room temperature. The reaction solution was extracted and partitioned with appropriate amounts of ethyl acetate and water, and the organic phase was washed twice with saturated brine and dried over anhydrous sodium sulfate, concentrated under reduced pressure, and purified by thick preparative plate to obtain 54 mg of the light yellow solid compound TA- 22, [ M + H ]$^+$ = 388.2, in 23.9% yield.
$^1$H NMR (400 MHz, Chloroform-d) $\delta$ 8.31 (s, 1H), 8.04 (d, *J* = 2.0 Hz, 1H), 7.69 (dd, *J*= 8.6, 1.8 Hz, 1H), 7.54 (d, *J* = 8.0 Hz, 1H), 7.43 (s, 1H), 5.31 (d, *J* = 4.0 Hz, 1H), 5.08 (s, 1H), 4.05 (dd, *J* = 12.0, 4.0 Hz, 1H), 3.98 (dd, *J* = 12.0, 4.0 Hz, 1H), 3.89 - 3.75 (m, 1H), 3.62 (td, *J* = 8.0, 4.0 Hz, 1H), 3.44 (td, *J* = 12.0, 2.0 Hz, 1H), 3.25 - 3.05 (m, 2H), 2.03 (dt, *J* = 12.0, 4.0 Hz, 1H), 1.74 - 1.67 (m, 1H), 1.38 (d, *J* = 8.0 Hz, 6H).
Referring to the synthesis method of Example A3, the following compound was synthesized:

| Compound | Structure | LC-MS | NMR |
|---|---|---|---|
| TA-23 | 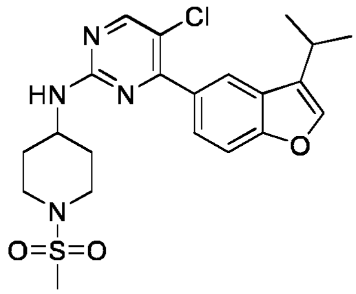 **5-chloro-4-(3-isopropylbenzofuran-5-yl)-*N*-(1-(methylsulfonyl)piperidin-4-yl)pyrimidin-2-amine** | 449.2 | |

## Example A4

Synthesis of the compound TA-24 of the present invention:

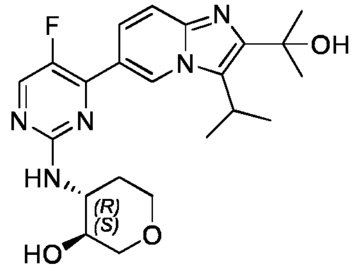

The synthesis route is as follows:

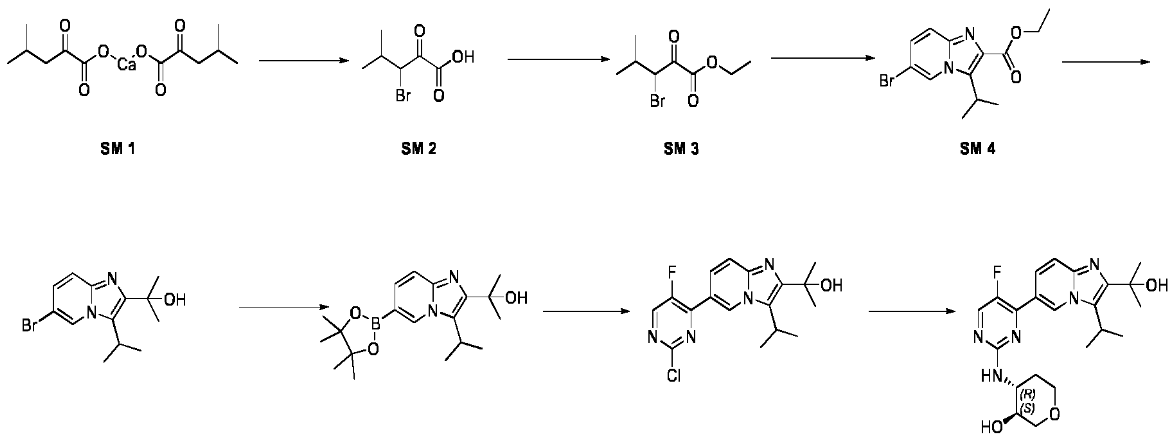

SM 1 SM 2 SM 3 SM 4

SM 5 SM 6 SM 7 TA-24

The experimental procedure is as follows:

1. Synthesis of compound SM2:

Calcium 4-methyl-2-ketovalerate (5 g, 1.0 e.q.) was added to a 250 mL three-necked flask under nitrogen, glacial acetic acid (100 mL) was added slowly, and pyridinium tribromide (13.4 g, 2.5 e.q.) was added during stirring and the reaction was carried out at room temperature. At the end of the reaction, most of the acetic acid solvent was removed by concentration under reduced pressure, and then extracted and partitioned with appropriate amount of water and EA, the organic phase was dried over anhydrous sodium sulfate, and the organic phase was concentrated under reduced pressure after suction filtration, to obtain the crude product of SM 2, which was directly put into the next step.

2. Synthesis of compound SM3:

The crude SM 2 (9.0 g) obtained in the previous step was dissolved in anhydrous ethanol, and concentrated sulfuric acid (1.78 mL, 1.0 e.q.) was slowly added dropwise to the ethanol solution, and the reaction was heated to reflux under nitrogen protection. At the end of the reaction, the reaction solution was cooled to room temperature and sodium bicarbonate solid was slowly added directly to the reaction system to adjust the pH to about 7. Most of the solvent was removed by concentration under reduced pressure, and then extracted and partitioned with EA and water, and the organic phase was dried over anhydrous sodium sulfate, suction filtered, concentrated under reduced pressure to remove the organic solvent, and then separated and purified by silica gel column chromatography to obtain 4.1 g of compound SM 3 in a two-step reaction yield of 52%.
$^1$H NMR (400 MHz, Chloroform-d) $\delta$ 4.85 (d, $J$ = 8.0 Hz, 1H), 4.37 (q, $J$ = 8.0 Hz, 2H), 2.49 - 2.26 (m, 1H), 1.39 (t, $J$ = 8.0 Hz, 3H), 1.14 (d, $J$ = 4.0 Hz, 3H), 1.05 (d, $J$ = 4.0 Hz, 3H).

3. Synthesis of compound SM 4:

Compound SM 3 (4.0 g, 1.0 e.q.) was dissolved in ultra-dry 1,4-dioxane solvent (80 mL), 2-amino-5-bromopyridine (2.9 g, 1.0 e.q.) and sodium bicarbonate solid (2.8 g, 2.8 e.q.) were added in sequence, and the reaction solution was heated up to reflux. At the end of the reaction, the reaction solution was cooled to room temperature, the reaction solution was extracted and partitioned with saturated ammonium chloride solution and EA, the organic phase was dried over

anhydrous sodium sulfate, suction filtered and concentrated under reduced pressure to remove the solvent, and then separated and purified by silica gel column chromatography to obtain 1.7 g of compound SM 4 in 32.4% yield.
$^1$H NMR (400 MHz, Chloroform-d) $\delta$ 8.29 (d, *J* = 4.0 Hz, 1H), 7.57 (dd, *J* = 8.0, 4.0 Hz, 1H), 4.45 (q, *J* = 7.1 Hz, 2H), 4.28 (m, 1H), 1.48 (d, *J* = 8.0 Hz, 6H), 1.44 (d, *J* = 8.0 Hz, 3H).

4. Synthesis of compound SM 5:

Compound SM 4 (1.7 g, 1.0 e.q.) was dissolved in ultra-dry tetrahydrofuran (35 mL) under nitrogen, placed in ice-water bath conditions, and 3.0 M MeMgBr solution (9.1 mL, 5.0 e.q.) was slowly dropwise added while the temperature was controlled. After the dropwise addition, the reaction solution was naturally warmed up to room temperature. At the end of the reaction, the excess of Grignard reagent was quenched with saturated ammonium chloride under the condition of ice-water bath, extracted and partitioned with EA, dried over anhydrous sodium sulfate, suction filtered and concentrated under reduced pressure to remove the solvent, and then isolated and purified by silica gel column chromatography to obtain 0.8 g of compound SM 5 in a yield of 49.4%. $^1$H NMR (400 MHz, Chloroform-d) $\delta$ 8.25 (dd, *J* = 1.8, 0.9 Hz, 1H), 7.45 (dd, *J* = 9.5, 0.9 Hz, 1H), 7.16 (dd, *J* = 9.5, 1.8 Hz, 1H), 3.93 (hept, *J* = 7.4 Hz, 1H), 1.64 (s, 7H), 1.45 (d, *J* = 7.4 Hz, 6H).

5. Synthesis of compound SM 6:

Compound SM 5 (0.8 g, 1.0 e.q.), pinacol bis(boronic acid) ester (1.4 g, 2.0 e.q.), Pd(dppf)$Cl_2$ (98.5 mg, 0.05 e.q.) and potassium acetate (0.8 g, 3.0 e.q.) were sequentially added to a dry three-necked flask under nitrogen protection, ultra-dry 1,4-dioxane solvent (20 mL) was added. The temperature was raised to reflux. At the end of the reaction, the reaction solution was cooled to room temperature, extracted and partitioned with EA and water, dried over anhydrous sodium sulfate, suction filtered and concentrated under reduced pressure to obtain a crude product containing SM 6.

6. Synthesis of compound SM 7:

Compound SM 6 (theoretical: 0.93 g, 1.0 e.q.), $Pd(PPh_3)_4$ (0.31 g, 0.1 e.q.), 2,4-dichloro-5-fluoropyrimidine (0.58 g, 1.3 e.q.), and sodium carbonate (0.86 g, 3.0 e.q.) were sequentially added to a dry three-necked flask protected with nitrogen, and a mixture solvent of ultra-dry 1,4-dioxane solvent and water (26 mL, v/v = 10: 3) was added and warmed to reflux reaction. At the end of the reaction, the reaction solution was cooled to room temperature, extracted and partitioned with EA and water, dried over anhydrous sodium sulfate, suction filtered and concentrated under reduced pressure to obtain 0.4 g of the compound SM 7. The two-step yield was 41%, LCMS: $[M + H]^+$ = 366.2.

7. Synthesis of compound TA-24:

Compound SM 7 (0.4 g, 1.0 e.q.), (3s, 4r)-4-aminooxan-3-ol hydrochloride (0.34 g, 2.0 e.q.) and DIPEA (0.8 mL, 4.0 e.q.) were sequentially added to 8.0 mL of DMSO solvent, and heated up to 90 °C under nitrogen protection. At the end of the reaction, the reaction solution was cooled down to room temperature, extracted and partitioned with saturated sodium chloride solution, water and EA in turn, the organic phase was dried over anhydrous sodium sulfate, concentrated and then separated and purified by silica gel column chromatography, 120 mg of compound TA-24 was obtained, the yield was 24.5%, the HPLC purity was 96.5% , LCMS: $[M + H]^+$ = 430.2.
$^1$H NMR (400 MHz, Chloroform-d) $\delta$ 9.03 (s, 1H), 8.24 (d, *J* = 3.8 Hz, 1H), 7.84 (d, *J* = *9.5* Hz, 1H), 7.62 (d, *J* = 9.5 Hz, 1H), 5.25 (d, *J* = 6.3 Hz, 1H), 4.09 (dd, *J* = 11.4, 4.9 Hz, 1H), 4.04 - 3.97 (m, 2H), 3.88 (tt, *J* = 11.0, 5.5 Hz, 1H), 3.66 (td, *J* = *9.5,* 4.9 Hz, 2H), 3.49 (td, *J* = 11.8, 2.3 Hz, 1H), 3.23 (dd, *J* = 11.4, 9.8 Hz, 1H), 2.17 - 2.06 (m, 1H), 1.73 (dd, *J* = 8.6, 3.9 Hz, 2H), 1.68 (s, 6H), 1.51 (dd, *J* = 7.4, 2.2 Hz, 6H).
Referring to the synthesis method of Example A4, the following compounds were synthesized:

| Compound | Structure | LC-MS | NMR |
|---|---|---|---|
| **TA-25** | 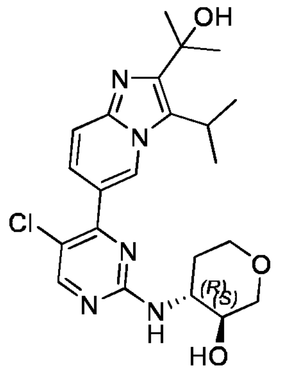 **(3*S*,4*R*)-4-((5-chloro-4-(2-(2-hydroxypropan-2-yl)-3-isopropylimidazo[1,2-*a*]pyridin-6-yl)pyrimidin-2-yl)amino)tetrahydro-2*H*-pyran-3-ol** | 446.2 | |
| **TA-26** | 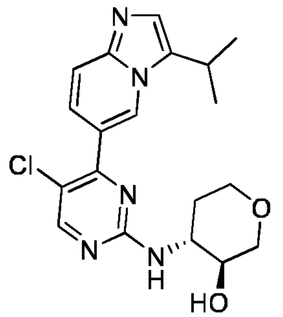 **(3*S*,4*R*)-4-((5-chloro-4-(3-isopropylimidazo[1,2-*a*]pyridin-6-yl)pyrimidin-2-yl)amino)tetrahydro-2*H*-pyran-3-ol** | 388.0 | |
| **TA-27** | 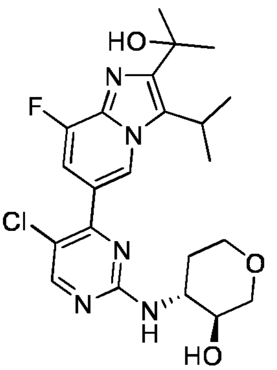 **(3S,4R)-4-((5-chloro-4-(8-fluoro-2-(2-hydroxypropan-2-yl)-3-isopropylimidazo[1,2-a]pyridin-6-yl)pyrimidin-2-yl)amino)tetrahydro-2*H*-pyran-3-ol** | 464.0 | $^1$H NMR (400 MHz, Chloroform-d) δ 8.72 (s, 1H), 8.34 (s, 1H), 7.40 (dd, *J* = 12.0, 4.0 Hz, 1H), 5.38 (d, *J* = 8.0 Hz, 1H), 4.30 (s, 1H), 4.11 - 3.95 (m, 3H), 3.89 (m, 1H), 3.64 (td, *J* = 8.0, 4.0 Hz, 1H), 3.48 (td, *J* = 12.0, 4.0 Hz, 2H), 3.20 (t, *J* = 8.0 Hz, 1H), 2.99 (s, 1H), 2.14 - 2.01 (m, 1H), 1.68 (s, 6H), 1.48 (dd, *J* = 8.0, 4.0 Hz, 6H). |

(continued)

| Compound | Structure | LC-MS | NMR |
|---|---|---|---|
| **TA-28** | 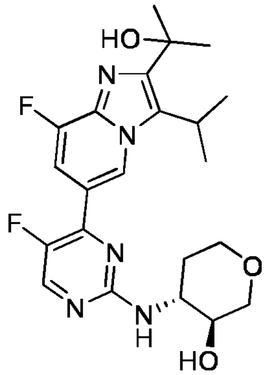 **(3*S*,4*R*)-4-((5-fluoro-4-(8-fluoro-2-(2-hydroxypropan-2-yl)-3-isopropylimidazo[1,2-a]pyridin-6-yl)pyrimidin-2-yl)amino) tetrahydro-2*H*-pyran-3-ol** | 448.2 | |
| **TA-48** | 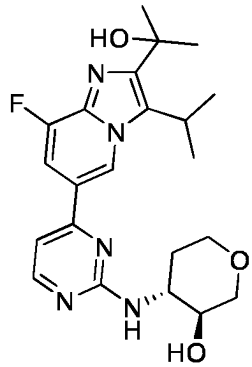 **(3*S*,4*R*)-4-((4-(8-fluoro-2-(2-hydroxypropan-2-yl)-3-isopropylimidazo[1, 2-a]pyridin-6-yl)pyrimidin-2-yl)amino)tetrahydro -2*H*-pyran-3-ol** | 430.2 | |

**Example A5**

Synthesis of the compound TA-30 of the present invention:

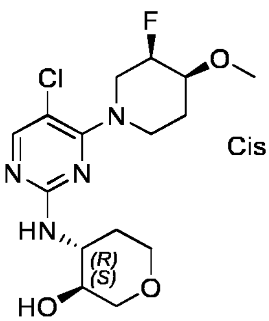

the synthesis route is as follows:

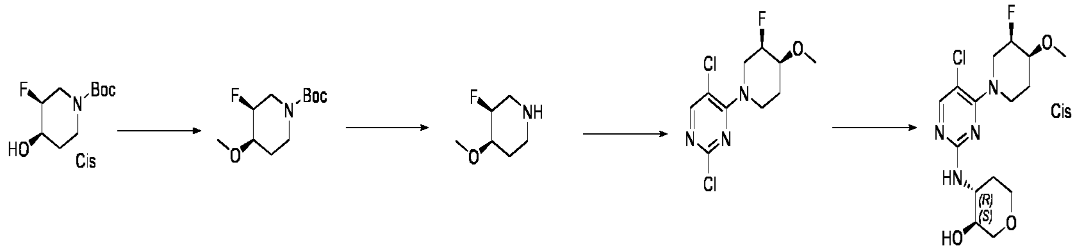

SM 1 SM 2 SM 3 SM 4 TA-30

The experimental procedure is as follows:

1. Synthesis of compound SM2:

Compound SM 1 (0.9 g, 1.0 e.q.) was solubilized in a solvent of ultra-dry DMF under nitrogen, placed in an ice-water bath, solid powder of sodium hydride (0.18 g, 1.1 e.q.) was added in batches, the reaction was carried out for fifteen minutes at controlled temperature, methyl iodide (0.64 g, 1.1 e.q.) was added, the reaction was carried out naturally at elevated temperature at the end of the dropwise addition, and at the end of the reaction, the reaction was quenched by the addition of ice-water. Extracted with EA, the organic phase was dried over anhydrous sodium sulfate, suction filtered and concentrated under reduced pressure, and purified by column chromatography to obtain compound SM 2 (0.85 g, 89%).

2. Synthesis of compound SM 3:

Compound SM 2 (0.85 g, 1.0 e.q.) was solubilized in ethyl acetate solvent under nitrogen, 4.0 M dioxane hydrochloride solution (9 mL, 10.0 e.q.) was added and the reaction was carried out at room temperature. At the end of the reaction, the reaction solution was concentrated under reduced pressure and used directly in the next step.

3. Synthesis of compound SM 4:

Compound SM 3 (0.49 g, 1.0 e.q.), 2,4,5-trichloropyrimidine (0.67 g, 1.0 e.q.) and DIPEA (1.9 g, 4.0 e.q.) were sequentially added to isopropanol solvent and the reaction solution was warmed up to 80 °C, at the end of the reaction, the reaction solution was cooled down to room temperature, extracted and partitioned with EA and water, and the organic phase was dried over anhydrous sodium sulfate, suction filtered and concentrated under reduced pressure and purified by column chromatography to obtain compound SM 4 (0.9 g, 59.4%), LCMS: $[M + H]^+$ = 280.0.

4. Synthesis of compound TA-30:

Compound SM 4 (0.1 g, 1.0 e.q.), (3s, 4r)-4-aminooxan-3-ol hydrochloride (0.083 g, 1.5 e.q.) and DIPEA (0.12 g, 2.5 e.q.) were added to DMSO solvent under nitrogen, and the reaction was heated up to 90 °C. After the reaction, the reaction solution was cooled down to room temperature, and then extracted and partitioned sequentially with saturated saline, water and EA. The organic phase was dried over anhydrous sodium sulfate, suction filtered and concentrated under reduced pressure, and purified by column chromatography to obtain the compound TA-30 (56 mg, 43.4%), LCMS: $[M + H]^+$ = 361.2, and the HPLC purity was 99.5%.
$^1$H NMR (400 MHz, Chloroform-d) & 7.85 (d, *J* = 2.0 Hz, 1H), 5.02 (s, 1H), 4.80 (ddd, *J* = 47.7, 7.2, 3.8 Hz, 1H), 4.28 - 4.12 (m, 1H), 4.04 (dd, *J*= 11.4, 5.0 Hz, 1H), 4.01 - 3.85 (m, 2H), 3.70 (dq, *J* = 7.4, 5.0, 3.7 Hz, 2H), 3.57 (dd, *J* = 9.6, 5.0 Hz, 3H), 3.49 - 3.45 (m, 3H), 3.45 - 3.28 (m, 2H), 3.15 (dd, *J* = 11.4, 9.8 Hz, 1H), 2.12 - 2.03 (m, 1H), 2.00 - 1.90 (m, 1H), 1.89 - 1.74 (m, 1H), 1.66 (qd, *J* = 12.1, 4.8 Hz, 1H).

Referring to the synthesis method of Example A5, the following compound was synthesized:

| Compound | Structure | LC-MS | NMR |
|---|---|---|---|
| **TA-31** | 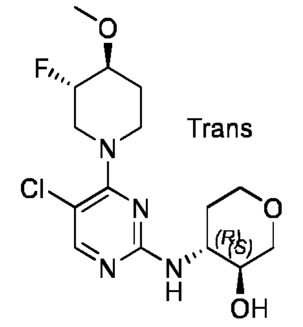  | 361.3 | |

**Example A6**

Synthesis of the compound TA-32 of the present invention:

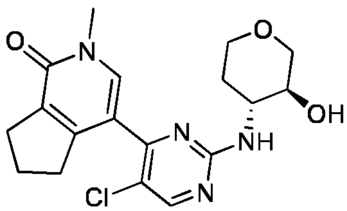

The Synthesis route is as follows:

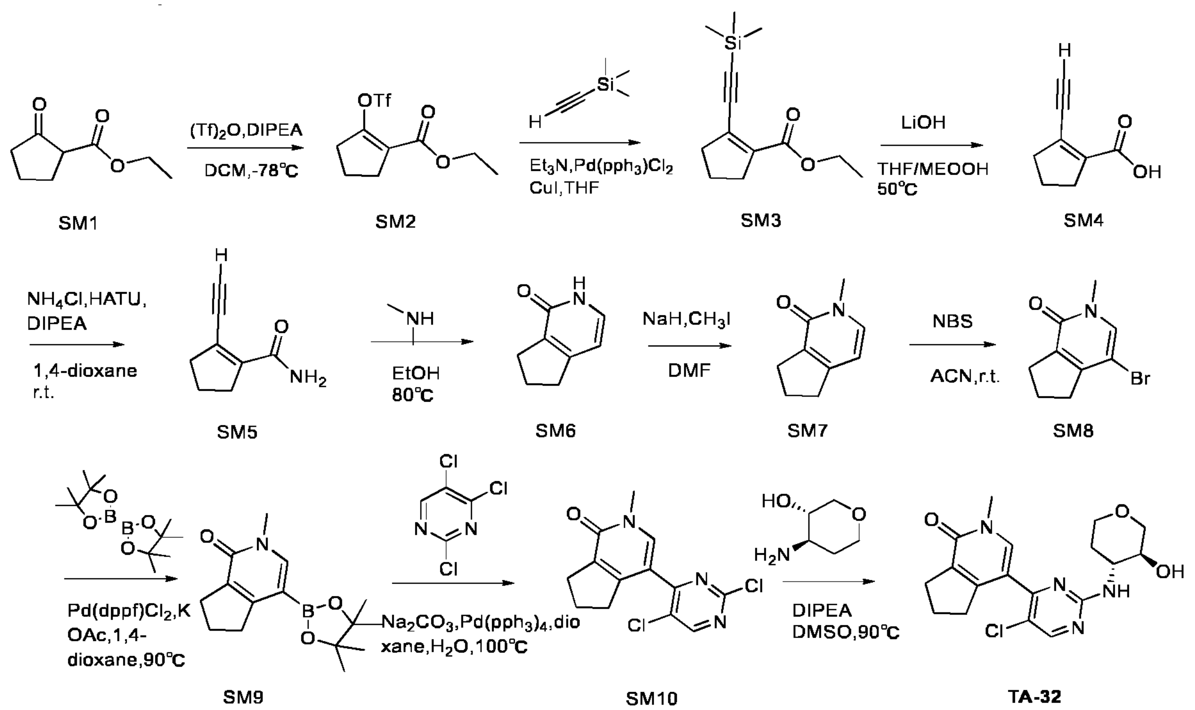


The experimental procedure is as follows:

1. Synthesis of compound SM2:

SM 1 (20.0g,1.0e.q.) was added into 1000ml three necked flask, DCM (500ml,25V) was added to dissolve it and then the solution was cooled down to -78°C using liquid nitrogen, DIPEA (110.82ml,5.3e.q.) was added, stirred for 10min and then $Tf_2O$ (25.83ml,1.2e.q.) was added slowly and then raised to room temperature. TLC showed that no raw material

remaining and the reaction was completed. The device was placed in an ice-water bath, quenched by slowly adding water (250 ml) to the reaction system, extracted with DCM and partitioned, the organic phase was washed twice with 5% aqueous citric acid and concentrated, and then separated and purified by silica gel column chromatography to obtain 35.58 g of light yellow oily SM 2, LCMS:[M+H]+=289, the yield was 96.4%.

2. Synthesis of compound SM3:

CuI (1.54g,0.066e.q.) and Pd(pph$_3$)Cl$_2$ (1.72g,0.02e.q.) were added to a 1000 ml triple-necked flask, a THF solution of SM 2 (35.32g,1.0e.q.) was added under $N_2$ protection, followed by the addition of THF solution of trimethylsilylacetylene (26.0 ml,1.5e.q.) and triethylamine ( 49.22 ml,2.89e.q.), a total of 500 ml of THF was added, nitrogen was replaced three times and the reaction was carried out at room temperature. TLC showed that no raw material remained and the reaction was completed. Extracted with saturated $NH_4Cl$ aqueous solution and ethyl acetate, the organic phase was dried and concentrated, and then separated and purified by silica gel column chromatography to obtain 30.66 g of brown oil SM 3 in 95% yield.

3. Synthesis of compound SM 4:

SM 3 (30.66g,1.0e.q.) was dissolved in THF/MEOH (600 ml, 10V:10V), added to 1000 ml three-necked flask, 1M aqueous lithium hydroxide (10.9g, 2.0e.q.) was added, and the reaction was carried out under nitrogen protection at 50°C. TLC showed that no raw material was remained, the reaction was completed, and the reaction solution was cooled down to room temperature. The system was concentrated to some extent and extracted with ethyl acetate and water, the aqueous phase was retained, the aqueous phase was adjusted pH to acidic with 2M HCl, extracted with ethyl acetate, the organic phase was dried and concentrated to give 15.3 g of orange solid SM 4 in 86.6% yield.

4. Synthesis of compound SM5:

SM 4 (15.3g,1.0e.q.), NH4Cl (30.04g,5.0e.q.), HATU (64.07g, 1.5e.q.) were added to 1000 ml triple-necked flask, 1,4-Dioxane (300 ml) was added, DIPEA (58.7 ml,3.0e.q.) was added, and the reaction was carried out at room temperature under nitrogen protection. TLC showed no raw material was remained and the reaction was completed. The system was suction filtered by diatomaceous earth, the filter cake was washed with appropriate amount of DCM, extracted with DCM and water, the organic phase was dried and concentrated, and then separated and purified by silica gel column chromatography to obtain 18.7 g of yellow solid SM 5, LCMS: $[M+H]^+$=136.00, the yield was 95%.

5. Synthesis of compound SM 6:

SM 5 (18.6g,1.0e.q.) was added to a 2000 ml three-necked flask, 2.0M ethanol solution of dimethylamine (688 ml, 10.0e.q.) was added, protected by nitrogen, and the temperature was slowly raised to 80°C. A large amount of gas was generated in the earlier reaction stage, and the temperature could be appropriately cooled down or the flow of condensate could be appropriately increased. TLC showed no raw material was remained, and the reaction was completed and the reaction solution was cooled down to room temperature. The system was concentrated directly and separated and purified by silica gel column chromatography to obtain 13.6 g of yellow-brown solid SM 6, LCMS: $[M+H]^+$=136.30, the yield was 73%.

6. Synthesis of compound SM7:

SM6 (520 mg,1.0 e.q.) was added to a 50 ml three-necked flask, solubilized by adding DMF (10 ml), after nitrogen displacement for three times, the reaction solution was cooled down to 0°C in ice-water bath, NaH (313 mg, 1.2 e.q.) with 60% content was added in batches, the reaction was carried out for 30 min at 0°C, and under the condition of nitrogen protection, $CH_3I$ (0.292 ml,1.2 e.q.) was slowly added to the system, followed by natural rewarming of the reaction to room temperature. TLC showed no raw material was remained and the reaction was completed. The reaction was quenched by the addition of ice water to the system. Extracted with appropriate amounts of ethyl acetate and water, the organic phase was dried and concentrated, and purified by silica gel column chromatography to obtain 344 mg of SM7 as a white solid, LCMS: $[M+H]^+$ = 150.30, yield 59.9%.

7. Synthesis of compound SM8:

SM 7 (344 mg,1.0 e.q.) was added to 100 ml single-necked flask, solubilized by adding 10 ml of ACN, and then NBS (493 mg,1.2 e.q.) was added, the reaction was carried out at room temperature under nitrogen protection. TLC

showed no raw material was remained and the reaction was completed. The reaction was extracted with ethyl acetate and water, the organic phase was dried and concentrated, and purified by silica gel column chromatography to obtain 400 mg of white solid SM 8, LCMS: $[M+H]^+$ = 150.30, yield 76.2%.

8. Synthesis of compound SM 9:

SM 8 (400 mg,1.0 e.q.), pinacol bis(boronic acid) ester (950 mg,2.0 e.q.), Pd(dppf)$Cl_2$ (68 mg,0.05 e.q.), KOAc (550 mg,3.0 e.q.) were added sequentially into 100 ml three-necked flask, nitrogen was replaced for 3 times and then 1,4-Dioxane (10 ml) was added. The reaction was warmed up to 90°C. TLC showed no raw material was remained, the reaction was completed and the reaction solution was cooled to room temperature. The reaction was extracted with ethyl acetate and water, the organic phase was dried and concentrated, and purified by silica gel column chromatography to obtain 150 mg of SM 9 as a white solid, LCMS: $[M+H]^+$=276.5, with a yield of 29%.

9. Synthesis of compound SM10:

SM9 (150 mg, 1.0e.q.) was added to a 50 ml three-necked flask, 1,4-Dioxane/$H_2O$ (10V:3V) was added, sodium carbonate (174 mg, 3.0e.q.) and 2,4,5-trichloropyrimidine (0.082 ml, 1.3e.q.) were added, nitrogen displacement for 3 times, Pd$(pph_3)_4$ (63 mg. 0.1e.q.) was added, nitrogen was displaced three times, warmed to 100 °C and reacted for 3h. TLC showed no raw material was remained, the reaction was completed, the reaction solution was cooled to room temperature. The reaction was extracted with ethyl acetate and water, the organic phase was dried and concentrated, and separated and purified by silica gel column chromatography to obtain 60 mg of SM10 as a white solid, LCMS: $[M+H]^+$=298.5, with a yield of 37.3%.

10. Synthesis of compound TA-32:

SM10 (60 mg, 1.0 e.q.) was solubilized with DMSO (2 ml), (3S,4R)-4-amino oxacyclohexan-3-ol hydrochloride (47 mg, 1.5 e.q.) was added, DIPEA (0.145 ml, 4.0 e.q.) was added, and the reaction was carried out under nitrogen protection by raising the temperature to 90°C. TLC showed that no raw material was remained, the reaction was completed, and the reaction solution was cooled to room temperature. The reaction was extracted with ethyl acetate and water, the organic phase was dried and concentrated, and purified by silica gel column chromatography to obtain 37 mg of TA-32 as a white solid, LC-MS: $[M+H]^+$=377.0, yield 44%.
$^1$H NMR (400 MHz, Chloroform-d) δ 8.27 (s, 1H), 7.53 (s, 1H), 4.05 (dd, *J* = 12.0, 4.0 Hz, 1H), 4.00 - 3.94 (m, 1H), 3.86 - 3.77 (m, 1H), 3.64 (t, *J* = 8.0 Hz, 2H), 3.60 (s, 3H), 3.46 (td, *J* = 12.0, 2.4 Hz, 1H), 3.18 (dd, *J* = 12.0, 8.0 Hz, 1H), 2.96 (m, 1H), 2.88 (t, *J* = 8.0 Hz, 3H).

Example A7

compound TA-33 synthesized in the present invention:

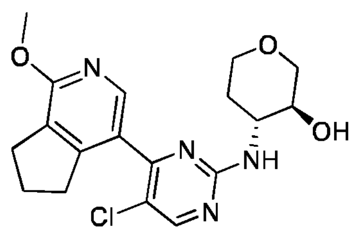

The synthesis route is as follows:

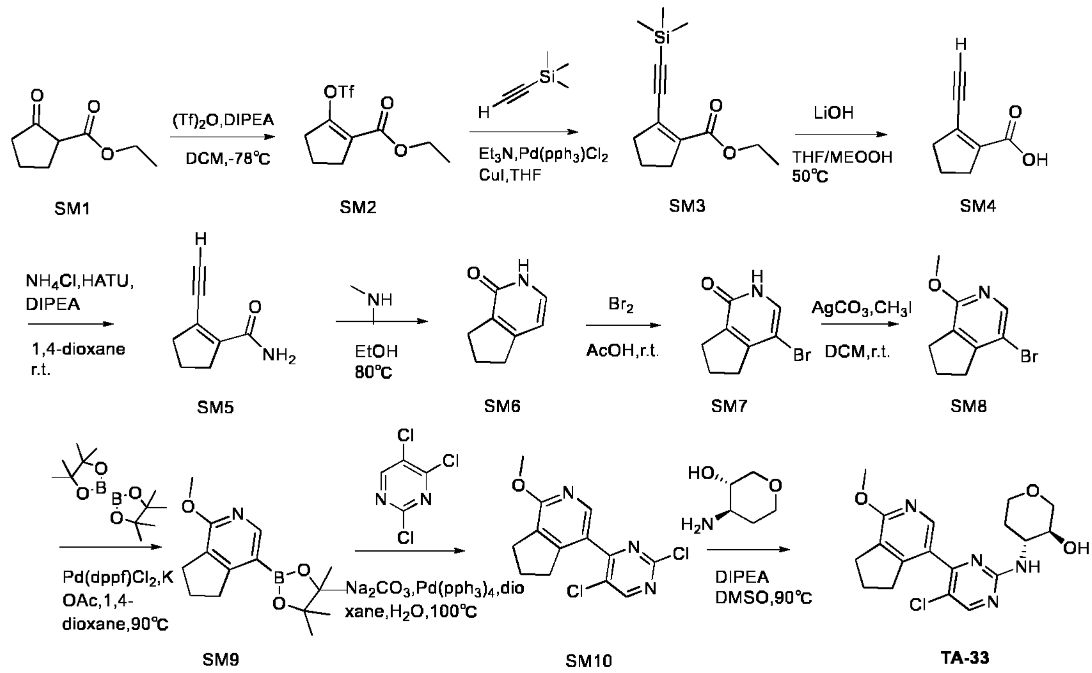


The experimental procedure is as follows:

1. Synthesis of compound SM7:

SM6 (5g,1.0e.q.) (refer to synthetic route **1,** Stepl-Step5) was added into 250ml three-necked flask, solubilized by adding 50ml acetic acid, liquid bromine (2.35ml,1.2e.q.) was added, and the reaction was carried out under nitrogen at room temperature for 2 h. The reaction was completed when TLC showed no raw material was remained. After concentration under reduced pressure, the reaction solution was purified by silica gel column chromatography, 7.25 g of yellow solid SM7 was obtained, LCMS: $[M+H]^+$=213.9/216.1, yield 91.5%.

2. Synthesis of compound SM8:

SM7(5g,1.0e.q.) was added into 250ml three-necked flask, silver carbonate(9.0g,1.4e.q.) was added, 150ml dichloromethane was added, nitrogen was displaced three times, iodomethane(15ml,10e.q.) was added, and the reaction was carried out at 37°C for 3h.The reaction was completed when TLC showed no raw material was remained, and the reaction solution was cooled to room temperature. The system was suction filtered by diatomaceous earth, the filter cake was washed with appropriate amount of dichloromethane, the filtrate was concentrated under reduced pressure and then separated and purified by silica gel column chromatography, 3.47g of white solid SM8 was obtained, the yield was 65%.

3. Synthesis of compound SM9:

SM8 (3.0g,1.0e.q.), pinacol bis(boronic acid) ester (8.34g,2.5e.q.), Pd(dppf)$Cl_2$ (480mg,0.05e.q.), KOAc (3.9g,3.0e.q.) were added to 100 ml three-necked flask in sequence, nitrogen was pumped three times and then 1,4-Dioxane (40 ml) was added. The reaction was warmed up to 90°C. TLC showed no raw material was remained, the reaction was completed and the reaction solution was cooled to room temperature. The reaction was extracted with ethyl acetate and water, the organic phase was dried and concentrated, separated and purified by silica gel column chromatography to obtain 3.9 g of white solid SM9, LCMS: $[M+H]^+$=276.00, the yield was 100%.

4. Synthesis of compound SM10:

SM9 (350 mg, 1.0e.q.) was added to a 50 ml three-necked flask, 1,4-Dioxane/$H_2O$ (10V:3V) was added, sodium carbonate (405 mg, 3.0e.q.) and 2,4,5-trichloropyrimidine (0.19 ml, 1.3e.q.) were added, nitrogen was displaced three times, Pd($pph_3$)$_4$ (147 mg. 0.1e.q.) was added, nitrogen was displaced three times, warmed to 100 °C and reacted for 3h. TLC showed no raw material was remained, the reaction was completed, the reaction solution was cooled to room temperature. The reaction solution was extracted with ethyl acetate and water, the organic phase was dried and concentrated, separated and purified by silica gel column chromatography to obtain 100 mg of white solid SM10, LCMS:

[M+H]+=295.9/297.9, the yield was 26.6%.

5. Synthesis of compound TA-33:

SM10 (100 mg,1.0e.q.) was solubilized with DMSO (2 ml), (3S,4R)-4-amino oxacyclohexan-3-ol hydrochloride (78 mg,1.5e.q.) was added, DIPEA (0.24 ml, 4.0e.q.) was added and the reaction was carried out under nitrogen protection by raising the temperature to 90°C overnight. The reaction was completed when the TLC showed no raw material was remained, and the reaction solution was cooled to room temperature. The reaction was extracted with ethyl acetate and water, the organic phase was washed twice with saturated aqueous sodium chloride solution and dried over anhydrous sodium sulfate, concentrated and purified by silica gel column chromatography to obtain 68 mg of white solid TA-33, LCMS: [M+H]+=377.10, yield 53.5%.

$^1$H NMR (400 MHz, Chloroform-d) δ 8.29 (s, 1H), 8.15 (s, 1H), 5.35 - 5.22 (m, 1H), 4.81 (m, 1H), 4.01 (m, 5H), 3.79 (m, 1H), 3.60 (m, 1H), 3.44 (t, *J* = 12.0 Hz, 1H), 3.15 (t, *J* = 8.0 Hz, 1H), 2.99 (m, 2H), 2.89 (d, *J* = 4.0 Hz, 3H), 2.18 - 2.07 (m, 2H), 2.06 - 1.98 (m, 1H).

Referring to the synthesis methods of Example A6 and Example A7, the following compounds were synthesized:

| Compound | Structure | LC-MS | NMR |
|---|---|---|---|
| TA-34 | 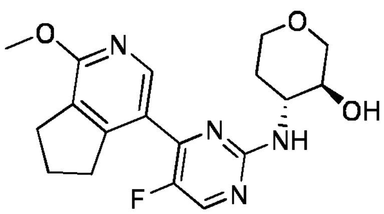 **(3*S*,4*R*)-4-((5-fluoro-4-(1-methoxy-6,7-dihydro-5*H*-cyclopenta[c]pyridin-4-yl)pyrimidin-2-yl)amino)tetrahydro-2*H*-pyran-3-ol** | [M+H]+=361.2 | $^1$H NMR (400 MHz, Chloroform-d) δ 8.31 - 8.30 (m, 1H), 8.19 (d, *J* = 2.7 Hz, 1H), 5.23 (d, *J* = 6.0 Hz, 1H), 4.07 - 4.04 (m, 1H), 4.02 (s, 3H), 4.00 - 3.95 (m, 1H), 3.84 - 3.75 (m, 1H), 3.64 - 3.57 (m, 1H), 3.45 (td, *J* = 12.0, 4.0 Hz, 1H), 3.17 (dd, *J* = 12.0, 8.0 Hz, 1H), 3.11 (t, *J* = 8.0 Hz, 1H), 3.00 (m, 1H), 2.90 - 2.85 (m, 2H), 2.15 - 2.08 (m, 2H), 2.05 (m, 1H), 2.02 (m, 1H), 1.69 (dd, *J* = 12.0, 4.0 Hz, 1H). |
| TA-35 | 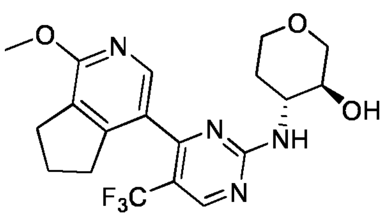 | [M+H]+=411.2 | $^1$H NMR (400 MHz, Chloroform-d) δ 8.59 (s, 1H), 7.94 (s, 1H), 5.62 (s, 1H), 4.00 (s, 3H), 3.96 (d, *J* = 4.0 Hz, 2H), 3.66 - 3.59 (m, 1H), 3.45 (m, 1H), 3.17 (m, 1H), 2.91 - 2.86 (m, 2H), 2.81 (q, *J* = 8.0 Hz, 2H), 2.10 (m, 3H), 2.06 - 2.01 (m, 1H), 1.69 (qd, *J* = 12.0, 4.0 Hz, 2H). |
| TA-36 | 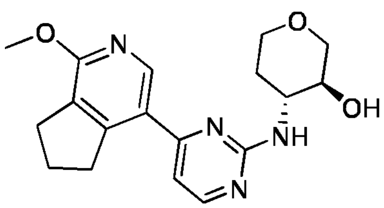 **(3*S*,4*R*)-4-((4-(1-methoxy-6,7-dihydro-5*H*-cyclopenta[*c*]pyridin-4-yl)pyrimidin-2-yl)amino)tetrahydro-2*H*-pyran-3-ol** | [M+H]+=343.5 | |

(continued)

| Compound | Structure | LC-MS | NMR |
|---|---|---|---|
| TA-37 | 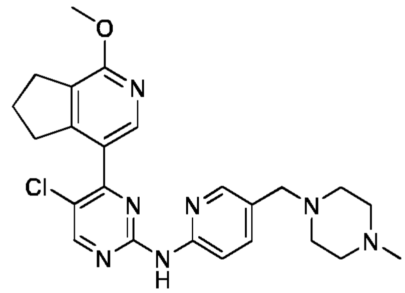 **5-chloro-4-(1-methoxy-6,7-dihydro-5*H*-cyclopenta[*c*]pyridin-4-yl)-*N*-(5-((4-methylpiperazin-1-yl)methyl)pyridin-2-yl)pyrimidin-2-amine** | $[M+H]^+$=466.3 | $^1$H NMR (400 MHz, Chloroform-*d*) δ 8.55 (s, 1H), 8.53 (s, 1H), 8.29 (dd, *J* = 8.0, 0.8 Hz, 1H), 8.26 (d, *J* = 4.0Hz, 2H), 7.63 (dd, *J* = 8.0, 4.0 Hz, 1H), 4.04 (s, 3H), 3.50 (s, 2H), 3.01 - 2.95 (m, 2H), 2.94 - 2.88 (m, 2H), 2.64 (s, 6H), 2.45 (s, 3H), 2.11 (p, *J* = 8.0Hz, 3H). |
| TA-38 | 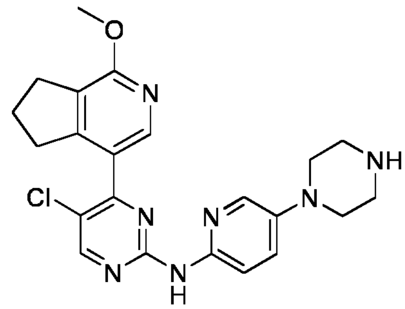 **5-chloro-4-(1-methoxy-6,7-dihydro-5*H*-cyclopenta[c]pyridin-4-yl)-*N*-(5-(piperazin-1-yl) pyridin-2-yl)pyrimidin-2-amine** | $[M+H]^+$=438.2 | |

**Example A8**

Synthesis of the compound TA-39 of the present invention:

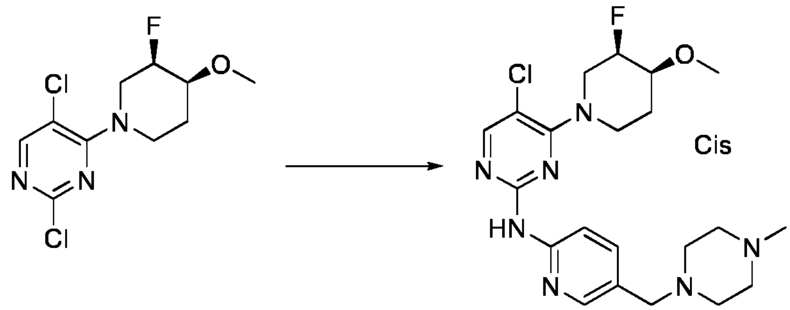

**SM 1** **TA-39**

The experimental procedure was as follows:

5-[(4-methylpiperazin-1-yl)methyl]pyridin-2-amine (100 mg, 1.0 e.q.) was dissolved in 3 mL of tetrahydrofuran solvent under nitrogen, cooled down to 0 °C in an ice-water bath, 1.0 M tetrahydrofuran solution of LiHDMS (0.73 mL, 1.5 e.q.) was slowly added, and the temperature of the reaction solution was held for half an hour and then compound SM 1 was added, and the temperature of the reaction solution was continued to be held for half an hour. After TLC monitor showed that the reaction was ended, quenched by adding appropriate amount of ice water, extracted and partitioned with ethyl acetate and water. The organic phase was dried over anhydrous sodium sulfate, after suction filtration, the organic phase was concentrated, and 50 mg of the compound TA-39 was obtained by isolation and purification by silica gel column chromatography, LCMS: $[M + H]^+$ = 450.5, and the purity of HPLC was 98.31%.

$^1$H NMR (400 MHz, Chloroform-d) $\delta$ 8.12 (d, *J* = 12.0 Hz, 1H), 8.10 (d, *J* = 1.6 Hz, 1H), 7.56 (dd, *J* = 8.0, 1.6 Hz, 1H), 7.36 (d,

*J* = 12.0 Hz, 1H), 6.83 (d, *J* = 8.0 Hz, 1H), 4.78 (dt, *J* = 48.0, 4.0 Hz, 1H), 4.21 - 4.07 (m, 1H), 3.92 (brs, 1H), 3.60 (m, 2H), 3.49 (s, 2H), 3.46 (s, 3H), 3.44 - 3.35 (m, 1H), 2.83 (s, 4H), 2.69 (s, 4H), 2.55 (s, 3H), 2.03 (m, 1H), 1.88 - 1.79 (m, 1H).

Referring to the synthesis method of Example A8, the following compound was synthesized:

| Compound | Structure | LC-MS | NMR |
|---|---|---|---|
| TA-40 | 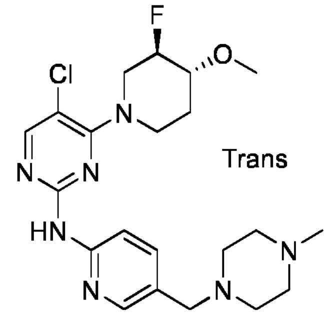 Trans | 450.5 | |

### Example A9

Synthesis of the compound TA-32 of the present invention:

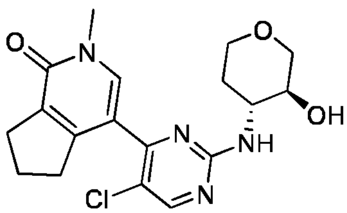

The synthesis route is as follows:

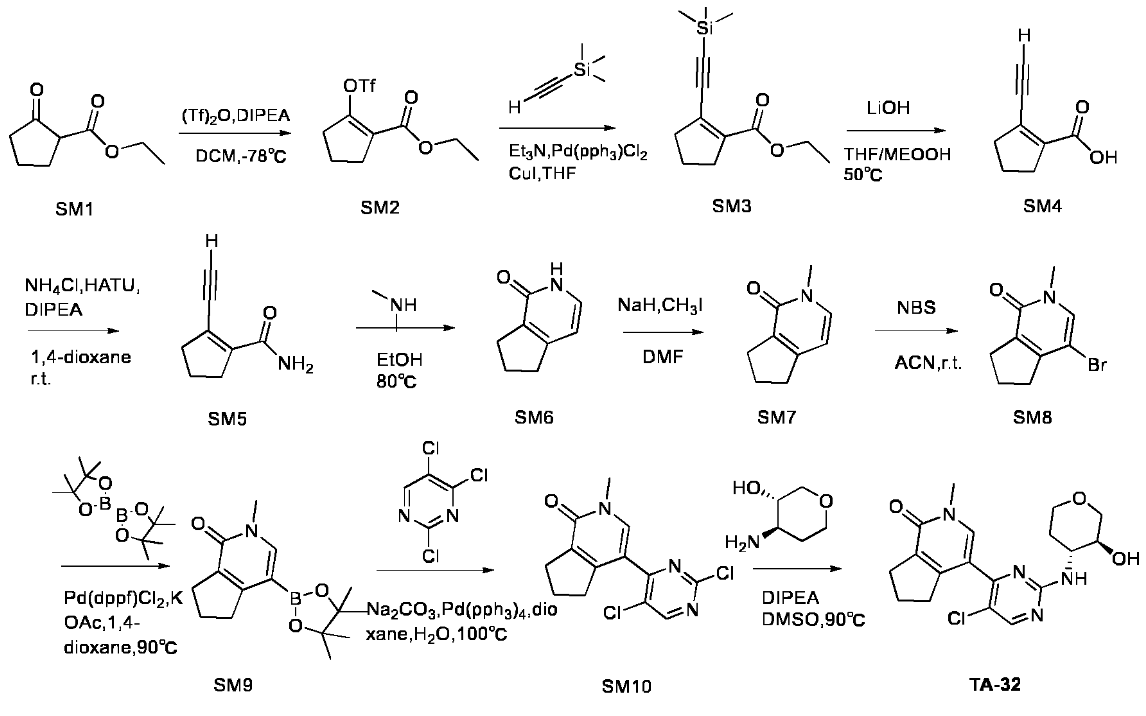


The experimental procedure is as follows:

Step 1:

SM 1 (20.0g,1.0e.q.) was added to 1000ml three-necked flask, solubilized by adding DCM (500ml,25V), the reaction solution was cooled down to -78 °C using liquid nitrogen, DIPEA (110.82 ml,5.3e.q.) was added, after stirring for 10min, $Tf_2O$ (25.83 ml,1.2e.q.) was added slowly, and then the reaction solution was brought to room temperature. TLC showed no raw material was remained and the reaction was completed. The device was placed in an ice-water bath, quenched by slowly adding water (250 ml) to the reaction system, extracted and partitioned with DCM, the organic phase was washed twice with 5% aqueous citric acid and concentrated, and then separated and purified by silica gel column chromatography to obtain 35.58 g of light yellow oily SM 2, LCMS:[M+H]+=289, the yield was 96.4%.

Step 2:

CuI (1.54g,0.066e.q.) and $Pd(pph_3)Cl_2$ (1.72g,0.02e.q.) were added to a 1000 ml three-necked flask, and a THF solution of SM 2 (35.32g,1.0e.q.) was added under the protection of $N_2$, followed by the addition of THF solution of trimethylsilyl acetylene (26.0ml,1.5e.q.) and triethylamine ( 49.22 ml,2.89e.q.), a total of 500 ml of THF was added, nitrogen was replaced three times and the reaction was carried out at room temperature. TLC showed no raw material was remained and the reaction was completed. Extracted with saturated $NH_4Cl$ aqueous solution and ethyl acetate, the organic phase was dried and concentrated, and then separated and purified by silica gel column chromatography to obtain 30.66 g of brown oil SM 3 in 95% yield.

Step 3:

SM 3 (30.66 g, 1.0 e.q.) was dissolved in THF/MEOH (600 ml, 10V:10V), added to a 1000 ml three-necked flask, 1M aqueous lithium hydroxide (10.9 g, 2.0 e.q.) was added, and the reaction was carried out under nitrogen protection at 50 °C. TLC showed no raw material was remained, the reaction was completed, and cooled down to room temperature. The system was concentrated to some extent and extracted with ethyl acetate and water, the aqueous phase was retained, the aqueous phase was adjusted pH to be acidic with 2M HCl, extracted with ethyl acetate, the organic phase was dried and concentrated to obtain 15.3 g of orange solid SM 4 in 86.6% yield.

Step 4:

SM 4 (15.3 g, 1.0 e.q.), $NH_4Cl$ (30.04 g, 5.0 e.q.), HATU (64.07 g, 1.5 e.q.) were added to 1000 ml three-necked flask, 1,4-Dioxane (300 ml) was added, DIPEA (58.7 ml, 3.0 e.q.) was added, and the reaction was carried out at room temperature under nitrogen protection. TLC showed no raw material was remained and the reaction was completed. The system was suction filtered by diatomaceous earth, the filter cake was washed with appropriate amount of DCM, extracted with DCM and water, the organic phase was dried and concentrated, and then separated and purified by silica gel column chromatography to obtain 18.7 g of yellow solid SM 5, LCMS: $[M+H]^+$=136.00, the yield was 95%.

Step 5:

SM 5 (18.6 g,1.0 e.q.) was added to a 2000 ml three-necked flask, 2.0 M ethanol solution of dimethylamine (688 ml, 10.0 e.q.) was added, under nitrogen protection, the temperature was slowly raised to 80 °C. A large amount of gas was generated in the earlier reaction stage, and the temperature could be appropriately cooled down or the flow of condensate could be appropriately increased. TLC showed no raw material was remained, the reaction was completed, and cooled down to room temperature. The system was concentrated directly and purified by silica gel column chromatography to obtain 13.6 g of yellow-brown solid SM 6, LCMS: $[M+H]^+$=136.30,yield 73%.

Step 6:

SM 6 (520 mg, 1.0 e.q.) was added to a 50 mL three-necked flask, solubilized by adding DMF (10 ml), and after three nitrogen exchanges, the reaction solution was cooled down to 0 °C in the ice-water bath, and 60% NaH (313 mg, 1.2 e.q.) was added in batches, and the reaction was carried out at 0 °C for 30 min, and under the protection of nitrogen, $CH_3I$ (0.292 ml, 1.2 e.q.) was added to the system slowly, followed by natural rewarming to room temperature. TLC showed no material was remained and the reaction was completed. The reaction was quenched by the addition of ice water to the system. Extracted with appropriate amounts of ethyl acetate and water, the organic phase was dried and concentrated, and purified by silica gel column chromatography to obtain 344 mg of SM 7 as a white solid, LCMS: $[M+H]^+$ = 150.30, yield 59.9%.

Step 7:

SM 7 (344 mg,1.0 e.q.) was added to a 100 ml single-necked flask, solubilized by adding 10 mL of acetonitrile solvent, and then NBS (493 mg,1.2 e.q.) was added, the reaction was carried out at room temperature under nitrogen protection. TLC showed no raw material was remained and the reaction was completed. The reaction was extracted with ethyl acetate and water, the organic phase was dried and concentrated, and purified by silica gel column chromatography to obtain 400 mg of SM 8 as a white solid, LCMS: $[M+H]^+$ = 150.30, yield 76.2%.

Step 8:

SM 8 (400 mg, 1.0 e.q.), pinacol bis(boronic acid) ester (950 mg, 2.0 e.q.), $Pd(dppf)Cl_2$ (68 mg, 0.05 e.q.), and KOAc (550 mg, 3.0 e.q.) were added sequentially to a 100 mL three-necked flask, and after displacing nitrogen three times, 1,4-Dioxane (10 ml) was added, and the reaction was warmed to 90 °C. TLC showed no raw material was remained, the reaction was completed and the reaction solution was cooled to room temperature. The reaction solution was extracted with ethyl acetate and water, the organic phase was dried and concentrated, and purified by silica gel column chromatography to obtain 150 mg of SM 9 as a white solid, LCMS: $[M+H]^+$=276.5, yield 29%.

Step 9:

SM 9 (150 mg, 1.0 e.q.) was added to a 50 mL three-necked flask, 1,4-Dioxane and $H_2O$ (10V:3V) were added, and sodium carbonate (174 mg, 3.0 e.q.) and 2,4,5-trichloropyrimidine (0.082 mL, 1.3 e.q.) were added, nitrogen was displaced three times, and $Pd(pph_3)_4$ (63 mg, 0.1 e.q.) was added, nitrogen was displaced three times, the reaction solution was warmed to 100 °C and reacted for 3 h. TLC showed no raw material was remained, and the reaction was completed, the reaction solution was cooled to room temperature. The reaction solution was extracted with ethyl acetate and water, the organic phase was dried and concentrated, and purified by silica gel column chromatography to obtain 60 mg of SM 10 as a white solid, LCMS: [M+H]+=298.5, with a yield of 37.3%.

Step 10:

SM 10 (60 mg, 1.0 e.q.) was solubilized with DMSO (2 ml), (3S,4R)-4-amino oxacyclohexan-3-ol hydrochloride (47 mg, 1.5 e.q.) was added, DIPEA (0.145 mL, 4.0 e.q.) was added, and the reaction was heated under nitrogen protection to 90 °C. TLC showed no raw material was remained, and the reaction was completed, the reaction solution was cooled to room temperature. The reaction solution was extracted with ethyl acetate and water, the organic phase was dried and concentrated, and 37 mg of white solid TA-32 was obtained by silica gel column chromatography, LC-MS: $[M+H]^+$ = 377.0, the yield was 44%.

$^1$H NMR (400 MHz, Chloroform-d) $\delta$ 8.27 (s, 1H), 7.53 (s, 1H), 4.05 (dd, *J*= 12.0, 4.0 Hz, 1H), 4.00 - 3.94 (m, 1H), 3.86 - 3.77 (m, 1H), 3.64 (t, *J* = 8.0 Hz, 2H), 3.60 (s, 3H), 3.46 (td, *J* = 12.0, 2.4 Hz, 1H), 3.18 (dd, *J* = 12.0, 8.0 Hz, 1H), 2.96 (m, 1H), 2.88 (t, *J* = 8.0 Hz, 3H).

## Example B1

Synthesis of the compound TB-1 of the present invention:

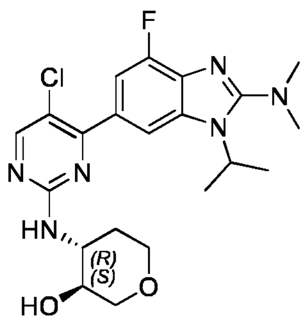

The synthesis route is as follows:

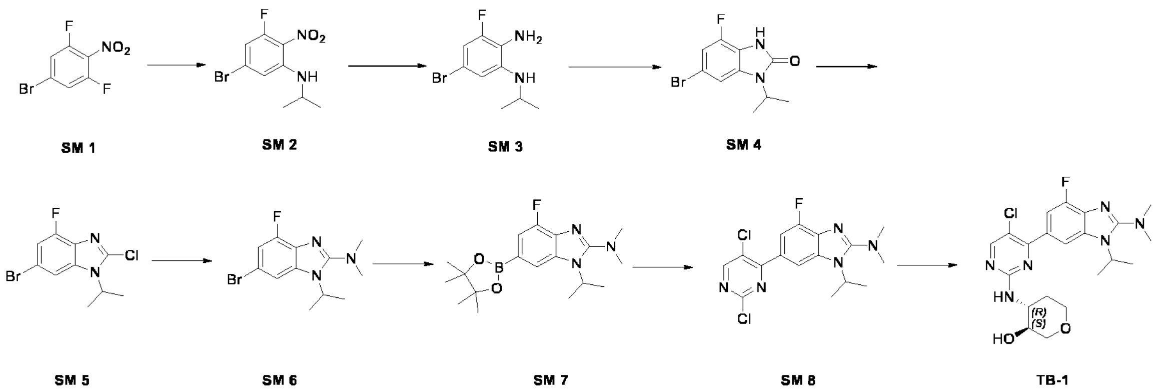

The experimental procedure is as follows:

Step 1:

Compound SM 1 (1.0 g, 1.0 e.q.) was solubilized in ultra-dry tetrahydrofuran solvent, cooled down in an ice-water bath, cesium carbonate solid (1.37 g, 1.0 e.q.) was added, and isopropylamine (0.25 g, 1.0 e.q.) was added dropwise under stirring conditions. After the dropwise addition, the reaction was heated **up** naturally. TLC monitor showed that the reaction was ended, the solid was removed by suction filtration, the filter cake was washed with appropriate amount of EA, extracted and separated by adding water, the organic phase was dried over anhydrous sodium sulfate, suction filtered and concentrated under reduced pressure, and purified by column chromatography to obtain SM 2 (750 mg, 64.7%) as a yellow solid.

$^1H$ NMR (400 MHz, Chloroform-d) δ 6.76 (t, *J* = 1.9 Hz, 1H), 6.57 (dd, *J* = 10.9, 2.0 Hz, 1H), 3.73 (dq, *J*= 13.0, 6.5 Hz, 1H), 1.31 (d, *J*= 6.3 Hz, 6H).

Step 2:

Compound SM 2 (0.75 g, 1.0 e.q.) was dissolved in a mixed solvent of anhydrous ethanol and water (v: v = 4:1), iron powder (0.91 g, 6.0 e.q.) and ammonium chloride solid (0.29 g, 2.0 e.q.) were added sequentially, and the reaction was heated to 90 °C. At the end of the reaction, most of the solvent was removed by concentration under reduced pressure, extracted and partitioned with saturated sodium bicarbonate solution and EA, the organic phase was dried over anhydrous sodium sulfate, suction filtered and concentrated under reduced pressure, and purified by column chromatography to obtain compound SM 3 (0.5 g, 74.7%).

$^1H$ NMR (400 MHz, Chloroform-d) *δ* 6.64 (dd, *J* = 9.4, 2.1 Hz, 1H), 6.53 (t, *J* = 1.8 Hz, 1H), 3.56 (p, *J* = 6.3 Hz, 1H), 3.50 (s, 1H), 3.15 (s, 2H), 1.23 (d, *J* = 6.2 Hz, 6H).

Step 3:

Compound SM 3 (1.3 g, 1.0 e.q.) and CDI (2.2 g, 2.5 e.q.) were sequentially dissolved in ultra-dry DMF solvent, the reaction was heated up to 110 °C. TLC monitor showed that the reaction was ended, then the reaction solution was cooled down to room temperature, extracted and partitioned with EA and water, and the organic phase was dried over anhydrous sodium sulphate, suction filtered, then concentrated under reduced pressure, and isolated and purified by column chromatography to obtain compound SM 4 (1.2 g, 83.3%)

$^1H$ NMR (400 MHz, Chloroform-d) *δ* 9.82 (s, 1H), 7.08 (s, 1H), 7.02 (dd, *J* = 8.0, 4.0 Hz, 1H), 4.70 (m, 1H), 1.53 (d, *J* = 8.0 Hz, 6H).

Step 4:

Compound SM 4 (1.2 g, 1.0 e.q.) was dissolved in dry toluene, phosphorus oxychloride (7.1 g, 1.0 e.q.) was added, and the reaction was heated up to 90 °C for two days, and at the end of the reaction, the reaction was cooled down to room temperature, quenched by pouring it into iced water, and extracted and partitioned with EA, and the organic phase was dried over anhydrous sodium sulfate, suction filtered, and then concentrated under reduced pressure, and separated and purified by column chromatography to obtain compound SM 5 ( 1.2 g, 94%).

$^1H$ NMR (400 MHz, Chloroform-d) *δ* 7.45 (d, *J* = 2.0 Hz, 1H), 7.14 (dd, *J* = 8.0, 2.0 Hz, 1H), 4.88 (m, 1H), 1.64 (d, *J* = 8.0 Hz, 6H).

Step 5:

Compound SM 5 (1.23 g, 1.0 e.q.) was dissolved in ultra-dry DMF, dimethylamine hydrochloride (3.5 g, 10.0 e.q.) and triethylamine (8.8 g, 20.0 e.q.) were added sequentially, and the reaction was heated to 110 °C. At the end of the reaction, the reaction solution was cooled to room temperature, extracted and partitioned with EA and water, the organic phase was dried over anhydrous sodium sulfate, suction filtered and concentrated under reduced pressure, and isolated and purified by column chromatography to obtain the compound SM 6 (1.2 g, 94.5%), LCMS: $[M + H]^+$ = 299.9, 301.8.

Step 6:

Compound SM 6 (0.27 g, 1.0 e.q.), pinacol bis(boronic acid) ester (0.46 g, 2.0 e.q.), Pd(dppf)$Cl_2$ (33 mg, 0.05 e.q.) and potassium acetate (0.26 g, 3.0 e.q.) were added sequentially to a dry 50 mL three-necked flask under nitrogen protection, after nitrogen displacement three times, and ultra-dry 1,4- Dioxane solvent was added, the reaction solution was warmed to reflux reaction, at the end of the reaction, the reaction solution was cooled to room temperature, extracted and partitioned with Ea and water, the organic phase was dried over anhydrous sodium sulfate, suction filtered and concentrated under reduced pressure, and purified by column chromatography to obtain the compound SM 7 (0.3 g, 97%), LCMS: $[M + H]^+$ = 348.3.

Step 7:

Under nitrogen protection, compound SM 7 (0.39 g, 1.0 e.q.), 2,4,5-trichloropyrimidine (0.27 g, 1.3 e.q.), palladium tetrakis(triphenylphosphine) (0.13 g, 0.1 e.q.), and sodium carbonate solid (0.36 g, 3.0 e.q.) were sequentially added to a 50 mL three-necked flask, and a mixture of 1,4-dioxane and water (v. v = 10: 3) was added, after nitrogen displacement three times, warmed to reflux, at the end of the reaction, cooled to room temperature, extracted and partitioned with EA and water, the organic phase was dried over anhydrous sodium sulfate, suction filtered and concentrated under reduced pressure, and purified by column chromatography to obtain compound SM 8 (0.2 g, 49%), LCMS: $[M + H]^+$ = 368.2.

Step 8:

Compound SM 8 (0.17 g, 1.0 e.q.), (3s, 4r)-4-aminooxan-3-ol hydrochloride (0.14 g, 2.0 e.q.) and DIPEA (0.24 g, 4.0 e.q.) were sequentially added to DMSO solvent under nitrogen, and the reaction was heated up to 90 °C. After the reaction was completed, the reaction solution was cooled down to room temperature, extracted and partitioned with saturated saline, water and EA, and the organic phase was dried over anhydrous sodium sulfate, suction filtered and concentrated under reduced pressure, and purified by column chromatography to obtain the compound TB-1 (90 mg, 43.3%), with a purity of 99.7% by HPLC, and an LCMS: $[M + H]^+$ = 449.4.

$^1$H NMR (400 MHz, Chloroform-d) $\delta$ 8.30 (s, 1H), 7.71 (d, *J* = 1.4 Hz, 1H), 7.41 (dd, *J* = 11.4, 1.4 Hz, 1H), 5.29 (d, *J* = 6.0 Hz, 1H), 4.98 (s, 1H), 4.68 (h, *J* = 7.0 Hz, 1H), 4.02 (ddd, *J = 27.1,* 11.7, 4.7 Hz, 2H), 3.92 - 3.77 (m, 1H), 3.63 (td, *J* = 9.5, 4.9 Hz, 1H), 3.46 (td, *J* = 11.9, 2.2 Hz, 1H), 3.17 (dd, *J* = 11.4, 9.8 Hz, 1H), 2.98 (s, 6H), 2.07 (s, 1H), 1.62 (dd, J = 7.0, 1.9 Hz, 6H).

Referring to the synthesis method of Example B1, the following compounds were synthesized:

| Compound | Structure | LC-MS | NMR |
|---|---|---|---|
| TB-2 | 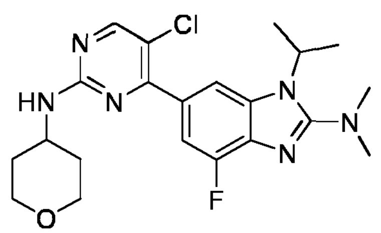<br>**6-(5-chloro-2-((tetrahydro-2*H*-pyran-4-yl)amino)pyrimidin-4-yl)-4-fluoro-1-isopropyl-*N,N*-dimethyl-1H-benzo[d]imidazol-2-amine** | $[M+H]^+$=433.4 | $^1$H NMR (400 MHz, Chloroform-*d*) $\delta$ 8.30 (s, 1H), 7.77 (s, 1H), 7.43 (*d, J* = 12.0 Hz, 1H), 5.16 (d, *J* = 8.0 Hz, 1H), 4.70 (m, 1H), 4.07 (m, 1H), 4.00 (m, 2H), 3.54 (td, *J* = 12.0, 4.0 Hz, 2H), 2.98 (s, 6H), 2.11 - 2.03 (m, 2H), 1.67 (m, 2H), 1.62 (d, *J* = 8.0 Hz, 6H). |

(continued)

| Compound | Structure | LC-MS | NMR |
|---|---|---|---|
| TB-3 | 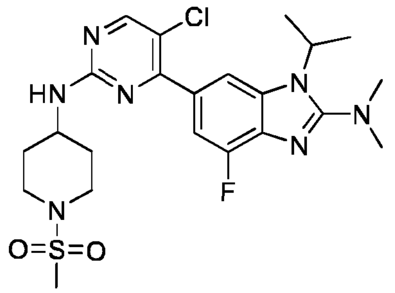 **6-(5-chloro-2-((1-(methylsulfonyl)piperidin-4-yl)amino)pyrimidin-4-yl)-4-fluoro-1-isopropyl-*N,N*-dimethyl-1*H*-benzo[*d*]imidazol-2-amine** | $[M+H]^+$=510.5 | $^1$H NMR (400 MHz, Chloroform-*d*) $\delta$ 8.30 (s, 1H), 7.77 (s, 1H), 7.41 (d, *J* = 8.0 Hz, 1H), 5.18 (d, *J* = 8.0 Hz, 1H), 4.70 (m, 1H), 4.03 - 3.95 (m, 1H), 3.80 - 3.73 (m, 2H), 2.98 (s, 6H), 2.81 (s, 2H), 2.20 (dd,*J* = 12.0, 4.0Hz, 2H), 1.66 (m, 4H), 1.62 (d, *J* = 8.0 Hz, 6H). |
| TB-4 | 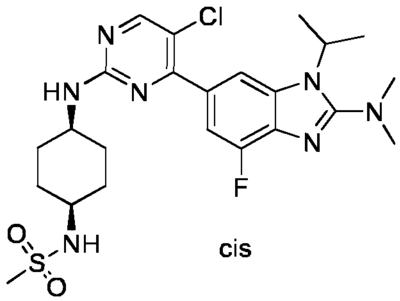 cis | $[M+H]^+$=524.5 | $^1$H NMR (400 MHz, Chloroform-*d*) $\delta$ 8.29 (s, 1H), 7.77 (s, 1H), 7.42 (d, *J* = 12.0 Hz, 1H), 5.29 (d, *J* = 8.0 Hz, 1H), 4.69 (m, 1H), 4.55 (d, *J* = 8.0 Hz, 1H), 4.01 (m, 1H), 3.55 (m, 1H), 3.00 (s, 3H), 2.98 (s, 6H), 1.89 - 1.83 (m, 4H), 1.71 (m, 4H), 1.62 *(d, J* = 4.0 Hz, 6H). |
| TB-5 | 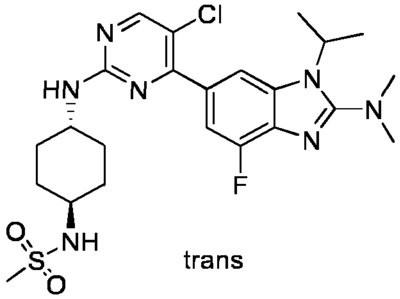 trans | $[M+H]^+$=524.4 | $^1$H NMR (400 MHz, Chloroform-*d*) $\delta$ 8.28 (s, 1H), 7.76 (s, 1H), 7.42 (d, *J*= 12.0 Hz, 1H), 5.11 (d, *J* = 8.0 Hz, 1H), 4.69 (m, 1H), 4.29 (m, 1H), 3.85 - 3.75 (m, 1H), 3.35 (m, 1H), 2.99 (s, 3H), 2.98 (s, 6H), 2.24 - 2.18 (m, 2H), 2.17 - 2.10 (m, 2H), 1.68 (m, 4H), 1.62 (d, *J* = 8.0 Hz, 6H). |
| TB-6 | 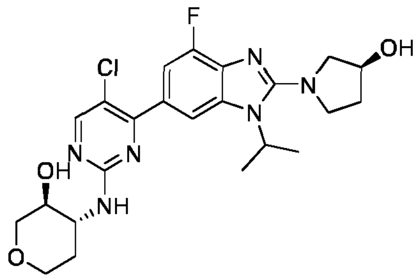 **(*S*)-1-(6-(5-chloro-2-(((3*S*,4*R*)-3-hydroxytetrahydro-2*H*-pyran-4-yl)amino)pyrimidin-4-yl)-4-fluoro-1-isopropyl-1*H*-benzo[*d*]imidazol-2-yl) pyrrolidin-3-ol** | $[M+H]^+$=491.3 | $^1$H NMR (400 MHz, Chloroform-*d*) $\delta$ 8.27 (s, 1H), 7.68 (d, *J* = 4.0 Hz, 1H), 7.40 (d, *J* = 12.0 Hz, 1H), 5.48 (d, *J* =4.0 Hz, 1H), 4.75 (m, 1H), 4.60 m, 1H), 4.05 (dd,*J* = 12.0, 4.0 Hz, 1H), 3.98 (dd, *J* = 12.0, 4.0 Hz, 1H), 3.93 - 3.87 (m, 1H), 3.85 (m, 1H), 3.78 (q, *J* = 12.0 Hz, 1H), 3.70 (dt, *J* = 12.0,4.0 Hz, 1H), 3.62 (qd, *J* = 16.0,8.0,4.0 Hz, 3H), 3.46 (td, *J* = 12.0,4.0 Hz, 1H), 3.18 (t, *J* =12.0 Hz, 1H), 2.26 (dt, *J* = 44.0,4.0 Hz, 1H), 2.18 - 2.09 (m, 2H), 2.09 - 1.98 (m, 2H), 1.67 (d, *J* = 8.0 Hz, 3H), 1.60 (d, *J* = 8.0 Hz, 3H). |

(continued)

| Compound | Structure | LC-MS | NMR |
|---|---|---|---|
| TB-7 | 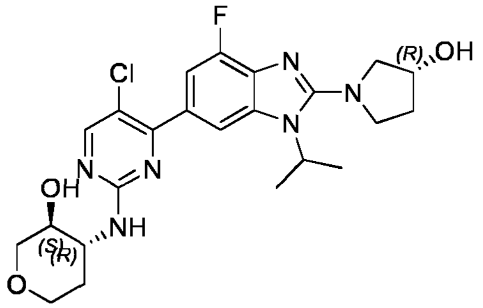 **(*R*)-1-(6-(5-chloro-2-(((3*S*,4*R*)-3-hyd roxytetrahyd ro-2*H*-pyran-4-yl)amino)pyrimidin-4-yl)-4-fluoro-1-isopropyl-1*H*-benzo[*d*]imidazol-2-yl) pyrrolidin-3-ol** | [M+H]+=491.4 | |
| TB-8 | 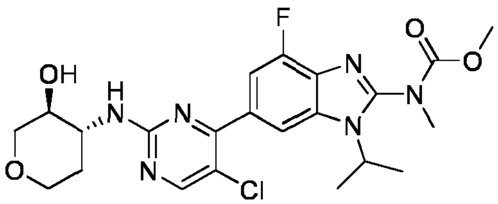 **methyl (6-(5-chloro-2-(((3*S*,4*R*)-3-hydroxytetrahydro-2*H*-pyran-4-yl)amino)pyrimidin-4-yl)-4-fluoro-1-isopropyl-1*H*-benzo[*d*]imidazol-2-yl)(methyl)carbamate** | [M+H]+=493.3 | $^1$H NMR (400 MHz, Chloroform-*d*) $\delta$ 8.34 (s, 1H), 7.84 (s, 1H), 7.46 (d, *J* = 12.0 Hz, 1H), 5.30 (d, *J* = 4.0 Hz, 1H), 4.84 (brs, 1H), 4.57 (m, 1H), 4.02 (dd, *J* = 12.0, 4.0 Hz, 2H), 3.85 (m, 1H), 3.75 (s, 3H), 3.69 - 3.58 (m, 1H), 3.46 (td,*J* = 12.0, 1.6 Hz 1H), 3.39 (s, 3H), 3.17 (t, *J* = 8.0 Hz, 1H), 1.74 (td, *J* = 8.0, 4.0 Hz, 2H), 1.64 (d, *J* = 8.0 Hz, 6H). |
| TB-9 | 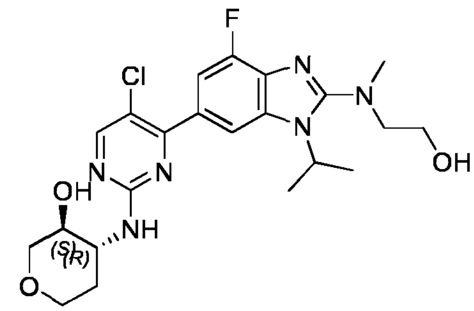 **(3*S*,4*R*)-4-((5-chloro-4-(4-fluoro-2-((2-hydroxyethyl)(methyl)amino)-1-isopropyl-1*H*-benzo [*d*]imidazol-6-yl)pyrimidin-2-yl)amino)tetrahydro-2*H*-pyran-3-ol** | [M+H]+=479.4 | $^1$H NMR (400 MHz, Chloroform-*d*) $\delta$ 8.30 (s, 1H), 7.71 (d, *J* = 1.2 Hz, 1H), 7.41 (dd, *J* = 120, 1.2 Hz, 1H), 5.32 (d,*J* = 4.0 Hz, 1H), 4.74 (m, 1H), 4.05 (dd, *J* = 12.0, 4.0 Hz, 1H), 3.98 (dd, *J* = 12.0, 4.0 Hz, 1H), 3.92 (t, *J* = 4.0 Hz, 2H), 3.85 (m, 1H), 3.63 (td, *J* = 8.0, 4.0 Hz, 1H), 3.53 (t, *J* = 4.0 Hz, 2H), 3.46 (td, *J* = 12.0, 2.0 Hz, 1H), 3.17 (t, *J* = 8.0 Hz, 2H), 3.04 (s, 3H), 2.19 - 1.94 (m, 2H), 1.77 - 1.65 (m, 2H), 1.63 (dd,*J* = 8.0, 1.2 Hz, 6H). |

(continued)

| Compound | Structure | LC-MS | NMR |
|---|---|---|---|
| TB-10 | 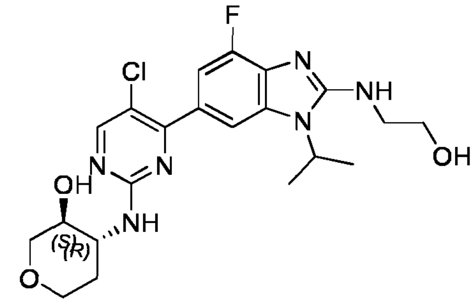 **(3*S*,4*R*)-4-((5-chloro-4-(4-fluoro-2-((2-hydroxyethyl)amino)-1-isopropyl-1*H*-benzo[*d*]imidazol-6-yl)pyrimidin-2-yl)amino)tetrahydro-2*H*-pyran-3-ol** | $[M+H]^+$=526.7 | |
| TB-11 | 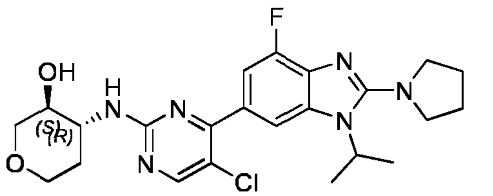 **(3*S*,4*R*)-4-((5-chloro-4-(4-fluoro-1-isopropyl-2-(pyrrolidin-1-yl)-1*H*-benzo[*d*]imidazol-6-yl)pyrimidin-2-yl)amino)tetrahydro-2*H*-pyran-3-ol** | $[M+H]^+$=475.7 | $^1$H NMR (400 MHz, Chloroform-*d*) $\delta$ 8.28 (s, 1H), 7.68 (d, *J* = 1.2 Hz, 1H), 7.41 (d, *J* = 11.2 Hz, 1H), 5.30 *(d, J*= 6.0 Hz, 1H), 4.72 (m, 1H), 4.05 (dd, *J* = 11.6, 5.2 Hz, 1H), 3.98 (dd,*J* = 11.2, 4.4 Hz, 1H), 3.84 (m, 1H), 3.65 - 3.61 (m, 4H), 3.45 (td, *J* = 11.9, 2.2 Hz, 1H), 3.21 - 3.12 (m, 1H), 2.03 (m, 1H), 2.03 - 1.98 (m, 4H), 1.76 - 1.67 (m, 2H), 1.63 (dd, *J* = 6.8, 2.0 Hz, 6H). |
| TB-12 | 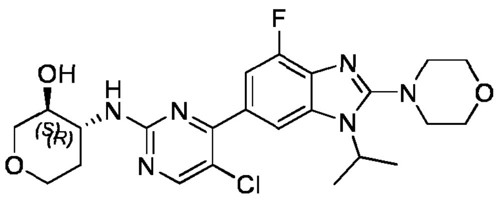 **(3*S*,4*R*)-4-((5-chloro-4-(4-fluoro-1-isopropyl-2-morpholino-1*H*-benzo[*d*]imidazol-6-yl)pyrimidin-2-yl)amino)tetrahydro-2*H*-pyran-3-ol** | $[M+H]^+$=491.7 | $^1$H NMR (400 MHz, Chloroform-*d*) $\delta$ 8.31 (s, 1H), 7.74 (d, *J* = 1.2 Hz, 1H), 7.42 (dd, *J* = 11.2, 0.8 Hz, 1H), 5.27 (d,*J* = 6.0 Hz, 1H), 4.93 (s, 1H), 4.68 (m, 1H), 4.05 (dd, *J* = 11.2, 4.8 Hz, 1H), 3.99 (dd, *J* = 11.2, 4.8 Hz, 1H), 3.95 - 3.88 (m, 4H), 3.84 (dq, *J* = 11.0, 5.4 Hz, 1H), 3.63 (q, *J* = 9.2 Hz, 1H), 3.46 (td, *J* = 11.9, 2.3 Hz, 1H), 3.37 - 3.26 (m, 4H), 3.17 (t, *J* = 10.6 Hz, 1H), 2.05 (ddd, *J* = 11.1, 4.8, 2.5 Hz, 1H), 1.73 (td, *J* = 12.3, 4.8 Hz, 1H), 1.63 (dd,*J* = 6.9, 1.7 Hz, 6H). |

(continued)

| Compound | Structure | LC-MS | NMR |
|---|---|---|---|
| TB-13 | 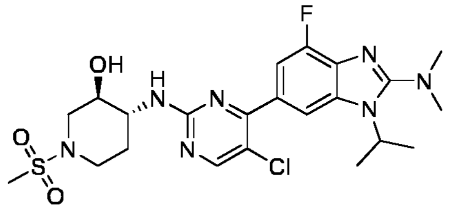 **(3*R*,4*R*)-4-((5-chloro-4-(2-(dimethylamino)-4-fluoro-1-isopropyl-1*H*-benzo[d]imidazol-6-yl)pyrimidin-2-yl)amino)-1-(methylsulfonyl)piperidin-3-ol** | $[M+H]^+$=526.7 | $^1$H NMR (400 MHz, Chloroform-*d*) δ 8.30 (s, 1H), 7.70 (d, *J* = 1.4 Hz, 1H), 7.39 (dd, *J* = 11.2, 1.4 Hz, 1H), 5.33 (d, *J* = 5.2 Hz, 1H), 4.98 (s, 1H), 4.69 (m, 1H), 3.95 (ddd, *J* = 11.7, 4.5, 2.2 Hz, 1H), 3.88 - 3.78 (m, 1H), 3.75 (m, 2H), 2.98 (s, 6H), 2.82 (s, 3H), 2.76 (dd, *J* = 12.2, 2.7 Hz, 1H), 2.53 (t, *J* = 10.2 Hz, 1H), 2.17 (dd, *J* = 13.2, 3.5 Hz, 1H), 1.80 - 1.71 (m, 1H), 1.62 (d, *J* = 7.2 Hz, 6H). |
| TB-14 | 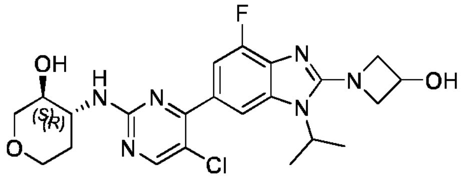 **1-(6-(5-chloro-2-(((3*S*,4*R*)-3-hydroxytetrahydro-2*H*-pyran-4-yl)amino)pyrimidin-4-yl)-4-fluoro-1-isopropyl-1*H*-benzo[*d*]imidazol-2-yl)azetidin-3-ol** | $[M+H]^+$=477.4 | |
| TB-15 | 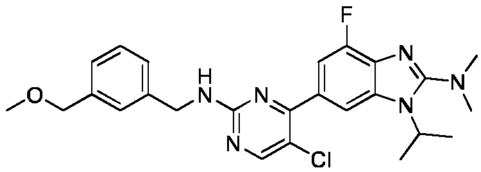 **6-(5-chloro-2-((3-(methoxymethyl)benzyl)amino)pyrimidin-4-yl)-4-fluoro-1-isopropyl-*N*,*N*-dimethyl-1*H*-benzo[*d*]imidazol-2-amine** | $[M+H]^+$=483.6 | $^1$H NMR (400 MHz, Chloroform-*d*) δ 8.30 (s, 1H), 7.77 (s, 1H), 7.43 (d, *J* = 12.0 Hz, 1H), 7.35 (s, 1H), 7.34 - 7.28 (m, 2H), 7.24 (m, 1H), 5.59 (t, *J* = 6.0 Hz, 1H), 4.70 (m, 1H), 4.67 (d, *J* = 6.0 Hz, 2H), 4.45 (s, 2H), 3.39 (s, 3H), 2.97 (s, 6H), 1.60 (d, *J* = 4.0 Hz, 6H). |

**Example B2**

Synthesis of the compound TB-16 of the present invention:

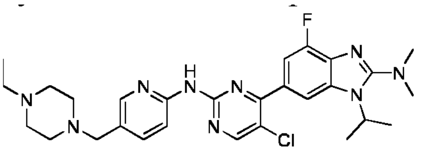

The synthesis route is as follows:

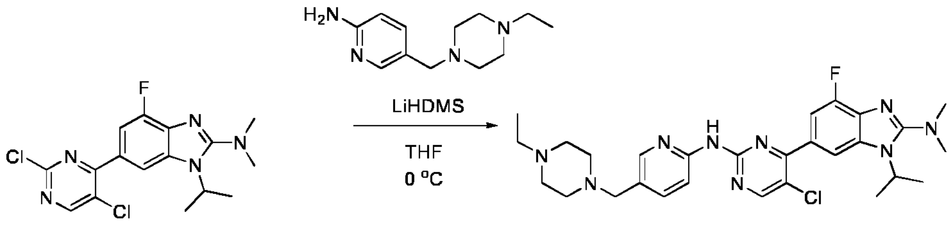


SM 1

TB-16

The experimental procedure is as follows:

Synthesis of compound TB-16

5-[(4-ethylpiperazin-1-yl)methyl]pyridin-2-amine (180 mg, 1.5 e.q.) was dissolved in 4 mL of tetrahydrofuran ultra-dry solvent under nitrogen protection, and placed in an ice-water bath, 1 M tetrahydrofuran solution of LiHDMS (0.81 mL, 1.5 e.q.) was added dropwise, the reaction was carried out in an ice-water bath for half an hour, and compound SM 1 (100 mg, 1.0 e.q.) was added, and the reaction was carried out while the temperature was kept. At the end of the reaction, extracted and partitioned with Ea and water, the organic phase was dried over anhydrous sodium sulfate, suction filtered and concentrated under reduced pressure, and 120 mg of compound TB- 16 was obtained after preparative isolation and purification, with a purity of 98.14% by HPLC, a yield of 82.0%, LCMS: [ M + H ]$^+$ = 552.5.

$^1$H NMR (400 MHz, Chloroform-*d*) $\delta$ 8.53 (s, 1H), 8.47 (s, 1H), 8.36 - 8.32 (d, *J* = 8.0 Hz, 1H), 8.21 (d, *J* = 4.0 Hz, 1H), 7.80 (d, *J* = 1.6 Hz, 1H), 7.57 (dd, *J* = 8.0, 4.0 Hz, 1H), 7.48 (dd, *J* = 12.0, 1.6 Hz, 1H), 4.65 (p, *J* = 8.0 Hz, 1H), 3.48 (s, 2H), 2.94 (s, 6H), 2.73 (m, 8H), 1.58 (d, *J* = 8.0 Hz, 6H), 1.25 (t, *J* = 6 Hz, 3H), 1.19 (m, 2H).

Referring to the synthesis method of Example B2, the following compounds were synthesized:

| Compound | Structure | LC-MS | NMR |
|---|---|---|---|
| TB-17 | 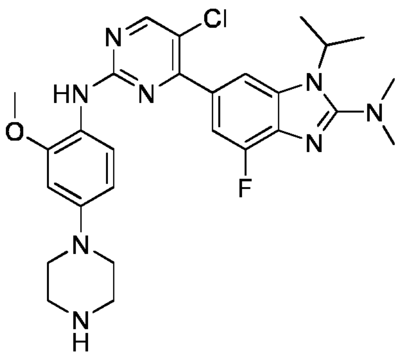<br>**6-(5-chloro-2-((2-methoxy-4-(piperazin-1-yl)phenyl)amino)pyrimidin-4-yl)-4-fluoro-1-isopropyl-*N,N*-dimethyl-1*H*-benzo[*d*]imidazol-2-amine** | [M+H]$^+$=539.5 | $^1$H NMR (400 MHz, Chloroform-*d*) $\delta$ 8.34 (s, 1H), 7.77 (d, *J* = 4.0 Hz, 1H), 7.60 (s, 1H), 7.49 - 7.44 (d, *J* = 12.0 Hz, 1H), 6.50 - 6.44 (m, 2H), 4.64 (p, *J* = 8.0 Hz, 1H), 3.83 (s, 3H), 3.33 (m, 8H), 2.93 (s, 6H), 1.57 (d, *J* = 4.0 Hz, 6H). |
| TB-18 | 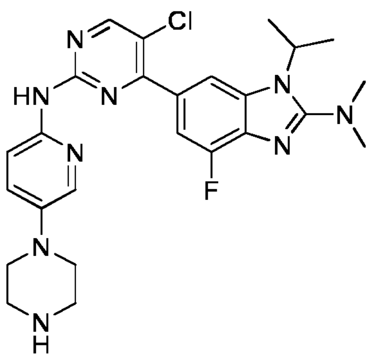<br>**6-(5-chloro-2-((5-(piperazin-1-yl)pyridin-2-yl)amino)pyrimidin-4-yl)-4-fluoro-1-isopropyl-*N,N*-dimethyl-1*H*-benzo[*d*]imidazol-2-amine** | [M+H]$^+$=510.4 | $^1$H NMR (400 MHz, DMSO-$d_6$) $\delta$ 9.98 (s, 1H), 8.62 (s, 1H), 8.12 - 8.02 (m, 2H), 7.91 (s, 1H), 7.47 (dd, *J* = 12, 1.3 Hz, 1H), 7.42 (dd, *J* = 10.0, 4.0 Hz, 1H), 4.69 (p, *J* = 8.0 Hz, 1H), 3.24 (m, 4H), 3.11 (m, 4H), 2.93 (s, 6H), 1.58 (d, *J* = 8.0 Hz, 6H). |

(continued)

| Compound | Structure | LC-MS | NMR |
|---|---|---|---|
| TB-19 | 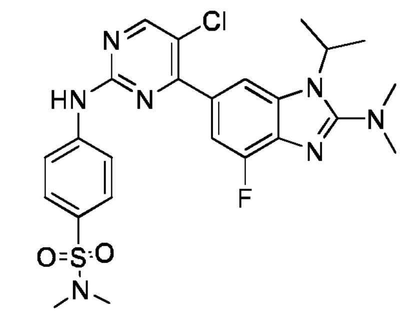 **4-((5-chloro-4-(2-(dimethylamino)-4-fluoro-1-isopropyl-1*H*-benzo[*d*]imidazol-6-yl)pyrimidin-2-yl)amino)-*N,N*-dimethylbenzenesulfonamide** | $[M+H]^+$=532.4 | $^1$H NMR (400 MHz, Chloroform-*d*) δ 8.50 (s, 1H), 7.87 (d, *J* = 4.0 Hz, 1H), 7.85 (d, *J* = 4.0 Hz, 2H), 7.73 (d, *J* = 8.0 Hz, 2H), 7.59 (s, 1H), 7.54 (dd, *J* = 12.0, 1.6 Hz, 1H), 4.72 (m, 1H), 3.00 (s, 6H), 2.70 (s, 6H), 1.65 (d, *J* = 8.0 Hz, 6H). |
| TB-20 | 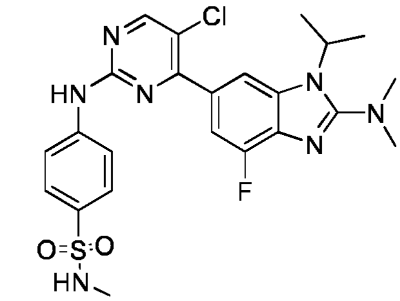 4-((5-chloro-4-(2-(dimethylamino)-4-fluoro-1-isopropyl-1*H*-benzo[*d*]imidazol-6-yl)pyrimidin-2-yl)amino)-*N*-methylbenzenesulfonamide | $[M+H]^+$=518.5 | |
| TB-21 | 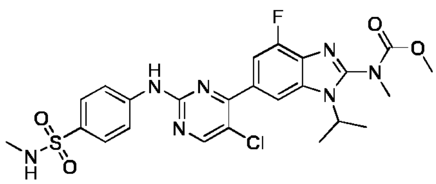 **methyl (6-(5-chloro-2-((4-(*N*-methylsulfamoyl)phenyl) amino)pyrimidin-4-yl)-4-fluoro-1-isopropyl-1*H*-benzo[*d*] imidazol-2-yl)(methyl)carbamate** | $[M+H]^+$=562.2 | $^1$H NMR (400 MHz, Chloroform-*d*) δ 8.54 (s, 1H), 7.98 (d, *J* = 0.8 Hz, 1H), 7.83 (q, *J* = 8.8 Hz, 4H), 7.62 (s, 1H), 7.58 (d, *J* = 12.0 Hz, 1H), 4.60 (m, 1H), 4.41 (q, *J* = 4.0 Hz, 1H), 3.77 (s, 3H), 3.41 (s, 3H), 2.66 (d, *J* = 4.0 Hz, 3H), 1.67 (d, *J* = 6.8 Hz, 6H). |

**Example B3**

Synthesis of the compound TB-23 of the present invention:

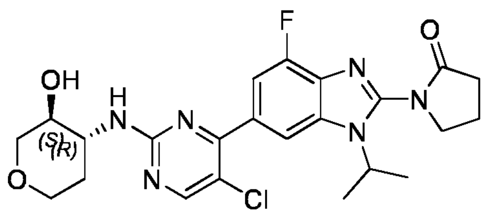

The synthesis route is as follows:

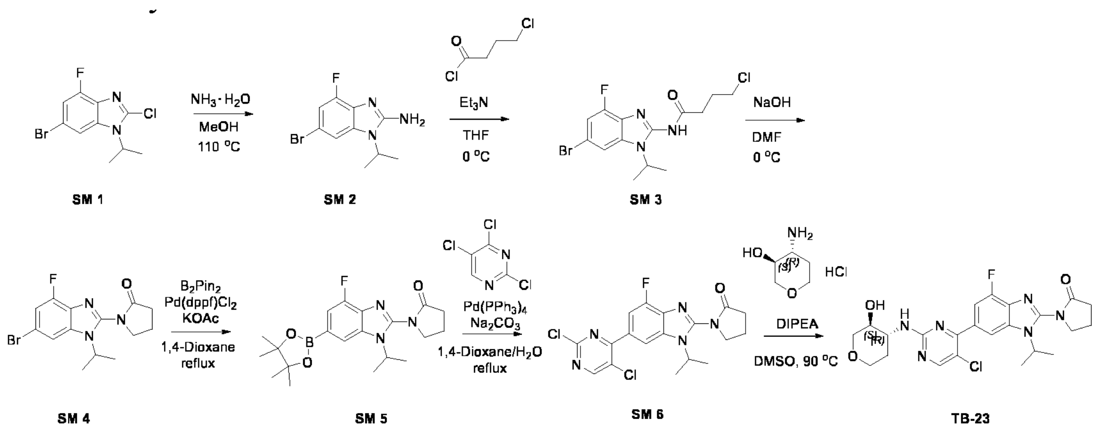


The experimental procedure is as follows:

Step 1:

Compound SM 1 (1.5 g, 1.0 e.q.) was dissolved in a solvent mixture of ammonia and methanol (45 mL, v : v = 2 : 1), and the reaction was carried out in a sealed autoclave at 110 °C for 24 hours. At the end of the reaction, the reaction solution was cooled to room temperature, the reaction solvent was concentrated under reduced pressure, and 1.1 g of compound SM 2 was obtained by isolation and purification directly by silica gel column chromatography, yield 78.6%, LCMS: $[M + H]^+$ = 272.0, 274.0.

Step 2:

Compound SM 2 (170 mg, 1.0 e.q.) was dissolved in 2 mL of ultra-dry THF solvent in an ice-water bath, then triethylamine (316 mg, 5.0 e.q.) and 4-chlorobutanoyl chloride (221 mg, 2.5 e.q.) were added sequentially, and the reaction was kept in an ice-water bath, after the reaction was completed, extracted and partitioned with EA and water, and the organic phase was dried over anhydrous sodium sulfate, and suction filtered. After concentration under reduced pressure, the filtrate was separated and purified by silica gel column chromatography to obtain 180 mg of compound SM 3 in 76.6% yield, LCMS: $[M + H]^+$ = 376.1,378.1.

Step 3:

Compound SM 3 (1.1 g, 1.0 e.q.) was solubilized in 22 mL of ultra-dry DMF solvent under ice-water bath conditions, and then NaOH solid (0.21 g, 1.8 e.q.) was added, and the reaction was kept stirred in the ice bath. At the end of the reaction, the reaction solution was extracted and partitioned with saturated ammonium chloride and EA, the organic phase was dried over anhydrous sodium sulfate, suction filtered, and after concentration under reduced pressure, the filtrate was separated and purified by silica gel column chromatography to obtain 340 mg of the compound SM 4 in 34% yield, LCMS: $[M + H]^+$ = 340.1,342.1.

Step 4:

Compound SM 4 (360 mg, 1.0 e.q.), pinacol bis(boronic acid) ester (537 mg, 2.0 e.q.), $Pd(dppf)Cl_2$ (38.7 mg, 0.05 e.q.) and potassium acetate (311 mg, 3.0 e.q.) were added sequentially to a dry 50 mL three-necked flask under nitrogen protection, after nitrogen displacement three times, and ultra-dry 1,4 - dioxane solvent was added, warmed to reflux reaction. At the end of the reaction, the reaction solution was cooled to room temperature, extracted and partitioned with Ea and water, the organic phase was dried over anhydrous sodium sulfate, suction filtered and concentrated under reduced pressure, isolated and purified by column chromatography to obtain the compound SM 5 (310 mg, 75.6%), LCMS: $[M + H]^+$ = 388.3.

Step 5:

Compound SM 5 (310 mg, 1.0 e.q.), 2,4,5-trichloropyrimidine (200 mg, 1.5 e.q.), palladium tetrakis(triphenylphosphine) (92.5 mg, 0.1 e.q.) and sodium carbonate solid (254.5 mg, 3.0 e.q.) were sequentially added to a 50 mL three-necked flask under nitrogen protection, and 13 mL mixture of 1,4-dioxane and water (v: v = 10: 3) was added, after nitrogen displacement three times, warmed to reflux. At the end of the reaction, the reaction solution was cooled to room temperature, extracted and partitioned with Ea and water, the organic phase was dried over anhydrous sodium sulfate, suction filtered and concentrated under reduced pressure, isolated and purified by column chromatography to obtain compound SM 6 (240 mg, 73.6%), LCMS: [ M + H ]$^+$ = 408.1.

Step 6:

Compound SM 6 (180 mg, 1.0 e.q.), (3s, 4r)-4-aminooxan-3-ol hydrochloride (102 mg, 1.5 e.q.) and DIPEA (228 mg, 4.0 e.q.) were sequentially added to 3 mL of DMSO solvent under nitrogen, and the reaction was heated up to 90 °C. After the reaction was ended, the reaction solution was cooled to room temperature, extracted and partitioned sequentially with saturated saline, water and EA, and the organic phase was dried over anhydrous sodium sulfate, suction filtered and concentrated under reduced pressure, and purified by column chromatography to obtain the compound TB-23 (120 mg, 56.0%), with the purity of 96.75% by HPLC, and LCMS: [ M + H ]$^+$ = 489.4.

$^1$H NMR (400 MHz, Chloroform-*d*) $\delta$ 8.32 (s, 1H), 7.88 (d, *J* = 1.2 Hz, 1H), 7.45 (dd, *J* = 12.0, 1.2 Hz, 1H), 5.33 (d, *J* = 8.0 Hz, 1H), 4.82 (s, 1H), 4.61 (m, 1H), 4.11 (t, *J* = 8.0 Hz, 3H), 4.05 (dd, *J* = 12.0, 4.0 Hz, 1H), 3.98 (dd, *J* = 12.0, 4.0 Hz, 1H), 3.92 - 3.79 (m, 1H), 3.63 (td, *J* = 8.0, 4.0 Hz, 1H), 3.46 (td, *J* = 12.0, 2.0 Hz, 1H), 3.17 (t, *J* = 8.0, 1H), 2.63 (t, *J* = 8.0 Hz, 2H), 2.32 (p, *J* = 8.0 Hz, 2H), 1.78 - 1.69 (m, 2H), 1.68 (d, *J* = 8.0 Hz, 6H).

Referring to the synthesis method of Example B3, the following compounds were synthesized:

**Example B4**

Synthesis of the compound TB-36 of the present invention:

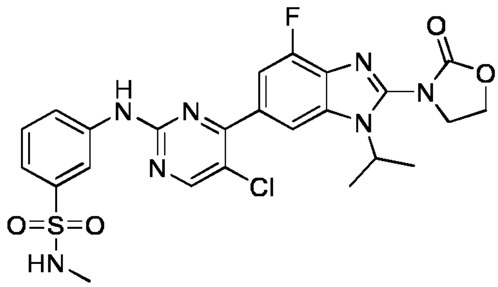

The synthesis route is as follows:

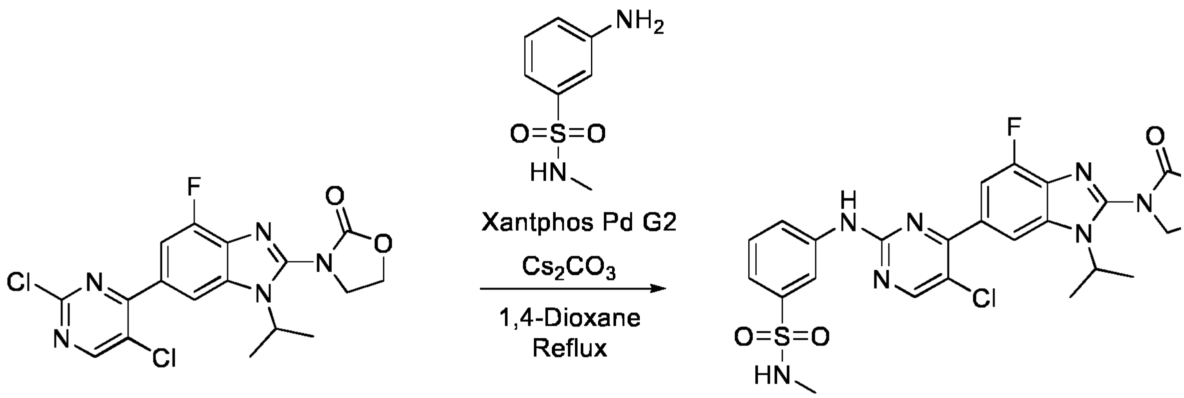


**Int-1** **TB-36**

The experimental procedure is as follows:

Step 1:

Int-1 (200 mg, 1.0 e.q.), N-methyl-3-aminobenzenesulfonamide (136.0 mg, 1.5 e.q.), cesium carbonate (476.5 mg, 3.0 e.q.), and Xantphos Pd G2 catalyst (21.6 mg, 0.05 e.q.) were added to a 25 mL three-necked flask under nitrogen

protection, then 10 mL of ultra-dry 1,4-dioxane solvent was added, after three nitrogen displacements, the reaction was started to warm up to reflux. At the end of the reaction, the reaction solution was cooled to room temperature, the system was extracted and partitioned with Ea and water, the organic phase was dried over anhydrous sodium sulfate, the organic phase was concentrated under reduced pressure after suction filtration, and the isomers were separated using preparative chromatography to obtain the compound TB-36 (20 mg, 7.3%) with a purity of 97.18% by HPLC, LCMS: [ M + H ]$^+$ = 561.0.

$^1$H NMR (400 MHz, Chloroform-*d*) δ 8.51 (s, 1H), 8.25 (t, *J* = 2.0 Hz, 1H), 8.03 (d, *J* = 1.3 Hz, 1H), 7.84 (d, *J* = 8.3 Hz, 1H), 7.62 - 7.43 (m, 4H), 4.79 (p, *J* = 6.9 Hz, 1H), 4.66 (t, *J* = 7.7 Hz, 2H), 4.51 (q, *J* = 5.4 Hz, 1H), 4.40 (t, *J* = 7.7 Hz, 2H), 2.69 (d, *J* = 5.4 Hz, 3H), 1.72 (d, *J* = 6.9 Hz, 6H).

Referring to the synthesis method of Example B4, the following compounds were synthesized:

| Compound | Structure | LC-MS | NMR |
|---|---|---|---|
| TB-38 | 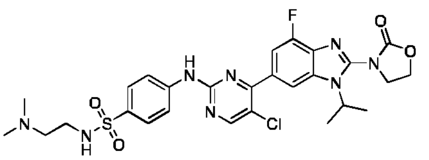 **4-((5-chloro-4-(4-fluoro-1-isopropyl-2-(2-oxooxazolidin-3-yl)-1*H*-benzo[*d*]imidazol-6-yl) pyrimidin-2-yl)amino)-*N*-(2-(dimethylamino) ethyl)benzenesulfonamide** | [M+H]$^+$= 617.6 | $^1$H NMR (400 MHz, Methanol-$d_4$) δ 8.61 (s, 1H), 8.17 (d, *J* = 1.2 Hz, 1H), 8.00 (d, *J* = 8.9 Hz, 2H), 7.78 (d, *J* = 8.9 Hz, 2H), 7.64 (dd, *J* = 12.0, 1.2 Hz, 1H), 4.86 (s, 1H), 4.70 (t, *J* = 8.0 Hz, 2H), 4.63 (s, 1H), 4.31 (t, *J* = 8.0 Hz, 2H), 3.05 (t, *J* = 6.4 Hz, 2H), 2.79 (t, *J* = 6.4 Hz, 2H), 2.52 (s, 6H), 1.72 (d, *J* = 7.0 Hz, 6H). |
| TB-39 | 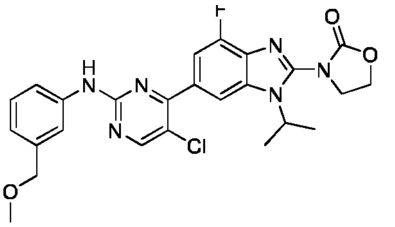 **3-(6-(5-chloro-2-((3-(methoxymethyl)phenyl) amino)pyrimidin -4-yl)-4-fluoro-1-isopropyl-1*H*-benzo[*d*]imidazol-2-yl)oxazolidin-2-one** | [M+H]$^+$=5 11.3 | $^1$H NMR (400 MHz, Chloroform-*d*) δ 8.47 (s, 1H), 8.03 (d, *J* = 1.2 Hz, 1H), 7.65 (dd, *J* = 8.0, 1.2 Hz, 1H), 7.64 - 7.61 (m, 1H), 7.60 (s, 1H), 7.32 (t, *J* = 8.0 Hz, 1H), 7.03 (d, *J* = 4.0 Hz, 1H), 4.77 (m, 1H), 4.65 (t, *J* = 8.0Hz, 2H), 4.47 (s, 2H), 4.39 (t, *J* = 8.0 Hz, 2H), 3.41 (s, 3H), 1.72 (d, *J* = 8.0 Hz, 6H). |
| TB-54 | 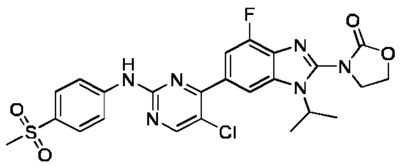 **3-(6-(5-chloro-2-((4-(methylsulfonyl)phenyl) amino)pyrimidin-4-yl)-4-fluoro-1-isopropyl-1*H*-benzo[*d*]imidazol-2-yl)oxazolidin-2-one** | [M+H]$^+$=5 45.3 | $^1$H NMR (400 MHz, Chloroform-*d*) δ 8.55 (s, 1H), 8.01 (d, *J* = 1.2 Hz, 1H), 7.88 (s, 4H), 7.67 (s, 1H), 7.58 (dd, *J* = 12.0, 1.2 Hz, 1H), 4.79 (m, 1H), 4.66 (t, *J* = 8.0 Hz, 2H), 4.40 (*t, J* = 8.0 Hz, 2H), 3.05 (s, 3H), 1.72 (d, *J* = 8.0 Hz, 6H). |
| TB-139 | 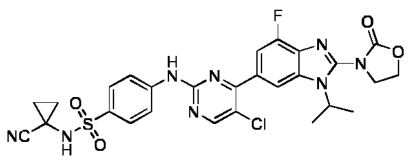 **4-((5-chloro-4-(4-fluoro-1-isopropyl-2-(2-oxooxazolidin-3-yl)-1*H*-benzo[*d*]imidazol-6-yl) pyrimidin-2-yl)amino)-*N*-(1-cyanocyclopropyl)benzenesulfonamide** | [M+H]$^+$=6 11.5 | $^1$H NMR (400 MHz, Chloroform-*d*) δ 8.52 (s, 1H), 8.02 (d, *J* = 1.4 Hz, 1H), 7.94 - 7.83 (m, 4H), 7.75 (s, 1H), 7.65 - 7.54 (m, 1H), 5.68 (s, 1H), 4.79 (p, *J* = 6.8 Hz, 1H), 4.66 (t, *J* = 7.7 Hz, 2H), 4.50 - 4.30 (m, 2H), 1.72 (d, *J* = 6.9 Hz, 6H), 1.56 (q, *J* = 5.4, 5.0 Hz, 2H), 1.42 (dd, *J* = 5.5, 2.8 Hz, 2H). |

(continued)

| Compound | Structure | LC-MS | NMR |
|---|---|---|---|
| TB-84 | 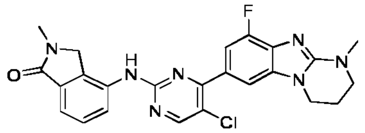 **4-((5-chloro-4-(9-fluoro-1-methyl-1,2,3,4-tetrahydrobenzo[4,5]imidazo[1,2-*a*]pyrimidin-7-yl) pyrimidin-2-yl)amino)-2-methylisoindolin-1-one** | $[M+H]^+$=4 78.5 | $^1H$ NMR (400 MHz, Chloroform-*d*) $\delta$ 8.40 (s, 1H), 8.09 (d, *J* = 8.0 Hz, 1H), 7.61 (d, *J* = 7.4 Hz, 1H), 7.53 (dd, *J* = 11.8, 1.5 Hz, 1H), 7.47 (t, *J* = 7.8 Hz, 1H), 7.41 (d,*J* = 1.5 Hz, 1H), 7.08 (s, 1H), 4.36 (s, 2H), 3.99 (t, *J* = 6.1 Hz, 2H), 3.44 (t, *J* = 5.6 Hz, 2H), 3.27 (s, 3H), 3.19 (s, 3H), 2.35 - 2.25 (m, 2H). |
| TB-53 | 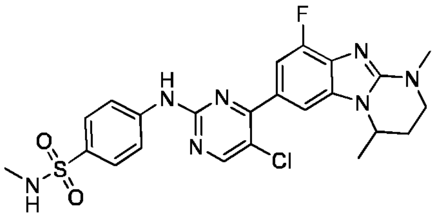 **4-(5-chloro-4-(9-fluoro-1,4-dimethyl-1,2,3,4-tetrahydrobenzo[4,5]imidazo[1,2-a]pyrimidin-7-yl)pyrimidin-2-yl)amino)-*N*-methylbenzenesulfonamide** | $[M+H]^+$= 516.5 | $^1H$ NMR (400 MHz, Chloroform-*d*) $\delta$ 8.45 (s, 1H), 7.81 - 7.74 (m, 4H), 7.69 (s, 1H), 7.54 - 7.46 (m, 2H), 4.65 (q, *J* = 5.6 Hz, 1H), 4.60 - 4.46 (m, 1H), 3.60 (td, *J* = 12.0, 3.2 Hz, 1H), 3.36 - 3.30 (m, 1H), 3.29 (s, 3H), 2.65 (d, *J* = 5.6 Hz, 3H), 2.41 (m, 1H), 2.04 - 1.97 (m, 1H), 1.52 (d,*J* = 6.4 Hz, 3H). |
| TB-130 | 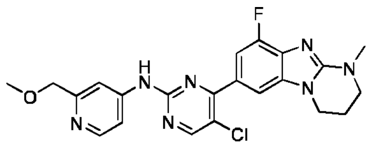 **5-chloro-4-(9-fluoro-1-methyl-1,2,3,4-tetrahydrobenzo[4,5]imidazo[1,2-*a*]pyrimidin-7-yl)-*N*-(2-(methoxymethyl)pyridin-4-yl)pyrimidin-2-amine** | $[M+H]^+$= 454.3 | |
| TB-133 | 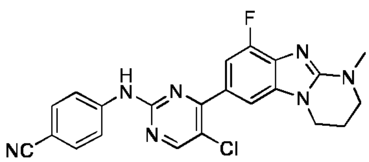 **4-((5-chloro-4-(9-fluoro-1-methyl-1,2,3,4-tetrahydrobenzo[4,5]imidazo[1,2-*a*]pyrimidin-7-yl) pyrimidin-2-yl)amino)benzonitrile** | $[M+H]^+$=4 34.5 | $^1H$ NMR (400 MHz, Chloroform-*d*) $\delta$ 8.46 (s, 1H), 7.78 (d, *J* = 8.8 Hz, 2H), 7.60 (d, *J* = 8.8 Hz, 2H), 7.55 (s, 1H), 7.52 (dd, *J* = 12.0, 1.5 Hz, 1H), 7.46 (d, *J* = 1.5 Hz, 1H), 4.03 (t, *J* = 6.0 Hz, 2H), 3.45 (t, *J* = 6.0 Hz, 2H), 3.28 (s, 3H), 2.31 (p, *J* = 6.0 Hz, 2H). |
| TB-135 | 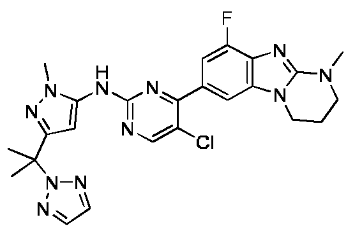 ***N*-(3-(2-(2*H*-1,2,3-triazol-2-yl)propan-2-yl)-1-methyl-1*H*-pyrazol-5-yl)-5-chloro-4-(9-fluoro-1-methyl-1,2,3,4-tetrahydrobenzo[4,5] imidazo[1,2-*a*]pyrimidin-7-yl)pyrimidin-2-amine** | $[M+H]^+$=5 22.4 | $^1H$ NMR (400 MHz, Chloroform-*d*) $\delta$ 8.39 (s, 1H), 7.63 *(d, J* = 1.2 Hz, 1H), 7.58 (d, *J* = 1.2 Hz, 1H), 7.56 - 7.51 (m, 1H), 7.43 (d, *J* = 1.6 Hz, 1H), 6.92 (s, 1H), 6.28 (s, 1H), 3.99 (t, *J* = 6.0 Hz, 2H), 3.79 (s, 3H), 3.52 - 3.42 (m, 2H), 3.30 (s, 3H), 2.32 (p, *J* = 6.2 Hz, 2H), 2.09 (s, 6H). |

(continued)

| Compound | Structure | LC-MS | NMR |
|---|---|---|---|
| TB-137 | 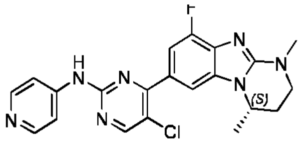 **(*S*)-5-chloro-4-(9-fluoro-1,4-dimethyl-1,2,3,4-tetrahydrobenzo[4,5]imidazo[1,2-*a*]pyrimidin-7-yl)-*N*-(pyridin-4-yl)pyrimidin-2-amine** | $[M+H]^+$=4 24.3 | |
| TB-141 | 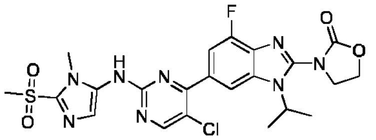 **3-(6-(5-chloro-2-((1-methyl-2-(methylsulfonyl)-1*H*-imidazol-5-yl)amino)pyrimidin-4-yl)-4-fluoro-1-isopropyl-1*H*-benzo[*d*]imidazol-2-yl) oxazolidin-2-one** | $[M+H]^+$=5 49.2 | $^1$H NMR (400 MHz, Chloroform-*d*) δ 8.45 (s, 1H), 7.93 (d, *J* = 1.2 Hz, 1H), 7.56 - 7.38 (m, 1H), 7.13 (s, 1H), 7.07 (s, 1H), 4.76 (m, 1H), 4.64 (t, *J* = 7.6 Hz, 2H), 4.38 (t, *J* = 7.6 Hz, 2H), 3.83 (s, 3H), 3.38 (s, 3H), 1.69 (d, *J* = 8.0 Hz, 6H). |
| TB-145 | 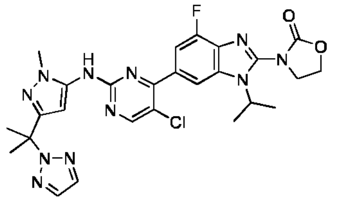 **3-(6-(2-((3-(2-(2H-1,2,3-triazol-2-yl)propan-2-yl)-1-methyl-1*H*-pyrazol-5-yl)amino)-5-chloropyrimidin-4-yl)-4-fluoro-1-isopropyl-1*H*-benzo [*d*]imidazol-2-yl)oxazolidin-2-one** | $[M+H]^+$=5 80.1 | $^1$H NMR (400 MHz, Chloroform-*d*) δ 8.46 (s, 1H), 7.96 (d, *J* = 1.1 Hz, 1H), 7.65 (d,J= 0.8 Hz, 1H), 7.57 (d, *J* = 0.8 Hz, 1H), 7.49 (dd, *J* = 11.2, 0.9 Hz, 1H), 6.90 (s, 1H), 6.21 (s, 1H), 4.76 (p, *J* = 6.9 Hz, 1H), 4.68 - 4.59 (m, 2H), 4.45 - 4.33 (m, 2H), 3.79 (s, 3H), 2.08 (s, 6H), 1.68 (d, *J* = 6.8 Hz, 6H). |

**Example B5**

Synthesis of the compound TB-40 of the present invention:

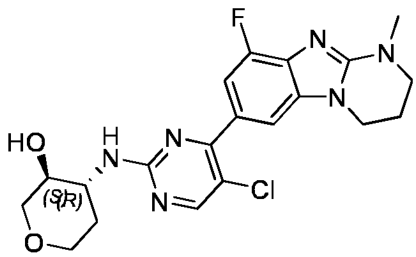

The synthesis route is as follows:

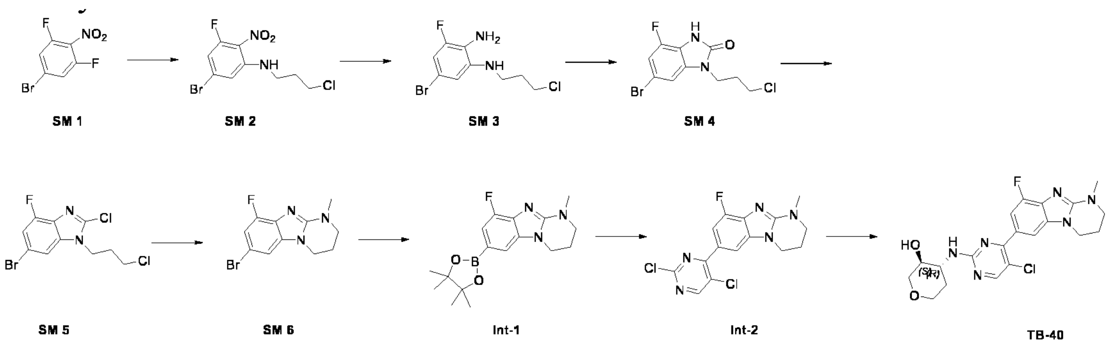

SM 1 SM 2 SM 3 SM 4

SM 5 SM 6 Int-1 Int-2 TB-40

The experimental procedure is as follows:

Step 1:

Compound SM 1 (5.0 g, 1.0 e.q.) was solubilized in ultra-dry tetrahydrofuran solvent, placed in an ice-water bath, cesium carbonate solid (17.1 g, 2.5 e.q.) was added, 3-chloropropylamine hydrochloride (2.7 g, 1.0 e.q.) was added under stirring conditions. After the addition, the reaction was brought to room temperature naturally. After TLC monitor showed the reaction was ended, the solids were removed by suction filtration, the filter cake was washed with an appropriate amount of EA, extracted and partitioned with water, and the organic phase was dried over anhydrous sodium sulfate. After suction filtration, the reaction solution was concentrated under reduced pressure, and purified by column chromatography to obtain SM 2 (4.7 g, 71.9%) as a yellow solid.

$^1$H NMR (400 MHz, Chloroform-*d*) $\delta$ 7.35 (s, 1H), 6.81 (t, $J$ = 2.0 Hz, 1H), 6.64 (dd, $J$ = 12.0, 2.0 Hz, 1H), 3.67 (t, $J$ = 4.0 Hz, 2H), 3.46 (q, $J$ = 8.0 Hz, 2H), 2.16 (p, $J$ = 8.0 Hz, H).

Step 2:

Compound SM 2 (4.3 g, 1.0 e.q.) was dissolved in a solvent mixture of methanol and water (v: v = 4:1), iron powder (3.9 g, 5.0 e.q.) and ammonium chloride solid (1.5 g, 2.0 e.q.) were added, and the reaction was warmed to reflux. TLC monitor showed the reaction was ended, the reaction solution was cooled down to room temperature, suction filtered, and the filter cake was washed with an appropriate amount of EA, and the filtrate was concentrated under reduced pressure to remove most of the solvent, extracted and partitioned with saturated sodium bicarbonate solution and EA, the organic phase was dried over anhydrous sodium sulfate, suction filtered and concentrated under reduced pressure, purified by column chromatography to obtain compound SM 3 (2.0 g, 51.4%).

Step 3:

Compound SM 3 (1.3 g, 1.0 e.q.) and CDI (2.2 g, 2.5 e.q.) were sequentially dissolved in ultra-dry DMF solvent, the reaction was heated up to 90 °C. TLC monitor showed the reaction was ended, the reaction solution was cooled down to room temperature, extracted and partitioned with EA and water, the organic phase was dried over anhydrous sodium sulphate, suction filtered, and then concentrated under reduced pressure, isolated and purified by column chromatography to obtain compound SM 4 ( 1.2 g, 83.3%).

Step 4:

Compound SM 4 (4.0 g, 1.0 e.q.) was solubilized in dry toluene, phosphorus oxychloride (36 mL, 30.0 e.q.) was added, and the reaction was heated up to reflux for two days. TLC monitor showed the reaction was ended, and the reaction solution was cooled down to room temperature, quenched by pouring it into iced water, extracted and partitioned with Ea, and the organic phase was dried over anhydrous sodium sulfate, suction filtered and concentrated under reduced pressure, and purified by column chromatography to obtain compound SM 5 (137 mg, 24.9%), LCMS: [ M + H ]$^+$ = 325.3, 327.3.

Step 5:

Compound SM 5 (1.25 g, 1.0 e.q.) was added to 60 mL of 30% methylamine ethanol solution, and the reaction was heated up to 60 °C. After the reaction was finished, the reaction solution was cooled down to room temperature, and then concentrated under reduced pressure to remove most of the solvent, and then extracted and partitioned with

saturated saline and EA, and the organic phase was dried over anhydrous sodium sulfate, and then suction filtered, and then the organic phase was concentrated, and then purified by silica gel column chromatography to obtain compound SM 6 (500 mg, 48.9%).
$^1$H NMR (400 MHz, Chloroform-*d*) $\delta$ 6.98 (dd, *J* = 10.0, 1.7 Hz, 1H), 6.93 (d, *J* = 1.7 Hz, 1H), 3.93 (t, *J* = 6.2 Hz, 2H), 3.48 - 3.31 (m, 2H), 3.22 (s, 3H), 2.26 (p, *J* = 6.0 Hz, 2H).

Step 6:

Compound SM 6 (0.26 g, 1.0 e.q.), pinacol bis(boronic acid) ester(0.46 g, 2.0 e.q.), Pd(dppf)$Cl_2$ (34 mg, 0.05 e.q.) and potassium acetate (0.27 g, 3.0 e.q.) were sequentially added to a dry 50 mL three-necked flask under nitrogen protection, nitrogen was displaced three times, and ultra-dry 1,4- Dioxane solvent was added, warmed up to reflux reaction. TLC monitor showed the reaction was ended, the reaction solution was cooled to room temperature, extracted and partitioned with Ea and water, the organic phase was dried over anhydrous sodium sulfate, suction filtered and concentrated under reduced pressure, and isolated and purified by column chromatography to obtain the compound Int-1 (0.21 g, 69.3%), LCMS: [ M + H ]$^+$ = 332.6.

Step 7:

Compound Int-1 (0.21 g, 1.0 e.q.), 2,4,5-trichloropyrimidine (0.21 g, 2.0 e.q.), palladium tetrakis(triphenylphosphine) (71.5 mg, 0.1 e.q.) and sodium carbonate solid (0.2 g, 3.0 e.q.) were sequentially added to a 50 mL three-necked flask under nitrogen protection, and a mixture of 1,4-dioxane and water ( v: v = 10: 3) was added, nitrogen was displaced three times, and warmed to reflux reaction. TLC monitor showed the reaction was ended, and the reaction solution was cooled to room temperature, extracted and partitioned with Ea and water, the organic phase was dried over anhydrous sodium sulfate, suction filtered and concentrated under reduced pressure, and isolated and purified by column chromatography to obtain the compound Int-2 (0.13 g, 59.2%), LCMS: [ M + H]$^+$ = 352.5.

Step 8:

Compound Int-2 (100 mg, 1.0 e.q.), (3s, 4r)-4-aminooxan-3-ol hydrochloride (87.2 mg, 2.0 e.q.) and DIPEA (0.2 mL, 4.0 e.q.) were sequentially added to a DMSO solvent under nitrogen, and the reaction was heated to 90 °C. At the end of the reaction, the reaction solution was cooled to room temperature, extracted and partitioned with saturated saline, water and EA in turn, the organic phase was dried over anhydrous sodium sulfate, suction filtered and concentrated under reduced pressure, purified by preparative liquid phase to obtain the compound TB-40 (50 mg, 40.7%), the purity of the HPLC was 91.7%, LCMS: [ M + H ]$^+$ = 433.4.

$^1$H NMR (400 MHz, Chloroform-*d*) $\delta$ 8.25 (s, 1H), 7.42 (dd, *J* = 11.8, 1.2 Hz, 1H), 7.32 (d, *J* = 1.2 Hz, 1H), 5.27 (d, *J* = 6.0 Hz, 1H), 5.12 (s, 1H), 4.05 (m, 2H), 4.00 (t, *J* = 6.0 Hz, 2H), 3.89 - 3.77 (m, 1H), 3.62 (td, *J* = 9.6, 4.8 Hz, 1H), 3.50 - 3.43 (m, 1H), 3.42 (t, *J* = 5.6 Hz, 2H), 3.26 (s, 3H), 3.24 - 3.14 (m, 1H), 2.28 (p, *J* = 5.8 Hz, 2H), 2.09 - 1.98 (m, 1H), 1.70 - 1.64 (m, 1H).

Referring to the synthesis method of Example B5, the following compounds were synthesized:

| Compound | Structure | LC-MS | NMR |
|---|---|---|---|
| TA-58 | 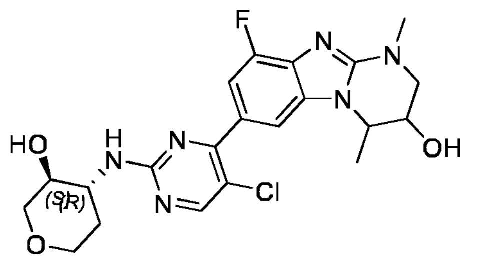 **7-(5-chloro-2-(((3*S*,4*R*)-3-hydroxytetrahydro-2*H*-pyran-4-yl)amino)pyrimidin-4-yl)-9-fluoro-1,4-dimethyl-1,2,3,4-tetrahydrobenzo[4,5]imidazo[1,2-*a*]pyrimidin-3-ol** | [M+H]$^+$= 463.2 | |

(continued)

| Compound | Structure | LC-MS | NMR |
|---|---|---|---|
| TB-37 | 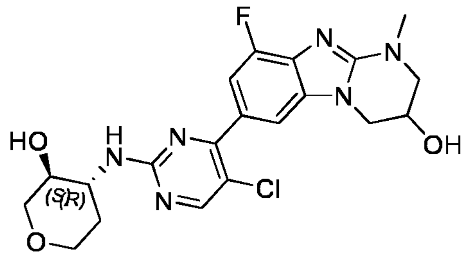<br>**7-(5-chloro-2-(((3*S*,4*R*)-3-hydroxytetrahydro-2*H*-pyran-4-yl)amino)pyrimidin-4-yl)-9-fluoro-1-methyl-1,2,3,4-tetrahydrobenzo[4,5]imidazo[1,2-*a*]pyrimidin-3-ol** | $[M+H]^+$= 449.2 | |
| TB-41 | 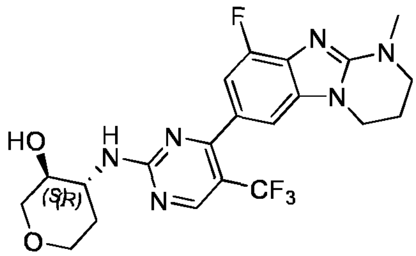<br>**(3*S*,4*R*)-4-((4-(9-fluoro-1-methyl-1,2,3,4-tetrahydrobenzo[4,5]imidazo[1,2-*a*]pyrimidin-7-yl)-5-(trifluoromethyl)pyrimidin-2-yl)amino)tetrahydro-2*H*-pyran-3-ol** | $[M+H]^+$= 467.6 | $^1$H NMR (400 MHz, Chloroform-*d*) $\delta$ 8.55 (s, 1H), 7.11 (d, *J* = 12.0 Hz, 1H), 7.03 (s, 1H), 5.60 (d, *J* = 5.2 Hz, 1H), 4.69 (s, 1H), 4.05 (m, 1H), 3.98 (t, *J* = 6.0 Hz, 2H), 3.96 (m, 2H), 3.63 (m, 1H), 3.47 (m, 1H), 3.42 (t, *J* = 5.2 Hz, 2H), 3.26 (s, 3H), 3.20 (m, 1H), 2.28 (p, *J* = 5.2 Hz, 2H), 2.07 (dd, *J* = 4.7, 2.3 Hz, 1H), 1.73 (d, *J* = 13.7 Hz, 1H). |
| TB-42 | 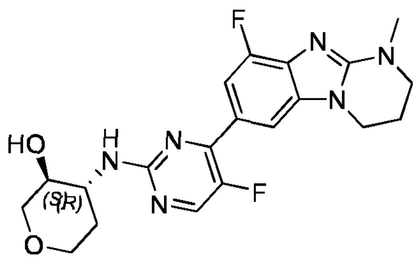<br>**(3*S*,4*R*)-4-((5-fluoro-4-(9-fluoro-1-methyl-1,2,3,4-tetrahydrobenzo[4,5]imidazo[1,2-*a*]pyrimidin-7-yl)pyrimidin-2-yl)amino)tetrahydro-2*H*-pyran-3-ol** | $[M+H]^+$= 417.6 | $^1$H NMR (400 MHz, Chloroform-*d*) $\delta$ 8.14 (d, *J* = 4.2 Hz, 1H), 7.66 (dd, *J* = 12.1, 1.5 Hz, 1H), 7.56 (d, *J* = 1.5 Hz, 1H), 5.17 (d, *J* = 5.8 Hz, 1H), 4.11 - 3.96 (m, 4H), 3.82 (td, *J* = 11.7, 10.7, 5.1 Hz, 1H), 3.67 - 3.61 (m, 1H), 3.53 - 3.40 (m, 3H), 3.27 (s, 3H), 3.27 - 3.17 (m, 1H), 2.30 (h, *J* = 5.9, 5.4 Hz, 3H), 2.08 - 2.01 (m, 1H), 1.73 - 1.68 (m, 2H). |

(continued)

| Compound | Structure | LC-MS | NMR |
|---|---|---|---|
| TB-43 | 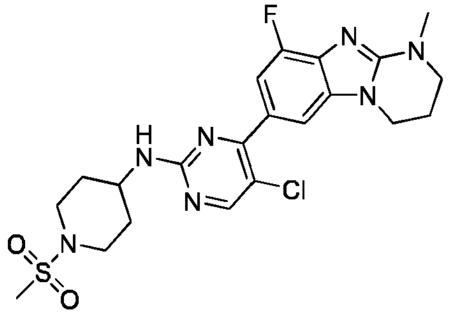 **5-chloro-4-(9-fluoro-1-methyl-1,2,3,4-tetrahydrobenzo [4,5]imidazo[1,2-a]pyrimidin-7-yl)-*N*-(1-(methylsulfonyl) piperidin-4-yl)pyrimidin-2-amine** | $[M+H]^+$= 494.8 | $^1$H NMR (400 MHz, Chloroform-*d*) $\delta$ 8.27 (s, 1H), 7.43 (dd, *J* = 12.0, 1.2 Hz, 1H), 7.40 (s, 1H), 5.14 (d, *J* = 8.0 Hz, 1H), 4.02 (t, *J* = 6.0 Hz, 2H), 4.00 - 3.91 (m, 1H), 3.75 (dt, *J* = 12.0 3.6 Hz, 2H), 3.43 (t, *J* = 5.6 Hz,2H), 3.27 (s, 3H), 2.93 (td, *J* = 12.0, 3.6 Hz, 2H), 2.81 (s, 3H), 2.33 - 2.24 (m, 2H), 2.18 (dd, *J* = 12.0, 3.6 Hz, 2H), 1.62 (dd, *J* = 12.0, 3.6 Hz, 2H). |
| TB-44 | 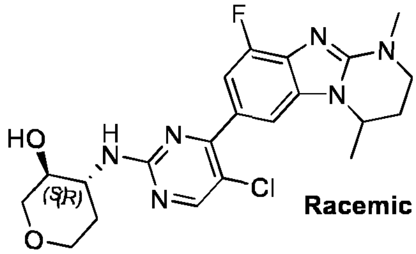 **Racemic** **(3*S*,4*R*)-4-((5-chloro-4-(9-fluoro-1,4-dimethyl-1,2,3,4-tetrahydrobenzo[4,5]imidazo[1,2-*a*]pyrimidin-7-yl)pyrimidin-2-yl)amino)tetrahydro-2*H*-pyran-3-ol** | $[M+H]^+$= 447.2 | |
| TB-45 | 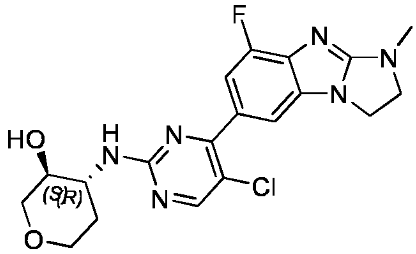 **(3*S*,4*R*)-4-((5-chloro-4-(8-fluoro-1-methyl-2,3-dihydro-1*H*-benzo[*d*]imidazo[1,2-*a*]imidazol-6-yl)pyrimidin-2-yl)amino)tetrahydro-2*H*-pyran-3-ol** | $[M+H]^+$= 419.2 | $^1$H NMR (400 MHz, DMSO-$d_6$) $\delta$ 8.43 (s, 1H), 7.55 (d, *J* = 4. 0Hz, 1H), 7.45 (d, *J* = 8.0 Hz, 1H), 7.37 (dd, *J* = 12.0, 4.0 Hz, 1H), 5.00 (d,*J* = 8.0 Hz, 1H), 4.26 (m, 2H), 4.04 (m, 2H), 3.91 - 3.82 (m, 3H), 3.55 (m, 1H), 3.11 (t, *J* = 12.0 Hz, 1H), 3.04 (s, 3H), 2.61 (s, 1H), 2.00 (m, 1H), 1.54 (m, 1H). |
| TB-48 | 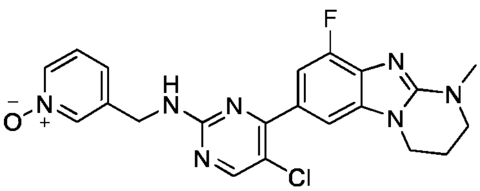 **3-(((5-chloro-4-(9-fluoro-1-methyl-1,2,3,4-tetrahydrobenzo[4,5]imidazo[1,2-a]pyrimidin-7-yl)pyrimidin-2-yl) amino)methyl)pyridine 1-oxide** | $[M+H]^+$= 440.6 | $^1$H NMR (400 MHz, Chloroform-*d*) $\delta$ 8.26 (s, 1H), 8.22 (s, 1H), 8.09 (dt, *J* = 4.3, 2.0 Hz, 1H), 7.39 (dd, *J* = 11.8, 1.5 Hz, 1H), 7.30 (d, *J* = 1.5 Hz, 1H), 7.21 (d,*J* = 4.8 Hz, 2H), 6.15 (s, 1H), 4.53 (d, *J*= 6.3 Hz, 2H), 4.02 (t, *J* = 6.1 Hz, 2H), 3.42 (t, *J* = 5.7 Hz, 2H), 3.26 (s, 3H), 2.28 (p, *J* = 6.0 Hz, 2H). |

(continued)

| Compound | Structure | LC-MS | NMR |
|---|---|---|---|
| TB-49 | 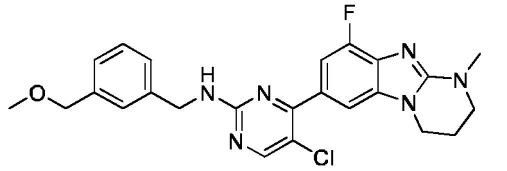 **5-chloro-4-(9-fluoro-1-methyl-1,2,3,4-tetrahydrobenzo [4,5]imidazo[1,2-*a*]pyrimidin-7-yl)-*N*-(3-(methoxymethyl)benzyl)pyrimidin-2-amine** | $[M+H]^+$= 467.5 | $^1$H NMR (400 MHz, Chloroform-*d*) $\delta$ 8.28 (s, 1H), 7.47 (d, *J* = 12.0 Hz, 1H), 7.38 (d, *J* = 1.2 Hz, 1H), 7.35 (s, 1H), 7.34 - 7.27 (m, 2H), 7.23 (s, 1H), 5.56 (t, *J* = 4.0 Hz, 1H), 4.66 (d, *J* = 4.0 Hz, 2H), 4.45 (s, 2H), 4.00 (t, *J* = 6.0 Hz, 2H), 3.42 (t, *J* = 6.0 Hz, 2H), 3.38 (s, 3H), 3.27 (s, 3H), 2.28 (p, *J* = 6.0 Hz, 2H). |
| TB-50 | 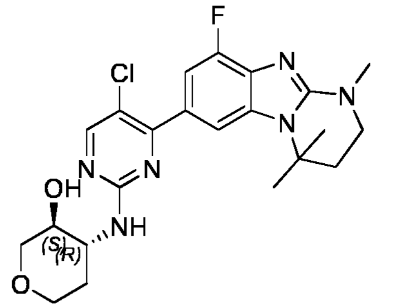 **(3*S*,4*R*)-4-((5-chloro-4-(9-fluoro-1,4,4-trimethyl-1,2,3,4-tetrahydrobenzo[4,5]imidazo[1 ,2-*a*]pyrimidin-7-yl)pyrimidin-2-yl)amino)tetrahydro-2*H*-pyran-3-ol** | $[M+H]^+$= 461.2 | |
| TB-51 | 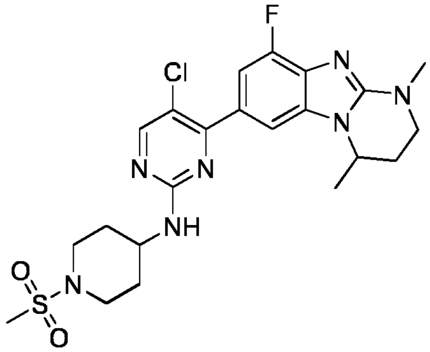 **5-chloro-4-(9-fluoro-1,4-dimethyl-1,2,3,4-tetrahydrobenzo[4,5]imidazo[1,2-a]pyrimidin-7-yl)-*N*-(1-(methylsulfonyl)piperidin-4-yl)pyrimidin-2-amine** | $[M+H]^+$= 508.50 | $^1$H NMR (400 MHz, Chloroform-*d*) $\delta$ 8.28 (s, 1H), 7.47 - 7.38 (m, 2H), 5.11 (d, *J* = 8.0 Hz, 1H), 4.57 - 4.52 (m, 1H), 4.0 - 3.97 (m, 1H), 3.76 (d, *J* = 12.0 Hz, 2H), 3.59 (td, *J* = 12.0, 4.0 Hz, 1H), 3.33 - 3.29 (m, 1H), 3.28 (s, 3H), 2.93 (td, *J* = 12.0, 4.0 Hz, 2H), 2.81 (s, 3H), 2.44 - 2.35 (m, 1H), 2.18 (d, *J*= 12.0 Hz, 2H), 2.00 (dq, *J* = 12.0, 8.0, 4.0 Hz, 1H), 1.70 - 1.61 (m, 2H), 1.50 (d, *J* = 8.0 Hz, 3H). |
| TB-52 | 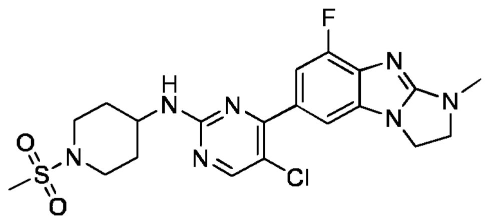 **5-chloro-4-(8-fluoro-1-methyl-2,3-dihydro-1*H*-benzo[*d*] imidazo[1,2-*a*]imidazol-6-yl)-*N*-(1-(methylsulfonyl)piperidin-4-yl)pyrimidin-2-amine** | $[M+H]^+$= 480.4 | $^1$H NMR (400 MHz, DMSO-$d_6$) $\delta$ 8.45 (s, 1H), 7.61 (s, 1H), 7.54 (s, 1H), 7.36 (dd, *J* = 12.0, 4.0 Hz, 1H), 4.25 (t, *J* = 16.0 Hz, 2H), 4.03 (t, *J* = 16.0 Hz, 2H), 3.94 (m, 1H), 3.59 (m, 2H), 3.04 (s, 3H), 2.93 (m, 5H), 2.04 (m, 2H), 1.63 (m, 2H). |

(continued)

| Compound | Structure | LC-MS | NMR |
|---|---|---|---|
| TB-64 | 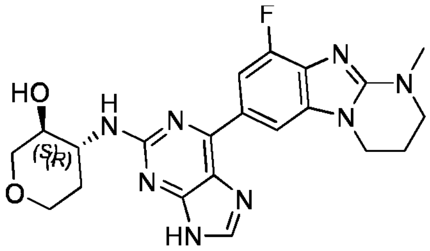 **(3*S*,4*R*)-4-((6-(9-fluoro-1-methyl-1,2,3,4-tetrahydrobenzo[4,5]imidazo[1,2-*a*]pyrimidin-7-yl)-9*H*-purin-2-yl)amino)tetrahydro-2*H*-pyran-3-ol** | $[M+H]^+$= 439.2 | |
| TB-74 | 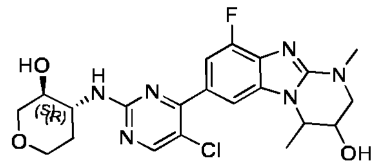 **7-(5-chloro-2-(((3*S*,4*R*)-3-hydroxytetrahydro-2*H*-pyran-4-yl)amino)pyrimidin-4-yl)-9-fluoro-1,4-dimethyl-1,2,3,4-tetrahydrobenzo[4,5]imidazo[1,2-*a*]pyrimidin-3-ol** | $[M+H]^+$= 463.2 | |
| TB-91 | 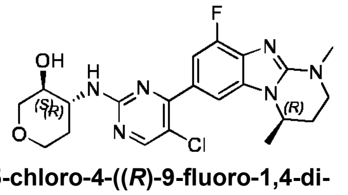 **(3*S*,4*R*)-4-((5-chloro-4-((*R*)-9-fluoro-1,4-dimethyl-1,2,3,4-tetrahydrobenzo[4,5]imidazo[1,2-*a*]pyrimidin-7-yl)pyrimidin-2-yl)amino)tetrahydro-2*H*-pyran-3-ol** | $[M+H]^+$= 447.2 | |
| TB-92 | 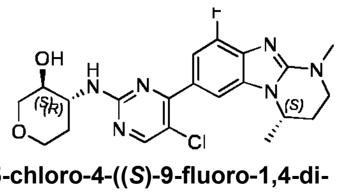 **(3*S*,4*R*)-4-((5-chloro-4-((*S*)-9-fluoro-1,4-dimethyl-1,2,3,4-tetrahydrobenzo[4,5]imidazo[1,2-*a*]pyrimidin-7-yl)pyrimidin-2-yl)amino)tetrahydro-2*H*-pyran-3-ol** | $[M+H]^+$= 447.2 | |
| TB-93 | 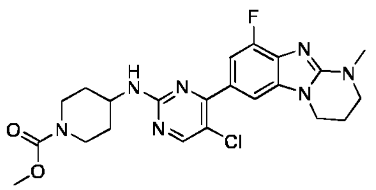 **methyl 4-((5-chloro-4-(9-fluoro-1-methyl-1,2,3,4-tetrahydrobenzo[4,5]imidazo[1,2-*a*]pyrimidin-7-yl)pyrimidin-2-yl)amino)piperidine-1-carboxylate** | $[M+H]^+$= 474.2 | |

(continued)

| Compound | Structure | LC-MS | NMR |
|---|---|---|---|
| TB-95 | 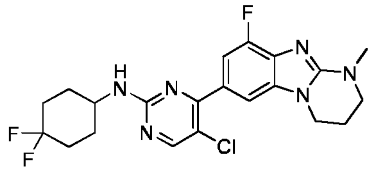 **5-chloro-*N*-(4,4-difluorocyclohexyl)-4-(9-fluoro-1-methyl-1,2,3,4-tetrahydrobenzo[4,5]imidazo[1,2-*a*]pyrimidin-7-yl)pyrimidin-2-amine** | $[M+H]^+$= 451.3 | $^1$H NMR (400 MHz, Methanol-$d_4$) $\delta$ 8.27 (s, 1H), 7.50 (d, *J* = 1.2 Hz, 1H), 7.40 (dd, *J* = 12.0, 1.2 Hz, 1H), 4.65 (s, 1H), 4.04 (t, *J* = 6.0 Hz, 2H), 3.96 (s, 1H), 3.47 (t, *J* = 6.0 Hz, 2H), 3.20 (s, 3H), 2.28 (p, *J* = 6.0 Hz, 2H), 2.08 (m, 4H), 1.99 - 1.84 (m, 2H), 1.76 - 1.62 (m, 2H). |
| TB-100 | 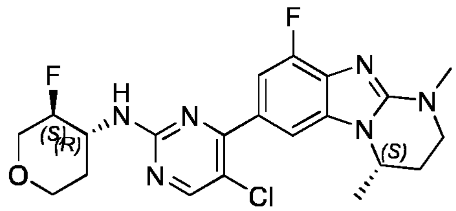 **5-chloro-4-((*S*)-9-fluoro-1,4-dimethyl-1,2,3,4-tetrahydrobenzo[4,5]imidazo[1,2-*a]pyrimidin-7-yl)-N-((3S,4R)-3-*fluorotetrahydro-2*H*-pyran-4-yl)pyrimidin-2-amine** | $[M+H]^+$= 449.3 | |
| TB-101 | 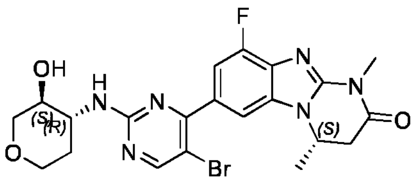 **(*S*)-7-(5-bromo-2-(((3*S*,4*R*)-3-hydroxytetrahydro -2*H*-pyran-4-yl)amino)pyrimidin-4-yl)-9-fluoro-1,4-dimethyl-3,4-dihydrobenzo[4,5]imidazo [1,2-*a*]pyrimidin-2(1*H*)-one** | $[M+H]^+$= 505.1 | $^1$H NMR (400 MHz, Chloroform-*d*) $\delta$ 8.32 (s, 1H), 7.53 (s, 1H), 7.48 (d, *J* = 11.4 Hz, 1H), 5.32 (d, *J* = 6.0 Hz, 1H), 4.75 (p, *J* = 6.9 Hz, 1H), 4.05 (dd, *J* = 11.5, 5.0 Hz, 1H), 3.99 (dd, *J* = 11.7, 4.1 Hz, 1H), 3.90 - 3.80 (m, 1H), 3.67 (dt, *J* = 8.5, 4.5 Hz, 1H), 3.63 (d, *J* = 4.9 Hz, 1H), 3.60 (s, 3H), 3.46 (ddd, *J* = 14.1, 9.8, 2.2 Hz, 2H), 3.27 - 3.14 (m, 2H), 2.11 - 1.99 (m, 1H), 1.76 - 1.69 (m, 1H), 1.47 (d, *J* = 6.7 Hz, 3H). |
| TB-102 | 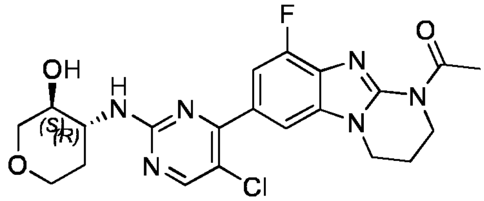 **1-(7-(5-chloro-2-(((3*S*,4*R*)-3-hydroxytetrahydro-2*H*-pyran-4-yl)amino)pyrimidin-4-yl)-9-fluoro-3,4-dihydrobenzo[4,5]imidazo[1,2-*a*]pyrimidin-1(2*H*)-yl)ethan-1-one** | $[M+H]^+$= 461.3 | $^1$H NMR (400 MHz, DMSO-d6) $\delta$ 8.41 (s, 1H), 7.72 (s, 1H), 7.44 (d, J = 12.4 Hz, 2H), 4.95 (d, J = 5.4 Hz, 1H), 4.19 (t, J = 6.1 Hz, 2H), 4.01 - 3.94 (m, 2H), 3.81 (dq, J = 10.4, 6.0, 5.3 Hz, 3H), 3.54 - 3.45 (m, 2H), 3.05 (t, J = 10.4 Hz, 1H), 2.72 (s, 3H), 2.19 (p, J = 5.9 Hz, 2H), 1.94 (m, 1H), 1.56 - 1.42 (m, 1H). |

(continued)

| Compound | Structure | LC-MS | NMR |
|---|---|---|---|
| TB-103 | 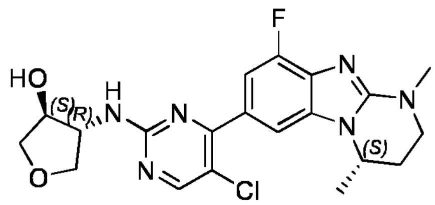 **(3*S*,4*R*)-4-((5-chloro-4-((*S*)-9-fluoro-1,4-dimethyl-1,2,3,4-tetrahydrobenzo[4,5]imidazo[1,2-*a*]pyrimidin-7-yl)pyrimidin-2-yl)amino)tetrahydrofuran-3-ol** | $[M+H]^+$= 433.2 | $^1$H NMR (400 MHz, Chloroform-*d*) $\delta$ 8.31 (s, 1H), 7.44 - 7.36 (m, 2H), 5.48 (d, *J* = 5.0 Hz, 1H), 4.53 (ddd, *J* = 11.5, 6.3, 3.3 Hz, 1H), 4.32 (ddd, *J* = 6.1, 3.9, 2.7 Hz, 1H), 4.27 (dd, *J* = 9.1, 6.4 Hz, 1H), 4.25 - 4.19 (m, 1H), 4.13 - 4.08 (m, 1H), 3.74 (ddd, *J* = 18.1, 9.4, 4.1 Hz, 2H), 3.58 (td, *J* = 12.3, 3.2 Hz, 1H), 3.34 - 3.28 (m, 1H), 3.27 (s, 3H), 2.38 (tt, *J* = 12.7, 5.1 Hz, 1H), 1.98 (dq, *J* = 13.8, 2.9 Hz, 1H), 1.49 (d, *J* = 6.6 Hz, 3H). |
| TB-104 | 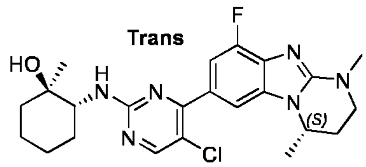 **(1*R*,2*R*)-2-((5-chloro-4-((*S*)-9-fluoro-1,4-dimethyl-1,2,3,4-tetrahydrobenzo[4,5]imidazo[1,2-*a*]pyrimidin-7-yl)pyrimidin-2-yl)amino)-1-methylcyclohexan-1-ol** | $[M+H]^+$= 459.4 | |
| TB-105 | 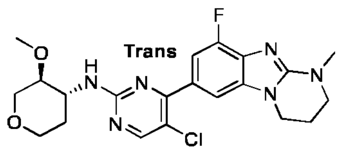 **5-chloro-4-(9-fluoro-1-methyl-1,2,3,4-tetrahydrobenzo[4,5]imidazo[1,2-*a*]pyrimidin-7-yl)-*N*-((3*S*,4*R*)-3-methoxytetrahydro-2*H*-pyran-4-yl)pyrimidin-2-amine** | $[M+H]^+$= 447.3 | $^1$H NMR (400 MHz, Chloroform-*d*) $\delta$ 8.28 (s, 1H), 7.44 (dd, *J* = 11.8, 1.5 Hz, 1H), 7.39 (s, 1H), 5.28 (d, *J* = 7.2 Hz, 1H), 4.10 (dd, *J* = 11.3, 4.0 Hz, 1H), 4.06 (d, *J* = 4.5 Hz, 1H), 4.02 (t, *J* = 6.1 Hz, 2H), 3.87 (dt, *J* = 11.8, 4.2 Hz, 1H), 3.60 - 3.52 (m, 1H), 3.44 (s, 3H), 3.42 (d, *J* = 5.9 Hz, 2H), 3.35 (dd, *J* = 11.4, 8.4 Hz, 1H), 3.26 (s, 3H), 3.23 (dd, *J* = 8.4, 4.0 Hz, 1H), 2.38 - 2.31 (m, 1H), 2.28 (q, *J* = 5.9 Hz, 2H), 1.61 - 1.53 (m, 1H). |

(continued)

| Compound | Structure | LC-MS | NMR |
|---|---|---|---|
| TB-106 | 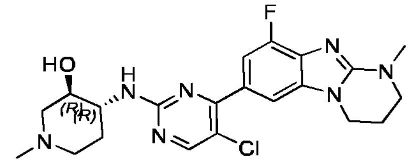 **(3*R*,4*R*)-4-((5-chloro-4-(9-fluoro-1-methyl-1,2,3,4-tetrahydrobenzo[4,5]imidazo[1,2-*a*]pyrimidin-7-yl)pyrimidin-2-yl)amino)-1-methylpiperidin-3-ol** | $[M+H]^+$= 446.3 | $^1$H NMR (400 MHz, Chloroform-*d*) $\delta$ 8.25 (s, 1H), 7.42 (dd, *J* = 11.8, 1.5 Hz, 1H), 7.33 (d, *J*= 1.5 Hz, 1H), 5.23 (d, *J* = 5.2 Hz, 1H), 4.02 (t, *J* = 6.1 Hz, 2H), 3.75 - 3.66 (m, 2H), 3.43 (t, *J* = 5.6 Hz, 2H), 3.27 (s, 3H), *3.06* (d, *J* = 11.3 Hz, 1H), 2.82 (d, *J* = 11.7 Hz, 1H), 2.34 (s, 3H), 2.28 (p, *J* = 5.9 Hz, 2H), 2.18 - 1.98 (m, 4H), 1.61 - 1.53 (m, 1H). |
| TB-107 | 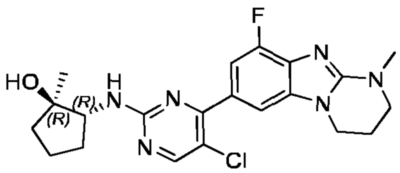 **(1*R*,2*R*)-2-((5-chloro-4-(9-fluoro-1-methyl-1,2,3,4-tetrahydrobenzo[4,5]imidazo[1,2-*a*]pyrimidin-7-yl)pyrimidin-2-yl)amino)-1-methylcyclopentan-1-ol** | $[M+H]^+$= 431.3 | $^1$H NMR (400 MHz, Methanol-$d_4$) $\delta$ 8.29 (s, 1H), 7.45 (s, 1H), 7.39 (dd, *J* = 12.0, 1.4 Hz, 1H), 4.61 (s, 1H), 4.18 (t, *J* = 8.2 Hz, 1H), 4.04 (t, *J* = 6.1 Hz, 2H), 3.52 - 3.43 (m, 2H), 3.20 (s, 3H), 2.26 (q, *J* = 5.4 Hz, 2H), 2.21 (dd, *J* = 8.9, 4.7 Hz, 1H), 1.92 - 1.55 (m, 6H), 1.19 (s, 3H). |
| TB-108 | 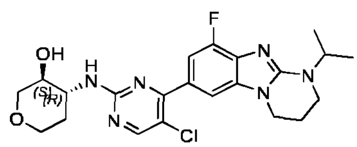 **(3*S*,4*R*)-4-((5-chloro-4-(9-fluoro-1-isopropyl-1,2,3,4-tetrahydrobenzo[4,5]imidazo[1,2-*a*]pyrimidin-7-yl)pyrimidin-2-yl)amino)tetrahydro-2*H*-pyran-3-ol** | $[M+H]^+$= 461.3 | $^1$H NMR (400 MHz, Chloroform-d) $\delta$ 8.19 (s, 1H), 7.35 (d, J = 12.0 Hz, 1H), 7.26 (s, 1H), 5.22 (d, J = 4.0 Hz, 1H), 4.91 (p, J = 6.8 Hz, 1H), 3.95 (dt, J = 20.0, 6.0 Hz, 4H), 3.75 (tt, J = 10.0, 4.0 Hz, 1H), 3.55 (td, J = 8.0, 4.0 Hz, 1H), 3.39 (td, J = 12.0, 4.0 Hz, 1H), 3.29 (t, J = 6.0 Hz, 2H), 3.11 (t, J = 10.0 Hz, 1H), 2.15 (p, J = 6.0 Hz, 2H), 2.01 - 1.92 (m, 1H), 1.59 (d, J = 6.0 Hz, 1H), 1.18 (d, J = 8.0 Hz, 6H). |

(continued)

| Compound | Structure | LC-MS | NMR |
|---|---|---|---|
| TB-109 | 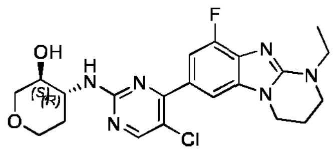 **(3*S*,4*R*)-4-((5-chloro-4-(1-ethyl-9-fluoro-1,2,3,4-tetrahydrobenzo[4,5]imidazo[1,2-*a*]pyrimidin-7-yl)pyrimidin-2-yl)amino)tetrahydro-2*H*-pyran-3-ol** | $[M+H]^+$= 447.3 | $^1$H NMR (400 MHz, Chloroform-*d*) δ 8.25 (s, 1H), 7.41 (dd, *J* = 11.8, 1.5 Hz, 1H), 7.32 (d, *J*= 1.5 Hz, 1H), 5.27 (d, *J* = 5.9 Hz, 1H), 4.01 (m, 4H), 3.89 - 3.78 (m, 1H), 3.74 (q, *J* = 7.1 Hz, 2H), 3.62 (td, *J* = 9.5, 4.9 Hz, 1H), 3.48 (d, *J* = 2.2 Hz, 1H), 3.43 (t, *J* = 5.8 Hz, 2H), 3.18 (t, *J* = 9.9 Hz, 1H), 2.26 (p, *J* = 6.0 Hz, 2H), 2.09 - 1.99 (m, 1H), 1.68 (m, 2H), 1.27 (t, *J* = 7.2 Hz, 3H). |
| TB-110 | 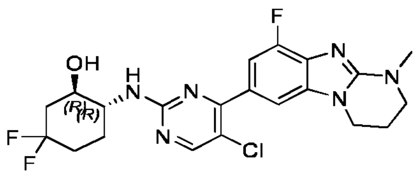 **(1*R*,2*R*)-2-((5-chloro-4-(9-fluoro-1-methyl-1,2,3,4-tetrahydrobenzo[4,5]imidazo[1,2-*a*]pyrimidin-7-yl)pyrimidin-2-yl)amino)-5,5-difluorocyclohexan-1-ol** | $[M+H]^+$= 467.2 | $^1$H NMR (400 MHz, Chloroform-d) δ 8.19 (s, 1H), 7.36 (dd, J = 12.0, 1.5 Hz, 1H), 7.26 (d, J = 4.0 Hz, 1H), 5.17 (d, J = 8.0 Hz, 1H), 3.94 (t, J = 12.0 Hz, 2H), 3.74 (m, 2H), 3.36 (t, J = 6.0 Hz, 2H), 3.20 (s, 3H), 2.54 - 2.45 (m, 1H), 2.22 (m, 2H), 2.05 (m, 2H), 1.93 - 1.84 (m, 2H), 1.84 - 1.74 (m, 2H). |
| TB-111 | 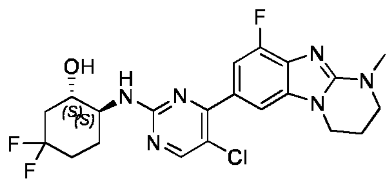 (1*S*,2*S*)-2-((5-chloro-4-(9-fluoro-1-methyl-1,2,3,4-tetrahydrobenzo[4,5]imidazo[1,2-*a*]pyrimidin-7-yl)pyrimidin-2-yl)amino)-5,5-difluorocyclohexan-1-ol | $[M+H]^+$= 467.1 | $^1$H NMR (400 MHz, Chloroform-d) δ 8.18 (s, 1H), 7.35 (d, J = 12.0 Hz, 1H), 7.26 (d, J = 4.0 Hz, 1H), 5.19 (d, J = 8.0 Hz, 1H), 3.93 (t, J = 4.0 Hz, 2H), 3.82 - 3.69 (m, 2H), 3.36 (t, J = 6.0 Hz, 2H), 3.19 (s, 3H), 2.56 - 2.44 (m, 1H), 2.21 (p, J = 6.0 Hz, 2H), 2.05 (td, J = 12.0, 12.0, 4.0 Hz, 2H), 1.88 (dq, J = 12.0, 4.0, 4.0 Hz, 1H), 1.84 - 1.72 (m, 2H). |

(continued)

| Compound | Structure | LC-MS | NMR |
|---|---|---|---|
| TB-112 | 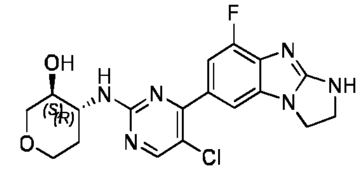 (3*S*,4*R*)-4-((5-chloro-4-(8-fluoro-2,3-**dihydro-1*H*-benzo[*d*] imidazo[1,2-*a*]imidazol-6-yl)pyrimidin-2-yl)amino)tetra-hydro-2*H*-pyran-3-ol** | $[M+H]^+$= 405.1 | $^1$H NMR (400 MHz, DMSO-d6) $\delta$ 7.63 (d, J = 1.4 Hz, 1H), 7.53 (s, 2H), 7.45 (dd, J = 12.4, 1.5 Hz, 1H), 5.09 (d, J = 5.4 Hz, 1H), 4.35 (dd, J = 9.4, 6.7 Hz, 2H), 4.19 (dd, J = 9.3, 6.9 Hz, 2H), 4.01 - 3.92 (m, 3H), 3.64 (dd, J = 10.0, 5.1 Hz, 2H), 3.19 (t, J = 10.4 Hz, 1H), 2.09 (d, J = 13.1 Hz, 1H), 1.71 - 1.54 (m, 2H). |
| TB-113 | 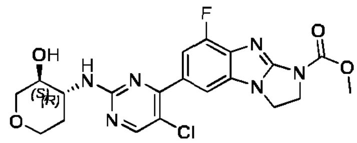 **methyl 6-(5-chloro-2-(((3*S*,4*R*)-3-hydroxytetrahydro-2H-pyran-4-yl)amino)pyrimidin-4-yl)-8-fluoro-2,3-dihydro-1*H*-benzo[*d*]imidazo[1,2-*a*]imidazole-1-carboxylate** | $[M+H]^+$= 463.3 | $^1$H NMR (400 MHz, DMSO-d6) $\delta$ 8.55 (s, 1H), 7.82 (s, 1H), 7.61 (s, 1H), 7.54 (d, J = 12.1 Hz, 1H), 5.08 (d, J = 5.3 Hz, 1H), 4.64 (dd, J = 9.7, 6.1 Hz, 2H), 4.54 - 4.40 (m, 2H), 4.00 (s, 3H), 3.97 (s, 2H), 3.19 (t, J = 10.4 Hz, 1H), 2.09 (s, 2H), 1.63 (d, J = 9.8 Hz, 2H), 1.46 (d, J = 15.4 Hz, 1H). |
| TB-114 | 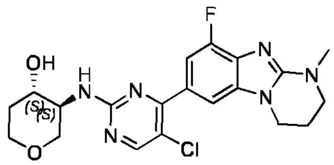 **(3*S*,4*S*)-3-((5-chloro-4-(9-fluoro-1-methyl-1,2,3,4-tetrahydrobenzo[4,5]imidazo[1,2-*a*]pyrimidin-7-yl)pyrimidin-2-yl)amino)tetrahydro-2*H*-pyran-4-ol** | $[M+H]^+$= 433.2 | $^1$H NMR (400 MHz, Chloroform-*d*) $\delta$ 8.26 (s, 1H), 7.44 (dd, *J* = 11.8, 1.5 Hz, 1H), 7.35 (d, *J* = 1.5 Hz, 1H), 5.26 (d, *J* = 7.4 Hz, 1H), 4.14 (dd, *J* = 11.2, 4.4 Hz, 1H), 4.02 (t, *J* = 6.1 Hz, 2H), 3.96 - 3.90 (m, 1H), 3.77 (m, 1H), 3.48 (td, *J* = 11.7, 2.8 Hz, 1H), 3.43 (t, *J* = 6.1 Hz, 2H), 3.27 (s, 3H), 3.25 (d, *J* = 2.8 Hz, 1H), 2.28 (p, *J* = 5.9 Hz, 2H), 2.10 - 2.04 (m, 1H), 1.73 (s, 1H), 0.89 - 0.82 (m, 2H). |

(continued)

| Compound | Structure | LC-MS | NMR |
|---|---|---|---|
| TB-115 | 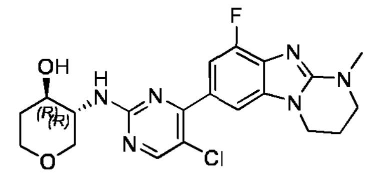 **(3*R*,4*R*)-3-((5-chloro-4-(9-fluoro-1-methyl-1,2,3,4-tetrahydrobenzo[4,5]imidazo[1,2-*a*]pyrimidin-7-yl)pyrimidin-2-yl)amino)tetrahydro-2*H*-pyran-4-ol** | $[M+H]^+$= 433.3 | $^1H$ NMR (400 MHz, Chloroform-*d*) $\delta$ 8.25 (s, 1H), 7.43 (d, *J* = 11.8 Hz, 1H), 7.36 - 7.31 (m, 1H), 5.28 (d, *J* = 7.4 Hz, 1H), 4.16 - 4.11 (m, 1H), 4.00 (t, *J* = 6.1 Hz, 2H), 3.92 (ddt, *J* = 15.9, 8.1, 4.3 Hz, 2H), 3.76 (td, *J* = 8.8, 4.5 Hz, 1H), 3.48 (ddd, *J* = 12.2, 10.4, 2.8 Hz, 1H), 3.42 (t, *J* = 5.7 Hz, 2H), 3.26 (s, 3H), 3.22 (s, 1H), 2.27 (p, *J* = 5.9 Hz, 2H), 2.13 - 1.99 (m, 2H), 0.88 - 0.83 (m, 1H). |
| TB-116 | 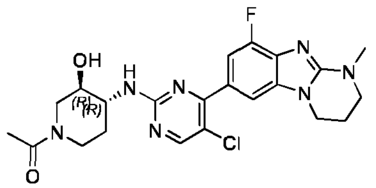 **1-((3*R*,4*R*)-4-((5-chloro-4-(9-fluoro-1-methyl-1,2,3,4-tetrahydrobenzo[4,5]imidazo[1,2-*a*]pyrimidin-7-yl)pyrimidin-2-yl)amino)-3-hydroxypiperidin-1-yl)ethan-1-one** | $[M+H]^+$= 474.2 | $^1H$ NMR (400 MHz, Chloroform-*d*) $\delta$ 8.26 (d, J = 2.3 Hz, 1H), 7.42 (dd, *J* = 11.7, 1.5 Hz, 1H), 7.33 (d, J = 1.6 Hz, 1H), 5.27 (d, J = 6.1 Hz, 1H), 4.68 - 4.59 (m, 1H), 4.06 - 3.94 (m, 3H), 3.87 - 3.76 (m, 1H), 3.53 (td, J = 9.6, 4.9 Hz, 1H), 3.43 (t, J = 5.6 Hz, 2H), 3.27 (s, 3H), 2.97 (dd, J = 13.5, 10.4 Hz, 1H), 2.70 - 2.53 (m, 1H), 2.28 (p, J = 6.0 Hz, 2H), 2.12 (d, J = 6.9 Hz, 3H), 2.05 (dt, J = 12.9, 3.8 Hz, 1H), 1.52 (qd, J = 12.4, 4.5 Hz, 2H). |
| TB-117 | 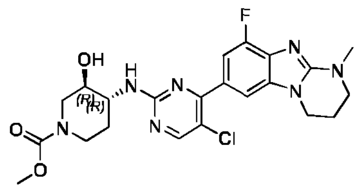 **methyl (3*R*,4*R*)-4-((5-chloro-4-(9-fluoro-1-methyl-1,2,3,4-tetrahydrobenzo[4,5]imidazo[1,2-*a*]pyrimidin-7-yl)pyrimidin-2-yl)amino)-3-hydroxypiperidine-1-carboxylate** | $[M+H]^+$= 490.2 | |

(continued)

| Compound | Structure | LC-MS | NMR |
|---|---|---|---|
| TB-118 | 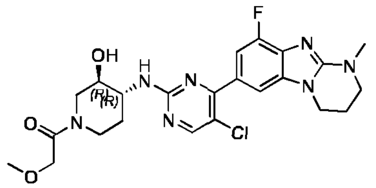 **1-((3*R*,4*R*)-4-((5-chloro-4-(9-fluoro-1-methyl-1,2,3,4-tetrahydrobenzo[4,5]imidazo[1,2-*a*]pyrimidin-7-yl)pyrimidin-2-yl)amino)-3-hydroxypiperidin-1-yl)-2-methoxyethan-1-one** | $[M+H]^+$= 504.3 | |
| TB-119 | 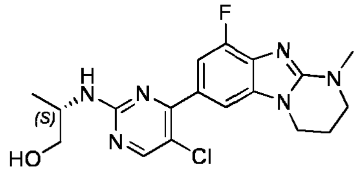 **(*S*)-2-((5-chloro-4-(9-fluoro-1-methyl-1,2,3,4-tetrahydrobenzo[4,5]imidazo[1,2-a]pyrimidin-7-yl)pyrimidin-2-yl)amino)propan-1-ol** | $[M+H]^+$= 391.5 | $^1$H NMR (400 MHz, Chloroform-d) δ 8.25 (s, 1H), 7.43 (dd, J = 11.8, 1.5 Hz, 1H), 7.34 (d, J = 1.5 Hz, 1H), 5.33 - 5.21 (m, 1H), 4.16 (qd, J = 6.7, 3.4 Hz, 1H), 4.01 (t, J = 6.1 Hz, 2H), 3.79 (dd, J = 11.0, 3.4 Hz, 1H), 3.63 (dd, J = 10.9, 6.5 Hz, 1H), 3.55 - 3.34 (m, 2H), 3.26 (s, 3H), 2.39 - 2.14 (m, 2H), 1.27 (d, J = 6.8 Hz, 3H). |
| TB-120 | 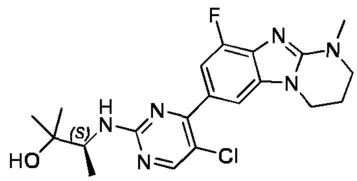 **(*S*)-3-((5-chloro-4-(9-fluoro-1-methyl-1,2,3,4-tetrahydrobenzo[4,5]imidazo[1,2-*a*]pyrimidin-7-yl)pyrimidin-2-yl)amino)-2-methylbutan-2-ol** | $[M+H]^+$= 419.7 | $^1$H NMR (400 MHz, Chloroform-d) δ 8.25 (s, 1H), 7.44 (dd, J = 11.8, 1.5 Hz, 1H), 7.37 (d, J = 1.6 Hz, 1H), 5.31 (d, J = 8.6 Hz, 1H), 4.05 (dt, J = 12.3, 6.4 Hz, 3H), 3.46 - 3.40 (m, 2H), 3.27 (s, 3H), 3.02 (s, 1H), 2.29 (p, J = 6.0 Hz, 3H), 1.29 (s, 3H), 1.25 (d, J = 1.6 Hz, 6H). |
| TB-121 | 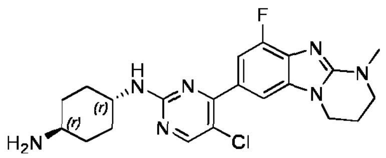 **(1*r*,4*r*)-*N*[1]-(5-chloro-4-(9-fluoro-1-methyl-1,2,3,4-tetrahydrobenzo[4,5]imidazo[1,2-a]pyrimidin-7-yl)pyrimidin-2-yl)cyclohexane-1,4-diamine** | $[M+H]^+$= 430.4 | |
| TB-122 | 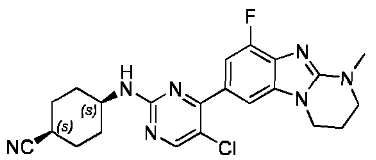 **(1*s*,4*s*)-4-((5-chloro-4-(9-fluoro-1-methyl-1,2,3,4-tetrahydrobenzo[4,5]imidazo[1,2-*a*]pyrimidin-7-yl)pyrimidin-2-yl)amino)cyclohexane-1-carbonitrile** | $[M+H]^+$= 440.7 | |

(continued)

| Compound | Structure | LC-MS | NMR |
|---|---|---|---|
| TB-123 | 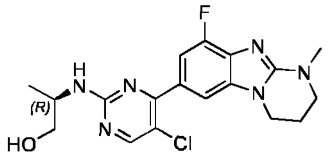 **(*R*)-2-((5-chloro-4-(9-fluoro-1-methyl-1,2,3,4-tetrahydrobenzo[4,5]imidazo[1,2-*a*]pyrimidin-7-yl)pyrimidin-2-yl)amino)propan-1-ol** | $[M+H]^+$= 391.2 | $^1H$ NMR (400 MHz, Chloroform-*d*) δ 8.25 (s, 1H), 7.43 (dd, *J* = 11.8, 1.5 Hz, 1H), 7.34 (d, *J* = 1.5 Hz, 1H), 5.26 (d, *J* = 6.9 Hz, 1H), 4.16 (ddt, *J* = 9.8, 6.5, 3.2 Hz, 1H), 4.01 (t, *J* = 6.1 Hz, 2H), 3.79 (dd, *J* = 10.9, 3.4 Hz, 1H), 3.64 (dd, *J* = 10.9, 6.5 Hz, 1H), 3.44 - 3.40 (m, 2H), 3.27 (s, 3H), 2.28 (p, *J* = 6.0 Hz, 2H), 1.28 (d, *J* = 6.8 Hz, 3H). |
| TB-124 | 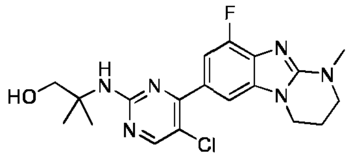 **2-((5-chloro-4-(9-fluoro-1-methyl-1,2,3,4-tetrahydrobenzo[4,5]imidazo[1,2-*a*]pyrimidin-7-yl)pyrimidin-2-yl)amino)-2-methylpropan-1-ol** | $[M+H]^+$= 405.3 | $^1H$ NMR (400 MHz, Chloroform-d) δ 8.24 (s, 1H), 7.41 (dd, J = 11.6, 1.4 Hz, 1H), 7.31 (d, J = 1.4 Hz, 1H), 5.63 (s, 1H), 5.34 (s, 1H), 4.02 (t, J = 6.0 Hz, 2H), 3.71 (s, 2H), 3.42 (t, J = 6.0 Hz, 2H), 3.27 (s, 3H), 2.32 - 2.24 (m, 2H), 1.39 (s, 6H). |
| TB-125 | 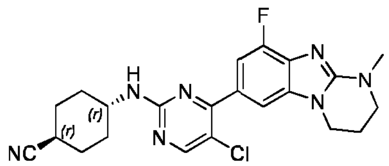 **(1*r*,4*r*)-4-((5-chloro-4-(9-fluoro-1-methyl-1,2,3,4-tetrahydrobenzo[4,5]imidazo[1,2-*a*]pyrimidin-7-yl)pyrimidin-2-yl)amino)cyclohexane-1-carbonitrile** | $[M+H]^+$= 440.3 | $^1H$ NMR (400 MHz, Chloroform-*d*) δ 8.26 (s, 1H), 7.44 (d, *J* = 12.0 Hz, 1H), 7.38 (s, 1H), 5.04 (d, *J* = 7.6 Hz, 1H), 4.02 (t, *J* = 6.0 Hz, 2H), 3.97 - 3.81 (m, 1H), 3.43 (t, *J* = 6.0 Hz, 2H), 3.27 (s, 3H), 2.49 (m, 1H), 2.29 (p, *J* = 6.0 Hz, 2H), 2.25 - 2.13 (m, 4H), 1.81 - 1.73 (m, 2H), 1.30 (q, *J* = 11.8 Hz, 2H). |
| TB-126 | 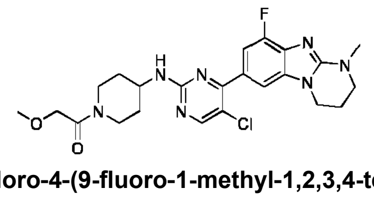 **1-(4-((5-chloro-4-(9-fluoro-1-methyl-1,2,3,4-tetrahydrobenzo[4,5]imidazo[1,2-*a*]pyrimidin-7-yl)pyrimidin-2-yl)amino)piperidin-1-yl)-2-methoxyethan-1-one** | $[M+H]^+$= 488.3 | $^1H$ NMR (400 MHz, Chloroform-d) δ 8.28 (s, 1H), 7.44 (d, J = 12.0 Hz, 1H), 7.39 (s, 1H), 5.11 (d, J = 8.0 Hz, 1H), 4.47 (d, J = 12.0 Hz, 1H), 4.14 - 4.10 (m, 2H), 4.08 (d, J = 6.2 Hz, 1H), 4.02 (t, J = 6.1 Hz, 2H), 3.87 (d, J = 13.9 Hz, 1H), 3.43 (s, 5H), 3.27 (s, 3H), 3.21 (t, J = 12.0 Hz, 1H), 2.93 (t, J = 12.0 Hz, 1H), 2.29 (m, 2H), 2.14 (t, J = 14.5 Hz, 2H), 1.44 (q, J = 12.0 Hz, 2H). |

(continued)

| Compound | Structure | LC-MS | NMR |
|---|---|---|---|
| TB-127 | 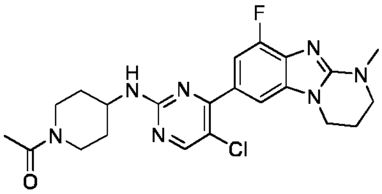 **1-(4-((5-chloro-4-(9-fluoro-1-methyl-1,2,3,4-tetrahydrobenzo[4,5]imidazo[1,2-*a*]pyrimidin-7-yl)pyrimidin-2-yl)amino)piperidin-1-yl)ethan-1-one** | $[M+H]^+$= 458.3 | $^1$H NMR (400 MHz, Chloroform-d) δ 8.27 (s, 1H), 7.44 (dd, J = 11.7, 1.4 Hz, 1H), 7.39 (s, 1H), 5.12 (d, J = 7.8 Hz, 1H), 4.55 - 4.43 (m, 1H), 4.17 - 4.05 (m, 1H), 4.02 (t, J = 6.1 Hz, 2H), 3.80 (dd, J = 13.9, 2.0 Hz, 1H), 3.47 - 3.39 (m, 2H), 3.27 (s, 3H), 3.26 - 3.19 (m, 1H), 2.94 - 2.82 (m, 1H), 2.35 - 2.23 (m, 2H), 2.21 - 2.13 (m, 1H), 2.11 (s, 3H), 2.06 (s, 1H), 1.74 (s, 2H). |
| TB-128 | 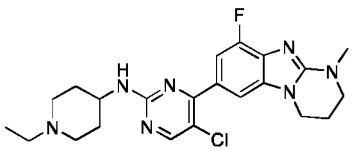 **5-chloro-*N*-(1-ethylpiperidin-4-yl)-4-(9-fluoro-1-methyl-1,2,3,4-tetrahydrobenzo[4,5]imidazo[1,2-*a*]pyrimidin-7-yl)pyrimidin-2-amine** | $[M+H]^+$= 444.3 | $^1$H NMR (400 MHz, Chloroform-d) δ 8.26 (s, 1H), 7.48 - 7.42 (d,J = 12.2 Hz, 1H), 7.40 (s, 1H), 5.25 (d, J = 7.8 Hz, 1H), 4.17 - 4.06 (m, 1H), 4.03 (t, J = 6.1 Hz, 2H), 3.43 (t, J = 5.6 Hz, 2H), 3.32 (m, 2H), 3.27 (s, 3H), 2.90 (d, J = 7.4 Hz, 2H), 2.61 (m, 2H), 2.29 (p, J = 5.9 Hz, 4H), 2.14 (s, 2H), 1.39 (t, J = 7.2 Hz, 3H). |
| TB-129 | 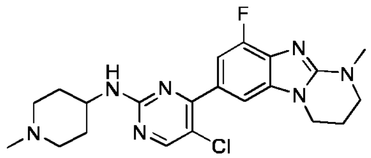 **5-chloro-4-(9-fluoro-1-methyl-1,2,3,4-tetrahydrobenzo[4,5]imidazo[1,2-*a*]pyrimidin-7-yl)-*N*-(1-methylpiperidin-4-yl)pyrimidin-2-amine** | $[M+H]^+$= 430.3 | $^1$H NMR (400 MHz, Chloroform-*d*) δ 8.26 (s, 1H), 7.44 (d, *J*= 120 Hz, 1H), 7.39 (s, 1H), 5.36 (s, 1H), 4.02 (t,*J* = 6.0 Hz, 2H), 3.48 (m, 1H), 3.43 (t, *J* = 6.0 Hz, 2H), 3.26 (s, 3H), 2.92 (m, 2H), 2.78 (s, 3H), 2.29 (m, 2H), 2.26 (s, 2H), 1.27 (d, *J*= 2.4 Hz, 2H), 0.86 (m, 2H). |
| TB-131 | 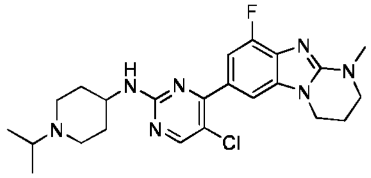 **5-chloro-4-(9-fluoro-1-methyl-1,2,3,4-tetrahydrobenzo[4,5]imidazo[1,2-*a*]pyrimidin-7-yl)-*N*-(1-isopropylpiperidin-4-yl)pyrimidin-2-amine** | $[M+H]^+$= 458.2 | $^1$H NMR (400 MHz, Chloroform-*d*) δ 8.26 (s, 1H), 7.45 (d, *J* = 12.0 Hz, 1H), 7.41 (s, 1H), 5.31 (s, 1H), 4.09 (m, 1H), 4.03 (t, *J* = 6.0 Hz, 2H), 3.43 (t, *J* = 5.6 Hz, 2H), 3.40 (m, 2H), 3.27 (s, 3H), 2.89 (m, 2H), 2.36 (m, 2H), 2.28 (d, *J* = 5.8 Hz, 2H), 1.43 (d, *J* = 6.8 Hz, 6H), 0.86 (m, 2H). |

(continued)

| Compound | Structure | LC-MS | NMR |
|---|---|---|---|
| TB-132 | 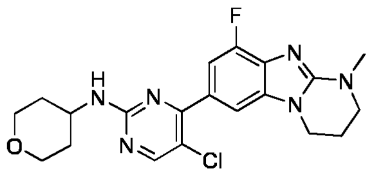 **5-chloro-4-(9-fluoro-1-methyl-1,2,3,4-tetrahydrobenzo [4,5]imidazo[1,2-*a*]pyrimidin-7-yl)-*N*-(tetrahydro-2*H*-pyran-4-yl)pyrimidin-2-amine** | $[M+H]^+$= 417.3 | $^1H$ NMR (400 MHz, Chloroform-*d*) δ 8.27 (s, 1H), 7.44 (d, *J* = 11.8 Hz, 1H), 7.39 (s, 1H), 5.11 (d, *J*= 7.9 Hz, 1H), 4.10 - 4.05 (m, 1H), 4.03 (d, *J* = 6.2 Hz, 2H), 3.98 (d, *J*= 3.7 Hz, 2H), 3.55 (td, *J*= 11.6, 2.2 Hz, 2H), 3.46 - 3.39 (m, 2H), 3.27 (s, 3H), 2.29 (p, *J*= 6.0 Hz, 2H), 2.08 - 2.02 (m, 2H), 1.62 - 1.50 (m, 2H). |
| TB-134 | 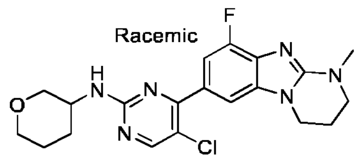 **5-chloro-4-(9-fluoro-1-methyl-1,2,3,4-tetrahydrobenzo [4,5]imidazo[1,2-*a*]pyrimidin-7-yl)-*N*-(tetrahydro-2*H*-pyran-3-yl)pyrimidin-2-amine** | $[M+H]^+$= 417.3 | $^1H$ NMR (400 MHz, Chloroform-*d*) δ 8.27 (s, 1H), 7.45 (d, *J* = 120 Hz, 1H), 7.39 (s, 1H), 5.38 (d, *J* = 8.0 Hz, 1H), 4.07 (s, 1H), 4.03 (t,*J* = 6.1 Hz, 2H), 3.94 (dd, *J* = 11.4, 3.2 Hz, 1H), 3.75 - 3.60 (m, 2H), 3.52 - 3.45 (m, 1H), 3.43 (t, *J*= 6.1 Hz, 2H), 3.27 (s, 3H), 2.37 - 2.22 (m, 2H), 1.99 (m, 1H), 1.85 - 1.78 (m, 1H), 1.72 - 1.63 (m, 2H). |
| TB-143 | 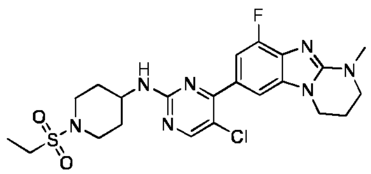 **5-chloro-*N*-(1-(ethylsulfonyl)piperidin-4-yl)-4-(9-fluoro-1-methyl-1,2,3,4-tetrahydrobenzo[4,5]imidazo [1,2-*a*]pyrimidin-7-yl)pyrimidin-2-amine** | $[M+H]^+$= 508.4 | $^1H$ NMR (400 MHz, Chloroform-*d*) δ 8.27 (s, 1H), 7.44 (dd, *J* = 12.0, 1.2 Hz, 1H), 7.39 (s, 1H), 5.14 (d, *J* = 7.0 Hz, 1H), 4.02 (t, *J* = 6.0 Hz, 2H), 4.00 - 3.93 (m, 1H), 3.78 (dt, *J* = 12.0, 3.2 Hz, 2H), 3.43 (t, *J* = 5.6 Hz, 2H), 3.27 (s, 3H), 3.07 - 3.00 (m, 2H), 2.97 (q, *J* = 7.4, 2H), 2.34 - 2.25 (m, 2H), 2.16 (dd, *J* = 13.1, 3.7 Hz, 2H), 1.61 (td, *J* = 10.7, 6.9 Hz, 2H), 1.38 (t, *J* = 7.4 Hz, 3H). |

(continued)

| Compound | Structure | LC-MS | NMR |
|---|---|---|---|
| TB-144 | 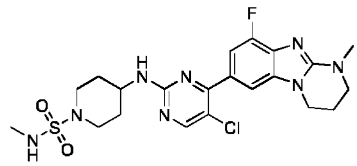 **4-((5-chloro-4-(9-fluoro-1-methyl-1,2,3,4-tetrahydrobenzo[4,5]imidazo[1,2-*a*]pyrimidin-7-yl)pyrimidin-2-yl)amino)-*N*-methylpiperidine-1-sulfonamide** | $[M+H]^+$= 509.2 | $^1$H NMR (400 MHz, Chloroform-*d*) $\delta$ 8.27 (s, 1H), 7.44 (d, *J* = 12.0 Hz, 1H), 7.39 (s, 1H), 5.22 (d, *J* = 8.0 Hz, 1H), 4.34 (m, 1H), 4.02 (t, *J* = 6.0 Hz, 2H), 4.00 - 3.89 (m, 1H), 3.68 (dt, *J* = 3.2 Hz, 12.5 Hz, 2H), 3.43 (t,*J* = 5.6 Hz, 2H), 3.27 (s, 3H), 3.01 (td, *J*= 12.0, 2.8 Hz, 2H), 2.71 (d, *J* = 5.2 Hz, 3H), 2.29 (p, *J* = 5.6 Hz, 2H), 2.13 (dd, *J* = 12.0, 4.0 Hz, 2H), 1.60 (ddd, *J* = 14.5, 10.7, 5.4 Hz, 2H). |

**Example B6**

compound synthesized in the present invention:

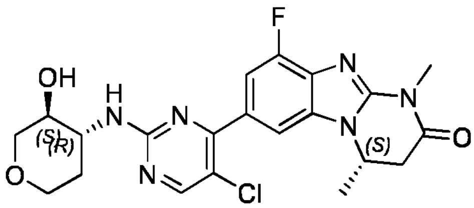

TB-98

The experimental procedure is as follows:

Synthesis of compound TB-98

The synthesis route is as follows:

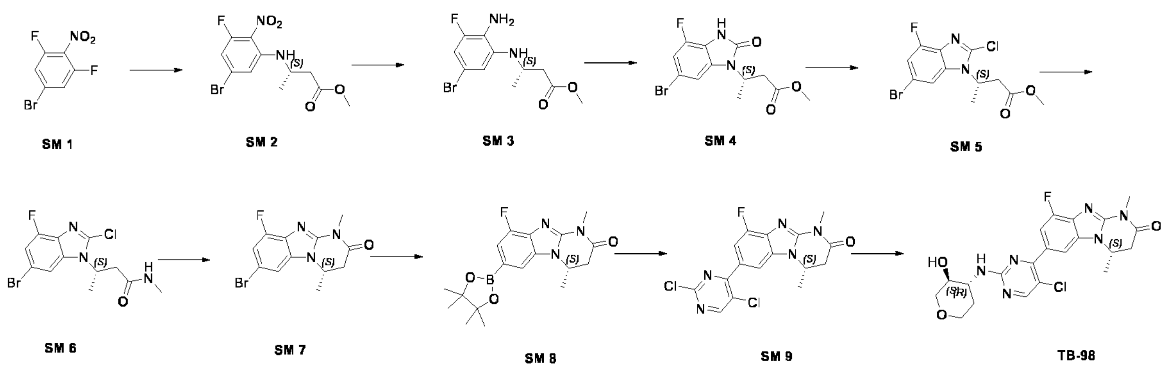

The experimental procedure is as follows:

Step 1:

Compound SM 1 (7.7 g, 1.0 e.q.) was solubilized in ultra-dry tetrahydrofuran solvent, placed in an ice-water bath,

cesium carbonate solid (31.8 g, 3.0 e.q.) was added, and (S)-3-aminobutanoic acid methyl ester hydrochloride (5.0 g, 1.0 e.q.) was added under stirring conditions. At the end of addition, the reaction was warmed to room temperature naturally. TLC monitor showed the reaction was ended, the solid was removed by suction filtration, the filter cake was washed with appropriate amount of EA, extracted and separated with water, the organic phase was dried over anhydrous sodium sulfate, suction filtrated and concentrated under reduced pressure, and purified by column chromatography to obtain SM 2 (6.7 g, 61.5%) as a yellow solid, LCMS: $[M+H]^+$ = 335.2, 337.2.

Step 2:

Compound SM 2 (6.7 g, 1.0 e.q.) was dissolved in a solvent mixture of methanol and water (v: v = 4:1), iron powder (6.7 g, 6.0 e.q.) and ammonium chloride solids (2.1 g, 2.0 e.q.) were added, and the reaction was warmed to reflux. TLC monitor showed the reaction was ended, the reaction solution was cooled down to room temperature, suction filtered, and the filter cake was washed with appropriate amount of EA, and the filtrate was concentrated under reduced pressure to remove most of the solvent, extracted and partitioned with saturated sodium bicarbonate solution and EA, the organic phase was dried over anhydrous sodium sulfate, suction filtered and concentrated under reduced pressure, and purified by column chromatography to obtain the compound SM 3 (5.5 g, 90.0%), LCMS: $[M+H]^+$ = 305.1, 307.1.

Step 3:

Compound SM 3 (5.5 g, 1.0 e.q.) and CDI (4.4 g, 1.5 e.q.) were sequentially solubilized in ultra-dry DMF solvent, the reaction was warmed up to 50 °C, TLC monitor showed the reaction was ended, the reaction solution was cooled down to room temperature, extracted and partitioned with EA and water, the organic phase was dried over anhydrous sodium sulphate, suction filtered and concentrated under reduced pressure, isolated and purified by column chromatography to obtain compound SM 4 ( 5.4 g, 90.4%), LCMS: $[M+H]^+$ = 331.2, 333.1.

Step 4:

Compound SM 4 (5.4 g, 1.0 e.q.) was dissolved in phosphorus oxychloride (18.2 mL, 12.0 e.q.), and the reaction was raised to 100 °C and reacted for 12 h. TLC monitor showed the reaction was ended, and the reaction solution was cooled to room temperature, quenched by pouring it into iced water, extracted and partitioned with EA, and the organic phase was dried over anhydrous sodium sulfate, suction filtered and concentrated under reduced pressure, and purified by column chromatography to obtain compound SM 5 (5.2 g, 91.2%), LCMS: $[M+H]^+$ = 349.0, 351.1.

Step 5:

Compound SM 5 (1.9 g, 1.0 e.q.) was added to 50 mL of 30% methanamine-ethanol solution and the reaction was kept in an ice-water bath. At the end of the reaction, most of the solvent was removed by concentration under reduced pressure, extracted and partitioned with saturated saline and EA, the organic phase was dried over anhydrous sodium sulfate, suction filtered, the organic phase was concentrated, and purified by silica gel column chromatography to obtain the compound SM 6 (1.7 g, 89.4%), LCMS: $[M+H]^+$ = 348.1, 350.1.

Step 6:

Compound SM 6 (1.5 g, 1.0 e.q.) was solubilized in 50 mL of ultra-dry tetrahydrofuran solvent, cooled in an ice-water bath, and 60% NaH powder was added in batches. At the end of addition, the reaction was held for 3 h. At the end of the reaction, cooled to 0 °C, quenched by adding appropriate amount of ice water, extracted and partitioned with saturated saline and EA, the organic phase was dried over anhydrous sodium sulfate, filtered, the organic phase was concentrated, and separated and purified by silica gel column chromatography to obtain the compound SM 7 (811 mg, 60.5%), LCMS: $[M+H]^+$ = 312.1, 314.0.

Step 7:

Compound SM 7 (811 mg, 1.0 e.q.), pinacol bis(boronic acid) ester (1.32 g, 2.0 e.q.), Pd(dppf)$Cl_2$ (95.1 mg, 0.05 e.q.) and potassium acetate (764.7 mg, 3.0 e.q.) were added sequentially to a dry 50 mL three-necked flask under nitrogen protection, and nitrogen was displaced three times, and ultra-dry 1 ,4-dioxane solvent was added, and the temperature was warmed to reflux reaction. TLC monitor showed the reaction was ended, cooled to room temperature, extracted and partitioned with Ea and water, the organic phase was dried over anhydrous sodium sulfate, suction filtered and concentrated under reduced pressure, and purified by column chromatography to obtain the compound SM 8 (890

mg, 95.4%), LCMS: [ M + H ]+ = 360.3.

Step 7:

Compound SM 8 (150 mg, 1.0 e.q.), 2,4,5-trichloropyrimidine (115 mg, 1.5 e.q.), palladium tetrakis(triphenylphosphine) (48.3 mg, 0.1 e.q.) and sodium carbonate solid (66.4 mg, 1.5 e.q.) were sequentially added to a 50 mL three-necked flask under nitrogen protection, a mixture of 1,4-dioxane and water (v: v = 10: 3) was added, nitrogen was displaced three times, and warmed to reflux reaction. TLC monitor showed the reaction was ended, cooled to room temperature, extracted and partitioned with EA and water, the organic phase was dried over anhydrous sodium sulfate, suction filtered and concentrated under reduced pressure, and purified by column chromatography to obtain the compound SM 9 (120 mg, 75.5%), LCMS: [ M + H ]+ = 380.1.

Step 8:

Compound SM 9 (150 mg, 1.0 e.q.), (3s, 4r)-4-aminooxan-3-ol hydrochloride (121.2 mg, 2.0 e.q.) and DIPEA (0.28 mL, 4.0 e.q.) were sequentially added to NMP solvent under nitrogen, and the reaction was warmed to 90 °C. At the end of the reaction, cooled to room temperature, extracted and partitioned sequentially with saturated saline, water and EA, the organic phase was dried over anhydrous sodium sulfate, suction filtered and concentrated under reduced pressure, and purified by preparative liquid-phase to obtain compound TB-98 (76 mg, 40.7%), HPLC purity of 99.38%, LCMS: [ M + H ]+ = 461.3.

$^1$H NMR (400 MHz, Chloroform-*d*) $\delta$ 8.32 (s, 1H), 7.53 (s, 1H), 7.48 (d, *J* = 11.4 Hz, 1H), 5.32 (d, *J* = 6.0 Hz, 1H), 4.75 (p, *J* = 6.9 Hz, 1H), 4.05 (dd, *J* = 11.5, 5.0 Hz, 1H), 3.99 (dd, *J* = 11.7, 4.1 Hz, 1H), 3.90 - 3.80 (m, 1H), 3.67 (dt, *J* = 8.5, 4.5 Hz, 1H), 3.63 (d, *J* = 4.9 Hz, 1H), 3.60 (s, 3H), 3.46 (ddd, *J* = 14.1, 9.8, 2.2 Hz, 2H), 3.27 - 3.14 (m, 2H), 2.11 - 1.99 (m, 1H), 1.76 - 1.69 (m, 1H), 1.47 (d, *J* = 6.7 Hz, 3H).

Referring to the synthesis method of Example B6, the following compounds were synthesized:

| Compound | Structure | LC-MS | NMR |
|---|---|---|---|
| TA-53 | 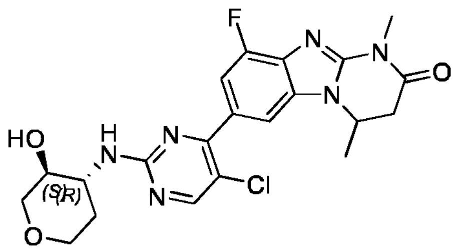 **7-(5-chloro-2-(((3*S*,4*R*)-3-hydroxytetrahydro-2*H*-pyran-4-yl)amino)pyrimidin-4-yl)-9-fluoro-1,4-dimethyl-3,4-dihydrobenzo[4,5]imidazo[1,2-*a*]pyrimidin-2(1*H*)-one** | [M+H]+=4 61.2 | |
| TA-60 | 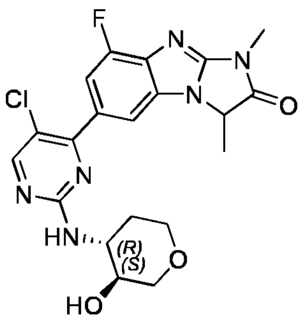 **6-(5-chloro-2-(((3*S*,4*R*)-3-hydroxytetrahydro-2*H*-pyran-4-yl)amino)pyrimidin-4-yl)-8-fluoro-1,3-dimethyl-1*H*-benzo[*d*]imidazo[1,2-*a*]imidazol-2(3*H*)-one** | [M+H]+=4 47.2 | |

(continued)

| Compound | Structure | LC-MS | NMR |
|---|---|---|---|
| TB-46 | 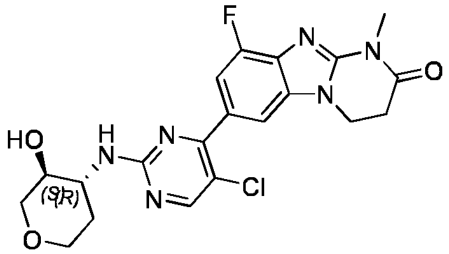 **7-(5-chloro-2-(((3*S*,4*R*)-3-hydroxytetrahydro-2*H*-pyran-4-yl)amino)pyrimidin-4-yl)-9-fluoro-1-methyl-3,4-dihydrobenzo[4,5]imidazo[1,2-*a*]pyrimidin-2(1*H*)-one** | $[M+H]^+$=4 47.3 | $^1$H NMR (400 MHz, Chloroform-*d*) $\delta$ 8.31 (s, 1H), 7.51 (s, 1H), 7.48 (d, *J* = 12.0 Hz, 1H), 5.31 (d, *J* = 6.0 Hz, 1H), 4.81 (s, 1H), 4.28 (t, *J* = 7.2 Hz, 2H), 4.05 (dd, *J* = 12.0, 4.0 Hz, 1H), 4.02 - 3.94 (m, 1H), 3.85 (m, 1H), 3.69 - 3.59 (m, 2H), 3.59 (s, 3H), 3.46 (td, *J* = 12.0, 2.0 Hz, 1H), 3.23 - 3.14 (m, 1H), 3.09 (t, *J* = 7.2 Hz, 2H), 2.13 - 1.93 (m, 1H). |
| TB-47 | 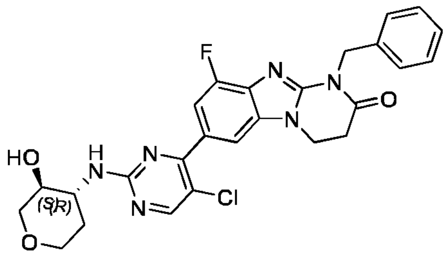 **1-benzyl-7-(5-chloro-2-(((3S,4R)-3-hydroxytetrahydro-2H-pyran-4-yl)amino)pyrimidin-4-yl)-9-fluoro-3,4-dihydrobenzo[4,5]imidazo[1,2-a]pyrimidin-2(1H)-one** | $[M+H]^+$=5 23.8 | |
| TB-101 | 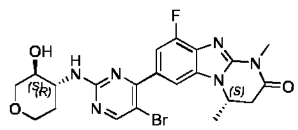 **(*S*)-7-(5-bromo-2-(((3*S*,4*R*)-3-hydroxytetrahydro-2*H*-pyran-4-yl)amino)pyrimidin-4-yl)-9-fluoro-1,4-dimethyl-3,4-dihydrobenzo[4,5]imidazo[1,2-*a*]pyrimidin-2(1*H*)-one** | $[M+H]^+$=5 05.1 | |

**Test Example 1 Enzyme activity test**

The following biological activity test experiments were performed on some of the compounds of the above embodiments and comparative compounds.

The procedure of the bioactivity test experiment is as follows:

1. Kinase activity test:

The tested compounds were subjected to CDK4 and CDK6 kinase IC50 values test.

(i) Reagent information

| **Reagent** | **Brand** | **Item number** |
|---|---|---|
| Palbociclib | MCE | HY-50767 |
| FL-Peptide18 | PerkinElmer | 760362 |

(continued)

| Reagent | Brand | Item number |
|---|---|---|
| FL-Peptide29 | PerkinElmer | 760429 |
| FL-Peptide34 | PerkinElmer | 760643 |

(ii) Equipment information

| Equipment | Brand | Item number |
|---|---|---|
| Incubator | Thermo Scientific | - |
| Shaker | QILINBEIER | - |
| EZ Reader | PerkinElmer | 122919 |
| Liquid Handler | Labcyte Inc. | Echo 550 |
| Liquid Handler | TECAN | EVO200 |

(iii) Research design

(1) Compound preparation:

① The tested compounds were prepared as 0.5 mM DMSO solution, while the positive control drug Palbociclib was also prepared as 0.5 mM DMSO solution.
② Triple dilutions were made to obtain 10 solutions of different concentrations of the compounds.

(2) Perform the enzyme assay:

① Preparing a 1.3 x enzyme solution containing enzyme, substrate and cofactor as shown below.

② Adding 15 μL of 1.3 x enzyme solution to each well and incubating for 30 min at room temperature.

③ Starting the reaction by adding 5 μL of 4 x ATP solution, with each test well containing the listed components and a final volume of 20 μL.

④ Incubating for 150 minutes and then stopping the reaction by adding 75 μL of buffer (containing 0.5 M EDTA).

⑤ Reading the data from each test well by EZ for analysis.

(3) Data analysis:

Percentage inhibition was calculated using the read conversion ratio (CR) according to the formula below: The wells treated with DMSO were used as positive control (positive control) and the wells without enzyme were used as negative control (negative control).

% (percentage inhibition) = 100-100*((CRPC-CRSample)/(CRPC- CRNC)).

| **Enzyme** | **Vendor** | **Cat No.** | **Refere nce** | **Enzy me conc.( nM)** | **Ass ay** | **ATP conc. ( μM)** | **Subst rate** | **Cofacto r** | **Reacti on time** |
|---|---|---|---|---|---|---|---|---|---|
| CDK4/Cy cD1 | Thermo Fisher | PR80 64A | Palboci clib | 2.50 | MS A | 180 | FL-Peptid e34 | 10mM $MgCl_2$ | 120 minute s |
| CDK6/Cy clinD3 | Carna | 04-107 | Palboci clib | 5.00 | MS A | 350 | FL-Peptid e34 | 10mM $MgCl_2$ | 120 minute s |

The inhibitory activities $IC_{50}$ (nM) values of the tested samples against CDK4 and CDK6 kinases were obtained by the above assays, as shown in Table 1.

Table 1

| Compound | CDK4 kinase $IC_{50}$(nM) | CDK6 kinase $IC_{50}$(nM) |
|---|---|---|
| Palbociclib | 7.8 | 3.6 |
| TA-1 | 0.849 | 23.632 |
| TA-2 | 42.250 | 109.275 |
| TA-4 | 10.416 | 115.634 |
| TA-5 | 3.111 | 30.158 |
| TA-6 | 4.286 | 13.694 |
| TA-7 | 17.671 | >500 |
| TA-8 | 21.401 | 67.273 |
| TA-10 | 57.637 | 206.461 |
| TA-11 | 7.195 | 24.748 |
| TA-14 | 69.436 | >500 |
| TA-16 | 15.338 | 99.133 |
| TA-17 | 22.697 | 83.438 |
| TA-20 | 107.677 | 319.426 |
| TA-27 | 21.823 | 202.994 |
| TA-34 | 729.175 | 2578.984 |
| TA-50 | 2.927 | 90.184 |
| TA-54 | 185 | >500 |
| TA-55 | 10.073 | 142.732 |
| TA-57 | 43.367 | >500 |
| TB-1 | 3.696 | 67.802 |
| TB-2 | 17.987 | 318.856 |
| TB-3 | 6.968 | 74.886 |
| TB-4 | 27.120 | >500 |
| TB-5 | 4.237 | 71.573 |
| TB-6 | 10.796 | 190.298 |
| TB-7 | 2.938 | 113.190 |
| TB-8 | 18.822 | 334.496 |
| TB-9 | 40.06 | 222.9 |

(continued)

| Compound | CDK4 kinase $IC_{50}$(nM) | CDK6 kinase $IC_{50}$(nM) |
|---|---|---|
| TB-11 | 6.446 | 201.534 |
| TB-12 | 6.006 | 151.182 |
| TB-13 | 1.268 | 18.630 |
| TB-14 | 6.371 | 234.955 |
| TB-16 | 6.915 | 44.636 |
| TB-17 | 33.843 | 297.064 |
| TB-18 | 2.604 | 13.715 |
| TB-20 | 44.565 | 140.674 |
| TB-23 | 185 | >500 |
| TB-24 | 10.073 | 142.732 |
| TB-25 | 43.367 | >500 |
| TB-26 | 18.554 | 200.051 |
| TB-28 | 8.568 | 154.037 |
| TB-31 | 15.289 | 181.628 |
| TB-32 | 12.287 | 145.373 |
| TB-33 | 8.373 | 61.66 |
| TB-35 | 15.496 | 166.793 |
| TB-38 | 18.504 | 76.613 |
| TB-40 | 7.939 | 228.859 |
| TB-41 | 160.593 | >500 |
| TB-42 | 8.358 | 195.291 |
| TB-43 | 18.554 | 200.051 |
| TB-44 | 2.927 | 90.184 |
| TB-45 | 9.439 | 241.422 |
| TB-46 | 15.275 | 243.864 |
| TB-51 | 2.234 | 27.601 |
| TB-52 | 8.940 | 40.623 |
| TB-53 | 10.572 | 56.852 |
| TB-54 | 101.298 | 410.857 |
| TB-68 | 301.466 | >500 |
| TB-70 | 18.504 | 76.613 |
| TB-90 | 12.665 | 95.239 |
| TB-91 | 4.154 | 68.376 |
| TB-92 | 4.546 | 123.496 |
| TB-93 | 51.787 | >500 |
| TB-94 | 44.364 | 458.443 |
| TB-95 | 103.169 | >500 |
| TB-130 | 28.567 | 154.898 |
| TB-132 | 46.788 | 245.603 |

(continued)

| Compound | CDK4 kinase $IC_{50}$(nM) | CDK6 kinase $IC_{50}$(nM) |
|---|---|---|
| TB-133 | 17.931 | 64.836 |
| TB-137 | 12.665 | 95.239 |
| TB-142 | 69.533 | 361.303 |
| TB-143 | 7.899 | 58.343 |
| TB-144 | 13.574 | 125.787 |

As can be seen from Table 1, the compounds synthesized by the present invention all have a very good inhibitory ability against CDK4 kinase by in vitro bioactivity screening, using Palbociclib as a control, and furthermore, the compounds synthesized by the present invention have a very good selectivity between the kinase activities of CDK4 and CDK6, which will have a great potential to reduce the side effects, such as hematologic, resulting from the inhibition of CDK6. Accordingly, the compounds synthesized by the present invention are expected to be further developed as drugs for use in the regulation of CDK4 kinase activity or in the treatment of CDK4-related diseases.

**Test Example 2 Cellular antiproliferative assay**

1. Experimental materials and equipment:

Human breast cancer cell MCF-7, ovarian cancer cell A2780, human mantle cell lymphoma cell JEKO-1. DMEM medium (Bio-Channel), DMSO (dimethyl sulfoxide), MTT (thiazolyl blue), 0.25% EDTA-Tripsin (tryptic digest), 1xPBS (phosphate buffer, PH7.2), 96-well plate (Corning), fetal bovine serum (FBS), 10,000 U/mL penicillin-G/streptomycin, high-speed cryo-centrifuge (EPPENDORF 5810R), enzyme-linked immunoassay (Tecan Spark).

2. Experimental preparation:

1. Cell spreading

A) Tumor cells were cultured in DMEM (high sugar with 10% FBS and 100 U/mL penicillin-G/streptomycin) at 37°C, 5% $CO_2$ and saturated humidity until 80-90% dense.
B) Removed the culture medium from 10 cm Petri dishes;
C) Rinsed the cells once with 10 ml 1xPBS;
D) Added 4 ml 0.25% EDTA-Tripsin into 37 °C, 5% $CO_2$ incubator for trypsin digestion for 5 minutes, transferred to 15 ml centrifuge tube, centrifuged at 200 g for 5 minutes, discarded the supernatant to get cell precipitate;
E) Resuspended with 4 ml DMEM medium, counted and adjust to 50,000 cells/ml.
F) Added cell suspension to 96-well plate with 100 μL per well volume and incubated overnight at 37°C, 5% $CO_2$ incubator.

2. Compound treatment

Compound Dilution

A) Preparation of gradient dilution solution of the tested compound: the test compound was configured into 1 mM reservoir solution. Then 1.5 μl of the reservoir solution was dissolved in 1.5 ml of DMSO-free culture solution, and then a 3-fold serial gradient dilution was performed with 0.1% DMSO culture solution for a total of 9 concentrations, and the concentrations of the compounds after dilution were as follows:
333.33 nM, 111.11 nM, 37.03 nM, 12.35 nM, 4.15 nM, 1.37 nM, 0.46 nM, 0.15 nM
(B) After thorough mixing, 100 μL of cultured compound solution was taken separately to replace the culture solution in the cell culture plate, with 4 replicate wells for each concentration;
(C) The cells were transferred to the incubator and incubated for 5 days.

3. MTT test

A) Taking the cell culture plate and adding 10 μL of 5 mg/ml MTT in the biosafety cabinet;
B) Putting the cell culture plate back into the incubator and continued incubation for 3 hours;
C) Taking the cell culture plate and removing the culture medium, adding 100 μL isopropanol (containing 0.4 mM HCl, 0.1% NP-40), and shaking the bed at room temperature for 30 min; and
D) Determining the absorbance value by selecting 570 nm on a TECAN enzyme-linked immunoassay detector.

4. Data Analysis

The % Cell Viability was calculated using the following formula :

$$\%\text{Cell Viability}=100\%\times(\text{Lum_Sample- Lum_LC})/(\text{Lum_HC-Lum_LC})$$

Lum_HC: 0.1% DMSO control cell reading
Lum_Sample: cell readings with compound added
Lum_LC: Blank medium reading

The IC50 values (in nM) were obtained by curve fitting with GraphPad Prism 8 software, as shown in Table 2, where the meanings represented by A/B/C are as follows: A ≤ 500 nM, 500 nM < B ≤ 2000 nM, C > 2000 nM.

Table 2

| Compound | MCF7 $IC_{50}$(nM) | A2780 $IC_{50}$(nM) | JEKO-1 $IC_{50}$(nM) |
|---|---|---|---|
| TA-1 | A | A | A |
| TA-2 | B | A | A |
| TA-4 | A | A | A |
| TA-5 | A | A | A |
| TA-6 | A | A | A |
| TA-7 | A | A | A |
| TA-11 | A | A | A |
| TA-55 | A | A | A |
| TB-1 | A | A | A |
| TB-2 | A | A | A |
| TB-3 | A | A | A |
| TB-5 | A | A | A |
| TB-6 | B | A | A |
| TB-7 | A | A | A |
| TB-8 | B | A | A |
| TB-9 | B | A | A |
| TB-11 | A | A | A |
| TB-12 | A | A | A |
| TB-13 | A | A | A |
| TB-14 | A | A | A |
| TB-16 | A | A | A |
| TB-17 | B | B | B |

(continued)

| Compound | MCF7 $IC_{50}$(nM) | A2780 $IC_{50}$(nM) | JEKO-1 $IC_{50}$(nM) |
|---|---|---|---|
| TB-18 | A | A | A |
| TB-24 | A | A | A |
| TB-25 | B | A | A |
| TB-26 | A | A | A |
| TB-28 | A | A | A |
| TB-31 | A | A | A |
| TB-32 | A | A | A |
| TB-33 | A | A | A |
| TB-35 | A | A | A |
| TB-38 | A | A | A |
| TB-40 | A | A | A |
| TB-41 | B | B | B |
| TB-42 | A | A | A |
| TB-43 | A | A | A |
| TB-44 | A | A | A |
| TB-45 | A | A | A |
| TB-46 | A | A | A |
| TB-51 | A | A | A |
| TB-52 | A | A | A |
| TB-53 | A | A | A |
| TB-70 | A | A | A |
| TB-90 | B | A | A |
| TB-91 | A | A | A |
| TB-92 | A | A | A |
| TB-93 | B | A | A |
| TB-130 | A | A | A |
| TB-132 | B | A | A |
| TB-133 | A | A | A |
| TB-137 | A | A | A |
| TB-143 | A | A | A |
| TB-144 | A | A | A |
| TB-146 | A | A | A |
| TB-149 | A | A | A |
| TB-150 | A | A | A |
| TB-153 | A | A | A |
| TB-154 | A | A | A |
| TB-158 | A | A | A |
| TB-162 | A | A | A |

As can be seen from Table 2, the compounds synthesized in the present invention showed good inhibitory ability

against human breast cancer cell MCF-7, ovarian cancer cell A2780, and human mantle cell lymphoma cell JEKO-1 through in vitro bioactivity screening.

**Test example 3 Preclinical rat pharmacokinetic test**

1. Experimental materials and equipment:

Healthy adult SD rats, male, 6-8 weeks old, weighing 200-300 g. EDTA-Na2 anticoagulant. Analytical balance, animal weight scale, magnetic stirrer, freezing centrifuge, single-channel manual pipette.

2. the experimental process:

(1) Drug preparation

About 10 mg of the sample to be tested was precisely weighed, the converted 5% DMSO was added to dissolve, and then 10 percent solutol HS-15 and 85% saline were added, ultrasonic treatment was performed, vortex mixed, to obtain the solution having a concentration of 1 mg/mL; freshly prepared before use.

0.2 mL of the sample was pipetted into a 1.5 mL centrifuge tube and stored at -80°C for concentration analysis of the drug delivery solution.

(2) Animal preparation

Animals were kept in rat cages and fasted (not less than 10 h) the day before the test, but not water fasted; on the day of the test, they were weighed and labeled at the tail. Blank blood was collected before drug administration. Blood was collected from the tail vein.

(3) Administration

Route of administration: gavage (p.o.).
Dose: 10 mg/kg
Administration volume: 10 mL/kg
Operation procedure: Hold the rat with the left hand with anti-biting gloves, made it stand upright, extent the 16-gauge gavage needle from the throat in the mouth, and then injected the drug into the stomach when there was no obvious resistance.

(4) Sample collection

Before and 0.5 h, 1 h, 2 h, 4 h, 6 h, 8 h, 12 h and 24 h after the administration of the drug, 0.1 ml of whole blood from the test animal was collected in EDTA-Na2 anticoagulant tubes, which was mixed upside down for 3-4 times and then centrifuged at 10,000 g for 5 min at 4 °C to isolate the plasma, which was stored at -80 °C for testing. Blood was collected from the tail vein.

For the specific operation, the rats were fixed on a fixator so that their tails could be completely exposed, and the tails were wiped with alcohol so that the skin on the surface absorbed the alcohol to achieve the effect of obvious venous dilatation, and the appropriate vein was selected from both sides and the needle was inserted about one-third of the way from the tip of the tail. The syringe used insulin syringe, the needle was inserted while the needle beveled upward, the needle was parallel immediately after feeling of piercing the skin , the operator could feel that the sliding resistance of the needle in the vein is small, there is blood return in the syringe. That is, the needle has entered the vein, and about 0.1-0.2ml of whole blood was drawn. After pulling out the needle, pressed to stop bleeding.

3. Sample analysis:

Preparation of standard curve: Pipetted 25μL of rat blank plasma in a centrifuge tube respectively, added 25μL of prepared standard series solution (prepared in methanol), then added 200 μL of internal standard solution (prepared in methanol), vortexed and mixed for 2min, centrifuged at 4°C, 10,000g for 10min.

Unknown plasma samples treatment: 25 μL of rat plasma containing drug was aspirated, 25 μL of methanol and 200 μL of internal standard solution were added sequentially, vortexed and mixed for 2 min, and then centrifuged at 4°C

and 10,000g for 10 min. The supernatant was extracted for LC/MS/MS detection.

4. Data processing

Shimadzu liquid phase and Triple Quad TM 6500+AB mass spectrometry were utilized to establish a quantitative assay for the compounds to be tested. The pro-drug concentration in plasma was determined. The blood drug concentration-time curves were plotted, and the main pharmacokinetic parameters were calculated using the non-compartment model in winnonlin Phoenix software, and the detailed data were shown in Table 3.

Table 3

| Compound | Tmax (h) | Cmax (ng/ml) | AUClast (h*ng/ml) | HL_ (h) |
|---|---|---|---|---|
| TA-25 | 3.33 | 611 | 4466 | 1.69 |
| TA-53 | 1.0 | 920 | 3530 | 1.77 |
| TB-12 | 1.50 | 677 | 4502 | 3.14 |
| TB-24 | 1.67 | 677 | 4502 | 3.14 |
| TB-27 | 2.33 | 1503 | 11472 | 3.69 |
| TB-31 | 4.00 | 923 | 4891 | 1.34 |
| TB-92 | 0.50 | 1943 | 5025 | 1.21 |

From Table 3, it was observed that all the compounds synthesized by the present invention have good pharmacokinetic properties as screened by PK test.

All literatures mentioned in the present invention are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

**1.** A compound for use as a CDK4 kinase inhibitor, wherein the compound is a compound of formula I, or a pharmaceutically acceptable salt, stereoisomer, tautomer, hydrate, solvate, isotope compound, or prodrug thereof,

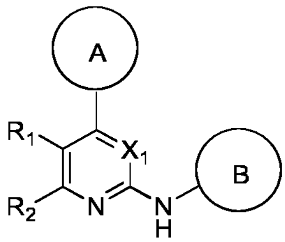

formula I

wherein,

$X_1$ is selected from the group consisting of N and $CR_3$;
$R_1$ is selected from the group consisting of H, $CF_3$, F, Cl, Br, methyl, ethyl, isopropyl, and cyclopropyl;
$R_2$ is selected from the group consisting of H, F, Cl, Br, $CF_3$, $CF_2H$, $NH_2$, and methyl;
or, $R_1$ and $R_2$ together with the C to which they are attached form a 5-membered heteroaryl containing 2 N atoms;
$R_3$ is selected from the group consisting of H, $CF_3$, F, Cl, Br, methyl, and cyano;
Ring A is selected from the group consisting of

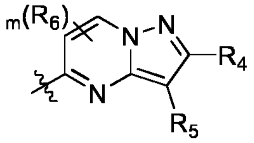,

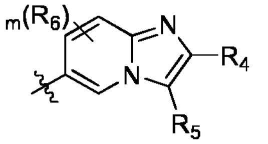, 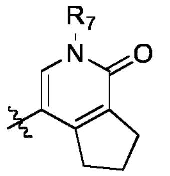, 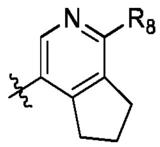, 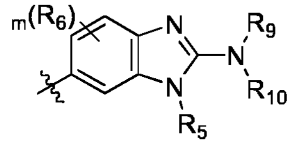,

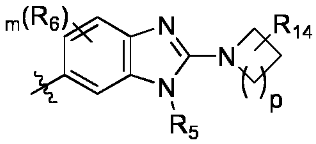, 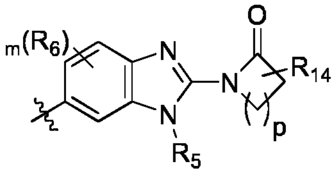, 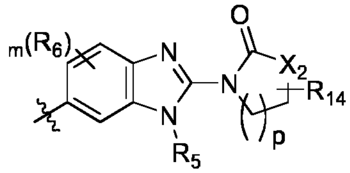,

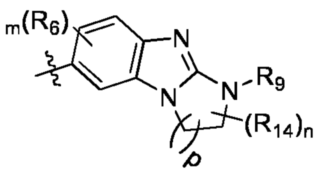, 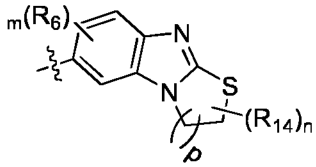, 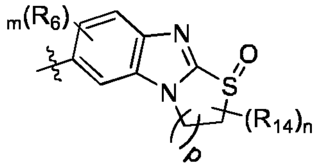,

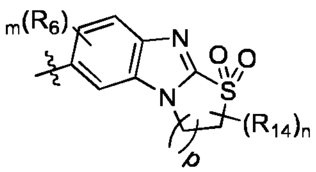, 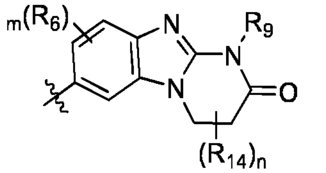, 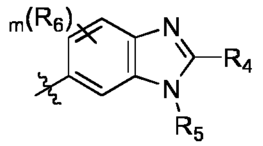,

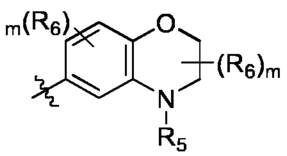, 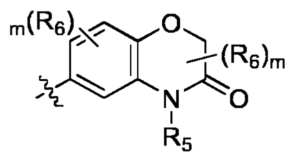, 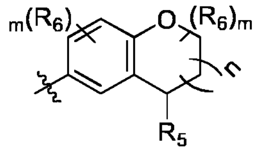,

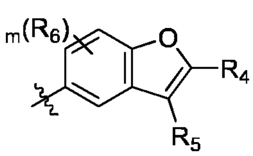, 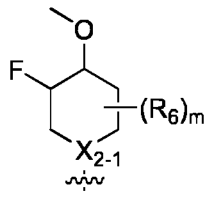, 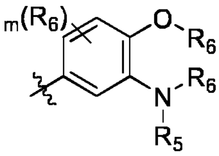, and 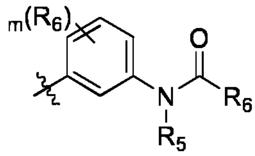,

wherein, $X_2$ is O or NR;

$X_{2-1}$ is selected from the group consisting of N and $CR_{11}$;

Ring B is selected from the group consisting of

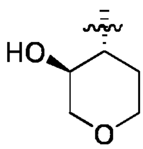, 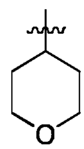, 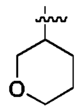,

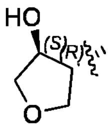, 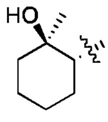, 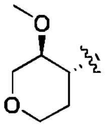, 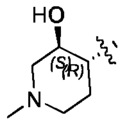, 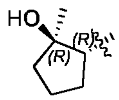, 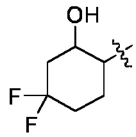, 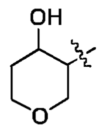,

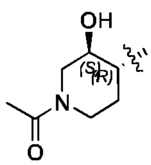, 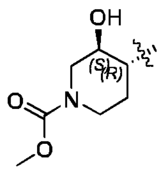, 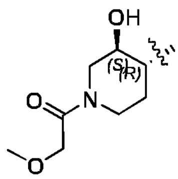, 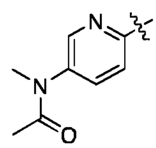, 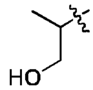, 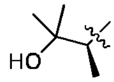,

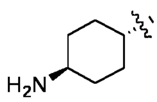, 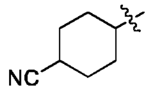, 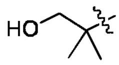, 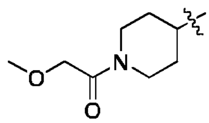, 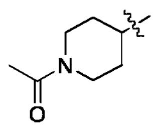, 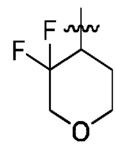,

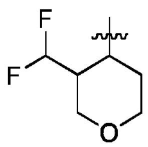, 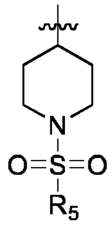, 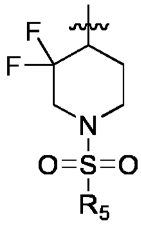, 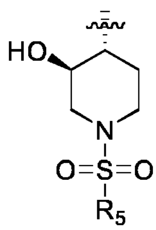, 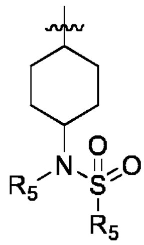, 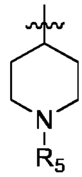,

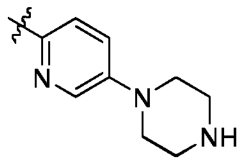, 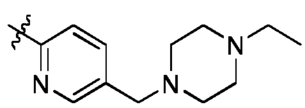, 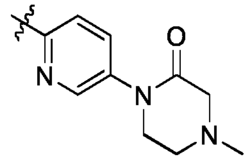,

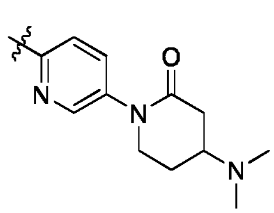, 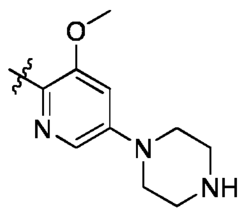, 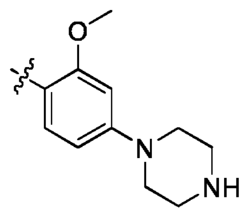, 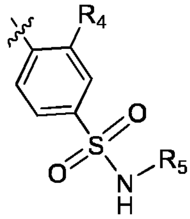,

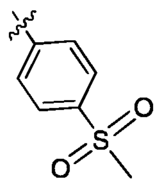 , 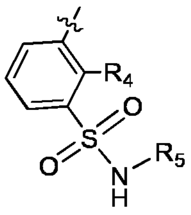 , 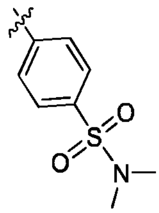 , 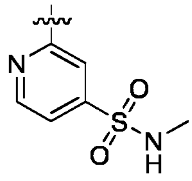 , 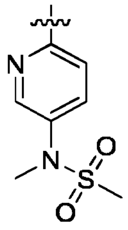 ,

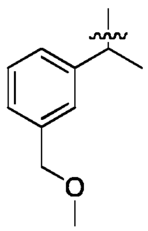 , 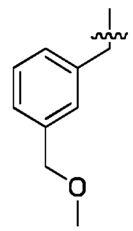 , 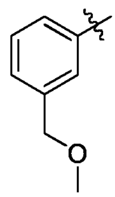 , 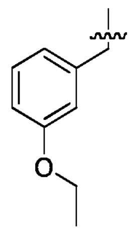 , 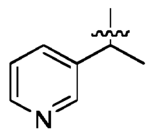 , 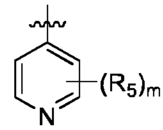 , 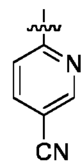 ,

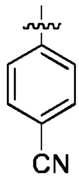 , 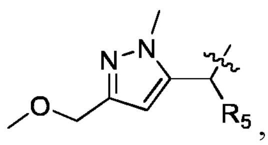 , 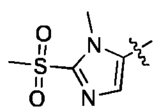 , 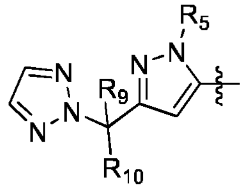 , 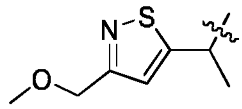 ,

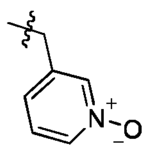 , 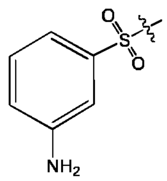 , 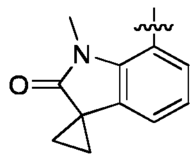 , 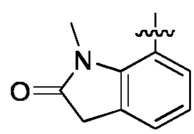 , 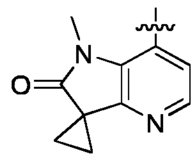 ,

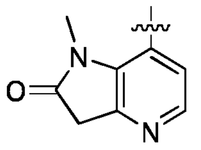 , 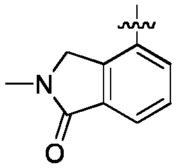 , 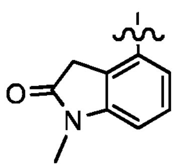 , 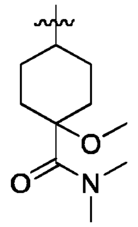 , 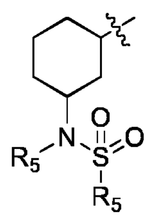 ,

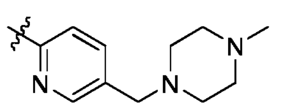 , 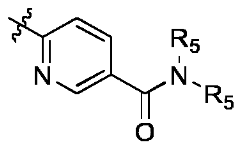 , 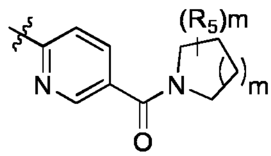 ,

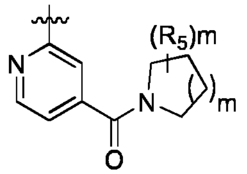 , 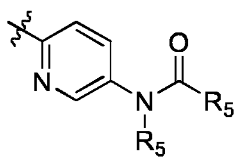 , 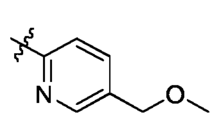 , 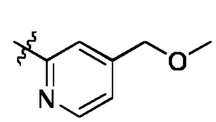 ,

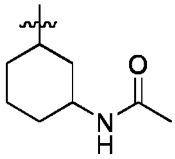, 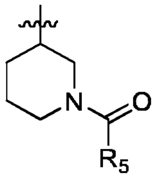, 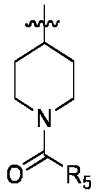, 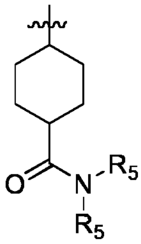, 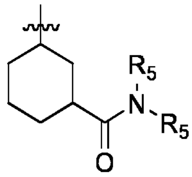, 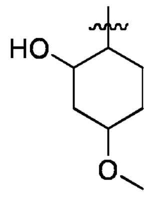,

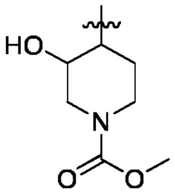, 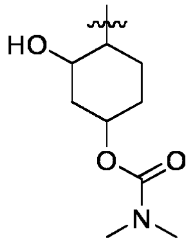, 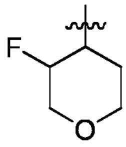, 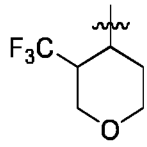, 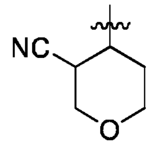, 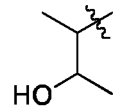,

and

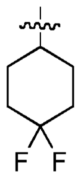;

wherein, each $R_4$ is independently selected from the group consisting of H, cyano, halogen, substituted or unsubstituted C1-C6 alkyl, substituted or unsubstituted C3-C6 cycloalkyl, -C(O)$NR_9R_{10}$, and substituted or unsubstituted -$NR_mR_n$;

each $R_5$ and $R_6$ is independently selected from the group consisting of H, halogen, hydroxyl, amino,

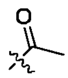, 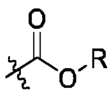, 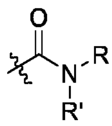,

substituted or unsubstituted C1-C6 alkyl, substituted or unsubstituted C3-C6 cycloalkyl, substituted or unsubstituted phenyl, substituted or unsubstituted $C_{1-6}$ alkoxy, and substituted or unsubstituted -$NR_mR_n$;

$R_7$ and $R_{11}$ are each independently selected from the group consisting of H, and substituted or unsubstituted $C_{1-6}$ alkyl;

$R_8$ is selected from the group consisting of H, $C_{1-6}$ alkoxy, halogenated $C_{1-6}$ alkoxy, -$NR_mR_n$, halogenated -$NR_mR_n$, cyano, substituted or unsubstituted $C_{1-6}$ alkyl, substituted or unsubstituted $C_{3-6}$ cycloalkyl, and -C(O)$NR_9R_{10}$;

each $R_9$ and $R_{10}$ is independently selected from the group consisting of H, halogen, hydroxyl, amino,

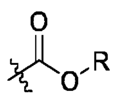, 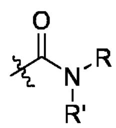,

substituted or unsubstituted C1-C6 alkyl, substituted or unsubstituted C3-C6 cycloalkyl, substituted or unsubstituted phenyl, -C(O)$R_{12}$, and -C(O)O$R_{13}$; or $R_9$ and $R_{10}$ together with the attached N form a substituted or unsubstituted 5-7 membered heterocyclic ring; or either $R_9$ or $R_{10}$ together with $R_5$ form a 5-7 membered ring;

each $R_{14}$ is independently selected from the group consisting of H, halogen, hydroxyl, amino,

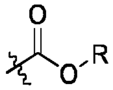, 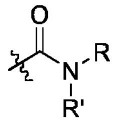,

substituted or unsubstituted C1-C6 alkyl, substituted or unsubstituted C3-C6 cycloalkyl, substituted or unsubstituted phenyl, and oxo; or two $R_{14}$ attached to the same C together with the C to which they are attached form C3-C6 cycloalkyl;

or $R_4$ and $R_5$ together with the C to which they are attached form a 5-7 membered heterocyclic ring containing 1, 2 or 3 heteroatoms selected from N, O, or S;

$R_{12}$ and $R_{13}$ are each independently selected from the group consisting of H, substituted or unsubstituted $C_{1-6}$ alkyl, and substituted or unsubstituted $C_{3-6}$ cycloalkyl;

each R and R' is independently selected from the group consisting of H, C1-C6 alkyl, halogenated C1-C6 alkyl, C3-C6 cycloalkyl, halogenated C3-C6 cycloalkyl, and phenyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each $R_m$ is selected from the group consisting of H, $C_{1-6}$ alkyl, $C_{3-6}$ cycloalkyl, $C_{6-10}$ aryl, and 6-10 membered heteroaryl containing 1, 2, or 3 heteroatoms selected from N, O, or S;

each $R_n$ is $C_{1-6}$ alkyl;

or $R_m$ and $R_n$ together with the N atom to which they are attached form a 3-10 membered N-containing monocyclic or bicyclic heterocyclic group;

each m, n, p is independently selected from the group consisting of 0, 1, 2, 3, 4, and 5;

wherein, when ring A is

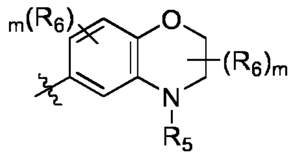,

ring B is selected from the group consisting of

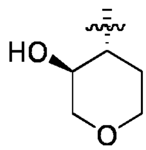, 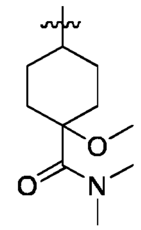, 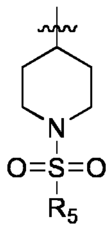, 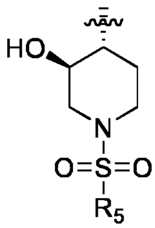, 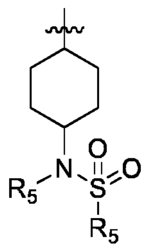,

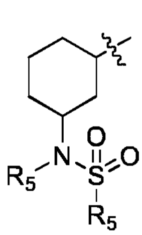, 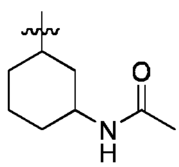, 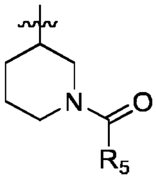, 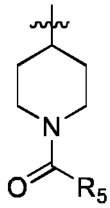, 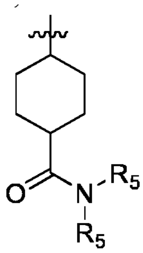, 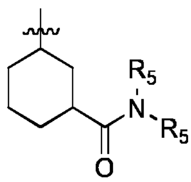,

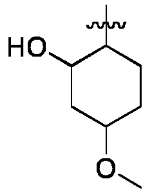, 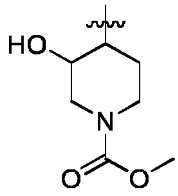, 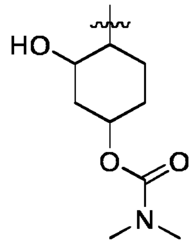, 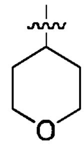, 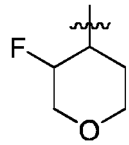, 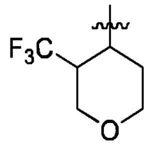,

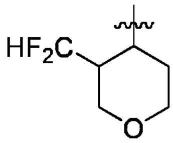, 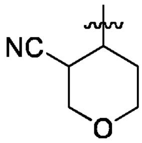, and 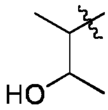.

2. The compound according to claim 1, wherein $X_1$ is selected from the group consisting of N and $CR_3$;

$R_1$ is selected from the group consisting of H, $CF_3$, F, Cl, Br, methyl, ethyl, isopropyl, and cyclopropyl;
$R_2$ is selected from the group consisting of H, $CF_3$, F, Cl, Br, and methyl;
$R_3$ is selected from the group consisting of H, $CF_3$, F, Cl, Br, methyl, and cyano;
Ring A is selected from the group consisting of

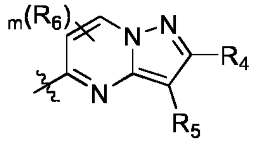,

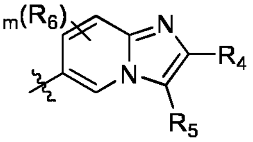, 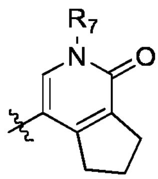, 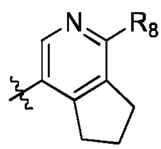, 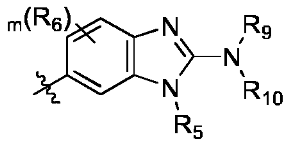,

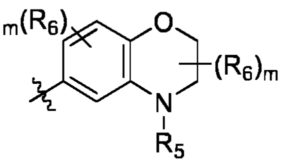, 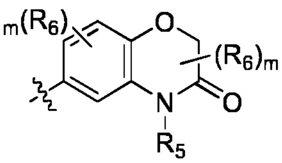, 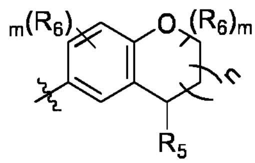,

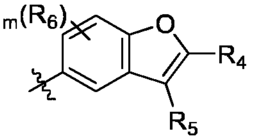, 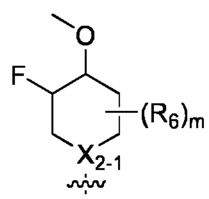, 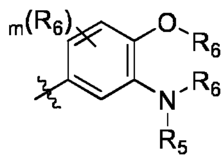, and 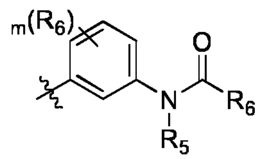,

wherein, $X_{2-1}$ is selected from the group consisting of N and $CR_{11}$;

$R_4$ is selected from the group consisting of H, substituted or unsubstituted $C_{1-6}$ alkyl, substituted or unsubstituted $C_{3-6}$ cycloalkyl, $-C(O)NR_9R_{10}$, and substituted or unsubstituted $-NR_mR_n$;
each $R_5$ is independently selected from the group consisting of H, substituted or unsubstituted $C_{1-6}$ alkyl, substituted or unsubstituted $C_{1-6}$ alkoxy, substituted or unsubstituted $C_{3-6}$ cycloalkyl, and substituted or unsubstituted $-NR_mR_n$;

each $R_6$ is respectively independently selected from the group consisting of halogen, substituted or unsubstituted $C_{1-6}$ alkyl, and substituted or unsubstituted $C_{3-6}$ cycloalkyl;

$R_7$ and $R_{11}$ are each independently selected from the group consisting of H and substituted or unsubstituted $C_{1-6}$ alkyl;

$R_8$ is selected from the group consisting of H, $C_{1-6}$ alkoxy, halogenated $C_{1-6}$ alkoxy, $-NR_mR_n$, halogenated $-NR_mR_n$, cyano, substituted or unsubstituted $C_{1-6}$ alkyl, substituted or unsubstituted $C_{3-6}$ cycloalkyl, and $-C(O)NR_9R_{10}$;

each $R_9$ and $R_{10}$ is independently selected from the group consisting of H, substituted or unsubstituted $C_{1-6}$ alkyl, substituted or unsubstituted $C_{3-6}$ cycloalkyl, $-C(O)R_{12}$, and $-C(O)OR_{13}$; or $R_9$ and $R_{10}$ together with the N to which they are attached form a substituted or unsubstituted 5-7 membered heterocyclic ring; or either $R_9$ or $R_{10}$ together with $R_5$ form a 5-7 membered ring;

or $R_4$ and $R_5$ together with the C to which they are attached form a 5-7membered heterocyclic ring containing 1, 2 or 3 heteroatoms selected from N, O, or S;

$R_{12}$ and $R_{13}$ are each independently selected from the group consisting of H, substituted or unsubstituted $C_{1-6}$ alkyl, and substituted or unsubstituted $C_{3-6}$ cycloalkyl;

the "substituted" each independently means to be substituted with 1, 2, 3 or 4 substituents selected from the group consisting of deuterium, halogen, hydroxyl, amino, $-NR_mR_n$, and $C_{1-6}$ alkoxy;

each $R_m$ is selected from the group consisting of H, $C_{1-6}$ alkyl, $C_{3-6}$ cycloalkyl, $C_{6-10}$ aryl, and 6-10 membered heteroaryl containing 1, 2 or 3 heteroatoms selected from N, O, or S;

each $R_n$ is $C_{1-6}$ alkyl;

each m, n is independently selected from the group consisting of 0, 1, 2, 3, 4, and 5;

Ring B is selected from the group consisting of

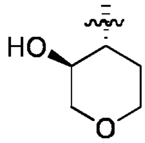, 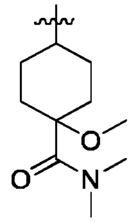,

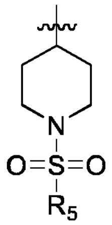, 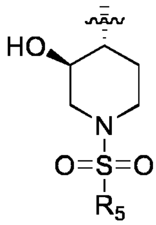, 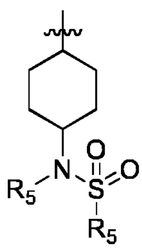, 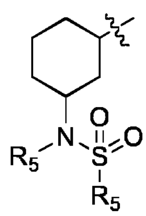, 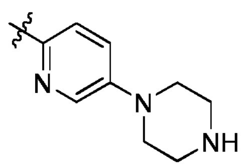,

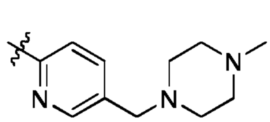, 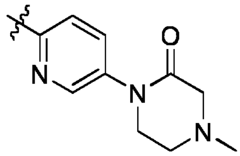, 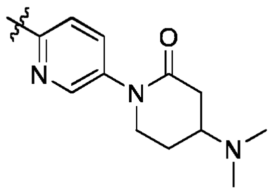, 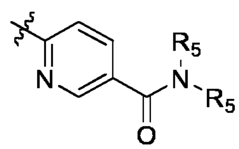,

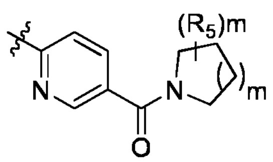, 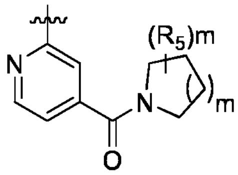, 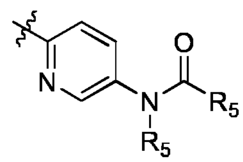, 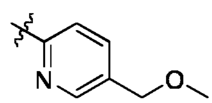,

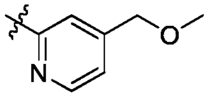, 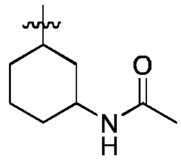, 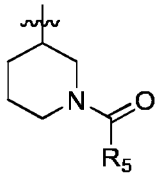, 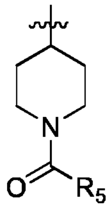, 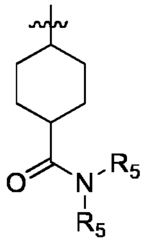,

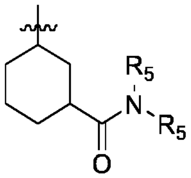, 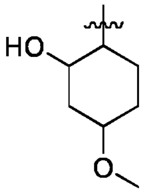, 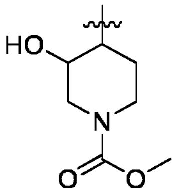, 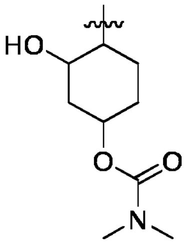, 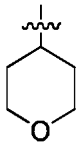, 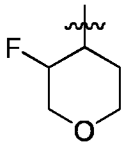,

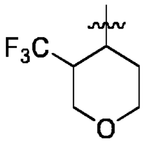, 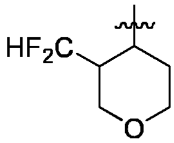, 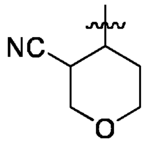, and 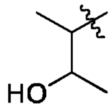;

wherein, when ring A is

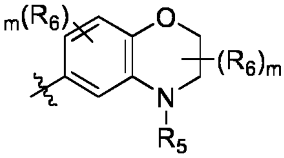,

ring B is selected from the group consisting of

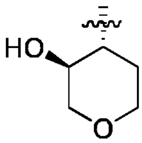, 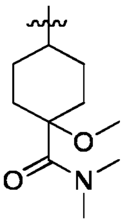, 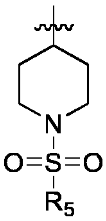, 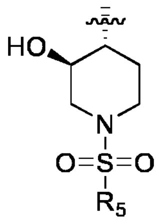, 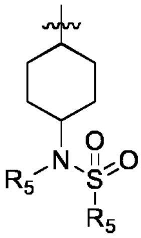,

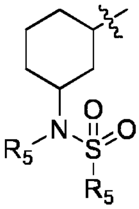, 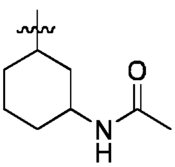, 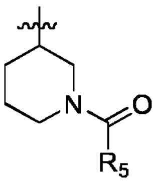, 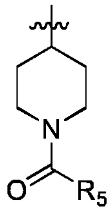, 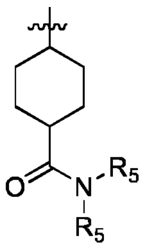, 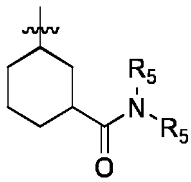,

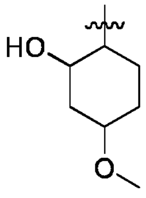, 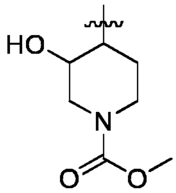, 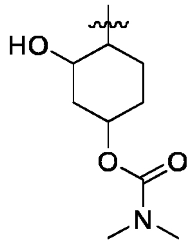, 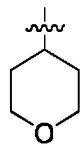, 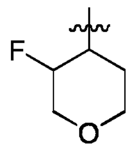, 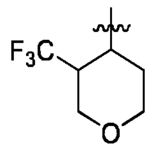,

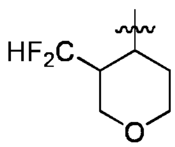, 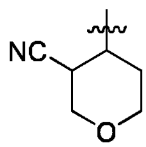, and 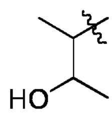.

3. A compound for use as a CDK4 kinase inhibitor, wherein the compound is a compound of formula II, or a pharmaceutically acceptable salt, stereoisomer, tautomer, hydrate, solvate, isotope compound, or prodrug thereof, wherein,

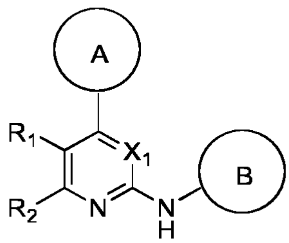
formula II

$X_1$ is selected from the group consisting of N and $CR_3$;
$R_1$ is selected from the group consisting of H, $CF_3$, F, Cl, Br, methyl, ethyl, isopropyl, and cyclopropyl;
$R_2$ is selected from the group consisting of H, F, $CF_3$, $CF_2H$, $NH_2$, and methyl;
or $R_1$ and $R_2$ together with the C to which they are attached form a 5-membered heteroaryl containing two N atoms;
$R_3$ is selected from the group consisting of H, $CF_3$, F, Cl, Br, methyl, and cyano;
Ring A is selected from the group consisting of

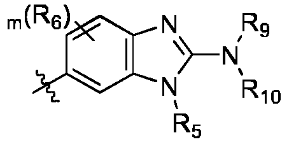,

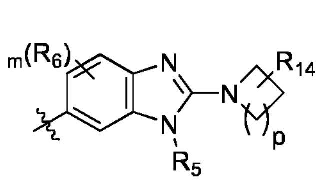, 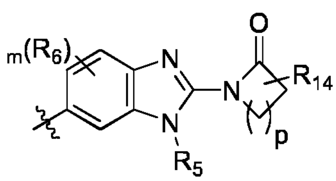, 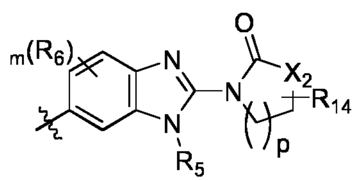,

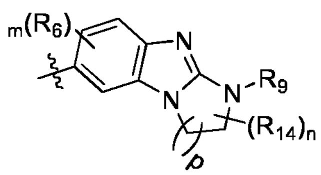, 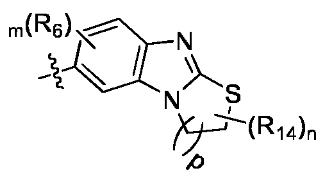, 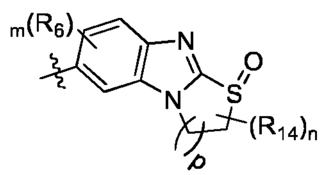,

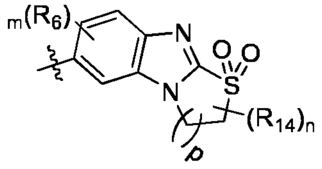, 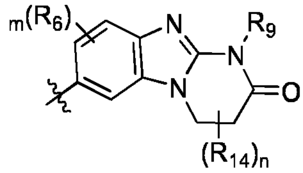, and 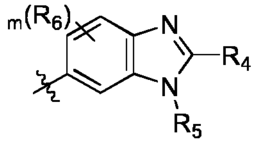,

wherein, $X_2$ is O or NR;
Ring B is selected from the group consisting of

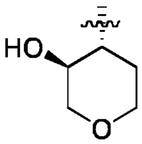, 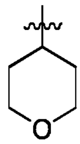, 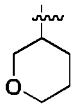,

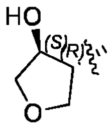, 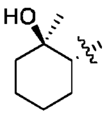, 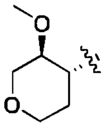, 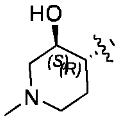, 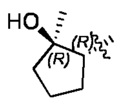, 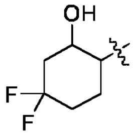, 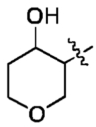,

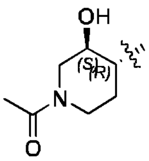, 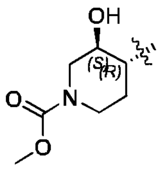, 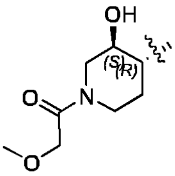, 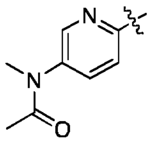, 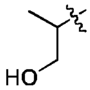, 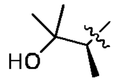,

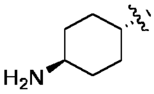, 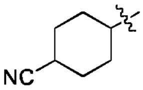, 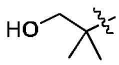, 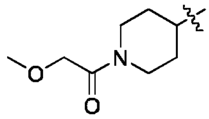, 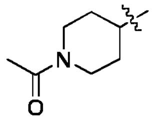, 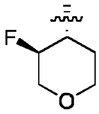,

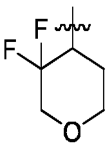, 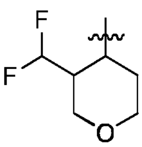, 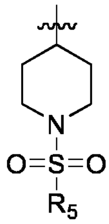, 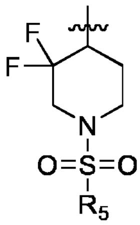, 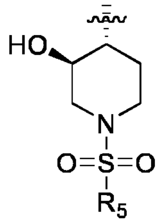, 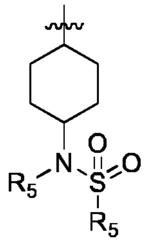, 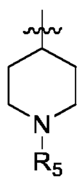,

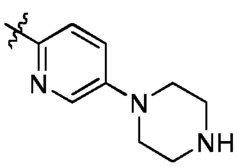, 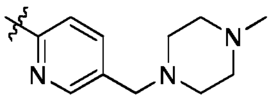, 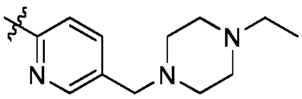,

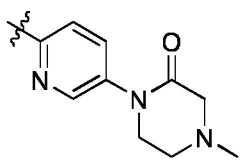, 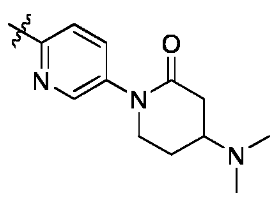, 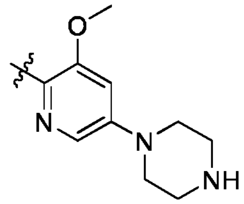, 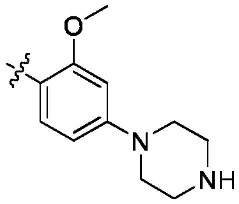,

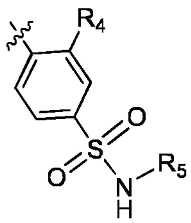, 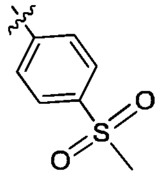, 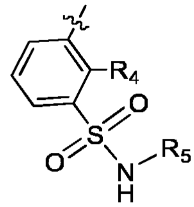, 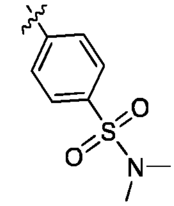, 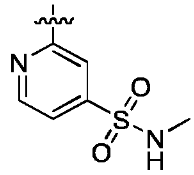,

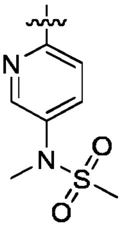, 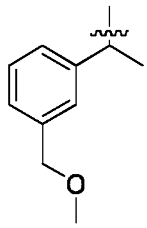, 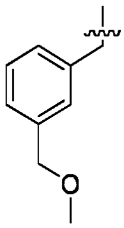, 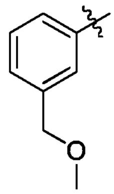, 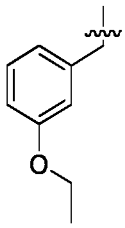, 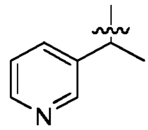, 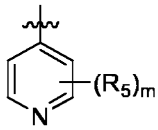,

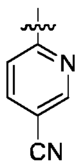, 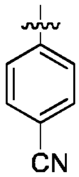, 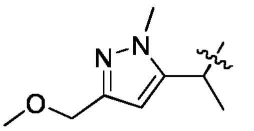, 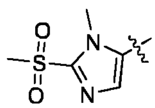, 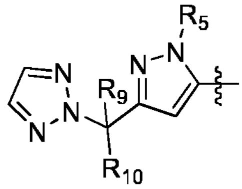,

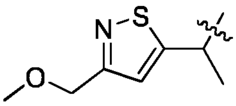, 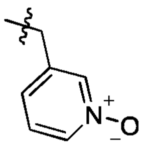, 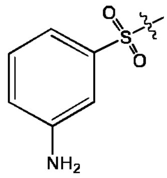, 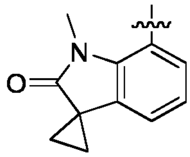, 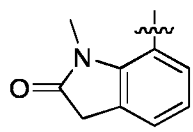,

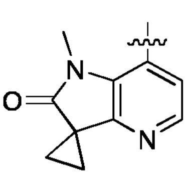, 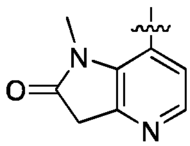, 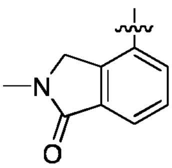, 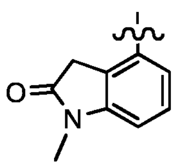, and 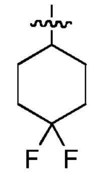;

wherein, each $R_4$ is independently selected from the group consisting of H, cyano, halogen, substituted or unsubstituted C1-C6 alkyl, and substituted or unsubstituted C3-C6 cycloalkyl;

each $R_5$, $R_6$, $R_9$, $R_{10}$, and $R_{14}$ is independently selected from the group consisting of H, halogen, hydroxyl, amino,

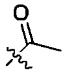, 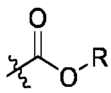, 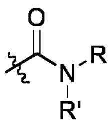,

oxo, substituted or unsubstituted C1-C6 alkyl, substituted or unsubstituted C3-C6 cycloalkyl, substituted or unsubstituted phenyl, substituted or unsubstituted $C_{1-6}$ alkoxy, and substituted or unsubstituted $-NR_mR_n$; or two $R_{14}$ attached to the same C together with the C to which they are attached form C3-C6 cycloalkyl; or $R_9$ and $R_{10}$ together with N to which they are attached form

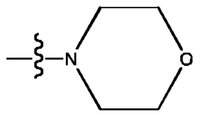 or 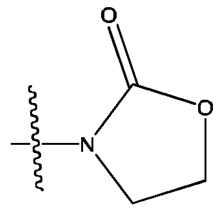 ;

or when ring A is

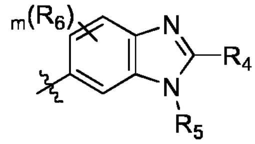 ,

$R_4$ and $R_5$ together with the ring to which they are attached form a 5-7 membered heterocycle containing 1, 2 or 3 heteroatoms selected from N, O, or S;
each R and R' is independently selected from the group consisting of H, C1-C6 alkyl, halogenated C1-C6 alkyl, C3-C6 cycloalkyl, halogenated C3-C6 cycloalkyl, and phenyl;
each $R_m$ is selected from the group consisting of H, $C_{1-6}$ alkyl, $C_{3-6}$ cycloalkyl, $C_{6-10}$ aryl, and 6-10 membered heteroaryl containing 1, 2 or 3 heteroatoms selected from N, O, or S;
each $R_n$ is $C_{1-6}$ alkyl;
the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;
each m, n, p is independently selected from the group consisting of 0, 1, 2, 3, and 4.

**4.** The compound according to claim 3, wherein,

$R_1$ is selected from the group consisting of H, $CF_3$, F, Cl, Br, methyl, ethyl, isopropyl, and cyclopropyl;
$R_2$ is selected from the group consisting of H, F, $CF_3$, $CF_2H$, $NH_2$, and methyl;
Ring A is selected from the group consisting of

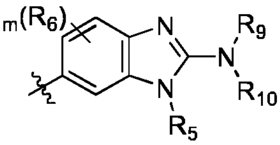 ,

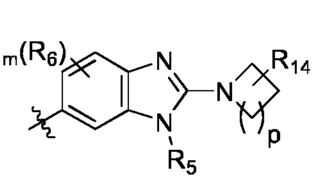 , 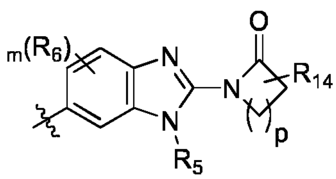 , 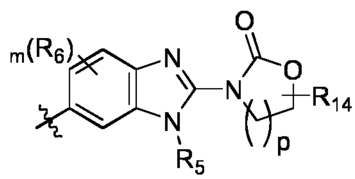 ,

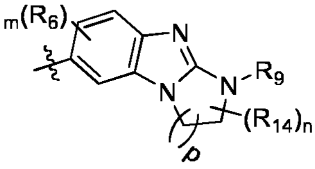 , and 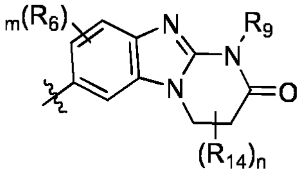 ,

Ring B is selected from the group consisting of

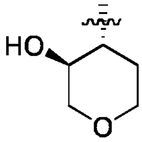 , 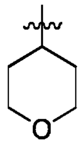 , 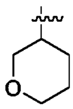 ,

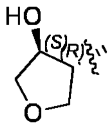 , 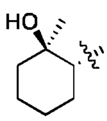 , 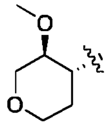 , 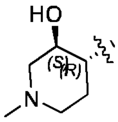 , 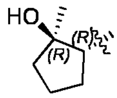 , 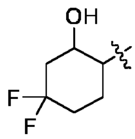 , 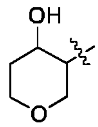 ,

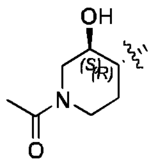 , 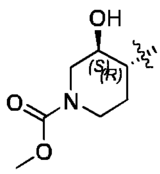 , 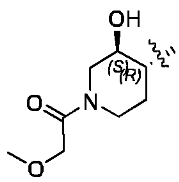 , 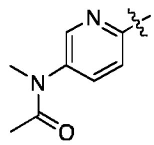 , 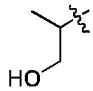 , 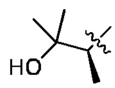 ,

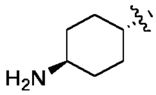 , 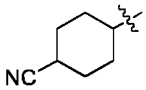 , 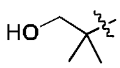 , 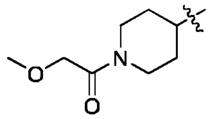 , 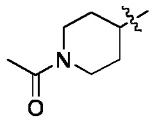 , 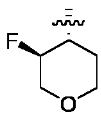 ,

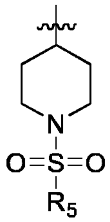 , 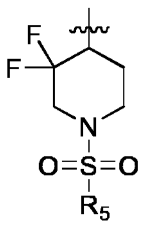 , 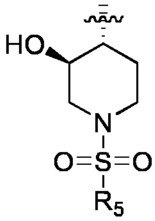 , 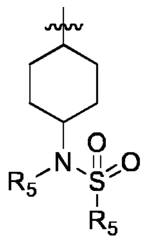 , 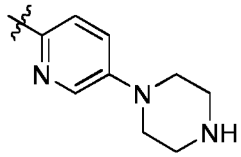 ,

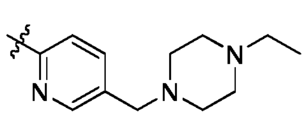 , 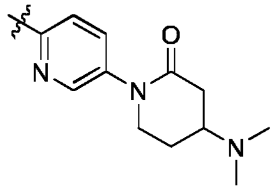 , 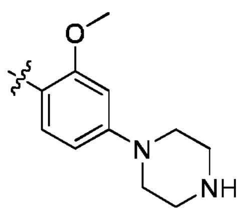 , 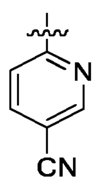 ,

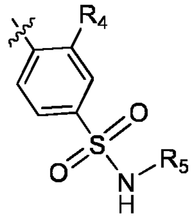 , 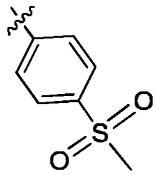 , 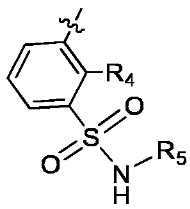 , 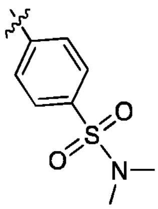 , 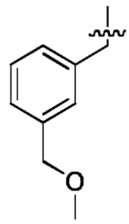 , 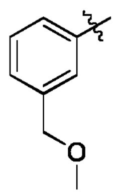 ,

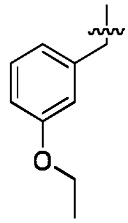 , 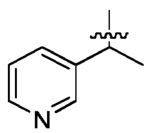 , 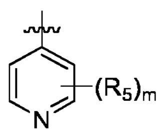 , 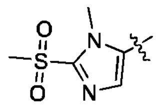 , 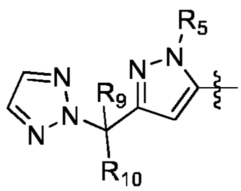 , 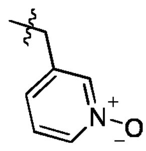 ,

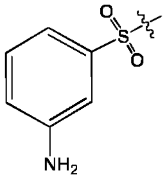 , 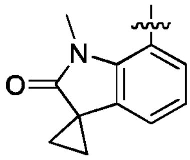 , 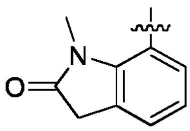 , 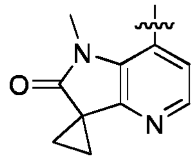 , 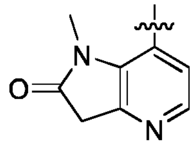 ,

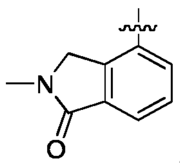 , and 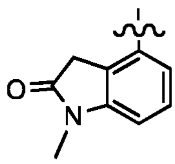 ;

wherein,

each $R_5$, $R_6$, $R_9$, $R_{10}$ and $R_{14}$ is independently selected from the group consisting of H, halogen, hydroxyl, amino,

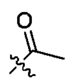 , 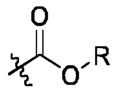 , 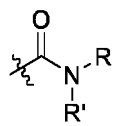 ,

substituted or unsubstituted C1-C6 alkyl, substituted or unsubstituted C3-C6 cycloalkyl, and substituted or unsubstituted phenyl;

or $R_9$, $R_{10}$ together with N to which they are attached form

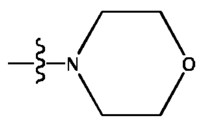 ;

R and R' are each independently selected from the group consisting of H, C1-C6 alkyl, halogenated C1-C6 alkyl, C3-C6 cycloalkyl, halogenated C3-C6 cycloalkyl, and phenyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each m, n, p is independently selected from the group consisting of 0, 1, 2, 3, and 4.

**5.** The compound according to claim 3, wherein,

$R_1$ is selected from the group consisting of Cl and Br;
$R_2$ is H;
Ring A is selected from the group consisting of

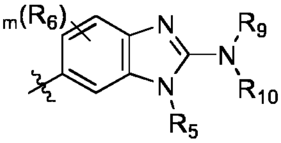

,

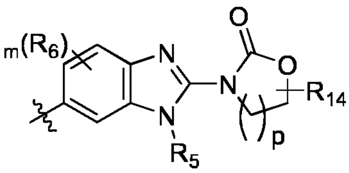

,

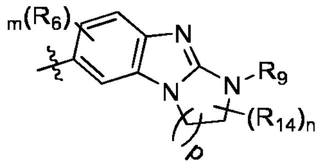

, and

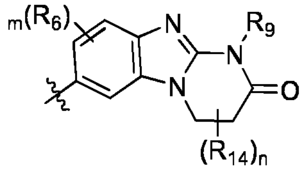

;

Ring B is selected from the group consisting of

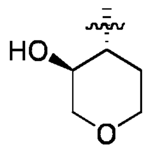

,

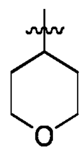

,

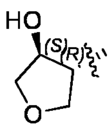

,

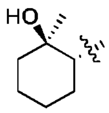

,

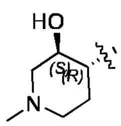

,

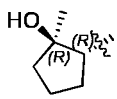

,

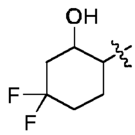

,

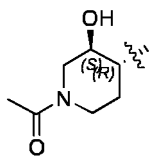

,

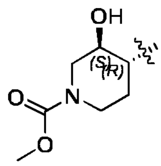

,

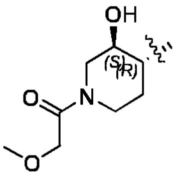

,

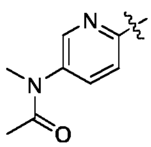

,

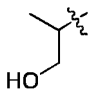

,

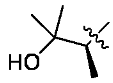

,

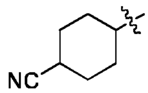

,

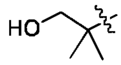

,

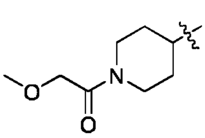

,

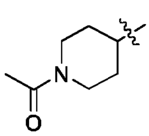

,

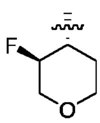

,

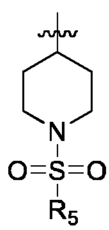

,

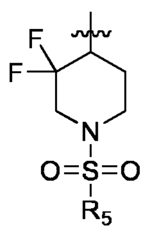

,

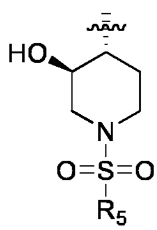

,

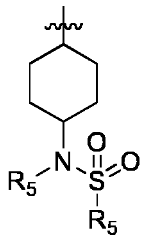, 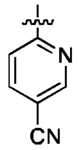, 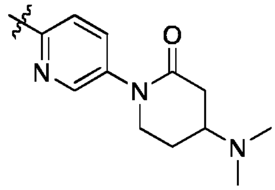, 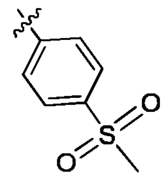, 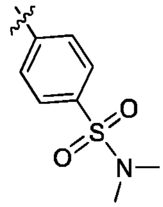, 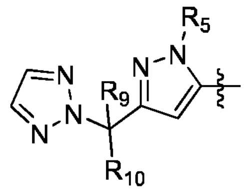, and 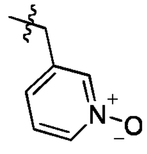;

wherein,

each $R_5$, $R_6$, $R_9$, $R_{10}$, and $R_{14}$ is independently selected from the group consisting of H, halogen, hydroxyl, amino,

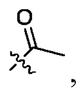, 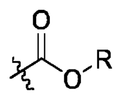, 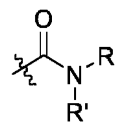,

substituted or unsubstituted C1-C6 alkyl, substituted or unsubstituted C3-C6 cycloalkyl, and substituted or unsubstituted phenyl;

or $R_9$ and $R_{10}$ together with N to which they are attached form

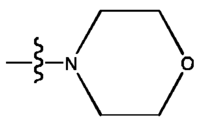;

R and R' is each independently selected from the group consisting of H, C1-C6 alkyl, halogenated C1-C6 alkyl, C3-C6 cycloalkyl, halogenated C3-C6 cycloalkyl, and phenyl;

the substituted in $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each m, n, p is independently selected from the group consisting of 0, 1, 2, 3, and 4.

**6.** The compound according to claim 3, wherein,

$R_1$ and $R_2$ together with the C to which they are attached form a 5-membered heteroaryl containing two N atoms;
Ring A is selected from the group consisting of

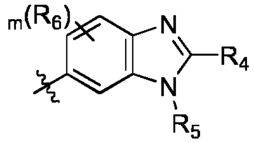,

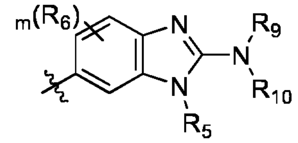 , 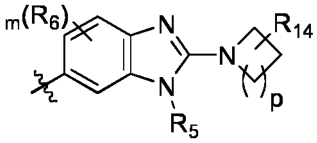 , 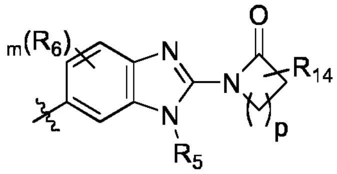 ,

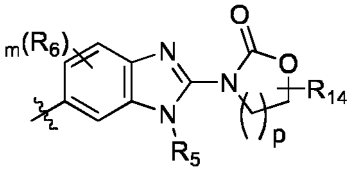 , 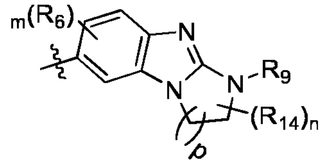 , and 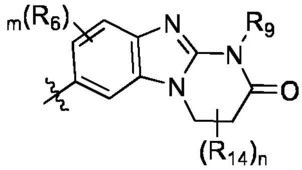 ,

Ring B is selected from the group consisting of

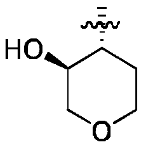 , 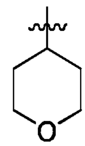 , 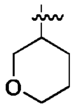 ,

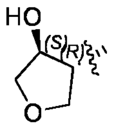 , 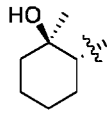 , 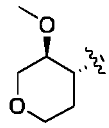 , 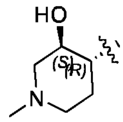 , 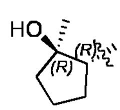 , 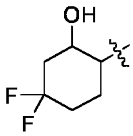 , 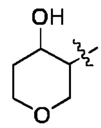 ,

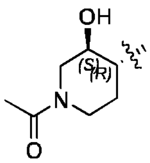 , 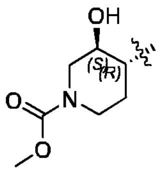 , 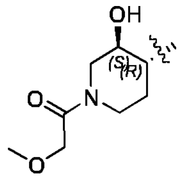 , 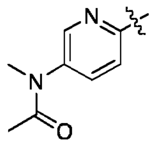 , 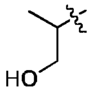 , 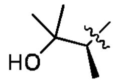 ,

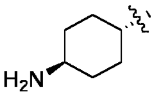 , 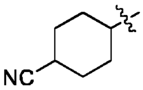 , 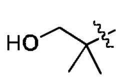 , 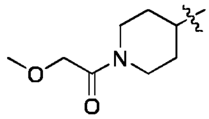 , 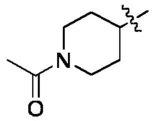 , 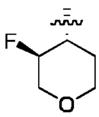 ,

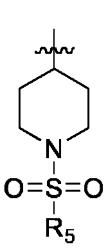 , 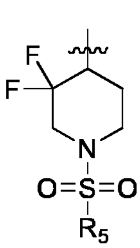 , 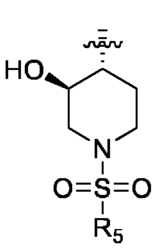 , 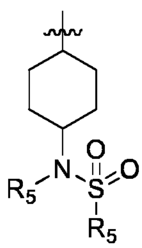 , 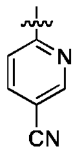 , 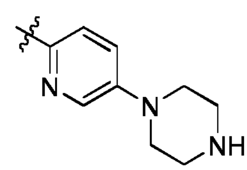 ,

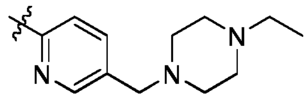, 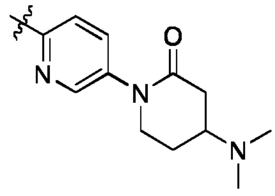, 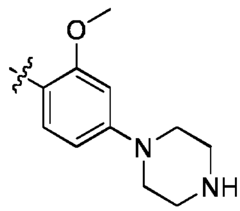, 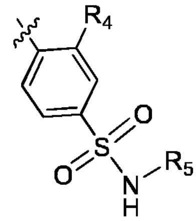,

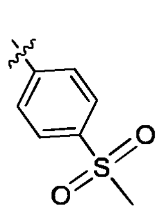, 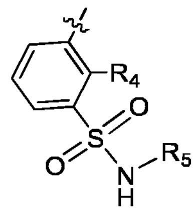, 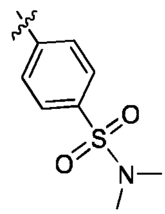, 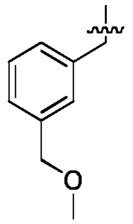, 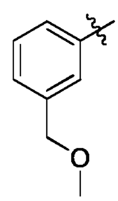, 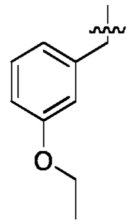,

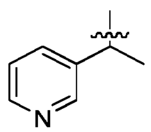, 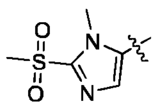, 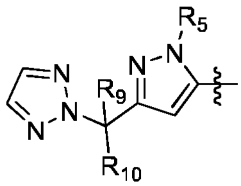, 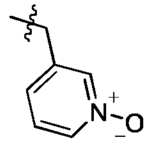, 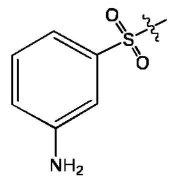,

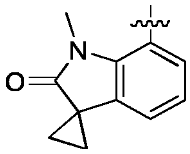, 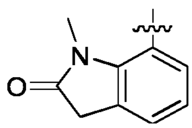, 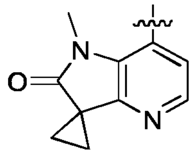, 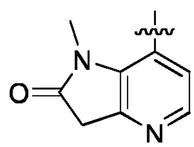, 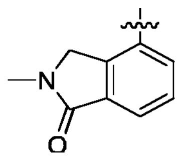,

and

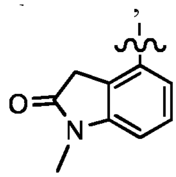;

wherein, each $R_4$ is independently selected from the group consisting of H, substituted or unsubstituted C1-C6 alkyl, and substituted or unsubstituted C3-C6 cycloalkyl;

each $R_5$, $R_6$, $R_9$, $R_{10}$, and $R_{14}$ is independently selected from the group consisting of H, halogen, hydroxyl, amino,

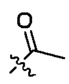, 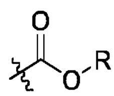, 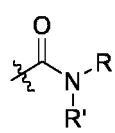,

substituted or unsubstituted C1-C6 alkyl, substituted or unsubstituted C3-C6 cycloalkyl, and substituted or unsubstituted phenyl;

R and R' is independently selected from the group consisting of H, C1-C6 alkyl, halogenated C1-C6 alkyl, C3-C6 cycloalkyl, halogenated C3-C6 cycloalkyl, and phenyl;

the substituted in $R_4$, $R_5$, $R_6$, $R_9$, $R_{10}$ and $R_{14}$ each independently refers to be substituted by 1, 2 or 3 substituents selected from the group consisting of deuterium, halogen, hydroxyl, amino, -N-(C1-C6 alkyl)$_2$, C1-C6 alkoxy, phenyl, and cyano;

each m, n, p is independently selected from the group consisting of 0, 1, 2, 3, and 4.

**7.** The compound according to claim 6, wherein,

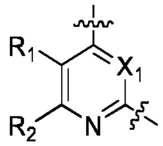

has the following structure:

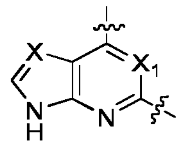
;

X is selected from the group consisting of N and $CR_3$;
$R_3$ is selected from the group consisting of H, $CF_3$, F, Cl, Br, methyl, and cyano.

**8.** A compound for use as a CDK4 kinase inhibitor, or a pharmaceutically acceptable salt, stereoisomer, tautomer, hydrate, solvate, isotope compound, or prodrug thereof, wherein, the compound is selected from the group consisting of:

| | | |
|---|---|---|
| 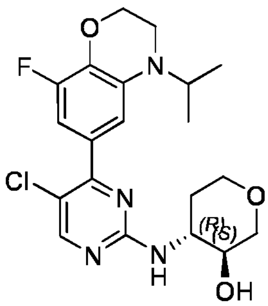 TA-1 | 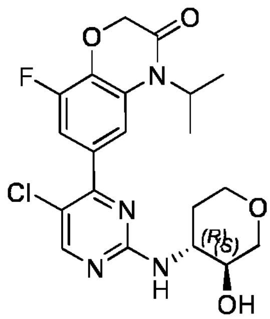 TA-2 | 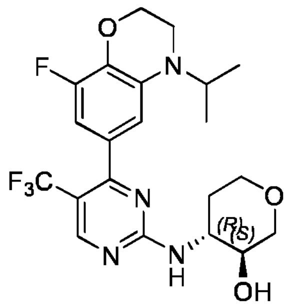 TA-3 |

| | | |
|---|---|---|
| 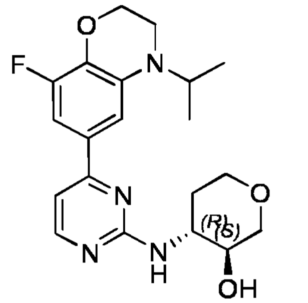 TA-4 | 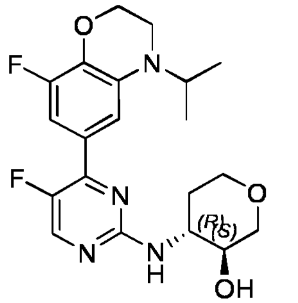 TA-5 | 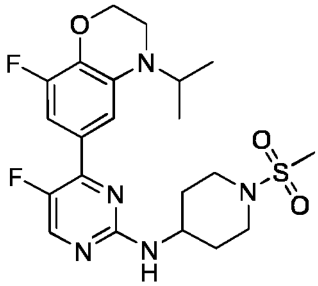 TA-6 |
| 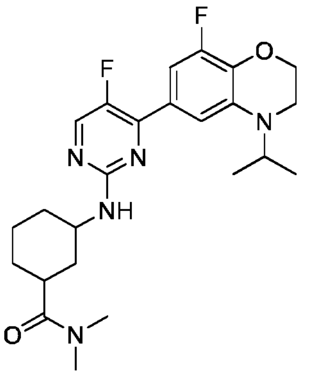 TA-7 | 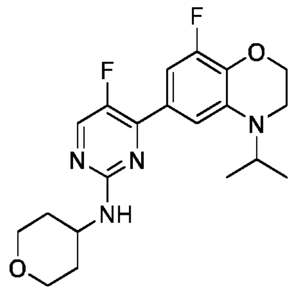 TA-8 | 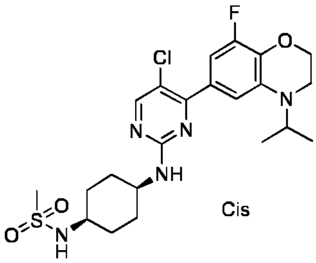  TA-9 |
| 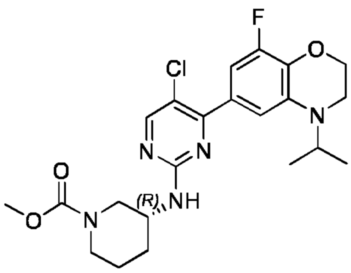 TA-10 | 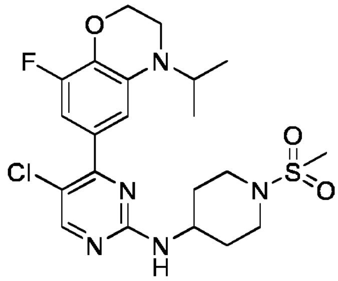 TA-11 | 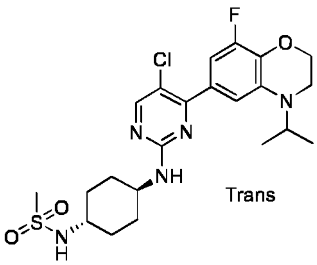  TA-12 |
| 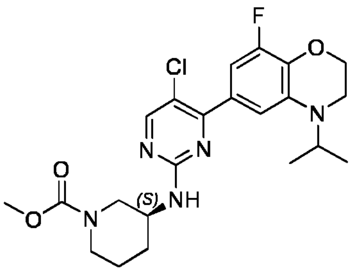 TA-13 | 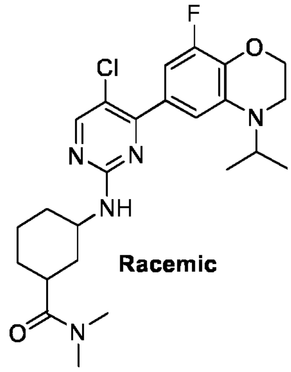  TA-14 | 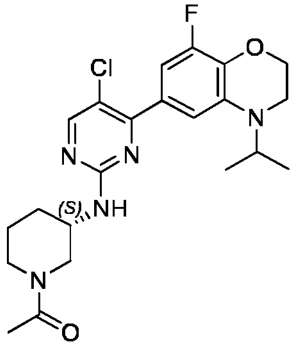 TA-15 |

| | | |
|---|---|---|
| 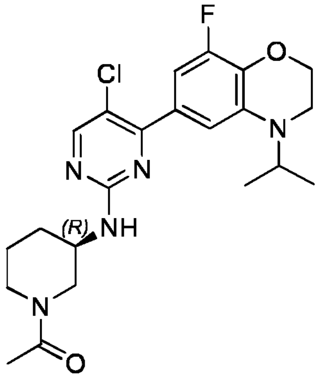 TA-16 | 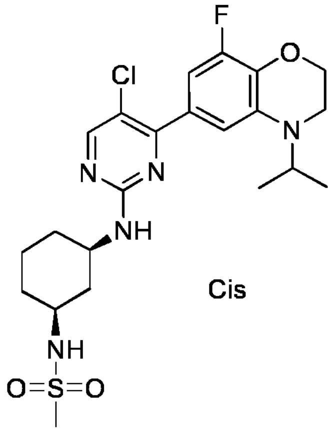 Cis TA-17 | 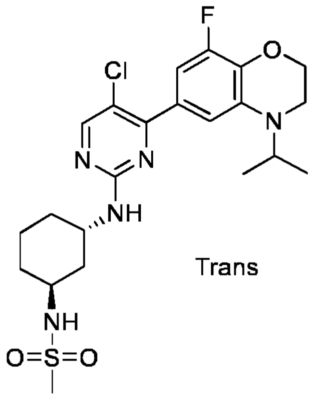 Trans TA-18 |
| 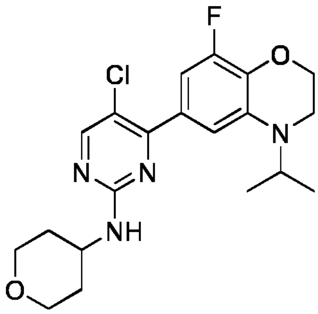 TA-19 | 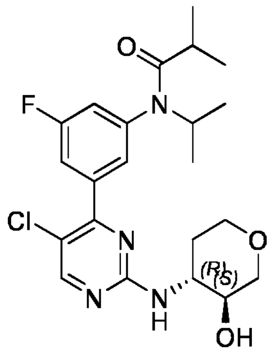 TA-20 | 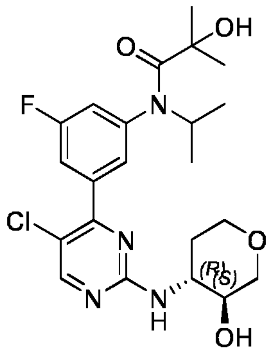 TA-21 |
| 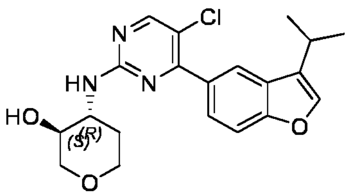 TA-22 | 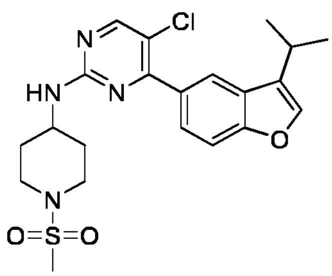 TA-23 | 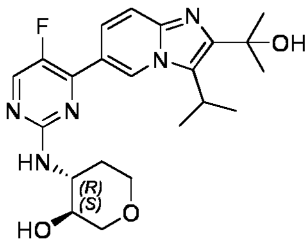 TA-24 |
| 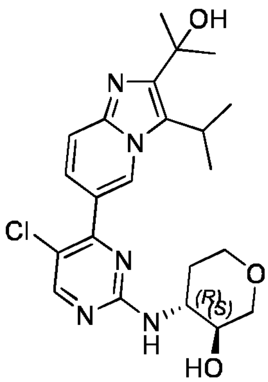 TA-25 | 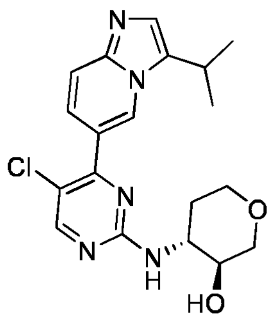 TA-26 | 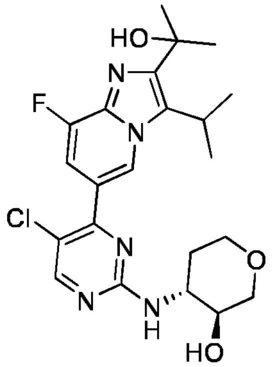 TA-27 |

| | | |
|---|---|---|
| 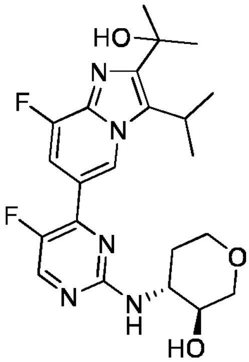 TA-28 | 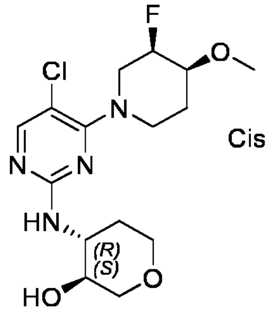 Cis TA-30 | 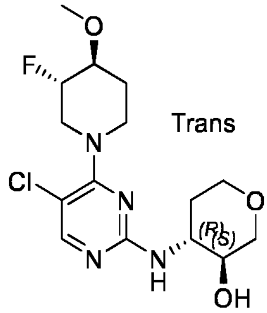 Trans TA-31 |
| 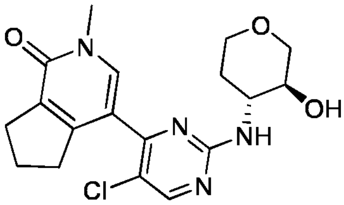 TA-32 | 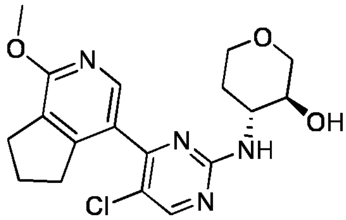 TA-33 | 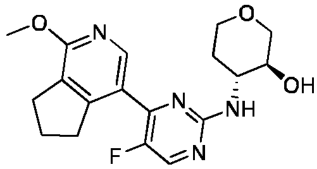 TA-34 |
| 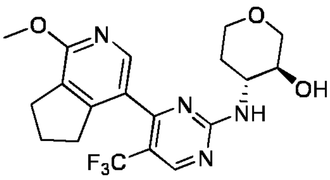 TA-35 | 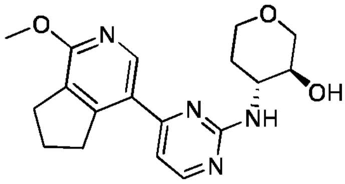 TA-36 | 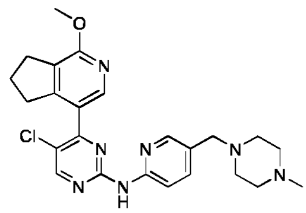 TA-37 |
| 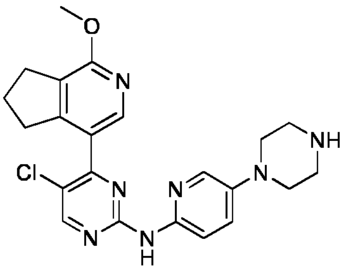 TA-38 | 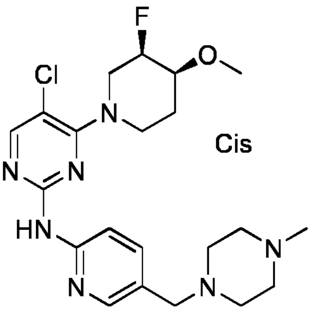 Cis TA-39 | 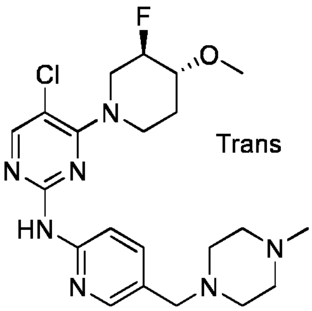 Trans TA-40 |
| 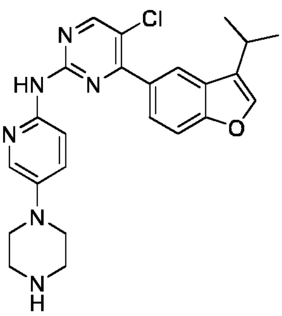 TA-41 | 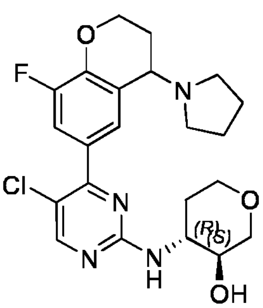 TA-42 | 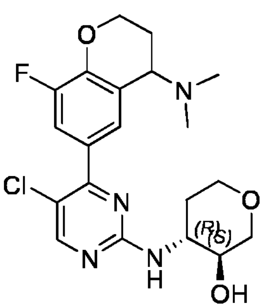 TA-43 |

| | | |
|---|---|---|
| 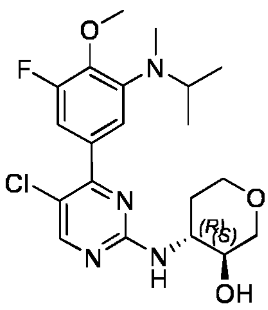 TA-44 | 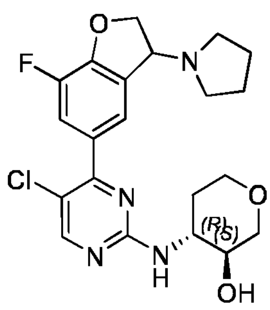 TA-45 | 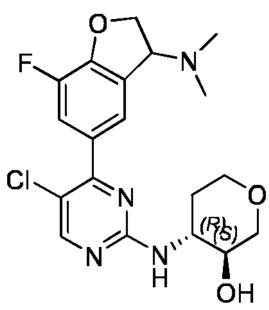 TA-46 |
| 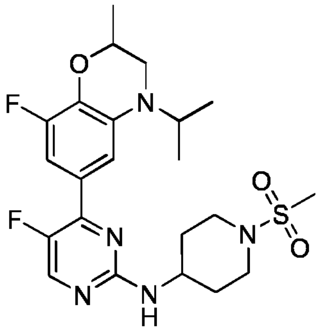 TA-47 | 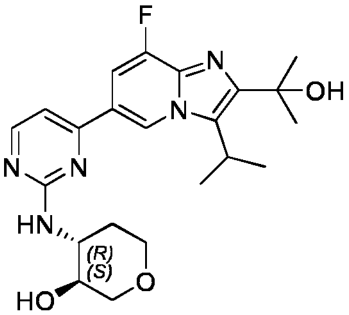 TA-48 | 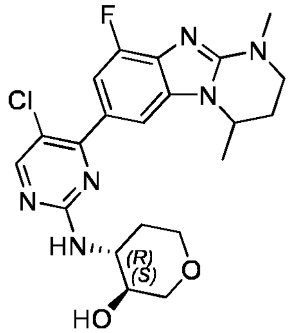 TA-50 |
| 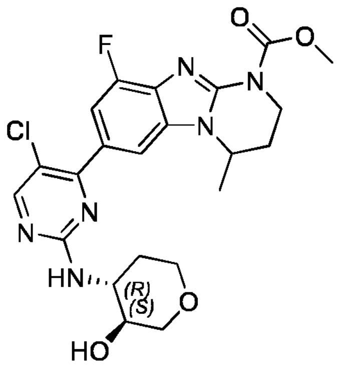 TA-52 | 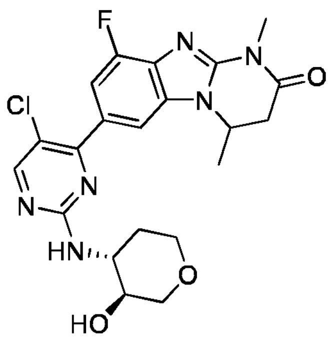 TA-53 | 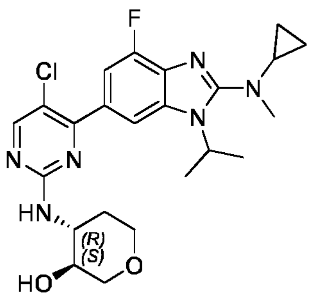 TA-56 |
| 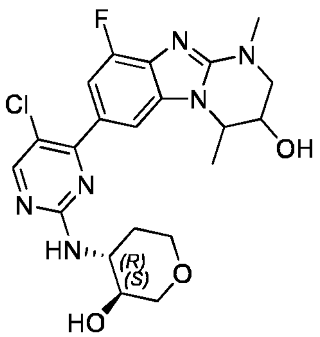 TA-58 | 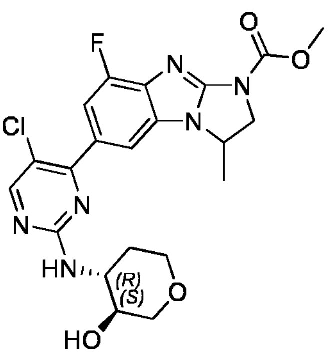 TA-59 | 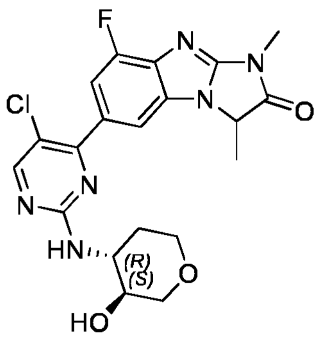 TA-60 |

| | | |
|---|---|---|
| 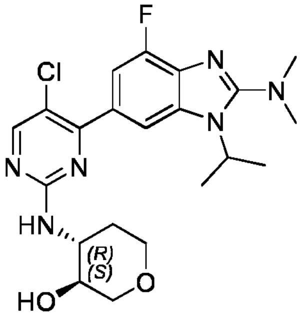 TB-1 | 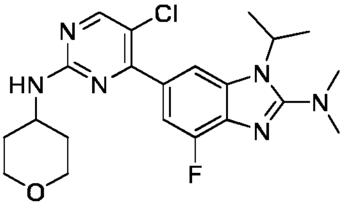 TB-2 | 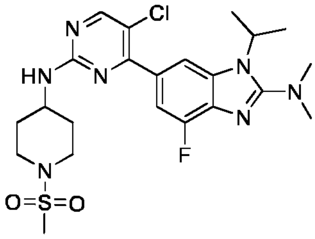 TB-3 |
| 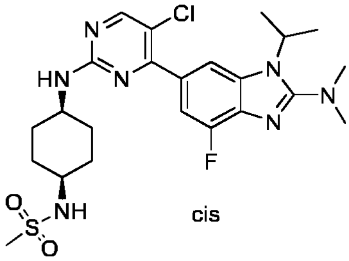 cis TB-4 | 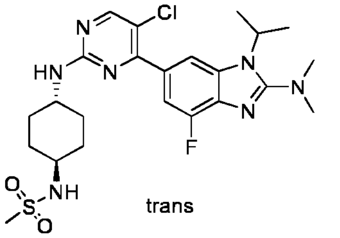 trans TB-5 | 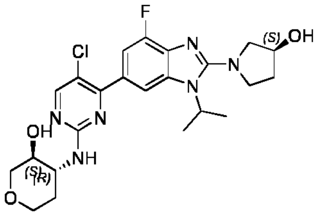 TB-6 |
| 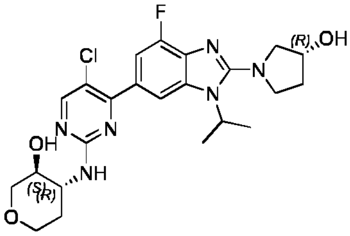 TB-7 | 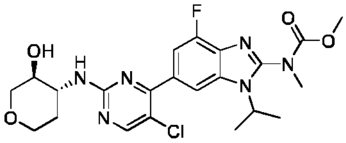 TB-8 | 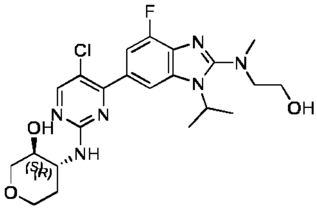 TB-9 |
| 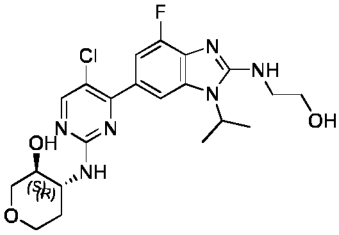 TB-10 | 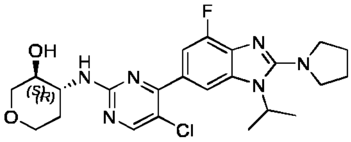 TB-11 | 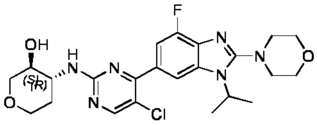 TB-12 |
| 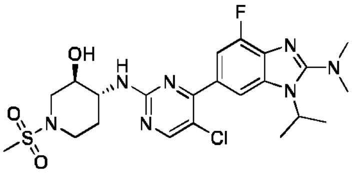 TB-13 | 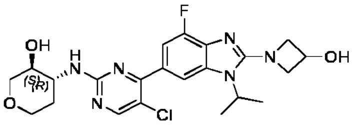 TB-14 | 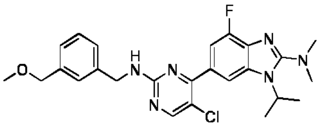 TB-15 |

| | | |
|---|---|---|
| 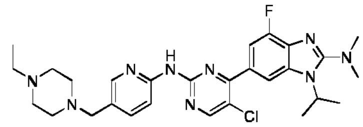 TB-16 | 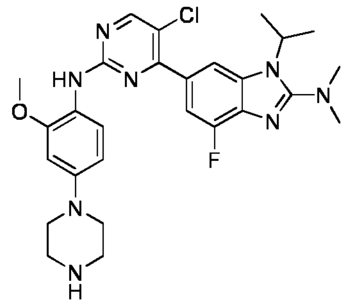 TB-17 | 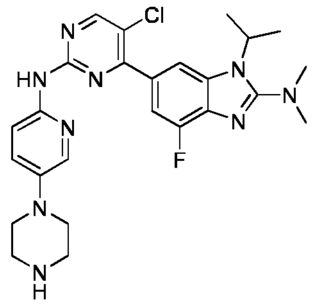 TB-18 |
| 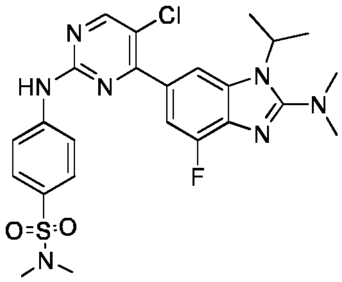 TB-19 | 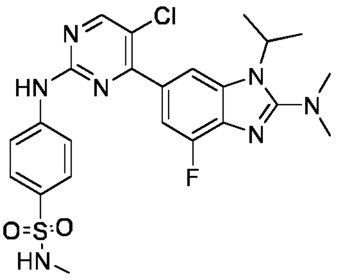 TB-20 | 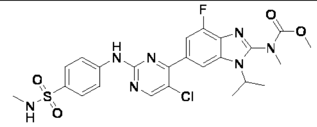 TB-21 |
| 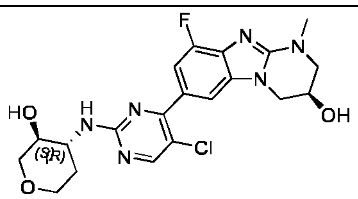 TB-22 | 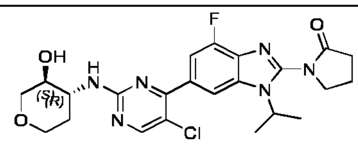 TB-23 | 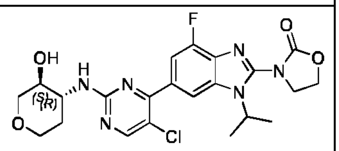 TB-24 |
| 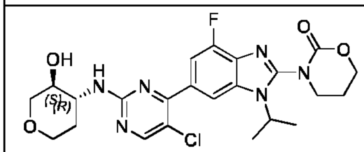 TB-25 | 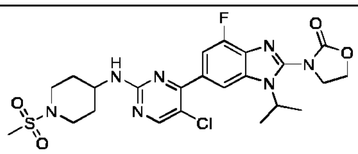 TB-26 | 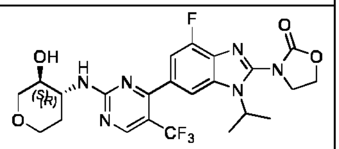 TB-27 |
| 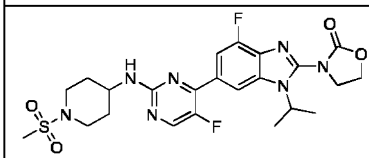 TB-28 | 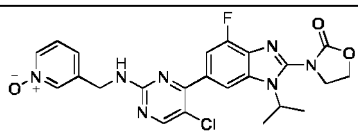 TB-29 | 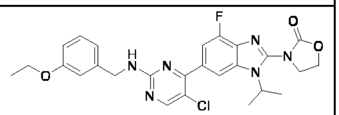 TB-30 |
| 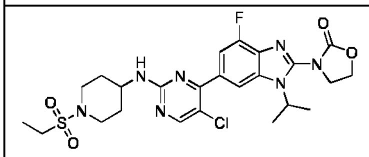 TB-31 | 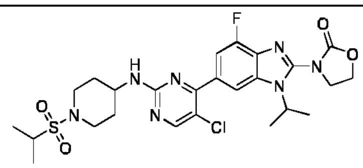 TB-32 | 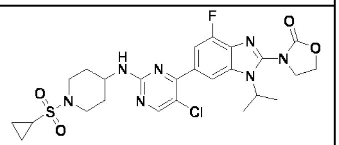 TB-33 |

| | | |
|---|---|---|
| 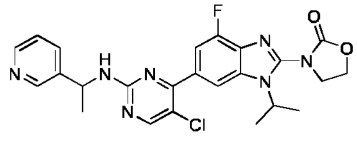 TB-34 | 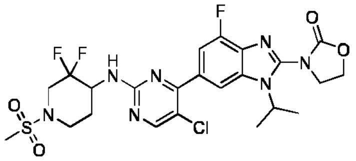 TB-35 | 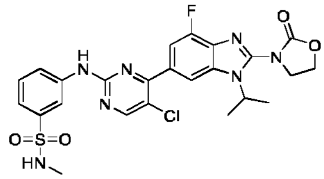 TB-36 |
| 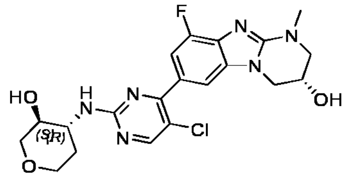 TB-37 | 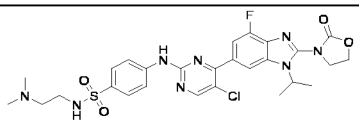 TB-38 | 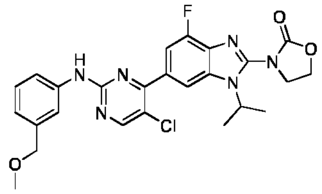 TB-39 |
| 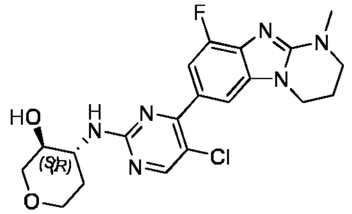 TB-40 | 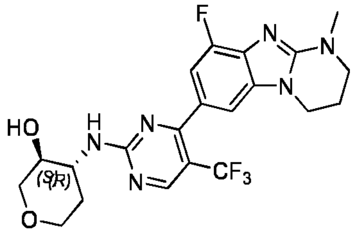 TB-41 | 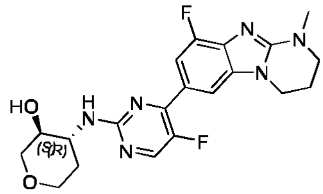 TB-42 |
| 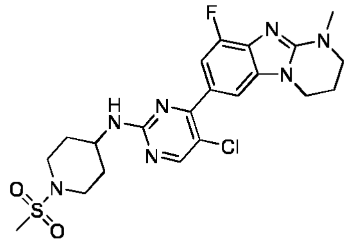 TB-43 | 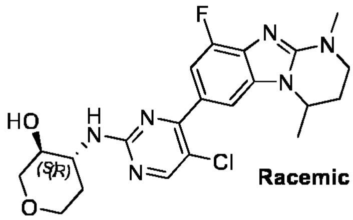 Racemic TB-44 | 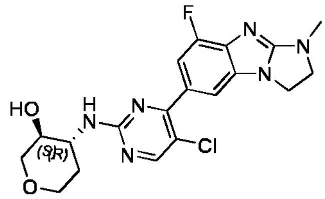 TB-45 |
| 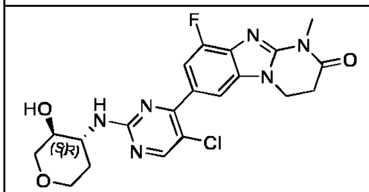 TB-46 | 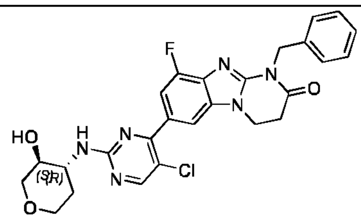 TB-47 | 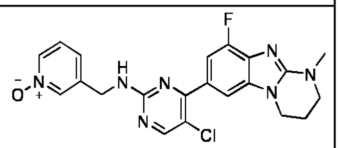 TB-48 |

| | | |
|---|---|---|
| 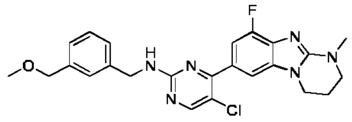<br>TB-49 | 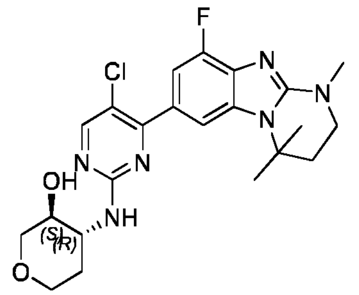<br>TB-50 | 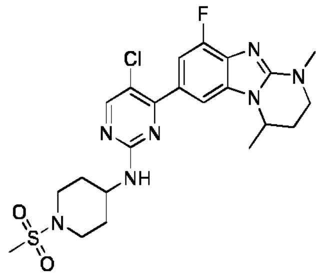<br>TB-51 |
| 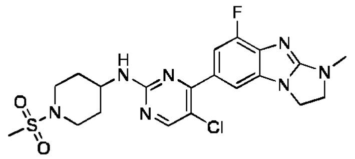<br>TB-52 | 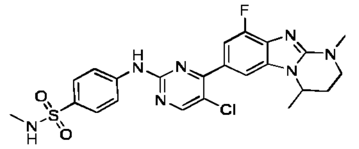<br>TB-53 | 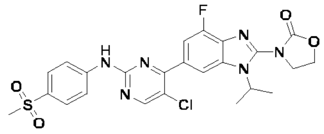<br>TB-54 |
| 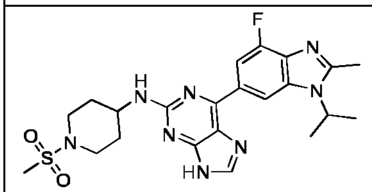<br>TB-55 | 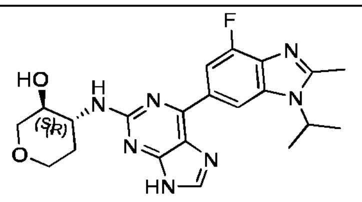<br>TB-56 | 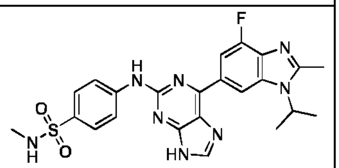<br>TB-57 |
| 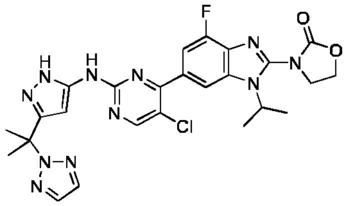<br>TB-58 | 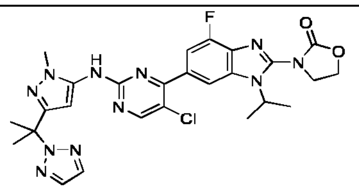<br>TB-59 | 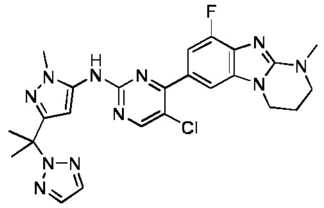<br>TB-60 |
| 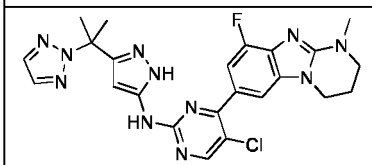<br>TB-61 | 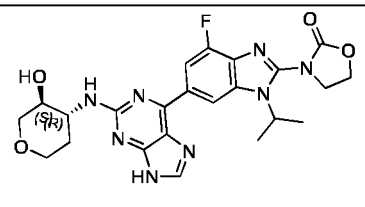<br>TB-62 | 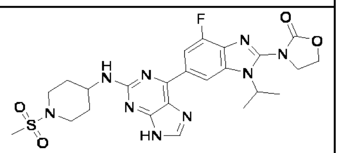<br>TB-63 |

| | | |
|---|---|---|
| 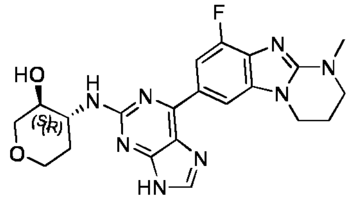 TB-64 | 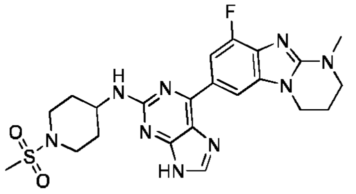 TB-65 | 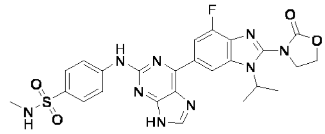 TB-66 |
| 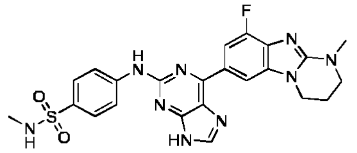 TB-67 | 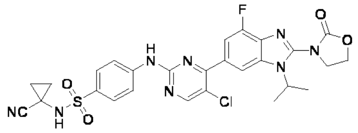 TB-68 | 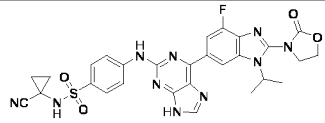 TB-69 |
| 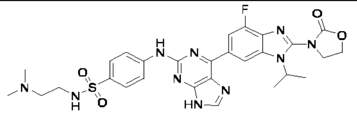 TB-70 | 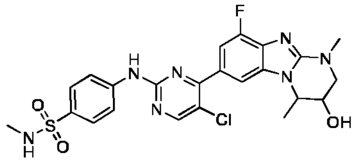 TB-71 | 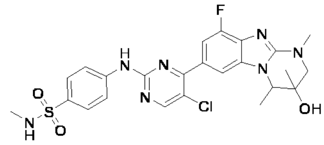 TB-72 |
| 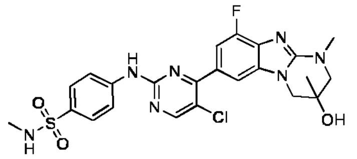 TB-73 | 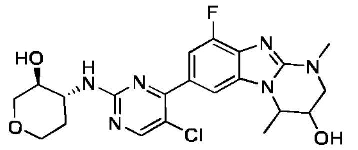 TB-74 | 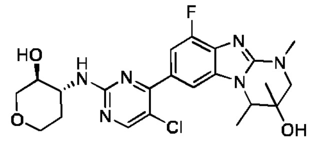 TB-75 |
| 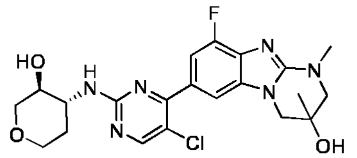 TB-76 | 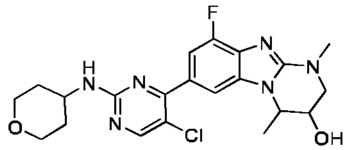 TB-77 | 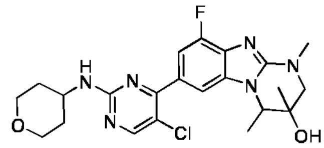 TB-78 |
| 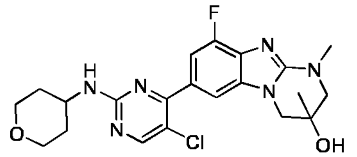 TB-79 | 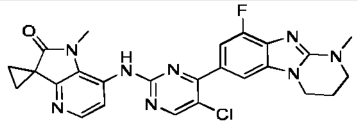 TB-80 | 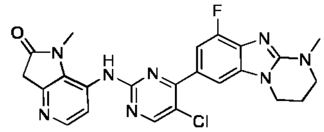 TB-81 |
| 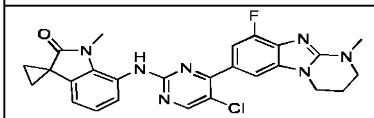 TB-82 | 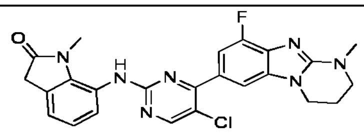 TB-83 | 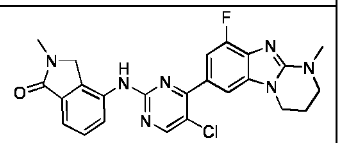 TB-84 |

| | | |
|---|---|---|
| 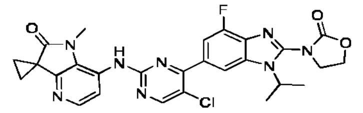 TB-85 | 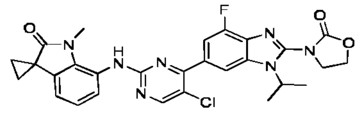 TB-86 | 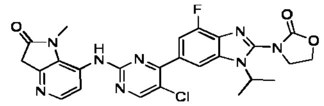 TB-87 |
| 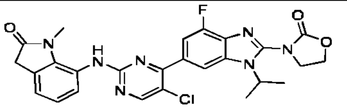 TB-88 | 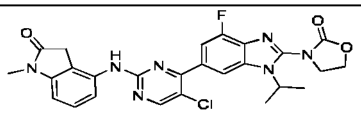 TB-89 | 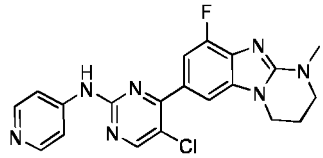 TB-90 |
| 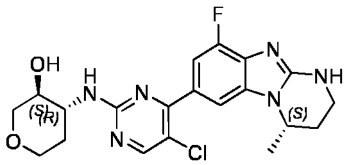 TB-91’ | 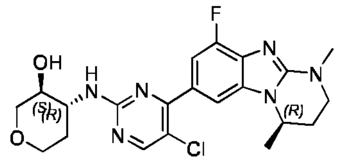 TB-91 | 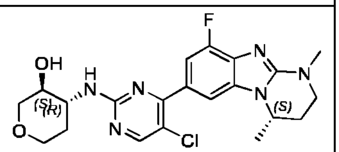 TB-92 |
| 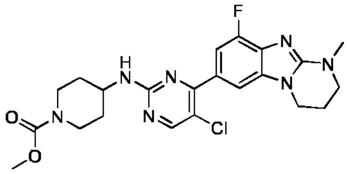 TB-93 | 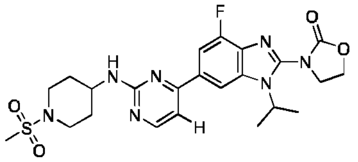 TB-94 | 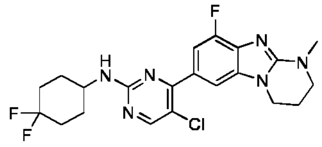 TB-95 |
| 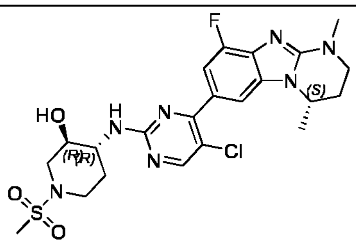 TB-96 | 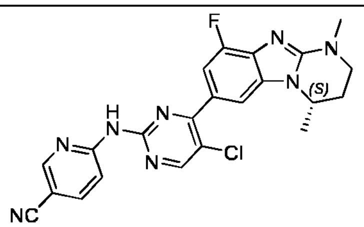 TB-97 | 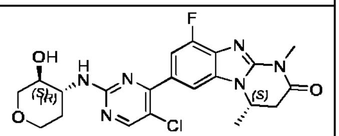 TB-98 |
| 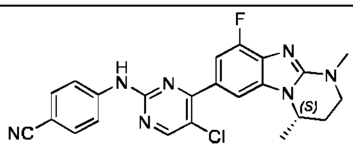 TB-99 | 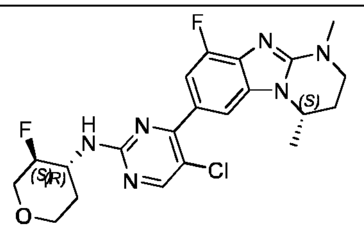 TB-100 | 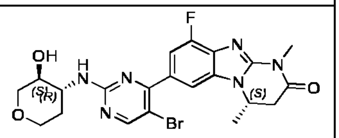 TB-101 |
| 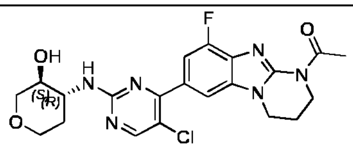 TB-102 | 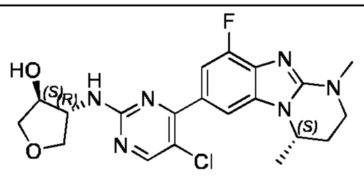 TB-103 | 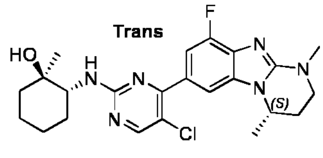 Trans TB-104 |

| | | |
|---|---|---|
| 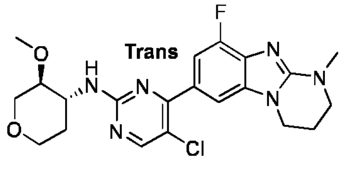 TB-105 | 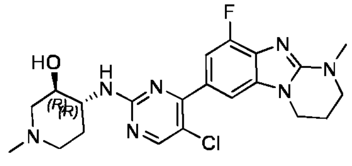 TB-106 | 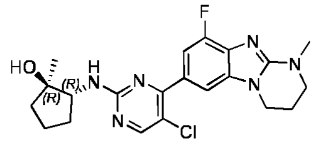 TB-107 |
| 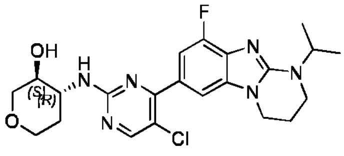 TB-108 | 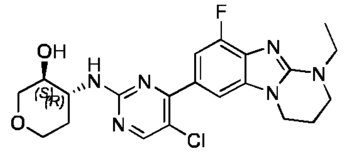 TB-109 | 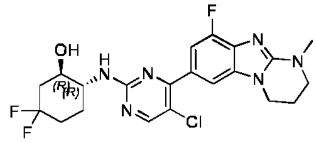 TB-110 |
| 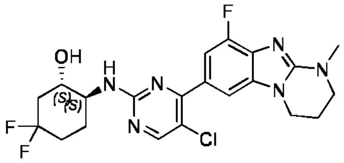 TB-111 | 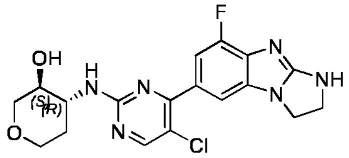 TB-112 | 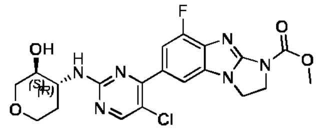 TB-113 |
| 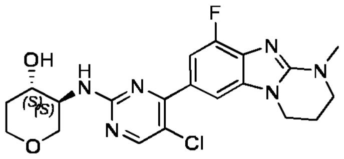 TB-114 | 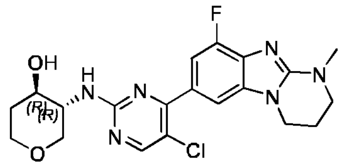 TB-115 | 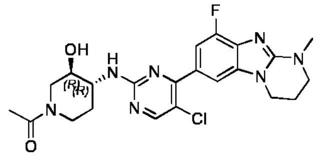 TB-116 |
| 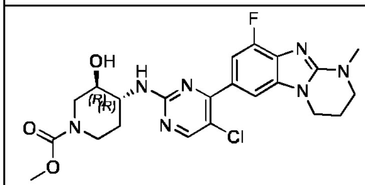 TB-117 | 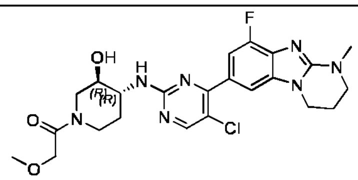 TB-118 | 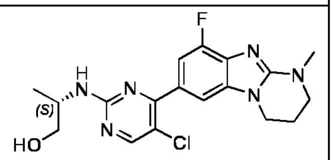 TB-119 |
| 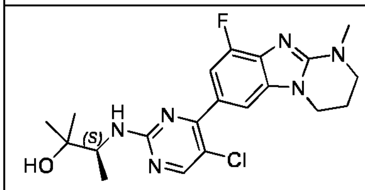 TB-120 | 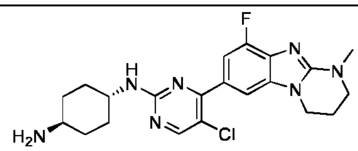 TB-121 | 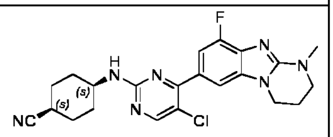 TB-122 |
| 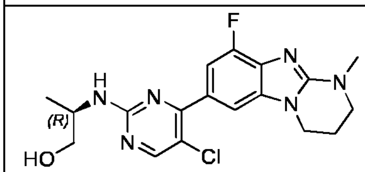 TB-123 | 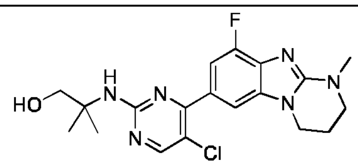 TB-124 | 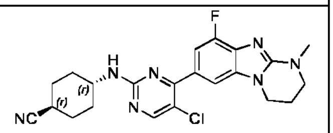 TB-125 |
| 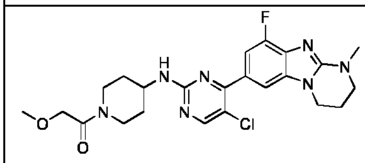 TB-126 | 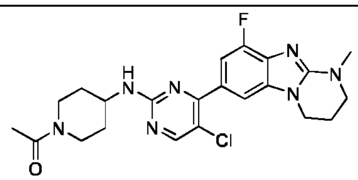 TB-127 | 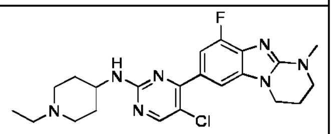 TB-128 |

| | | |
|---|---|---|
| 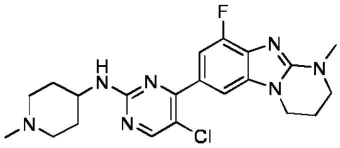 TB-129 | 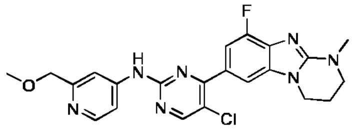 TB-130 | 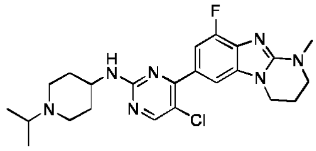 TB-131 |
| 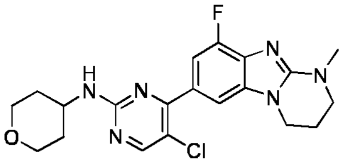 TB-132 | 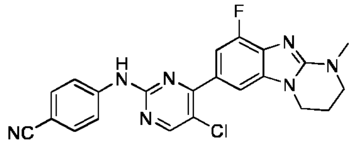 TB-133 | Racemic 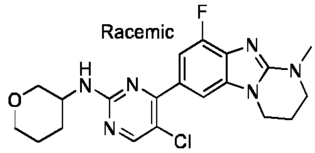 TB-134 |
| 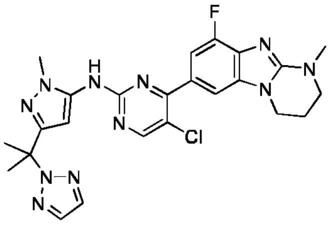 TB-135 | Trans 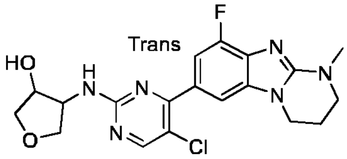 TB-136 | 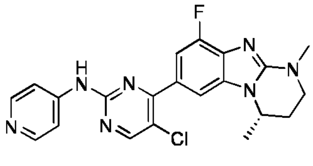 TB-137 |
| 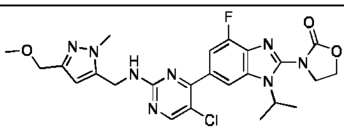 TB-138 | 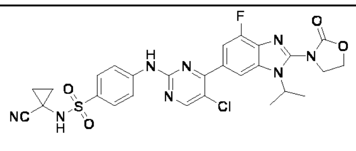 TB-139 | 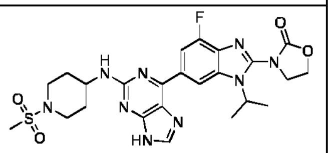 TB-140 |
| 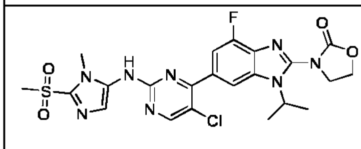 TB-141 | 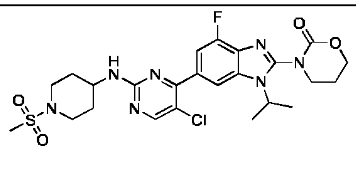 TB-142 | 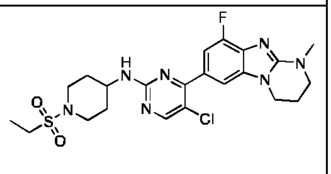 TB-143 |
| 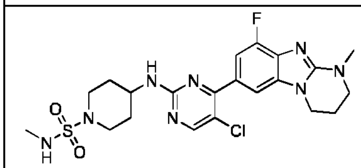 TB-144 | 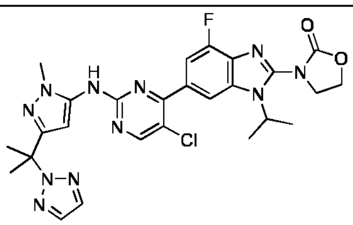 TB-145 | 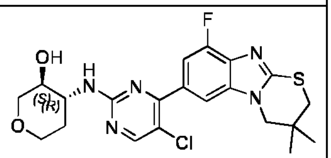 TB-146 |
| 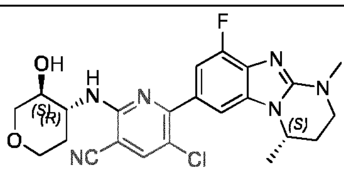 TB-147 | 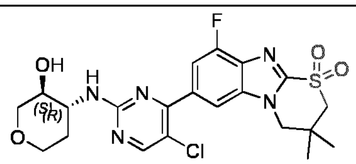 TB-148 | 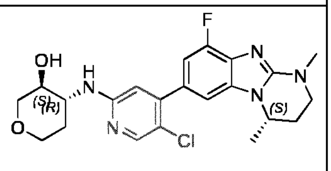 TB-149 |
| 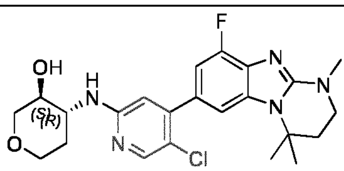 | 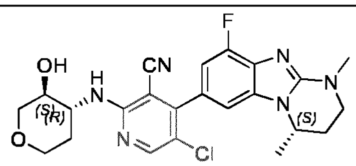 | 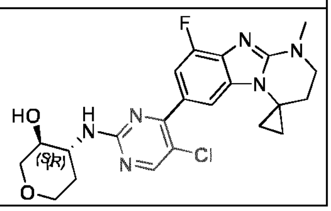 |

| TB-150 | TB-151 | TB-152 |
|---|---|---|
| 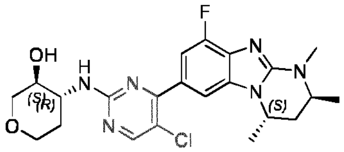<br>TB-153 | 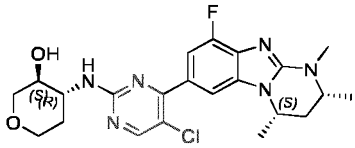<br>TB-154 | 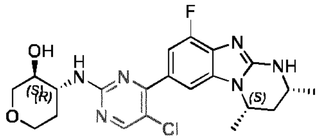<br>TB-155 |
| 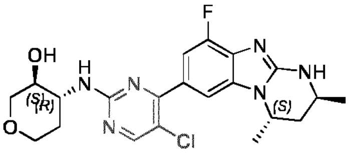<br>TB-156 | 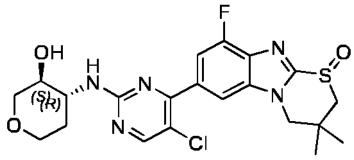<br>TB-157 | 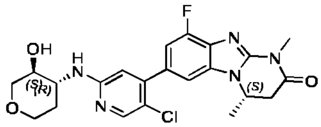<br>TB-158 |
| 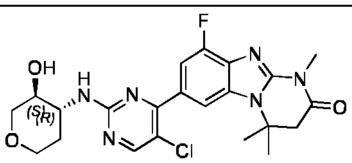<br>TB-159 | 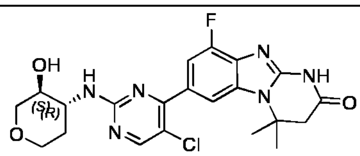<br>TB-160 | 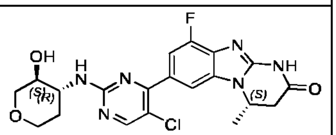<br>TB-161 |
| 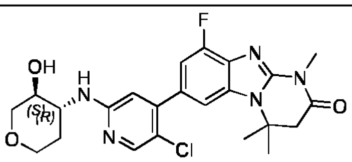<br>TB-162 | 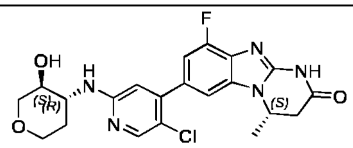<br>TB-163 | |

.

**9.** A pharmaceutical composition comprising a safe and effective amount of the compound of any one of claims 1-8, and a pharmaceutically acceptable carrier.

**10.** A use of the compound of any one of claims 1-8 for the preparation of a drug for modulating CDK4 kinase activity or treating a CDK4-related disease, the disease is selected from the group consisting of inflammation, cancer, cardiovascular diseases, infections, immune diseases, and metabolic diseases.

**INTERNATIONAL SEARCH REPORT**

International application No.
**PCT/CN2023/117094**

**A. CLASSIFICATION OF SUBJECT MATTER**

C07D413/14(2006.01)i; C07D413/10(2006.01)i; C07D405/12(2006.01)i; C07D405/14(2006.01)i; C07D487/04(2006.01)i; C07D401/14(2006.01)i; C07D403/10(2006.01)i; C07D473/34(2006.01)i; C07D513/04(2006.01)i; A61K31/538(2006.01)i; A61K31/506(2006.01)i; A61K31/5355(2006.01)i; A61K31/519(2006.01)i; A61K31/52(2006.01)i; A61K31/542(2006.01)i; A61K31/527(2006.01)i; A61P3/00(2006.01)i; A61P9/00(2006.01)i; A61P29/00(2006.01)i; A61P31/00(2006.01)i; A61P37/00(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, CNABS, VEN, CNKI, 万方数据 WANFANG DATABASE, STNext (Caplus, Registry, Marpat): 嘧啶, 苯并咪唑, 苯并恶嗪, 细胞周期蛋白依赖性激酶, CDK4, benzoxazin, benzo, oxazine, pyrimidin, cyclin-dependent kinase, structure search

**C. DOCUMENTS CONSIDERED TO BE RELEVANT**

| Category* | Citation of document, with indication, where appropriate, of the relevant passages | Relevant to claim No. |
|---|---|---|
| E | CN 116947840 A (RISEN (SUZHOU) PHARMACEUTICAL TECHNOLOGY CO., LTD. et al.) 27 October 2023 (2023-10-27)<br>see embodiment 22, intermediates K1 | 1-2 |
| E | WO 2023205892 A1 (RISEN (SUZHOU) PHARMACEUTICAL TECHNOLOGY CO., LTD. et al.) 02 November 2023 (2023-11-02)<br>see description, embodiment 16 | 1-2 |
| E | WO 2023196517 A1 (BIOLEXIS THERAPEUTICS INC.) 12 October 2023 (2023-10-12)<br>claims 39-52, and description, table 1, compounds I-8 | 1, 2, 9, 10 |
| X | WO 2021030623 A1 (NUVATION BIO INC.) 18 February 2021 (2021-02-18)<br>see entire document, in particular claims 1-52, description, table 1, and embodiment 5 | 1-2, 8-10 |
| X | WO 2020224568 A1 (QILU REGOR THERAPEUTICS INC.) 12 November 2020 (2020-11-12)<br>see entire document, in particular claims 1-30, and description, synthesis example 259 | 1, 2, 9, 10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 December 2023** | **14 December 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
**PCT/CN2023/117094**

**C. DOCUMENTS CONSIDERED TO BE RELEVANT**

| Category* | Citation of document, with indication, where appropriate, of the relevant passages | Relevant to claim No. |
|---|---|---|
| X | WO 2012066065 A1 (NOVARTIS AG et al.) 24 May 2012 (2012-05-24) see entire document, in particular claims 1-42, and description embodiments 1-2 | 1, 2, 9, 10 |
| X | WO 2004041810 A1 (VERTEX PHARMACEUTICALS INCORPORATED et al.) 21 May 2004 (2004-05-21) see entire document, in particular claims 1, 18-19, and 22 | 1, 2, 9, 10 |
| X | CN 112313219 A (PFIZER INC.) 02 February 2021 (2021-02-02) see entire document, in particular claims 1 and 20-23, and description, embodiments A55, A56, etc. | 1-10 |
| X | CN 107286134 A (SHANGHAI XUNHE PHARMACEUTICAL TECHNOLOGY CO., LTD.) 24 October 2017 (2017-10-24) see entire document, paticularly claims 1-7 | 1, 2, 9, 10 |
| X | WO 2018045957 A1 (JIANGSU HANSOH PHARMACEUTICAL GROUP CO., LTD. et al.) 15 March 2018 (2018-03-15) see claims 1-16, and description, embodiments 1-163 | 1-10 |
| X | CN 105916848 A (SHANDONG XUANZHUPHARMA CO., LTD.) 31 August 2016 (2016-08-31) see claims 1-23 | 1-10 |
| X | CN 113382991 A (SHENZHEN TARGETRX, INC.) 10 September 2021 (2021-09-10) see claims 1-10 | 1-10 |
| X | CN 112334451 A (NUVATION BIO INC.) 05 February 2021 (2021-02-05) see claims 1, 50, and 65-68 | 1-2, 8-10 |

Form PCT/ISA/210 (second sheet) (July 2022)

**INTERNATIONAL SEARCH REPORT**
**Information on patent family members**

International application No.
**PCT/CN2023/117094**

| Patent document cited in search report | Publication date (day/month/year) | Patent family member(s) | Publication date (day/month/year) |
|---|---|---|---|
| CN 116947840 A | 27 October 2023 | WO 2023205892 A1 | 02 November 2023 |
| WO 2023205892 A1 | 02 November 2023 | None | |
| WO 2023196517 A1 | 12 October 2023 | US 2023322792 A1 | 12 October 2023 |
| WO 2021030623 A1 | 18 February 2021 | US 2022347187 A1 | 03 November 2022 |
| | | AU 2020329288 A1 | 10 March 2022 |
| | | IL 290508 A | 01 April 2022 |
| | | KR 20220047329 A | 15 April 2022 |
| | | JP 2022544516 A | 19 October 2022 |
| | | CA 3150689 A1 | 18 February 2021 |
| | | EP 4013743 A1 | 22 June 2022 |
| | | BR 112022002532 A2 | 19 July 2022 |
| | | CN 114502536A A | 13 May 2022 |
| WO 2020224568 A1 | 12 November 2020 | AU 2021268648 A1 | 19 January 2023 |
| | | IL 287767 A | 01 January 2022 |
| | | TW 202144351 A | 01 December 2021 |
| | | JP 2023525005 A | 14 June 2023 |
| | | JP 2022531687 A | 08 July 2022 |
| | | SG 11202112229 UA | 30 December 2021 |
| | | MA 55909 A | 16 March 2022 |
| | | MX 2021013531 A | 11 February 2022 |
| | | TW 202108572 A | 01 March 2021 |
| | | US 2022296595 A1 | 22 September 2022 |
| | | CA 3138973 A1 | 12 November 2020 |
| | | KR 20220004755 A | 11 January 2022 |
| | | EA 202193015 A1 | 17 March 2022 |
| | | WO 2021226140 A1 | 11 November 2021 |
| | | CO 2021016504 A2 | 17 January 2022 |
| | | EP 4146647 A1 | 15 March 2023 |
| | | US 2023174512 A1 | 08 June 2023 |
| | | AU 2020269469 A1 | 09 December 2021 |
| | | CL 2021002903 A1 | 08 July 2022 |
| | | EP 3966213 A1 | 16 March 2022 |
| | | EP 3966213 A4 | 19 April 2023 |
| | | CN 114364675 A | 15 April 2022 |
| | | CN 115803327 A | 14 March 2023 |
| WO 2012066065 A1 | 24 May 2012 | None | |
| WO 2004041810 A1 | 21 May 2004 | EP 1560824 A1 | 10 August 2005 |
| | | CA 2507406 A1 | 21 May 2004 |
| | | US 2004176271 A1 | 09 September 2004 |
| | | US 7348335 B2 | 25 March 2008 |
| | | JP 2006508107 A | 09 March 2006 |
| | | AU 2010246324 A1 | 09 December 2010 |
| | | AU 2010246324 B2 | 15 December 2011 |
| | | JP 2010195838 A | 09 September 2010 |
| | | AU 2003286895 A1 | 07 June 2004 |
| CN 112313219 A | 02 February 2021 | US 2019330196 A1 | 31 October 2019 |
| | | US 10766884 B2 | 08 September 2020 |
| | | EP 3784664 A1 | 03 March 2021 |
| | | ECSP 20067394 A | 31 December 2020 |

Form PCT/ISA/210 (patent family annex) (July 2022)

**INTERNATIONAL SEARCH REPORT**
**Information on patent family members**

International application No.
**PCT/CN2023/117094**

| Patent document cited in search report | Publication date (day/month/year) | Patent family member(s) | Publication date (day/month/year) |
|---|---|---|---|
| | | US 2022089580 A1 | 24 March 2022 |
| | | MX 2020011294 A | 18 November 2020 |
| | | CL 2020002748 A1 | 12 February 2021 |
| | | DOP 2020000191 A | 28 February 2021 |
| | | RU 2021136543 A | 16 December 2021 |
| | | MA 52360 A | 03 March 2021 |
| | | TW 202000661 A | 01 January 2020 |
| | | TWI 740135 B | 21 September 2021 |
| | | CU 20200070 A7 | 12 May 2021 |
| | | CO 2020013149 A2 | 10 November 2020 |
| | | PE 20201202 A1 | 11 November 2020 |
| | | BR 112020021689 A2 | 26 January 2021 |
| | | BR 112020021689 B1 | 28 February 2023 |
| | | CR 20200503 A | 17 December 2020 |
| | | GEP 20227433 B | 25 October 2022 |
| | | US 2020354350 A1 | 12 November 2020 |
| | | US 11220494 B2 | 11 January 2022 |
| | | UA 126177 C2 | 25 August 2022 |
| | | CA 3098283 A1 | 31 October 2019 |
| | | CA 3098283 C | 23 May 2023 |
| | | KR 20210002642 A | 08 January 2021 |
| | | KR 102596598 B1 | 03 November 2023 |
| | | KR 20230152182 A | 02 November 2023 |
| | | JP 2021522275 A | 30 August 2021 |
| | | JP 7089061 B2 | 21 June 2022 |
| | | NI 202000072 A | 23 March 2021 |
| | | RU 2762557 C1 | 21 December 2021 |
| | | ZA 202006944 B | 26 July 2023 |
| | | JP 2022120096 A | 17 August 2022 |
| | | WO 2019207463 A1 | 31 October 2019 |
| | | UY 38196 A | 29 November 2019 |
| | | PH 12020551692 A1 | 19 July 2021 |
| | | IL 278075 A | 30 November 2020 |
| | | IL 278075 B1 | 01 January 2023 |
| | | IL 278075 B2 | 01 May 2023 |
| | | AU 2019259653 A1 | 29 October 2020 |
| | | AU 2019259653 B2 | 19 January 2023 |
| | | SG 11202009992 VA | 27 November 2020 |
| CN 107286134 A | 24 October 2017 | US 2019071427 A1 | 07 March 2019 |
| | | US 10464927 B2 | 05 November 2019 |
| | | JP 2019510821 A | 18 April 2019 |
| | | JP 6661048 B2 | 11 March 2020 |
| | | WO 2017177836 A1 | 19 October 2017 |
| | | EP 3444246 A1 | 20 February 2019 |
| | | EP 3444246 A4 | 03 April 2019 |
| | | EP 3444246 B1 | 06 November 2019 |
| WO 2018045957 A1 | 15 March 2018 | CN 109952295 A | 28 June 2019 |
| | | CN 109952295 B | 06 April 2021 |
| CN 105916848 A | 31 August 2016 | JP 2017501187 A | 12 January 2017 |
| | | JP 6263269 B2 | 17 January 2018 |

Form PCT/ISA/210 (patent family annex) (July 2022)

**INTERNATIONAL SEARCH REPORT**
**Information on patent family members**

International application No.
**PCT/CN2023/117094**

| Patent document cited in search report | | | Publication date (day/month/year) | Patent family member(s) | | | Publication date (day/month/year) |
|---|---|---|---|---|---|---|---|
| | | | | PL | 3091008 | T3 | 31 December 2018 |
| | | | | HK | 1223089 | A1 | 21 July 2017 |
| | | | | US | 2016332989 | A1 | 17 November 2016 |
| | | | | US | 9796701 | B2 | 24 October 2017 |
| | | | | CA | 2935103 | A1 | 09 July 2015 |
| | | | | CA | 2935103 | C | 28 August 2018 |
| | | | | HUE | 039504 | T2 | 28 January 2019 |
| | | | | WO | 2015101293 | A1 | 09 July 2015 |
| | | | | RU | 2016130986 | A | 02 February 2018 |
| | | | | RU | 2016130986 | A3 | 25 May 2018 |
| | | | | RU | 2670762 | C2 | 25 October 2018 |
| | | | | ES | 2687477 | T3 | 25 October 2018 |
| | | | | KR | 20160104072 | A | 02 September 2016 |
| | | | | KR | 101787680 | B1 | 19 October 2017 |
| | | | | EP | 3091008 | A1 | 09 November 2016 |
| | | | | EP | 3091008 | A4 | 28 June 2017 |
| | | | | EP | 3091008 | B1 | 27 June 2018 |
| | | | | AU | 2014375500 | A1 | 18 August 2016 |
| | | | | AU | 2014375500 | B2 | 16 March 2017 |
| CN | 113382991 | A | 10 September 2021 | None | | | |
| CN | 112334451 | A | 05 February 2021 | KR | 20200131246 | A | 23 November 2020 |
| | | | | CA | 3089592 | A1 | 22 August 2019 |
| | | | | EP | 3752491 | A1 | 23 December 2020 |
| | | | | EP | 3752491 | A4 | 01 December 2021 |
| | | | | BR | 112020015431 | A2 | 08 December 2020 |
| | | | | IL | 276509 | A | 30 September 2020 |
| | | | | MA | 51846 | A | 21 April 2021 |
| | | | | US | 2023062022 | A1 | 02 March 2023 |
| | | | | MX | 2020008559 | A | 08 January 2021 |
| | | | | SG | 11202007754 | UA | 29 September 2020 |
| | | | | JP | 2021514359 | A | 10 June 2021 |
| | | | | US | 2019248774 | A1 | 15 August 2019 |
| | | | | US | 11174252 | B2 | 16 November 2021 |
| | | | | AU | 2019220746 | A1 | 27 August 2020 |
| | | | | WO | 2019161224 | A1 | 22 August 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Periodic Table of the Elements. Handbook of Chemistry and Physics
- **THOMAS SORRELL**. Organic Chemistry. University Science Books, 1999
- **SAMBROOK et al.** Molecular Cloning: Laboratory Manual. Cold Spring Harbor Laboratory Press, 1989